(19) Europäisches Patentamt
European Patent Office
Office européen des brevets

(11) Publication number: **0 146 263 B1**

# (12) EUROPEAN PATENT SPECIFICATION

(45) Date of publication of patent specification: **15.05.91**

(51) Int. Cl.⁵: **C07D 495/04**, A01N 47/36, //(C07D495/04,335:00,333:00), (C07D495/04,333:00,333:00)

(21) Application number: **84307916.1**

(22) Date of filing: **14.11.84**

The file contains technical information submitted after the application was filed and not included in this specification

## (54) Herbicidal substituted-thiophene, furan and pyrrole sulfonamides.

(30) Priority: **15.11.83 US 551943**
**17.10.84 US 660233**

(43) Date of publication of application:
**26.06.85 Bulletin 85/26**

(45) Publication of the grant of the patent:
**15.05.91 Bulletin 91/20**

(84) Designated Contracting States:
**AT BE CH DE FR GB IT LI LU NL**

(56) References cited:
**EP-A- 0 001 514**
**EP-A- 0 039 239**
**EP-A- 0 046 677**
**EP-A- 0 070 698**
**EP-A- 0 097 122**
**EP-A- 0 009 419**
**EP-A- 0 045 196**
**EP-A- 0 057 546**
**EP-A- 0 084 224**

(73) Proprietor: **E.I. DU PONT DE NEMOURS AND COMPANY**
**1007 Market Street**
**Wilmington Delaware 19898(US)**

(72) Inventor: **Pasteris, Robert James**
**305 Plymouth Road, Fairfax**
**Wilmington, DE 19803(US)**

(74) Representative: **Hildyard, Edward Martin et al**
**Frank B. Dehn & Co. Imperial House 15-19 Kingsway**
**London WC2B 6UZ(GB)**

**EP 0 146 263 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to novel N-(heterocyclicaminocarbonyl) substituted thiophene, furan and pyrrolesulfonamides which are useful as herbicides, and their method-of-use as general pre-emergence or post-emergence herbicides or plant growth regulants.

In U.S. Patent 4,127,405, issued November 28, 1978 to Levitt, herbicidal benzene- and thiophenesulfonylureas are disclosed.

U.S. Patent 4,398,939, issued August 16, 1983 to Levitt discloses herbicidal thiophenesulfonylureas substituted by alkyl, $OCH_3$, $NO_2$, halogen, or sulfamoyl groups.

European Patent Application (EP-A)-30,142, published June 10, 1981 teaches herbicidal thiophenesulfonylureas bearing ortho-carboxylic acid ester groups as well as other derivatives of carboxylic acids.

European Patent Application (EP-A)-64,804, published November 17, 1982, discloses herbicidal thiophenesulfonylureas bearing ortho-alkylsulfonyl groups.

U.S. Patent 4,391,627, issued July 5, 1983 to Levitt discloses benzothiophenesulfonylureas.

European Patent Application (EP-A)-79,683, published May 25, 1983, teaches herbicidal sulfonylureas including those of the general formulae:

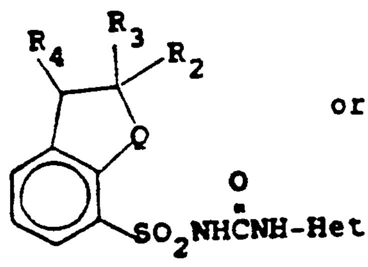

or

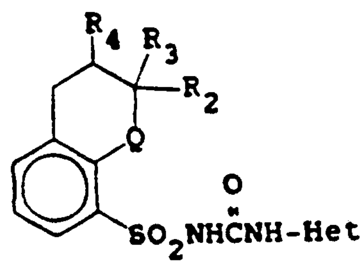

wherein

Q is O, S or $SO_2$;

$R_2$ is H or $C_1$-$C_3$ alkyl;

$R_3$ is H or $CH_3$; and

$R_4$ is H or $CH_3$.

EPA-107,979, published May 9, 1984, teaches herbicidal sulfonylureas including those of general Formulae III-VI:

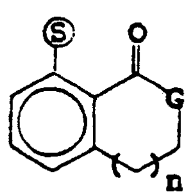

III

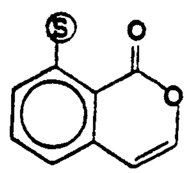

IV

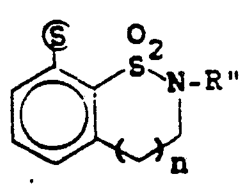

V

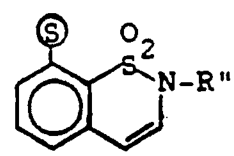

VI

wherein

Ⓢ is 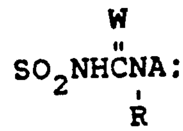

n is O, 1 or 2;
G is O or NR"; and
R" is alkyl.

Still further herbicidal sulfonylureas are disclosed in our EP-A-45,196 and EP-A-70,698.

## Summary of the Invention

This invention relates to novel compounds of Formula I, suitable agricultural compositions containing them and their method-of-use as general pre-emergence and/or post-emergence herbicides or plant growth regulants.

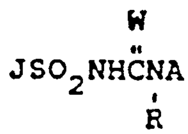

I

wherein
J is

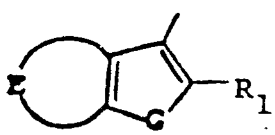

or

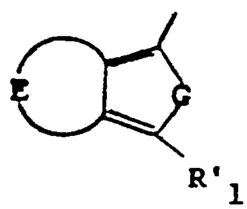

E is a bridge of 3 to 4 atoms. containing 0-2 heteroatoms selected from the group consisting of oxygen, sulfur and nitrogen and also containing 1 to 4 atoms of carbon, said bridge together with the two carbon attachment sites forming a non-aromatic 5 to 6 membered carbocyclic ring, an aromatic 5 to 6 membered heterocyclic ring, or a non-aromatic 5 to 6 membered heterocyclic ring, with the proviso that two oxygen atoms must be separated by at least one atom of carbon and that oxygen and sulfur are only linked to each other if the sulfur is in the form of -SO- or $-SO_2-$;
G is O, S or N-R;
W is O or S;
R is H or $CH_3$;
$R'_1$ is H, $CH_3$ or Cl when G is S and is H when G is O or N-R;
$R_1$ is H, $CH_3$, $OCH_3$, Cl, Br, $SCH_3$, $SO_2CH_3$ or $NO_2$; and

A is 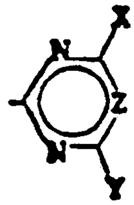 , 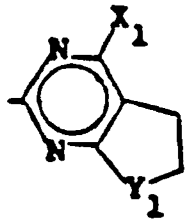 , 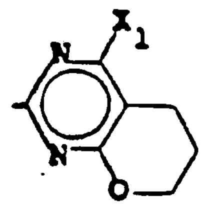 ,

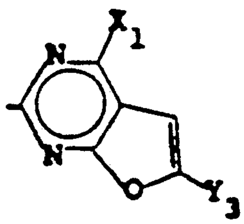 , 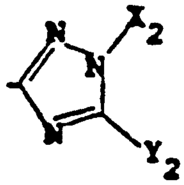 or 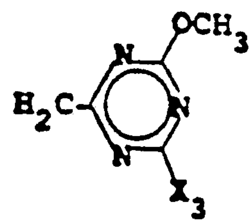 ;

A-4 A-5 A-6

wherein

X is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio,$C_1$-$C_4$ alkythio, halogen, $C_2$-$C_5$ alkoxyalkoxy, amino, $C_1$-$C_3$ alkylamino or di($C_1$-$C_3$ alkyl)amino;

Y is H, $C_1$-$C_4$ alkyl, $C_1$-$C_4$ alkoxy, $C_1$-$C_4$ haloalkoxy, $C_1$-$C_4$ haloalkylthio $C_1$-$C_4$ alkylthio, halogen, $C_2$-$C_5$ alkoxyalkyl, $C_2$-$C_5$ alkoxyalkoxy, amino, $C_1$-$C_3$ alkylamino di($C_1$-$C_3$ alkyl)amino, $C_3$-$C_4$ alkenyloxy, $C_3$-$C_4$ alkylnyloxy, $C_2$-$C_5$ alkylthioalkyl, $C_1$-$C_4$ haloalkyl, $C_3$-$C_5$ cycloalkyl, $C_2$-$C_4$ alkynyl;

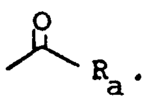 , 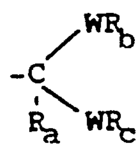 , 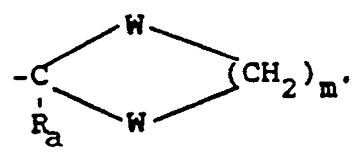 ,

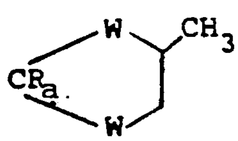 or $N(OCH_3)CH_3$ ;

W is O or S;

m is 2 or 3;

$R_a$ is H or $CH_3$;

$R_b$ is $C_1$-$C_2$ alkyl;

$R_c$ is $C_1$-$C_2$ alkyl;

Z is CH, N, $CCH_3$, $CC_2H_5$, CCl or CBr;

$Y_1$ is O or $CH_2$;

$X_1$ is $CH_3$, $OCH_3$, $OC_2H_5$ or $OCF_2H$;

$X_2$ is $CH_3$, $C_2H_5$ or $CH_2CF_3$;

$Y_2$ is $OCH_3$, $OC_2H_5$, $SCH_3$, $SC_2H_5$, $OCF_2H$, $SCF_2H$, $CH_3$ or $CH_2CH_3$;

$X_3$ is $CH_3$ or $OCH_3$; and $Y_3$ is H or $CH_3$;

provided that

a) when W is S, then R is H, A is A-1, Z is CH or N, and
Y is $CH_3$, $OCH_3$, $OC_2H_5$, $CH_2OCH_3$, $C_2H_5$, $CF_3$, $SCH_3$, $OCH_2CH=CH_2$, $OCH_2C\equiv CH$, $OCH_2CH_2OCH_3$, $CH(OCH_3)_2$, $OCH_2CF_3$

or 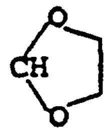 ;

c) when X is Cl, F or Br, then Z is CH and Y is $OCH_3$, $OC_2H_5$, $NH_2$, $NHCH_3$ $N(CH_3)_2$ or $OCF_2H$;

g) when X or Y is $OCF_2H$, then Z is CH;

and their agriculturally suitable salts.

Other compounds of the present invention have the formula

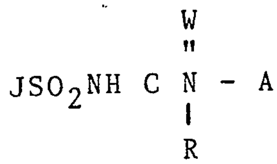 I

wherein J is

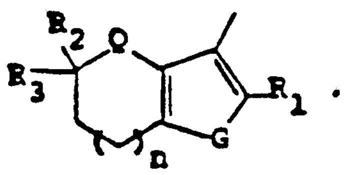

J1

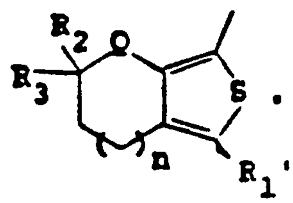

J2

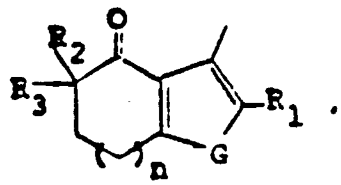

J3

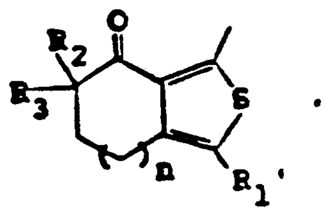

J4

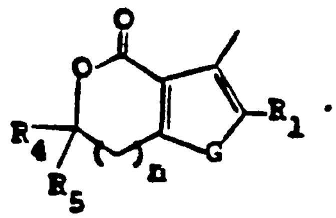

J5

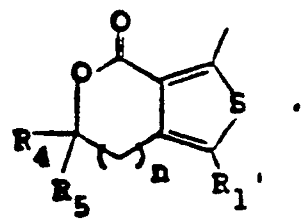

J6

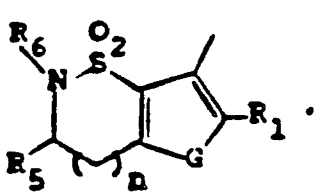

J7

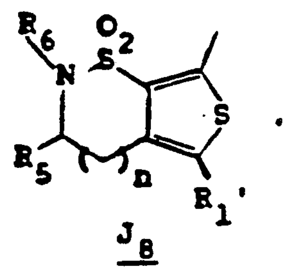

J8

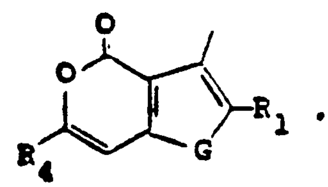

J9

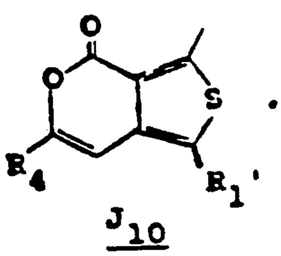

J10

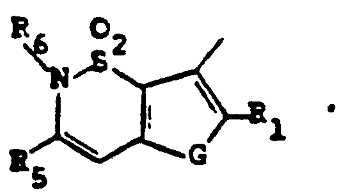

J11

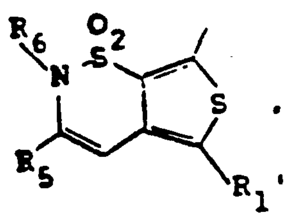

J12

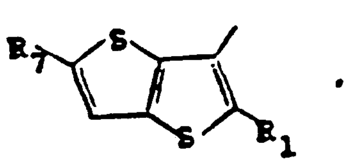

J13

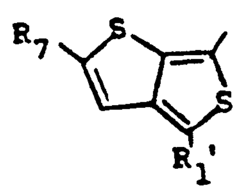

J14

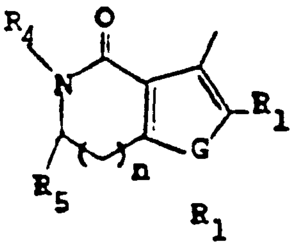 .
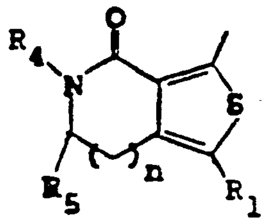 .
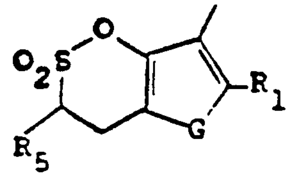 .

$J_{15}$ $J_{16}$ $J_{17}$

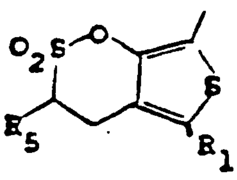 .
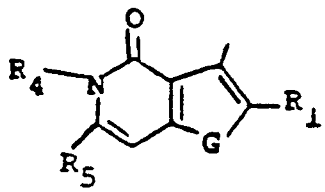 .
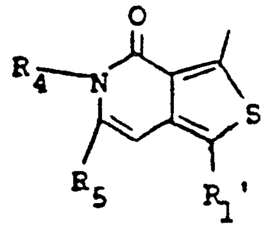

$J_{18}$ $J_{19}$ $J_{20}$

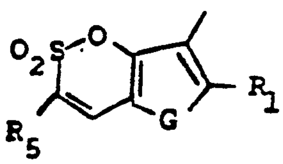 .
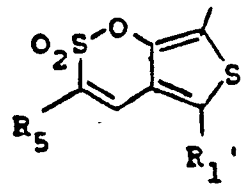

$J_{21}$ $J_{22}$

R is H or $CH_3$;
$R_1'$ is H, $CH_3$ or Cl;
$R_1$ is H, $CH_3$, $OCH_3$, Cl, Br, $SCH_3$, $SO_2CH_3$, or $NO_2$;
$R_2$ is H, Cl or $C_1$-$C_4$ alkyl;
$R_3$ is H, Cl or $C_1$-$C_4$ alkyl;
$R_4$ is H or $C_1$-$C_4$ alkyl;
$R_5$ is H or $CH_3$;
$R_6$ is H, $R_8$, $SR_8$, $SO_2R_8$, $OR_8$, $C(O)R_8$, $CO_2R_8$, $NR_8R_4$, CN or $Si(CH_3)_2R_9$;
$R_7$ is H, $C_1$-$C_6$ alkyl, Cl, Br, CN, $NO_2$, $SR_9$, $SO_2R_9$, $CO_2R_9$ or $C(O)R_9$;
$R_8$ is $C_1$-$C_6$ alkyl, $C_2$-$C_6$ alkenyl, or $C_2$-$C_6$ alkynyl, each optionally substituted by one or more halogens and/or $R_{11}$, $C_2$-$C_6$ epoxyalkyl, $C_3$-$C_6$ cycloalkyl, $C_4$-$C_7$ cycloalkylaklyl.

or 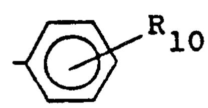 ;

$R_9$ is $C_1$-$C_4$ alkyl,

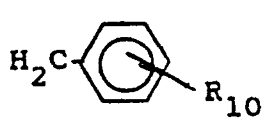 or 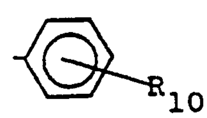

$R_{10}$ is H, F, Cl Br, $CH_3$, $OCH_3$, CN, $NO_2$, $SCH_3$, $SO_2CH_3$ or $CF_3$;

$R_{11}$ is $OR_{12}$, $OC(O)R_{12}$, $OC(O)NR_9R_{12}$, $OSO_2R_{12}$, $OSi(CH_3)_2R_9$, $Si(CH_3)_2R_9$, $SR_{12}$, $SOR_{12}$, $SO_2R_{12}$, SCN, CN, $NO_2$, $C(O)R_{12}$, $C(O)OR_{12}$, $C(O)NR_4R_{12}$,

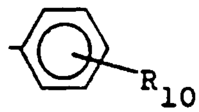

or L.

$R_{12}$ is H, $C_1$-$C_6$ alkyl, $C_1$-$C_6$ haloalkyl, $C_2$-$C_6$ alkynyl; $C_2$-$C_6$ alkynyl, $C_3$-$C_6$ cycloalkyl or

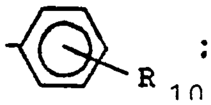

;

n is O or 1; Q is O, S, SO, $SO_2$, $CH_2$ or $CHCH_3$; and

G is O, S or NR;

L is a 5 or 6 membered aromatic heterocycle, a 5-6-membered dihydroaromatic heterocycle or a 5- or 6-membered tetrahydroaromatic heterocycle each containing 1-4 heteroatoms selected from the group consisting of 0-1 oxygen atoms, 0-1 sulfur atoms and 0-4 nitrogen atoms, also each optionally substituted with 1-4 $CH_3$, 1-2 $OCH_3$, 1 $SCH_3$, 1 Cl, 1 $N(CH_3)_2$or 1 CN or L is a 5- or 6-membered lactone, lactam or cycloalkanone optionally substituted with 1-4 $CH_3$ groups;

W is O or S;

$R_1$, $R_1'$ and A are as defined above;

said provisos a), c) and g) as defined above apply;

and provided further that

b) the total number of carbon atoms in $R_2$ and $R_3$ is less than or equal to 4;

d) when J is $J_1$ or $J_2$, then $R_2$ and $R_3$ are other than Cl;

e) the total number of carbon atoms in $R_4$ and $R_5$ is less than or equal to 4; and

f) when J is $J_5$, $J_6$, $J_7$ or $J_8$ and n = O, then $R_4$ is H;

and their agriculturally suitable salts.

In the above formula, preferably J is

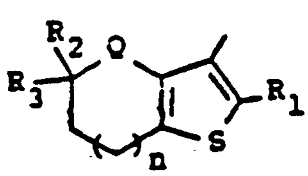 ,

$J_1$

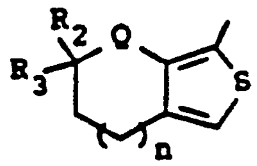 ,

$J_2$

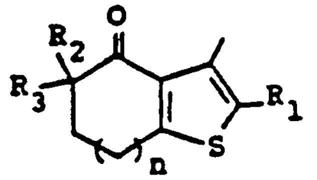 ,

$J_3$

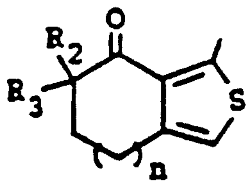 ,

$J_4$

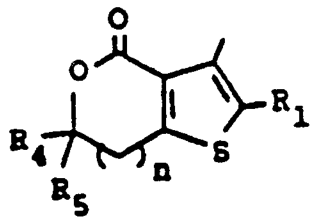 ,

$J_5$

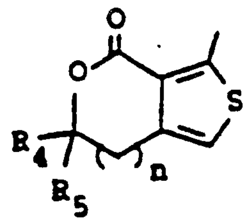 ,

$J_6$

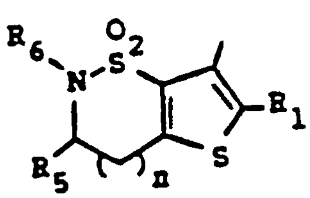 ,

$J_7$

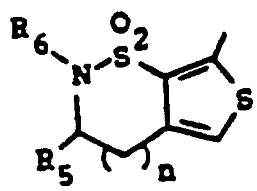 ,

$J_8$

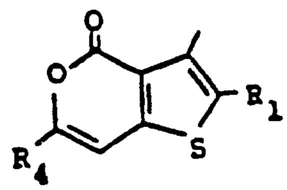 ,

$J_9$

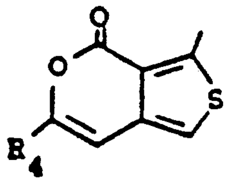 ,
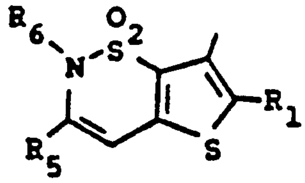 ,
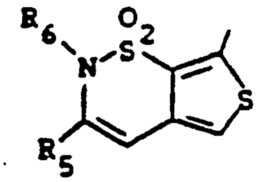 ,

$\underline{J_{10}}$ $\underline{J_{11}}$ $\underline{J_{12}}$

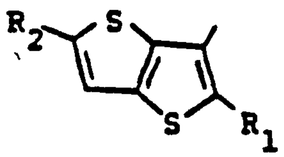

or

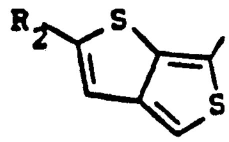 ;

$\underline{J_{13}}$ $\underline{J_{14}}$

R is H or $CH_3$;
$R_1$ is H, $CH_3$, $OCH_3$, Cl or Br;
$R_2$ is H, Cl or $C_1$-$C_4$ alkyl;
$R_3$ is H, Cl or $C_1$-$C_4$ alkyl;
$R_4$ is H or $C_1$-$C_4$ alkyl;
$R_5$ is H or $CH_3$;
$R_6$ is H or $C_1$-$C_4$ alkyl;
Q is O, S, SO, $SO_2$, $CH_2$ or $CHCH_3$;
n is 0 or 1;

A is 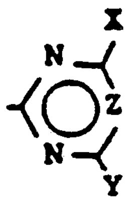 , 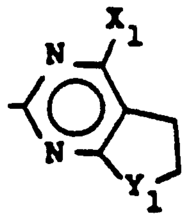 , 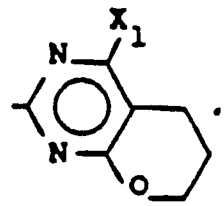 ,

A-1 A-2 A-3

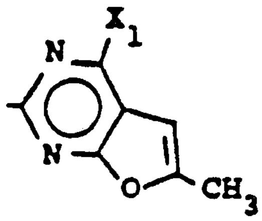 , 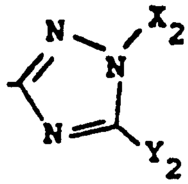 or 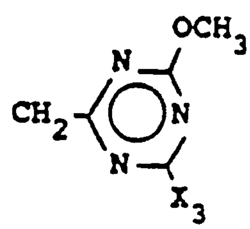 ;

A-4 A-5 A-6

X is $CH_3$, $OCH_3$, $OCH_2CH_3$, Cl, F, Br, $CH_2F$, $OCF_2H$ or $CF_3$;
Y is H, $CH_3$, $OCH_3$, $OC_2H_5$, $CH_2OCH_3$, $NH_2$, $NHCH_3$, $N(CH_3)_2$, $C_2H_5$, $CF_3$, $SCH_3$, $OCH_2CH=CH_2$, $OCH_2C{\equiv}CH$, $OCH_2CF_3$, CN, $N_3$, $OCH_2CH_2OCH_3$, $CH_2SCH_3$, $CR_a(WCH_3)_2$,

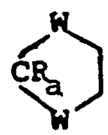 , 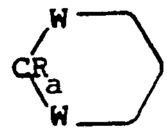 ,

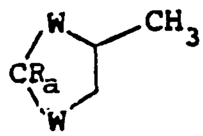 ,

$CR_a(WCH_2CH_3)_2$, or $WCF_2T$
wherein W is O or S, $R_a$ is H or $CH_3$ and T is H, CHClF, CHBrF or $CHFCF_3$;
Z is CH, N, $CCH_3$, $CC_2H_5$, CCl or CBr;
$Y_1$ is O or $CH_2$;
$X_1$ is $CH_3$, $OCH_3$, $OC_2H_5$ or $OCF_2H$;
$X_2$ is $CH_3$, $C_2H_5$ or $CH_2CF_3$;
$Y_2$ is $OCH_3$, $OC_2H_5$, $SCH_3$, $SC_2H_5$, $CH_3$ or $CH_2CH_3$; and
$X_3$ is $CH_3$ or $OCH_3$,

Preferred for reasons of their higher herbicidal activity, greater plant growth regulant activity or more favorable ease of synthesis are:

1) Compounds of Formula I wherein R is H, W is O, and G is O or S.
2) Compounds of Formula I wherein J is $J_1$-$J_{22}$, defined above.
3) Compounds of Preferred 2 wherein R is H, W is O, and G is O or S.
4) Compounds of Preferred 3 wherein A is A-1; X is $CH_3$, $OCH_3$, Cl, Br, $OCH_2CF_3$ or $OCF_2H$; Y is $C_1$-$C_3$ alkyl, cyclopropyl, C≡CH, $OCH_3$, $OC_2H_5$, $CH_2OCH_3$, $NH_2$, $NHCH_3$, $N(CH_3)_2$, $C_2H_5$, $CF_3$, $SCH_3$, $OCH_2CH=CH_2$, $OCH_2C≡CH$, $OCH_2CH_2OCH_3$, $CR(OCH_3)_2$,

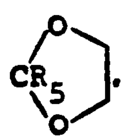 , 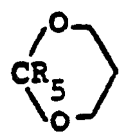 ,

$CR_5(OCH_2CH_3)_2$ or $OCF_2H$; and Z is CH or N.
5) Compounds of Preferred 4 wherein $R_1$ is H, Cl, Br or $CH_3$; $R_2$, $R_3$ and $R_4$ are H or $C_1$-$C_3$ alkyl; $R_6$ is H, $R_8$, $C(O)R_8$ or $CO_2R_8$; $R_7$ is Cl, Br, $CO_2CH_3$ or $CO_2CH_2CH_3$; $R_8$ is $C_1$-$C_6$ alkyl, $C_1$-$C_6$ haloalkyl, $C_2$-$C_6$ alkenyl, $C_2$-$C_6$ alkynyl, $C_3$-$C_6$ cycloalkyl or $C_4$-$C_7$ cycloalkylalkyl and $R_{11}$ is $OCH_3$, $OCH_2CH_3$, CN, $CO_2(C_1$-$C_4$ alkyl), OH or $C(O)CH_3$.
6) Compounds of Preferred 5 wherein G is S, Y is $CH_3$ or $OCH_3$ and X is $CH_3$, $OCH_3$, Cl or Br.
7) Compounds of Preferred 6 where J is $J_1$.
8) Compounds of Preferred 6 where J is $J_3$.
9) Compounds of Preferred 6 where J is $J_5$.
10) Compounds of Preferred 6 where J is $J_7$.
11) Compounds of Preferred 6 where J is $J_9$.
12) Compounds of Preferred 6 where J is $J_{11}$.
13) Compounds of Preferred 6 where J is $J_{13}$.

Specifically Preferred for reasons of their highest herbicidal activity, greatest plant growth regulant activity and/or most favorable ease of synthesis are:

- N-[(4-methoxy-6-methylpyrimidin-2-yl)aminocarbonyl]-6,7-dihydro-5H-thieno[3,2-B]thiopyran-3-sulfonamide, 4,4-dioxide, m.p. 224-225.5° C.
- N-[(4,6-dimethoxypyrimidin-2-yl)aminocarbonyl]-6,7-dihydro-5H-thieno[3,2-B]thiopyran-3-sulfonamide, 4,4-dioxide, m.p. 228-230° C.
- N-[(4,6-dimethoxypyrimidin-2-yl)aminocarbonyl]-5,6-dihydro-5-methylthieno[3,2-B]-thiophene-3-sulfonamide-4,4-dioxide, m.p. 186-187° C.
- N-[(4-methoxy-6-methyl-1,3,5-triazin-2-yl)aminocarbonyl]-5,6-dihydro-5-methythieno[3,2-B]-thiophene-3-sulfonamide-4,4-dioxide, m.p. 192-193° C.

- N-[(4-chloro-6-methoxypyrimidin-2-yl)aminocarbonyl]-5,6-dihydro-5-methylthieno[3,2-B]-thiophene-3-sulfonamide-4,4-dioxide, m.p. 203-204° C.

## DETAILED DESCRIPTION OF THE INVENTION

### Synthesis

The following discussion represents a general outline for the preparation of the compounds of this invention. All of the syntheses described below are multistep with one or more methods being taught for each step. This allows for a wide variety of possible synthetic pathways to prepare a particular compound of Formula 1. The proper choice of the synthetic pathway and the best ordering of the reaction sequences for each individual compound will be knovn to one skilled in the art.

The compounds of Formula I can be prepared by one or more of the methods described below in Equations 1 to 6.

As shown in Equation 1, many of the compounds of Formula I can be prepared by reacting a sulfonylisocyanate (W = 0) or a sulfonylisothiocyanate (W = S) of Formula II with an appropriate heterocyclic amine of Formula III. R, A and W are as previously defined.

Equation 1

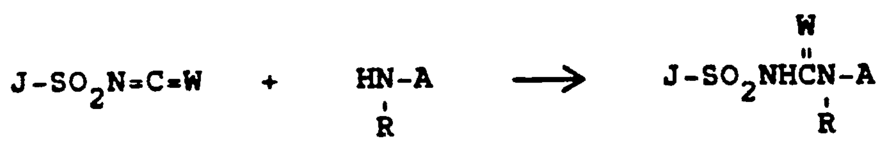

II III I

The reaction is carried out at 25° to 100° C in an inert, aprotic solvent such as methylene chloride or xylene for 0.5 to 24 hours as taught in U.S. Patent 4,127,405.

Many of the compounds of Formula I, where W is S and R is H, (Ia) can be prepared by reacting the appropriate sulfonamide of Formula IV with a heterocyclic isothiocyanate of Formula V, as shown in Equation 2.

Equation 2

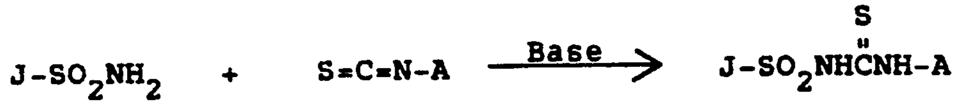


IV V Ia

The reaction is carried out at 25° to 80° C in an inert, aprotic solvent such as acetone or acetonitrile in the presence of a base such as potassium carbonate for 0.5 to 24 hours. The required heterocyclic isothiocyanates V are prepared from the corresponding amines III as taught in EPO Publication 35,893.

Many of the compounds of Formula I, where W is O (Ib) and J is other than $J_5$, $J_6$, $J_9$ and $J_{10}$, can be prepared by reacting the sulfonamides of Formula IV with an appropriate methylcarbamate of Formula VI in the presence of an equimolar amount of trimethylaluminum, as shown in Equation 3.

Equation 3

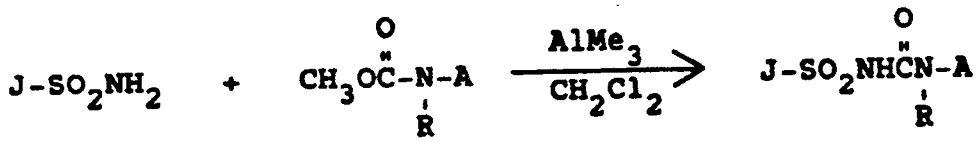


IV VI Ib

The reaction is carried out at 25° to 40° C in a solvent such as methylene chloride for 10 to 96 hours under an inert atmosphere as taught in EPO Publication 82,681. The required carbamates VI are prepared by reacting the corresponding amines III with dimethylcarbonate or methyl chloroformate in the presence of a strong base.

Alternatively, many of the compounds of Formula Ib, can be prepared by reacting a sulfonylcarbamate of Formula VII with an appropriate amine of Formula III, as shown in Equation 4.

Equation 4

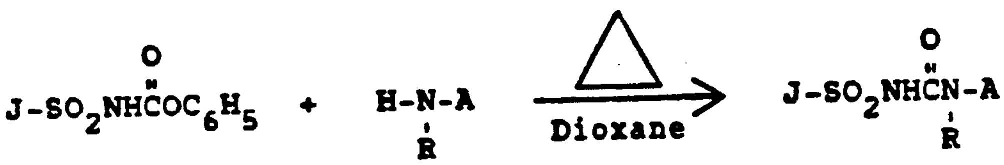


VII III Ib

The reaction is carried out at 50° to 100° C in a solvent such as dioxane for 0.5 to 24 hours as taught in EPO publication No. 44,807. The required carbamates VII are prepared by reacting the corresponding sulfonamides IV vith diphenylcarbonate in the presence of a strong base, such as sodium hydride.

Compounds of Formula Ib can also be prepared, as shown in Equation 5, by reacting a heterocyclic carbamate of Formula VIII with an appropriate sulfonamide of Formula IV.

Equation 5

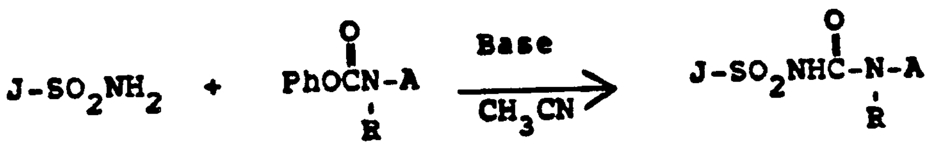


IV VIII Ib

The reaction is carried out at 0° to 50° C in a solvent such as acetonitrile or dioxane in the presence of a non-nucleophilic base such as DBU for 0.2 to 24 hours. The required phenylcarbamate VIII are prepared by reacting the corresponding heterocyclic amines III with diphenylcarbonate or phenylchloroformate in the presence of a strong base, such as sodium hydride.

Many of the compounds of Formula Ib, particularly when G is NH or $NCH_3$ can be prepared by reacting the bicyclic heterocycles IX with a sulfamoyl chloride X as shown in Equation 6.

Equation 6

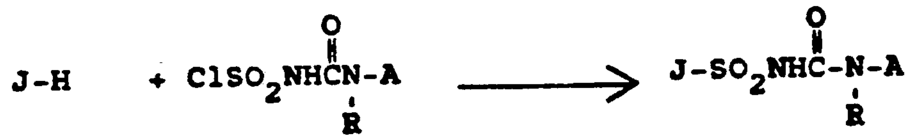

IX X Ib

The reaction is carried out at -78° C to 80° C in a solvent such as tetrahydrofuran or nitroethane for 1 to 48 hours alone or in the presence of a Friedel-Crafts catalyst as taught in U.S. 4,368,067 and 4,473,394. The sulfamoyl chloride intermediates X are prepared by reacting the heterocyclic amines III with chlorosulfonylisocyanate by methods taught in U.S. 4,401,816.

The intermediate sulfonylisocyanates (W = 0) and sulfonylisothiocyanates (W = S) of Formula II from Equation 1 can be prepared as shown in Equations 7, 8 and 9.

As shown in Equation 7, many of the sulfonylisocyanates of Formula IIa where J is other than $J_3$ and $J_4$ can be prepared by the reaction of sulfonamides of Formula IV with phosgene, in the presence of *n*-butylisocyanate and a tertiary amine catalyst, at reflux in a solvent such as xylene by the method of U.S. Patent 4,238,621.

Equation 7

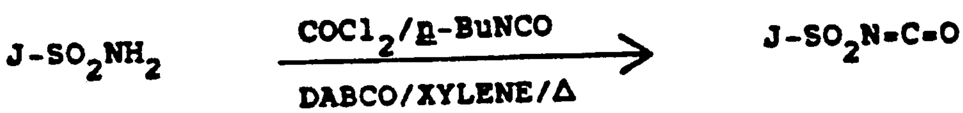


IV IIa

The sulfonylisocyanates can also be prepared from the sulfonamides by a two step procedure involving (a) reacting the sulfonamides with *n*-butylisocyanate in the presence of a base such as $K_2CO_3$ at reflux in an inert solvent such as 2-butanone forming a *n*-butylsulfonylurea; and (b) reacting this compound with phosgene and a tertiary amine catalyst at reflux in xylene solvent. The method is similar to a procedure taught by Ulrich and Sayigh, Newer Methods of Preparative Organic Chemistry, Vol. VI, p. 223-241, Academic Press, New York and London, W. Foerst Ed.

Alternatively, as shown in Equation 8, many of the sulfonylisocyanates of Formula IIa can be prepared by reacting the corresponding sulfonyl chlorides VIII with cyanic acid salts.

Equation 8

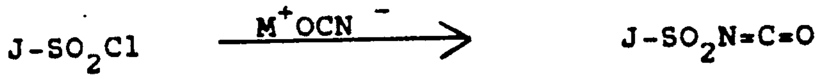


XI IIa

The reaction is carried out at 25° to 100° C in an inert aprotic solvent such as acetonitrile for 0.5-24 hours in the presence of phosphorus pentoxide and an alkali metal salt such as lithium iodide according to the teachings of Japanese Patent No. 76/26,816 (Chem. Abst., 85:77892e (1976)).

Many of the sulfonylisothiocyanates of Formula IIb where J is other than $J_3$ and $J_4$ can be prepared, as shown in Equation 9, by contacting the sulfonamides of Formula IV with carbon disulfide in the presence of two equivalents of a strong base. The resulting salt is then reacted with phosgene according to the

teachings of K. Hartke, Arch. Pharm., 299, 174 (1966).

Equation 9

$$J\text{-}SO_2NH_2 \xrightarrow[2)\ COCl_2]{1)\ CS_2/BASE} J\text{-}SO_2N{=}C{=}S$$

IV IIb

The sulfonamides of Formula IV of Equations 2, 3, 5, 7 and 9 are important intermediates for the preparation of the compounds of this invention. The syntheses of the required sulfonamide intermediates are described in Equations 10 to 15.

As shown in Equation 10, sulfonamides of Formula IV can be prepared from the corresponding sulfonyl chlorides of Formula XI by contacting with either anhydrous or aqueous ammonia.

Equation 10

$$J\text{-}SO_2Cl \xrightarrow[or\ NH_3]{NH_4OH} J\text{-}SO_2NH_2$$

XI IV

The preparation of sulfonamides from sulfonyl chlorides is widely reported in the literature, for reviews see: F. Hawking and J. S. Lawrence, "The Sulfonamides," H. K. Lewis and Co., London, 1950 and E. H. Northey, "The Sulfonamides and Allied Compounds," Reinhold Publishing Corp., New York, 1948.

Alternatively, many sulfonamides IV can be prepared by dealkylation of their corresponding N-t-butyl sulfonamides XII as shown in Equation 11.

Equation 11

$$J\text{-}SO_2NHC(CH_3)_3 \xrightarrow[\text{or}\ HCl/CH_3OH]{CF_3CO_2H} \text{IV}$$

XII

The reaction is carried out by contacting the N-t-butyl sulfonamide XII with a strong acid such as trifluoroacetic acid or methanolic HCl at 25° to 50° C for 0.5 to 24 hours. The N-t-butyl sulfonamides XII are readily prepared by reacting sulfonylchlorides XI with t-butylamine and are useful either as an aid in purification, to enhance solubility for subsequent reactions such as Equation 12 below or to protect the sulfonamide function from competing with reactions at other parts of the molecule.

Many of the unsaturated sulfonamides of Formula IVa and IVb can be prepared from the corresponding saturated sulfonamides of Formula IVc and IVd by the two-step procedure shown in Equation 12. $G_1$-$G_2$ is CO-O, $SO_2$-$NR_6$, CO-$NR_4$ or O-$SO_2$ and R" is H or $C(CH_3)_3$.

Equation 12

a)

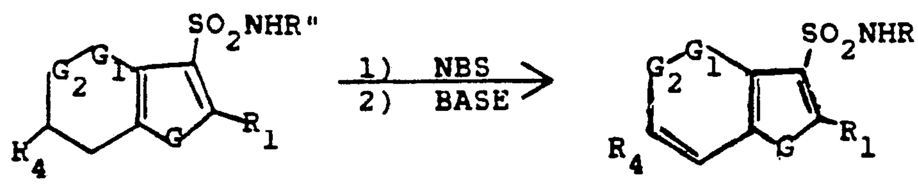


IVc IVa

b)

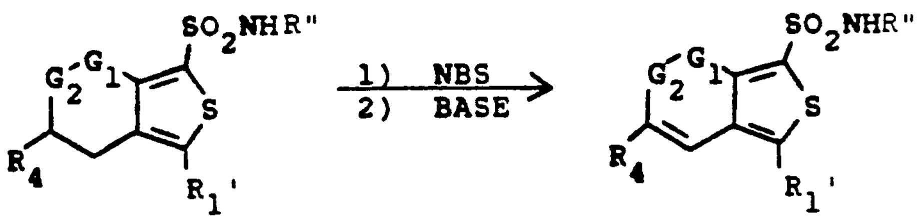


IVd IVb

The first step involves $\alpha$-bromination by N-bromosuccinimide to give a monobromide which is subsequently dehydrobrominated in a second step by reaction with a suitable base such as triethylamine or potassium-t-butoxide in an inert solvent such as THF. This method has been used to prepare isocoumarins from 3,4-dihydroisocoumarins, see R. Barry, Chem. Rev., 64, 229 (1964). In cases where $R_1$ or $R_1$' is a methyl function, competitive bromination at this site may occur resulting in a mixture. The desired bromide may be separated at this stage, or after treatment with the base, by standard methods.

Many of the sulfonamides of Formula IVe and IVf can be prepared from sulfonamides of Formula IVg and IVh, respectively by dehalogenation as shown in Equation 13. $G_1$-$G_2$-$G_3$ is Q-$CR_2R_3$-$CH_2$, CO-$CR_2R_3$-$CH_2$, CO-O-$CR_4R_5$, $SO_2$-$NR_6$-$CHR_5$, CO-$NR_4$-$CHR_5$ or O-$SO_2$-$CHR_5$; $R_1$ and R' are Cl or Br and R" is H or $C(CH_3)_3$.

Equation 13

a)

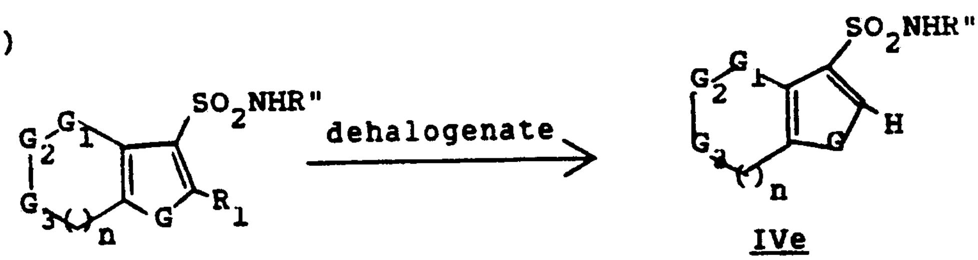


IVg

b)

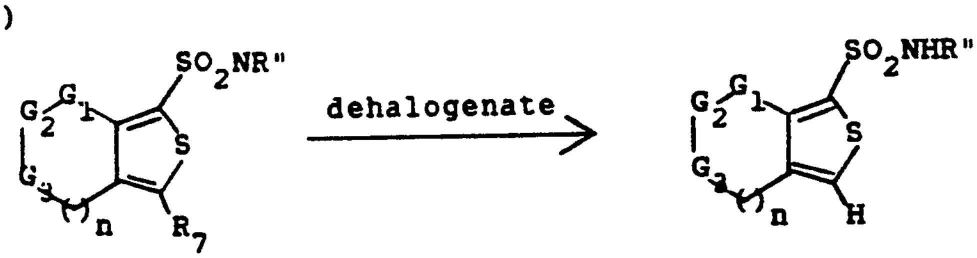


IVh IVf

A typical dehalogenation method involves contacting the halo compound with zinc in aqueous acetic acid at 25° to 100° C for 0.5 to 24 hours. A variety of dehalogenation methods are known, for example, see H. D. Hartough, Thiophene and Derivatives, Vol. III of The Chemistry of Heterocyclic Compounds, Interscience, New York, 1952.

Additionally, as shown in Equation 14, many of the sulfonamides of Formula IVi and IVj can be prepared by oxidation of sulfonamides of Formula IVk and IVl, respectively. m is 1 or 2. $R_1$ is not $SCH_3$ and R" is H or $C(CH_3)_3$.

Equation 14

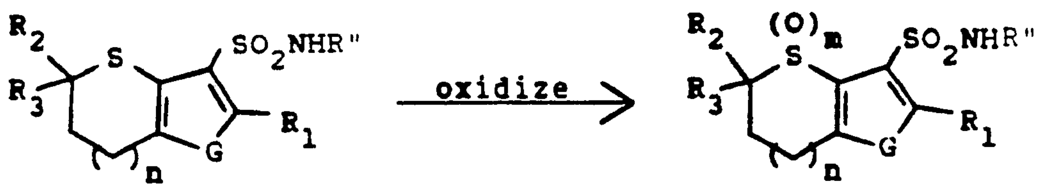


IVk IVi

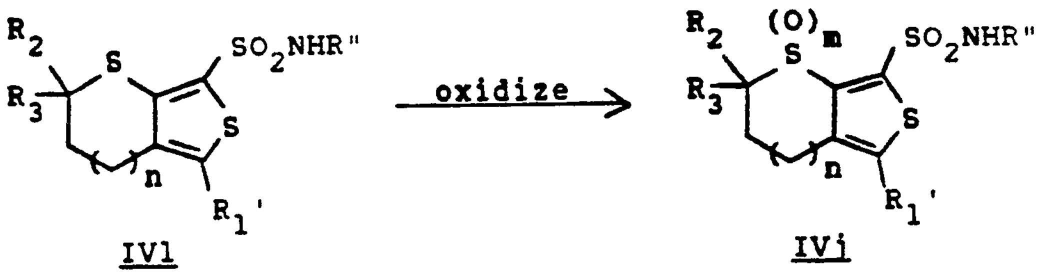


IVl IVj

The oxidation of sulfides to sulfoxides and sulfones are widely reported in the literature, for an overview see: "Organic Chemistry of Sulfur", Plenum Press, New York, 1977, S. Oae Ed.

Many of the sulfonamides of Formula IVm and IVn can be prepared by functionalization of the corresponding N-unsubstituted sulfonamides of Formula IVo and IVp, respectively, as shown in Equation 15. $G_1$-$G_2$ is $(CH_2)_n$-$CHR_5$ or $CH=CR_5$, R" is H or $C(CH_3)_3$ and X is Cl, Br, I or other readily displaceable groups.

Equation 15

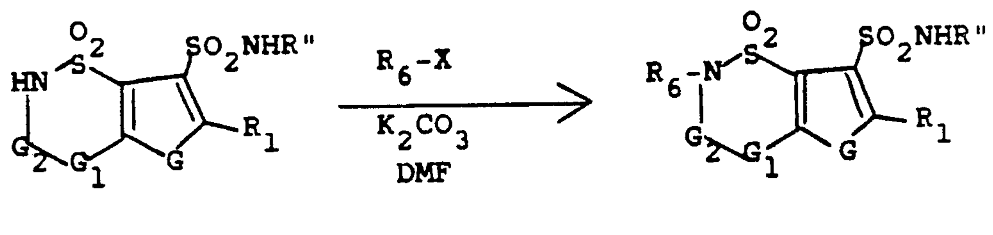


IVo IVm

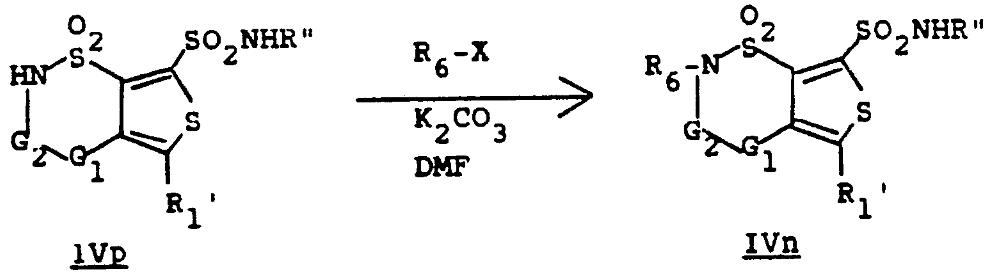


IVp IVn

The reaction is carried out by contacting the sulfonamides IVo or IVp with the appropriate electrophile in the presence of a suitable base such as $K_2CO_3$ in an inert, polar solvent such as DMF at 0° to 100° C for 0.5 to 24 hours. In some instances the $R_6$ function can also be introduced by Michael addition of IVo or IVp to the appropriate Michael acceptor as known to one skilled in the art.

While many of the $R_6$ groups can be introduced directly, as described above, some of the $R_6$ groups may best be prepared by standard functional group manipulations upon compounds of Formula IVm or IVn containing an appropriate $R_6$ group precursor as will be known to one skilled in the art. Some examples of these manipulations are the preparation of IVm or IVn where $R_6$ contains an epoxide by the expoxidation of IVm or IVn where $R_6$ contains a carbon-carbon double bond, the preparation of IVm or IVn where $R_6$ contains a sulfone by the oxidation of IVm or IVn where $R_6$ contains a thioether function, the preparation of IVm or IVn where $R_6$ contains $OC(O)CH_3$ by acetylation of IVm or IVn where $R_6$ contains OH, or the preparation of IVm or IVn where $R_6$ contains $NH_2$ by the reduction of IVm or IVn where $R_6$ contains $NO_2$.

The sulfonyl chlorides of Formula XI of Equations 8 and 10 can be prepared by methods outlined in Equations 16 to 18.

As shown in Equation 16, many of the sulfonyl chlorides of Formula XIa and XIb can be prepared from thiophenes of Formula XIIIa and XIIIb, respectively by direct chlorosulfonation. $R_1$ is not H.

Equation 16

a)

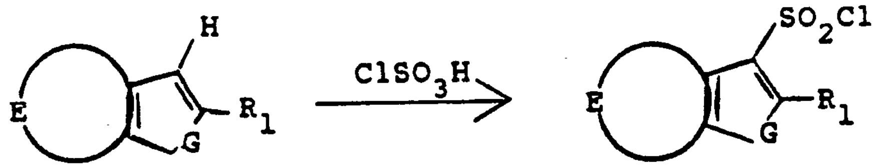


XIIIa XIa

b)

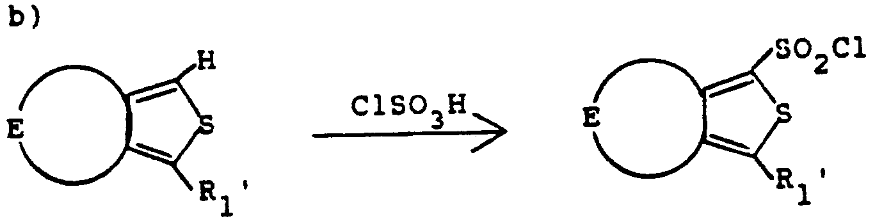


XIIIb XIb

Direct chlorosulfonation can be carried out by standard methods such as those cited by Hartough (loc. cit.) for the preparation of thiophene sulfonylchlorides. For compounds of Formula XIIIa, $R_1$ cannot be hydrogen in order to avoid chlorosulfonation at the more reactive 2-position of the heterocyclic ring. For compounds of Formula IXb, mixtures may result when $R_1$' is hydrogen due to competitive chlorosulfonation at the 5-position of the heterocyclic ring. These isomers can be separated by standard fractional crystallization, fractional distillation or chromatographic methods.

When G is NH or $NCH_3$, direct ring sulfonation with pyridine-sulfur trioxide complex may be preferable as known to one skilled in the art. The resulting sulfonic acid can be converted to the sulfonyl chlorides by standard methods.

Alternatively, many of the sulfonyl chlorides of Formula XIa and XIb can be prepared from compounds of Formula XIIIa and XIIIb by the four step sequence shown in Equation 17.

Equation 17

a)

XIIIa → XIa

1) Nitrate
2) Reduce
3) Diazotize
4) $SO_2$/HCl

b)

XIIIb → XIb

1) Nitrate
2) Reduce
3) Diazotize
4) $SO_2$/HCl

Nitration of oompounds can be carried out by standard methods such as those cited by Hartough (loc. cit.) for the thiophene ring system. The same regioselectivity in nitration is observed as described for the chlorosulfonation of XIIIa and XIIIb (vide supra). The reduction of the intermediate nitro compounds to

amines can be carried out by any of several methods as described in Preparative Organic Chemistry, 4th Edition, p. 557-563, John Wiley and Sons, New York and London, G. Hilgetag and A. Martini Ed. Diazotization of the amines with sodium nitrite in HCl, followed by reaction of the diazonium salt with sulfur dioxide and cupric chloride in acetic acid analogous to the teachings of Yale and Sowinski, J. Org. Chem., 25, 1824 (1960) provides the desired sulfonyl chlorides.

Alternatively, the sulfonyl chlorides can be prepared by a modification of the above procedure whereby the diazotization reaction is carried out in dilute sulfuric acid and the resulting diazonium salt is reacted with sulfur dioxide, HCl and cupric chloride in a cosolvent mixture consisting of acetic acid-water (1:1) and an immiscible, inert solvent such as 1-chlorobutane or methylene chloride at 0°-40°C for 1 to 24 hours.

Sulfonyl chlorides of Formula XIc and XId can also be prepared from the bromothiophenes XIIIc and XIIId by the three-step sequence shown in Equation 18. Q is O, S, $CH_2$ or $CHCH_3$; $R_1$ is not $SO_2CH_3$, Br or $NO_2$.

Equation 18

a)

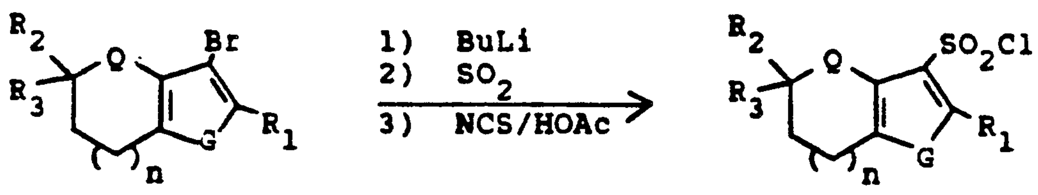


XIIIc XIc

b)

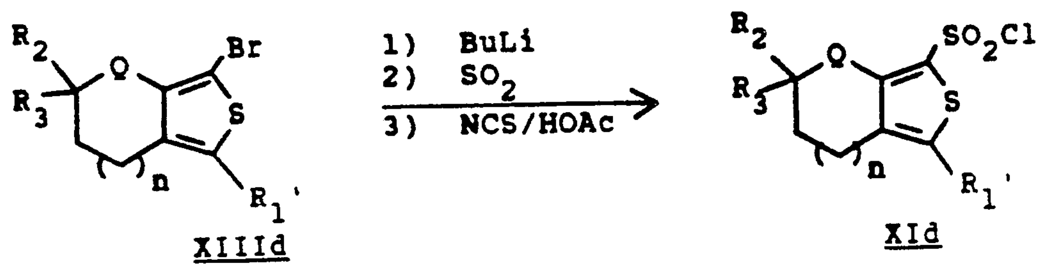


XIIId XId

The compounds are contacted with 1.0 equivalents of n-BuLi at -78°C in a solvent such as THF to give a 3-lithio species via metal-halogen exchange. The lithio species is reacted with sulfur dioxide to give an intermediate sulfinate salt which precipitates and is isolated by filtration. The salt is dissolved in a solvent such as acetic acid and treated with a chlorine source such as N-chlorosuccinimide to give the desired sulfonyl chloride.

The bicyclic heterocycles XIII, many of which are reported in the literature, can be prepared by standard methods known to one skilled in the art.

The amines of Formula III in Equations 1 and 4 are also important intermediates for the preparation of the compounds of this invention and are described below.

The pyrimidines and triazines of Formula (IIIa) to (IIId) below are either known or can be prepared by obvious methods known to one skilled in the art.

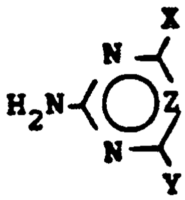

IIIa

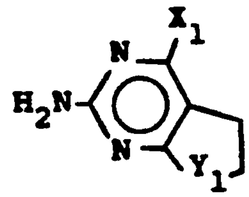

IIIb

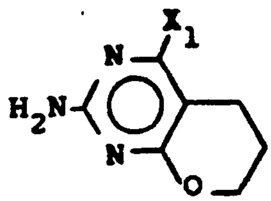

IIIc

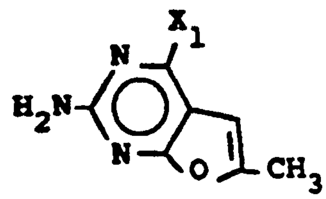

IIId

For a review of the synthesis and reactions of 2-aminopyrimidines (IIIa, Z = CR') see The Chemistry of Heterocyclic Compounds, Vol. 16, John Wiley and Sons, New York (1962). For a review of the synthesis and reactions of 2-amino-s-triazines (IIIa, Z = N) see The Chemistry of Heterocyclic Compounds, Vol. 13, John Wiley, New York (1959), F. C. Schaefer, U.S. Patent 3,154,547 and F. C. Schaefer and K. R. Huffman. J. Org. Chem., 28, 1812 (1963). The synthesis of the bicyclic amines IIIb and IIIc is taught in European Patent Application 15,683. The synthesis of bicyclic amines IIId is taught in European Patent Application 46,677.

The amines of Formula III where X is $OCF_2H$ or $CF_3$; or $X_1$ is $OCF_2H$ and/or Y is $WCF_2T$ wherein W is O or S and T is H, CHClF, CHBrF or $CHFCF_3$ can be prepared by methods taught in South African Patent Application 82/5045, or by suitable modifications that would be obvious to one skilled in the art.

The pyrimidines of Formula IIIa (Z = CH) where Y is $-CR_5(OCH_3)_2$, $-CR_5(OCH_2CH_3)_2$,

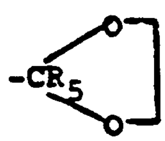
,
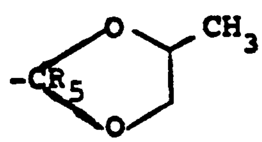

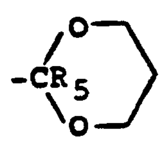

can be prepared according to the methods taught in European Patent Application 84,224 or suitable modifications thereof known to one skilled in the art.

The triazine amines of Formula IIIe where $X_3$ is $CH_3$ or $OCH_3$ and R is H or $CH_3$ can be prepared according to the methods of European Patent Application 94,260.

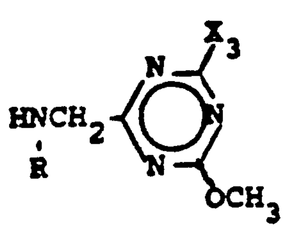

IIIe

Preparations of 3-amino-1,2,4-triazoles intermediates of Formula (IIIf) are described in European Patent Application 73,562.

Many of the aminoheterocyclic intermediates of Formula (III) where R is methyl may be prepared by a two-step procedure as described for IIIg in Equation 19. X, Y and Z are as previously defined.

Equation 19

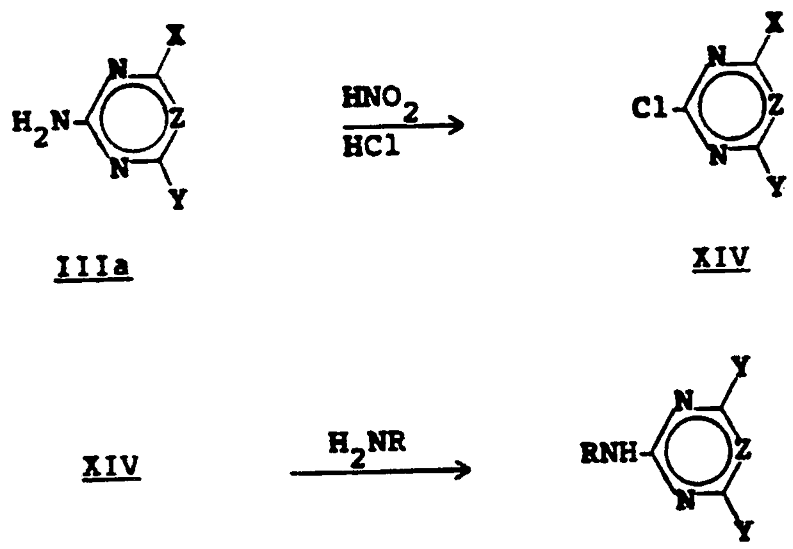


IIIa XIV

XIV IIIg

A solution of the amine IIIa in concentrated hydrochloric acid is treated with sodium nitrite solution and the chloro compound X is isolated in the usual manner by filtration of the acidic solution. A representative procedure is described by Bee and Rose in J. Chem. Soc. C, 2031 (1966), for the case in which Z = CH, and X = Y = $OCH_3$. Displacement of the chlorine of X may be accomplished by heating with an excess of methylamine in water to obtain the methylamino heterocycle (IIIg).

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide, carbonate or hydride). Quaternary amine salts can be made by similar techniques.

Salts of compounds of Formula I can also be prepared by exchange of one cation for another. Cationic exchange can be effected by direct contacting of an aqueous solution of a salt of a compound of Formula I (e.g., alkali metal or quaternary amine salt) with a solution containing the cation to be exchanged. This method is most effective when the desired salt containing the exchanged cation is insoluble in water. e.g., a copper salt, and can be separated by filtration.

Exchange may also be effected by passing an aqueous solution of a salt of a compound of Formula I (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of Formula I with a suitable acid, e.g., p-toluenesulfonic acid, trichloroacetic acid or the like.

In the following examples, all parts are by weight and temperatures in °C unless otherwise indicated. "Nujol" is a Trade Mark.

Example 1

3-Allylthio-4-bromothiophene

48,4 g (0.20 mol) of 3,4-dibromothiophene was added to a -78° C solution of 0.21 mol of n-butyllithium (130 mls of a 1.6 M solution in hexanes, Aldrich) in 200 mls of anhydrous ether under a nitrogen atmosphere at such a rate that the temperature did not rise above -70° C. The mixture was stirred at -78° C for 15 minutes and treated with 6.4 g (0.21 mol) of elemental sulfur causing the temperature to rise to -50° C. The suspension was recooled to -78° C, stirred for 1 hour, treated with 18.2 g (0.21 mol) of allyl bromide and allowed to warm to 20° C. The solution was washed with water and brine, dried over $MgSO_4$ and concentrated to give 47.5 g of an orange oil. 38 g of this oil was chromatographed on 500 g of $SiO_2$ eluting with hexane to give 30.2 g of the desired product as a colorless oil.

90 MHz NMR ($CDCl_3$):

δ 7.4-7.1 (AB, 2H, arom);
6.2-5.6 (m, 1H, vinyl);
5.2-4.9 (m, 2H, vinyl);
3.45 (d, 2H, $CH_2$).

IR (neat) 3100, 1630, 1470, 1320, 980, 930 $cm^{-1}$.

Example 2

3-Bromo-6,7-dihydro-5H-thieno[3,2-b]thiopyran

A solution of 30.2 g (0.13 mol) of 3-allylthio-4-bromothiophene and 10.3 mls (0.13 mol) of pyridine in 250 mls of toluene was refluxed for 68 hours, cooled, washed with 1N HCl solution. 1N NaOH solution, water and brine and dried over $MgSO_4$ and concentrated to give 27.2 g of an amber oil. The oil was chromatographed on 500 g of $SiO_2$ eluting with hexane to give 16.2 g of the desired product as a colorless oil (90% purity).

90 MHz NMR ($CDCl_3$):

δ 7.15 (s, 1H, arom);
3.1-2.7 (m, 4H, $CH_2$'s);
2.3-2.0 (m, 2H, $CH_2$).

IR (neat) 3100, 2920, 1510, 1310, 1110, 960, 900 $cm^{-1}$.

Example 3

6,7-Dihydro-5H-thieno[3,2-b]thiopyran-3-sulfonamide

A solution of 11.8 g (0.05 mol) of 3-bromo-6,7-dihydro-5H-thieno[3,2-b]thiopyran in 50 mls of anhydrous ether was added dropwise to a solution of 0.055 mol of n-butyllithium (1.6 M in hexane) in 100 mls of anhydrous ether such that the temperature remained below -70° C. The solution was stirred for 15 minutes at -78° C, treated with 3.0 mls (0.07 mol) of liquified $SO_2$ (exotherm to -50° C), allowed to warm to 20° C and stirred for 16 hours. The resulting precipitate was filtered, washed with ether and hexane and dried to give 12.5 g of the sulfinate salt as a cream colored powder, m.p. >300° C.

9.7 g (0.043 mol) of this salt was dissolved in 80 mls of glacial acetic acid and treated with 5.7 g of N-chlorosuccinimide. After 5 minutes, the solution was poured into ice-water and the resulting precipitate was filtered, washed with water and hexane and dried to give 8.15 g of the sulfonyl chloride as a yellow powder, m.p. 80-87° C.

The sulfonyl chloride was dissolved in 100 mls of methylene chloride and treated with 2.5 mls of liquified ammonia at -78° C. The mixture was warmed to 20° C, 100 mls of 1N HCl was added and the methylene chloride phase was separated, washed with brine, dried over $MgSO_4$ and concentrated to give 6.2 g of the desired sulfonamide as a white powder, m.p. 146-149° C.

200 MHz NMR ($CDCl_3$):

δ 7.9 (s, 1H, arom);
4.95 (b, 2H, $NH_2$);
3.10 (m, 2H, $CH_2$);
2.90 (t, 2H, $CH_2$);
2.30 (m, 2H, $CH_2$).
IR (nujol) 3360, 3260, 3100, 1545, 1320, 1150, 960 $cm^{-1}$.

Example 4

6,7-Dihydro-5H-thieno[3,2-b]thiopyran-3-sulfonamide, 4,4-dioxide

A suspension of 9.0 4 (0.038 mol) of 6,7-dihydro-5H-thieno[3,2-b]thiopyran-3-sulfonamide in 200 mls of methylene chloride was treated with 2.0 equivalents of m-chloroperbenzoic acid at -5° C to 0° C. The mixture was stirred for 30 minutes at 0° C, 200 mls of saturated sodium bicarbonate solution was added and the methylene chloride layer and suspended solids were separated. The methylene chloride layer was concentrated and the combined solids were washed with water and ether and dried to give 7.8 g of the desired sulfonamide as a white powder, m.p. 213-215° C.

90 MHz NMR ($CDCl_3$):
δ 8.2 (s, 1H, arom);
7.2 (b, 2H, $NH_2$);
3.7-2.2 (m, 6H, $CH_2$'s).
IR (nujol) 3390, 3270, 3100, 1345, 1280, 1150, 1115 $cm^{-1}$.

Example 5

N-[(4,6-dimethoxypyrimidin-2-yl)aminocarbonyl]-6,7-dihydro-5H-thieno[3,2-b]thiopyran-3-sulfonamide, 4,4-dioxide

A mixture of 10.5 g (0.039 mol) of 6,7-dihydro-5H-thieno[3,2-b]thiopyran-3-sulfonamide-4,4-dioxide, 6.9 g of potassium carbonate and 5.3 ml of n-butyl isocyanate were refluxed in 300 mls of dry acetonitrile for 16 hours, cooled and filtered. The solid was dissolved in water, acidified to pH 3 with concentrated HCl and the resulting precipitate was filtered, washed with water and ether and dried in vacuo at 70° C to give 10.7 g of the n-butylurea as a cream colored solid, m.p. 193-195° C.

A mixture of 10.2 g of the butylurea, 3.1 mls of butyl isocyanate and 0.1 g of DABCO were heated in 200 mls of dry xylenes and treated with 3.0 ml of liquified phosgene at such a rate that the temperature remained above 134° C. The mixture was refluxed for 1.5 hours, excess phosgene and HCl were purged with dry nitrogen and the mixture was cooled to 20° C. The resulting solid was filtered under nitrogen and washed with dry xylenes to give 7.0 g of the sulfonyl isocyanate as a light gray powder, m.p. 216-230° C, IR 2240 $cm^{-1}$.

1.4 g of the sulfonyl isocyanate was dissolved in 20 mls of dry methylene chloride and filtered to remove some insolubles. The filtrate was treated with 0.31 g of 2-amino-4,6-dimethoxypyrimidine, heated to reflux for 1 minute and allowed to stir at 20° C for 1.5 hours. The resulting precipitate was filtered, washed with dry methylene chloride and air dried to give 0.37 g of the desired sulfonylurea as a white powder, m.p. 228-230° C.

200 MHz NMR ($CDCl_3$):
δ 12.57 (b, 1H, NH);
8.33 (s, 1H, arom);
7.20 (d, 1H, NH);
5.75 (s, 1H, CH);
3.97 (s, 6H, $OCH_3$'s);
3.40 (m, 2H, $CH_2$);
3.05 (m, 2H, $CH_2$);
and
2.60 (m, 2H, $CH_2$).
1R (nujol) 3380, 1720, 1700, 1600, 1445, 1375, 1195, 1155 $cm^{-1}$.

Following the procedures described in Examples 1-5, the following compounds can be prepared.

## GENERAL TABLE OF STRUCTURES

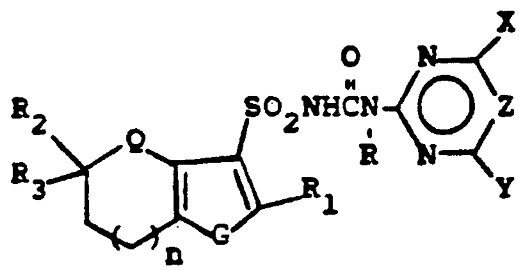

Structure Ia

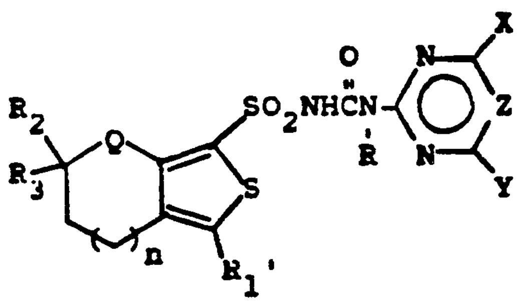

Structure Ib

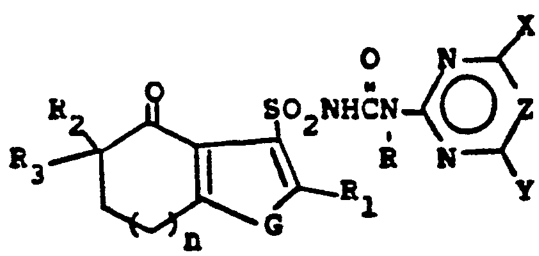

Structure Ic

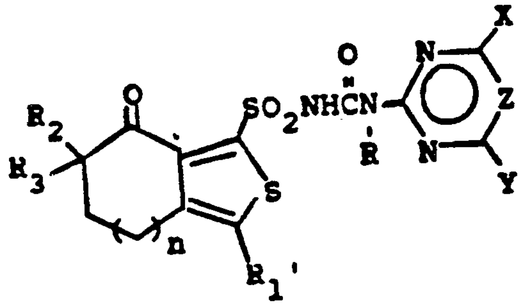

Structure Id

GENERAL TABLE OF STRUCTURES (CONTINUED)

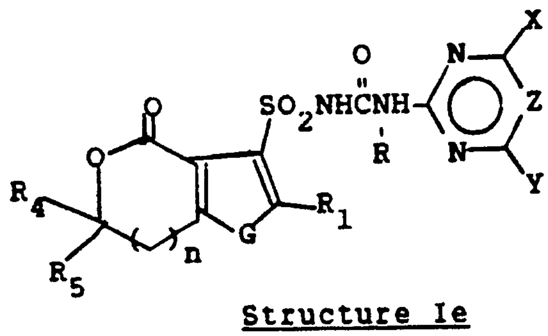

Structure Ie

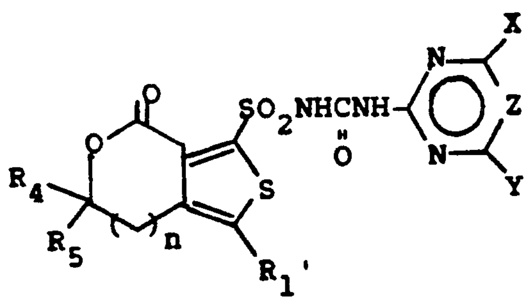

Structure If

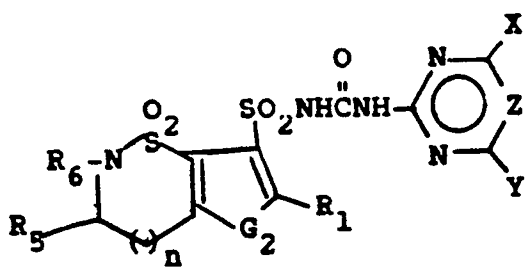

Structure Ig

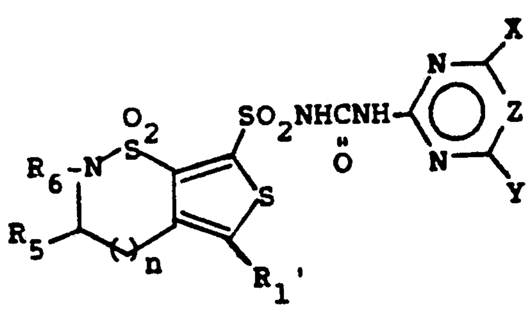

Structure Ih

GENERAL TABLE OF STRUCTURES (CONTINUED)

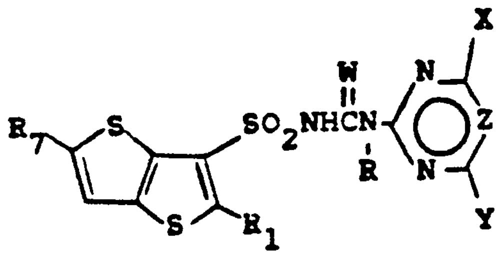

Structure Ii

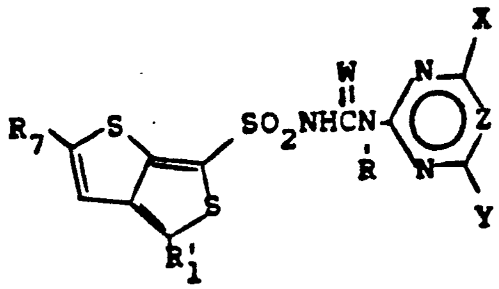

Structure Ij

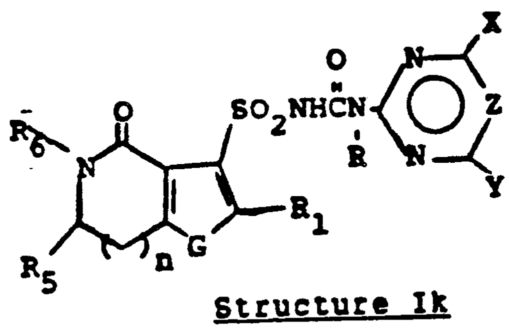

Structure Ik

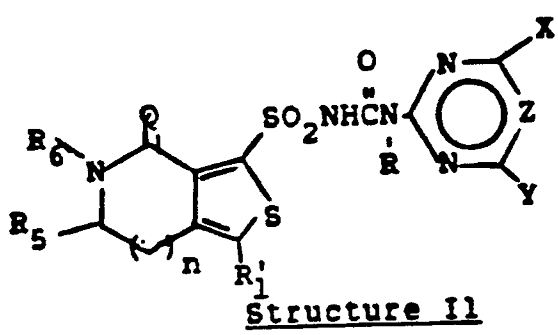

Structure Il

GENERAL TABLE OF STRUCTURES (CONTINUED)

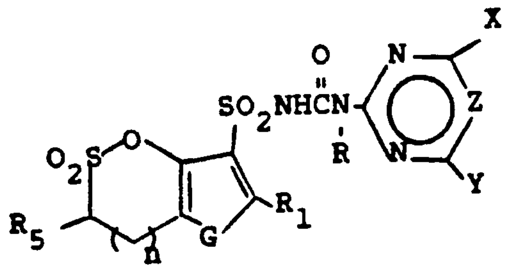

Structure Im

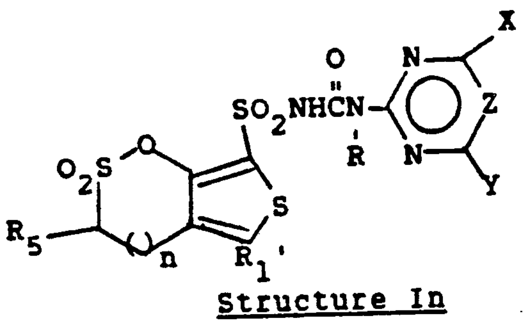

Structure In

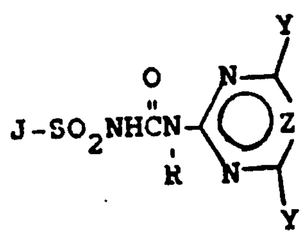

Structure Io

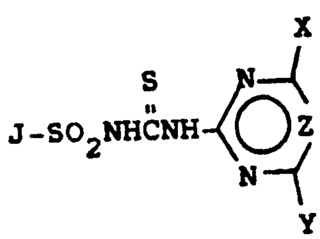

Structure Ip

GENERAL TABLE OF STRUCTURES (CONTINUED)

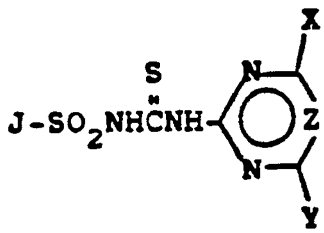

Structure Iq

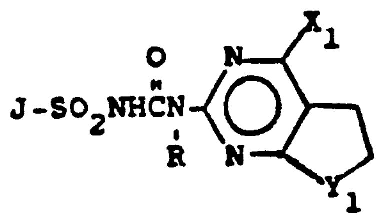

Structure IIa

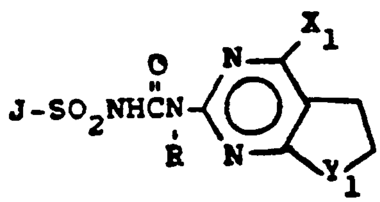

Structure IIb

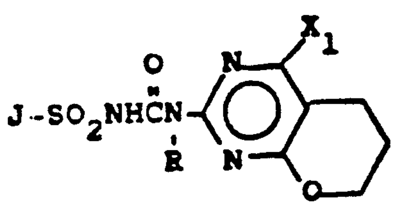

Structure IIIa

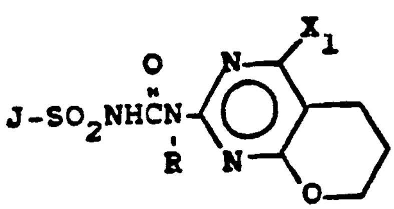

Structure IIIb

GENERAL TABLE OF STRUCTURES (CONTINUED)

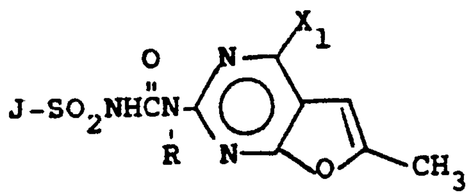

Structure IVa

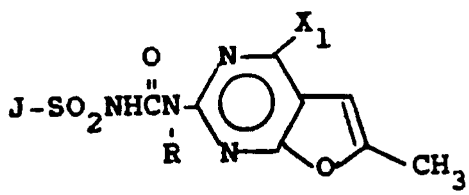

Structure IVb

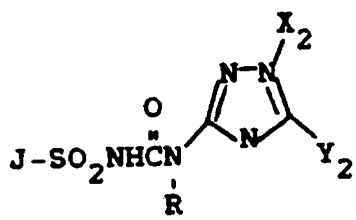

Structure Va

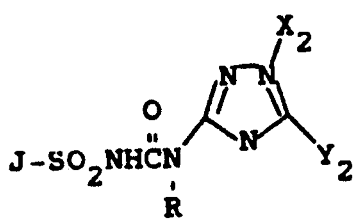

Structure Vb

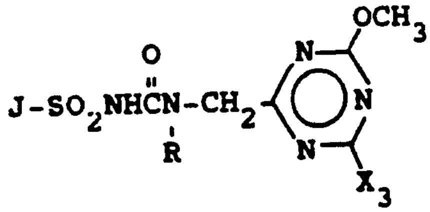

Structure VIa

GENERAL TABLE OF STRUCTURES (CONTINUED)

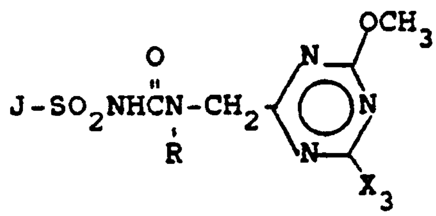

Structure VIb

## Table Ia

## Structure Ia

| n | Q | G | R | $R_1$ | $R_2$ | $R_3$ | X | Y | Z | m.p.(°C) |
|---|---|---|---|---|---|---|---|---|---|---|
| 1 | $SO_2$ | S | H | H | H | H | $CH_3$ | $CH_3$ | CH | 214-220° |
| 1 | $SO_2$ | S | H | H | H | H | $CH_3$ | $OCH_3$ | CH | 224-226° |
| 1 | $SO_2$ | S | H | H | H | H | $OCH_3$ | $OCH_3$ | CH | 228-230° |
| 1 | $SO_2$ | S | H | H | H | H | Cl | $OCH_3$ | CH | 214-220° |
| 1 | $SO_2$ | S | H | H | H | H | $CH_3$ | $OCH_3$ | N | 195-197° |
| 1 | $SO_2$ | S | H | H | H | H | $OCH_3$ | $OCH_3$ | N | 190-194° |
| 0 | $SO_2$ | S | H | H | H | H | $CH_3$ | $CH_3$ | CH | |
| 0 | $SO_2$ | S | H | H | H | H | $CH_3$ | $OCH_3$ | CH | |
| 0 | $SO_2$ | S | H | H | H | H | $OCH_3$ | $OCH_3$ | CH | |
| 0 | $SO_2$ | S | H | H | H | H | Cl | $OCH_3$ | CH | |
| 0 | $SO_2$ | S | H | H | H | H | Br | $OCH_3$ | CH | |
| 0 | $SO_2$ | S | H | H | H | H | $CH_3$ | $OCH_3$ | N | |
| 0 | $SO_2$ | S | H | H | H | H | $OCH_3$ | $OCH_3$ | N | |
| 0 | $SO_2$ | S | H | H | $CH_3$ | H | $CH_3$ | $CH_3$ | CH | 218-219 |
| 0 | $SO_2$ | S | H | H | $CH_3$ | H | $CH_3$ | $OCH_3$ | CH | 208-209 |
| 0 | $SO_2$ | S | H | H | $CH_3$ | H | $OCH_3$ | $OCH_3$ | CH | 186-187 |
| 0 | $SO_2$ | S | H | H | $CH_3$ | H | Cl | $OCH_3$ | CH | 203-204 |
| 0 | $SO_2$ | S | H | H | $CH_3$ | H | Br | $OCH_3$ | CH | |
| 0 | $SO_2$ | S | H | H | $CH_3$ | H | $CH_3$ | $OCH_3$ | N | 192-193 |
| 0 | $SO_2$ | S | H | H | $CH_3$ | H | $OCH_3$ | $OCH_3$ | N | 176-178 |
| 0 | $SO_2$ | S | H | H | $CH_3$ | $CH_3$ | $CH_3$ | $CH_3$ | CH | 189-191 |
| 0 | $SO_2$ | S | H | H | $CH_3$ | $CH_3$ | $CH_3$ | $OCH_3$ | CH | 208-210 |
| 0 | $SO_2$ | S | H | H | $CH_3$ | $CH_3$ | $OCH_3$ | $OCH_3$ | CH | 237-238 |
| 0 | $SO_2$ | S | H | H | $CH_3$ | $CH_3$ | Cl | $OCH_3$ | CH | 231-232 |
| 0 | $SO_2$ | S | H | H | $CH_3$ | $CH_3$ | Br | $OCH_3$ | CH | |
| 0 | $SO_2$ | S | H | H | $CH_3$ | $CH_3$ | $CH_3$ | $OCH_3$ | N | 136-140 |
| 0 | $SO_2$ | S | H | H | $CH_3$ | $CH_3$ | $OCH_3$ | $OCH_3$ | N | 202-203 |
| 1 | $SO_2$ | S | H | H | $CH_3$ | H | $CH_3$ | $CH_3$ | CH | |
| 1 | $SO_2$ | S | H | H | $CH_3$ | H | $CH_3$ | $OCH_3$ | CH | |
| 1 | $SO_2$ | S | H | H | $CH_3$ | H | $OCH_3$ | $OCH_3$ | CH | |
| 1 | $SO_2$ | S | H | H | $CH_3$ | H | Cl | $OCH_3$ | CH | |

Table Ia (Continued)

| n | Q | G | R | $R_1$ | $R_2$ | $R_3$ | X | Y | Z | m.p. (°C) |
|---|---|---|---|---|---|---|---|---|---|---|
| 1 | $SO_2$ | S | H | H | $CH_3$ | H | Br | $OCH_3$ | CH | |
| 1 | $SO_2$ | S | H | H | $CH_3$ | H | $CH_3$ | $OCH_3$ | N | |
| 1 | $SO_2$ | S | H | H | $CH_3$ | H | $OCH_3$ | $OCH_3$ | N | |
| 1 | $SO_2$ | S | H | H | $CH_3$ | $CH_3$ | $CH_3$ | $OCH_3$ | CH | |
| 1 | $SO_2$ | S | H | H | $CH_3$ | $CH_3$ | $OCH_3$ | $OCH_3$ | CH | |
| 1 | S | S | H | H | H | H | $CH_3$ | $OCH_3$ | N | |
| 1 | S | S | H | H | H | H | $OCH_3$ | $OCH_3$ | CH | |
| 1 | S | S | H | H | $CH_3$ | H | $CH_3$ | $OCH_3$ | N | |
| 1 | S | S | H | H | $CH_3$ | H | $OCH_3$ | $OCH_3$ | CH | |
| 1 | S | S | H | H | $CH_3$ | $CH_3$ | $CH_3$ | $OCH_3$ | N | |
| 1 | S | S | H | H | $CH_3$ | $CH_3$ | $OCH_3$ | $OCH_3$ | CH | |
| 0 | S | S | H | H | H | H | $CH_3$ | $CH_3$ | CH | |
| 0 | S | S | H | H | H | H | $CH_3$ | $OCH_3$ | CH | |
| 0 | S | S | H | H | H | H | $OCH_3$ | $OCH_3$ | CH | |
| 0 | S | S | H | H | H | H | Cl | $OCH_3$ | CH | |
| 0 | S | S | H | H | H | H | Br | $OCH_3$ | CH | |
| 0 | S | S | H | H | H | H | $CH_3$ | $OCH_3$ | N | |
| 0 | S | S | H | H | H | H | $OCH_3$ | $OCH_3$ | N | |
| 0 | S | S | H | H | $CH_3$ | H | $CH_3$ | $CH_3$ | CH | 197-198 |
| 0 | S | S | H | H | $CH_3$ | H | $CH_3$ | $OCH_3$ | CH | 199-200 |
| 0 | S | S | H | H | $CH_3$ | H | $OCH_3$ | $OCH_3$ | CH | 190-191 |
| 0 | S | S | H | H | $CH_3$ | H | Cl | $OCH_3$ | CH | 185-186 |
| 0 | S | S | H | H | $CH_3$ | H | Br | $OCH_3$ | CH | |
| 0 | S | S | H | H | $CH_3$ | H | $CH_3$ | $OCH_3$ | N | 182-183 |
| 0 | S | S | H | H | $CH_3$ | H | $OCH_3$ | $OCH_3$ | N | 179-181 |
| 0 | S | S | H | H | $CH_3$ | $CH_3$ | $CH_3$ | $CH_3$ | CH | 190-191 |
| 0 | S | S | H | H | $CH_3$ | $CH_3$ | $CH_3$ | $OCH_3$ | CH | 163-164 |
| 0 | S | S | H | H | $CH_3$ | $CH_3$ | $OCH_3$ | $OCH_3$ | CH | 180-181 |
| 0 | S | S | H | H | $CH_3$ | $CH_3$ | Cl | $OCH_3$ | CH | 166-167 |
| 0 | S | S | H | H | $CH_3$ | $CH_3$ | Br | $OCH_3$ | CH | |
| 0 | S | S | H | H | $CH_3$ | $CH_3$ | $CH_3$ | $OCH_3$ | N | 187-188 |
| 0 | S | S | H | H | $CH_3$ | $CH_3$ | $OCH_3$ | $OCH_3$ | N | 173-174 |

Table Ia (Continued)

| n | Q | G | R | $R_1$ | $R_2$ | $R_3$ | X | Y | Z | m.p. (°C) |
|---|---|---|---|---|---|---|---|---|---|---|
| 0 | SO | S | H | H | $CH_3$ | H | $CH_3$ | $CH_3$ | CH | |
| 0 | SO | S | H | H | $CH_3$ | H | $CH_3$ | $OCH_3$ | CH | |
| 0 | SO | S | H | H | $CH_3$ | H | $OCH_3$ | $OCH_3$ | CH | |
| 0 | SO | S | H | H | $CH_3$ | H | Cl | $OCH_3$ | CH | |
| 0 | SO | S | H | H | $CH_3$ | H | Br | $OCH_3$ | CH | |
| 0 | SO | S | H | H | $CH_3$ | H | $CH_3$ | $OCH_3$ | N | |
| 0 | SO | S | H | H | $CH_3$ | H | $OCH_3$ | $OCH_3$ | N | |
| 0 | O | S | H | H | H | H | $CH_3$ | $OCH_3$ | N | |
| 0 | O | S | H | H | H | H | $OCH_3$ | $OCH_3$ | CH | |
| 0 | O | S | H | H | $CH_3$ | H | $CH_3$ | $OCH_3$ | N | |
| 0 | O | S | H | H | $CH_3$ | H | $OCH_3$ | $OCH_3$ | CH | |
| 0 | O | S | H | H | $CH_3$ | $CH_3$ | $CH_3$ | $OCH_3$ | N | |
| 0 | O | S | H | H | $CH_3$ | $CH_3$ | $OCH_3$ | $OCH_3$ | CH | |
| 1 | O | S | H | H | $CH_3$ | H | $OCH_3$ | $OCH_3$ | N | |
| 1 | O | S | H | H | $CH_3$ | H | $OCH_3$ | $OCH_3$ | CH | |
| 0 | $CH_2$ | S | H | H | H | H | $CH_3$ | $OCH_3$ | N | |
| 0 | $CH_2$ | S | H | H | H | H | $OCH_3$ | $OCH_3$ | CH | |
| 1 | $CH_2$ | S | H | H | H | H | $CH_3$ | $OCH_3$ | N | |
| 1 | $CH_2$ | S | H | H | H | H | $OCH_3$ | $OCH_3$ | CH | |
| 0 | $CH_2$ | O | H | H | H | H | $CH_3$ | $OCH_3$ | N | |
| 0 | $CH_2$ | O | H | H | H | H | $OCH_3$ | $OCH_3$ | CH | |
| 1 | $CH_2$ | O | H | H | H | H | $CH_3$ | $OCH_3$ | N | |
| 1 | $CH_2$ | O | H | H | H | H | $OCH_3$ | $OCH_3$ | CH | |
| 0 | $CH_2$ | NH | H | H | H | H | $CH_3$ | $OCH_3$ | N | |
| 0 | $CH_2$ | NH | H | H | H | H | $OCH_3$ | $OCH_3$ | CH | |
| 1 | $CH_2$ | NH | H | H | H | H | $CH_3$ | $OCH_3$ | N | |
| 1 | $CH_2$ | NH | H | H | H | H | $OCH_3$ | $OCH_3$ | CH | |
| 0 | $CH_2$ | $NCH_3$ | H | H | H | H | $CH_3$ | $OCH_3$ | N | |
| 0 | $CH_2$ | $NCH_3$ | H | H | H | H | $OCH_3$ | $OCH_3$ | CH | |
| 1 | $CH_2$ | $NCH_3$ | H | H | H | H | $CH_3$ | $OCH_3$ | N | |
| 1 | $CH_2$ | $NCH_3$ | H | H | H | H | $OCH_3$ | $OCH_3$ | CH | |

Table Ia (Continued)

| n | Q | G | R | $R_1$ | $R_2$ | $R_3$ | X | Y | Z | m.p. (°C) |
|---|---|---|---|---|---|---|---|---|---|---|
| 0 | $CHCH_3$ | S | H | H | H | H | $CH_3$ | $OCH_3$ | N | |
| 0 | $CHCH_3$ | S | H | H | H | H | $OCH_3$ | $OCH_3$ | CH | |
| 0 | $SO_2$ | O | H | $CH_3$ | H | H | $CH_3$ | $CH_3$ | CH | |
| 0 | $SO_2$ | O | H | $CH_3$ | H | H | $CH_3$ | $OCH_3$ | CH | |
| 0 | $SO_2$ | O | H | $CH_3$ | H | H | $OCH_3$ | $OCH_3$ | CH | |
| 0 | $SO_2$ | O | H | $CH_3$ | H | H | Cl | $OCH_3$ | CH | |
| 0 | $SO_2$ | O | H | $CH_3$ | H | H | Br | $OCH_3$ | CH | |
| 0 | $SO_2$ | O | H | $CH_3$ | H | H | $CH_3$ | $OCH_3$ | N | |
| 0 | $SO_2$ | O | H | $CH_3$ | H | H | $OCH_3$ | $OCH_3$ | N | |
| 0 | $SO_2$ | NH | H | $CH_3$ | H | H | $CH_3$ | $CH_3$ | CH | |
| 0 | $SO_2$ | NH | H | $CH_3$ | H | H | $CH_3$ | $OCH_3$ | CH | |
| 0 | $SO_2$ | NH | H | $CH_3$ | H | H | $OCH_3$ | $OCH_3$ | CH | |
| 0 | $SO_2$ | NH | H | $CH_3$ | H | H | Cl | $OCH_3$ | CH | |
| 0 | $SO_2$ | NH | H | $CH_3$ | H | H | Br | $OCH_3$ | CH | |
| 0 | $SO_2$ | NH | H | $CH_3$ | H | H | $CH_3$ | $OCH_3$ | N | |
| 0 | $SO_2$ | NH | H | $CH_3$ | H | H | $OCH_3$ | $OCH_3$ | N | |
| 0 | $SO_2$ | $NCH_3$ | H | $CH_3$ | H | H | $CH_3$ | $CH_3$ | CH | |
| 0 | $SO_2$ | $NCH_3$ | H | $CH_3$ | H | H | $CH_3$ | $OCH_3$ | CH | |
| 0 | $SO_2$ | $NCH_3$ | H | $CH_3$ | H | H | $OCH_3$ | $OCH_3$ | CH | |
| 0 | $SO_2$ | $NCH_3$ | H | $CH_3$ | H | H | Cl | $OCH_3$ | CH | |
| 0 | $SO_2$ | $NCH_3$ | H | $CH_3$ | H | H | Br | $OCH_3$ | CH | |
| 0 | $SO_2$ | $NCH_3$ | H | $CH_3$ | H | H | $CH_3$ | $OCH_3$ | N | |
| 0 | $SO_2$ | $NCH_3$ | H | $CH_3$ | H | H | $OCH_3$ | $OCH_3$ | N | |
| 0 | $SO_2$ | S | H | $CH_3$ | H | H | $CH_3$ | $CH_3$ | CH | |
| 0 | $SO_2$ | S | H | $CH_3$ | H | H | $CH_3$ | $OCH_3$ | CH | |
| 0 | $SO_2$ | S | H | $CH_3$ | H | H | $OCH_3$ | $OCH_3$ | CH | |
| 0 | $SO_2$ | S | H | $CH_3$ | H | H | Cl | $OCH_3$ | CH | |
| 0 | $SO_2$ | S | H | $CH_3$ | H | H | Br | $OCH_3$ | CH | |
| 0 | $SO_2$ | S | H | $CH_3$ | H | H | $CH_3$ | $OCH_3$ | N | |
| 0 | $SO_2$ | S | H | $CH_3$ | H | H | $OCH_3$ | $OCH_3$ | N | |
| 0 | $SO_2$ | S | H | $CH_3$ | $CH_3$ | H | $CH_3$ | $CH_3$ | CH | |
| 0 | $SO_2$ | S | H | $CH_3$ | $CH_3$ | H | $CH_3$ | $OCH_3$ | CH | |

Table Ia (Continued)

| n | Q | G | R | $R_1$ | $R_2$ | $R_3$ | X | Y | Z | m.p. (°C) |
|---|---|---|---|---|---|---|---|---|---|---|
| 0 | $SO_2$ | S | H | $CH_3$ | $CH_3$ | H | $OCH_3$ | $OCH_3$ | CH | |
| 0 | $SO_2$ | S | H | $CH_3$ | $CH_3$ | H | Cl | $OCH_3$ | CH | |
| 0 | $SO_2$ | S | H | $CH_3$ | $CH_3$ | H | $CH_3$ | $OCH_3$ | CH | |
| 0 | $SO_2$ | S | H | $CH_3$ | $CH_3$ | H | $CH_3$ | $OCH_3$ | N | |
| 0 | $SO_2$ | S | H | $CH_3$ | $CH_3$ | H | $OCH_3$ | $OCH_3$ | N | |
| 0 | $SO_2$ | S | H | $CH_3$ | $CH_3$ | $CH_3$ | $CH_3$ | $CH_3$ | CH | |
| 0 | $SO_2$ | S | H | $CH_3$ | $CH_3$ | $CH_3$ | $CH_3$ | $OCH_3$ | CH | |
| 0 | $SO_2$ | S | H | $CH_3$ | $CH_3$ | $CH_3$ | $OCH_3$ | $OCH_3$ | CH | |
| 0 | $SO_2$ | S | H | $CH_3$ | $CH_3$ | $CH_3$ | Cl | $OCH_3$ | CH | |
| 0 | $SO_2$ | S | H | $CH_3$ | $CH_3$ | $CH_3$ | Br | $OCH_3$ | CH | |
| 0 | $SO_2$ | S | H | $CH_3$ | $CH_3$ | $CH_3$ | $CH_3$ | $OCH_3$ | N | |
| 0 | $SO_2$ | S | H | $CH_3$ | $CH_3$ | $CH_3$ | $OCH_3$ | $OCH_3$ | N | |
| 0 | $SO_2$ | S | H | Cl | H | H | $CH_3$ | $CH_3$ | CH | |
| 0 | $SO_2$ | S | H | Cl | H | H | $CH_3$ | $OCH_3$ | CH | |
| 0 | $SO_2$ | S | H | Cl | H | H | $OCH_3$ | $OCH_3$ | CH | |
| 0 | $SO_2$ | S | H | Cl | H | H | Cl | $OCH_3$ | CH | |
| 0 | $SO_2$ | S | H | Cl | H | H | Br | $OCH_3$ | CH | |
| 0 | $SO_2$ | S | H | Cl | H | H | $CH_3$ | $OCH_3$ | N | |
| 0 | $SO_2$ | S | H | Cl | H | H | $OCH_3$ | $OCH_3$ | N | |
| 0 | $SO_2$ | S | H | Cl | $CH_3$ | H | $CH_3$ | $CH_3$ | CH | |
| 0 | $SO_2$ | S | H | Cl | $CH_3$ | H | $CH_3$ | $OCH_3$ | CH | |
| 0 | $SO_2$ | S | H | Cl | $CH_3$ | H | $OCH_3$ | $OCH_3$ | CH | |
| 0 | $SO_2$ | S | H | Cl | $CH_3$ | H | Cl | $OCH_3$ | CH | |
| 0 | $SO_2$ | S | H | Cl | $CH_3$ | H | Br | $OCH_3$ | CH | |
| 0 | $SO_2$ | S | H | Cl | $CH_3$ | H | $CH_3$ | $OCH_3$ | N | |
| 0 | $SO_2$ | S | H | Cl | $CH_3$ | H | $OCH_3$ | $OCH_3$ | N | |
| 0 | $SO_2$ | S | H | Cl | $CH_3$ | $CH_3$ | $OCH_3$ | $OCH_3$ | CH | |
| 0 | $SO_2$ | S | H | Br | H | H | $CH_3$ | $OCH_3$ | N | |
| 0 | $SO_2$ | S | H | Br | H | H | $CH_3$ | $OCH_3$ | CH | |
| 0 | $SO_2$ | S | H | Br | H | H | $OCH_3$ | $OCH_3$ | CH | |
| 0 | $SO_2$ | S | H | Br | $CH_3$ | H | $CH_3$ | $OCH_3$ | N | |
| 0 | $SO_2$ | S | H | Br | $CH_3$ | H | $CH_3$ | $OCH_3$ | CH | |

Table Ia (Continued)

| n | Q | G | R | $R_1$ | $R_2$ | $R_3$ | X | Y | Z | m.p. (°C) |
|---|---|---|---|---|---|---|---|---|---|---|
| 0 | $SO_2$ | S | H | Br | $CH_3$ | H | $OCH_3$ | $OCH_3$ | CH | |
| 0 | $SO_2$ | S | H | $OCH_3$ | H | H | $CH_3$ | $OCH_3$ | N | |
| 0 | $SO_2$ | S | H | $OCH_3$ | H | H | $CH_3$ | $OCH_3$ | CH | |
| 0 | $SO_2$ | S | H | $OCH_3$ | H | H | $OCH_3$ | $OCH_3$ | CH | |
| 0 | $SO_2$ | S | H | $OCH_3$ | $CH_3$ | H | $CH_3$ | $OCH_3$ | N | |
| 0 | $SO_2$ | S | H | $OCH_3$ | $CH_3$ | H | $CH_3$ | $OCH_3$ | CH | |
| 0 | $SO_2$ | S | H | $OCH_3$ | $CH_3$ | H | $OCH_3$ | $OCH_3$ | CH | |
| 0 | $SO_2$ | S | H | $SCH_3$ | H | H | $CH_3$ | $OCH_3$ | N | |
| 0 | $SO_2$ | S | H | $SCH_3$ | H | H | $CH_3$ | $OCH_3$ | CH | |
| 0 | $SO_2$ | S | H | $SCH_3$ | H | H | $OCH_3$ | $OCH_3$ | CH | |
| 0 | $SO_2$ | S | H | $SCH_3$ | $CH_3$ | H | $CH_3$ | $OCH_3$ | N | |
| 0 | $SO_2$ | S | H | $SCH_3$ | $CH_3$ | H | $CH_3$ | $OCH_3$ | CH | |
| 0 | $SO_2$ | S | H | $SCH_3$ | $CH_3$ | H | $OCH_3$ | $OCH_3$ | CH | |
| 0 | $SO_2$ | S | H | $SO_2CH_3$ | H | H | $CH_3$ | $OCH_3$ | N | |
| 0 | $SO_2$ | S | H | $SO_2CH_3$ | H | H | $CH_3$ | $OCH_3$ | CH | |
| 0 | $SO_2$ | S | H | $SO_2CH_3$ | H | H | $OCH_3$ | $OCH_3$ | CH | |
| 0 | $SO_2$ | S | H | $SO_2CH_3$ | $CH_3$ | H | $CH_3$ | $OCH_3$ | N | |
| 0 | $SO_2$ | S | H | $SO_2CH_3$ | $CH_3$ | H | $CH_3$ | $OCH_3$ | CH | |
| 0 | $SO_2$ | S | H | $SO_2CH_3$ | $CH_3$ | H | $OCH_3$ | $OCH_3$ | CH | |
| 0 | $SO_2$ | S | H | $NO_2$ | H | H | $CH_3$ | $OCH_3$ | N | |
| 0 | $SO_2$ | S | H | $NO_2$ | H | H | $CH_3$ | $OCH_3$ | CH | |
| 0 | $SO_2$ | S | H | $NO_2$ | H | H | $OCH_3$ | $OCH_3$ | CH | |
| 0 | $SO_2$ | S | H | $NO_2$ | $CH_3$ | H | $CH_3$ | $OCH_3$ | N | |
| 0 | $SO_2$ | S | H | $NO_2$ | $CH_3$ | H | $CH_3$ | $OCH_3$ | CH | |
| 0 | $SO_2$ | S | H | $NO_2$ | $CH_3$ | H | $OCH_3$ | $OCH_3$ | CH | |
| 0 | S | S | H | $CH_3$ | $CH_3$ | H | $CH_3$ | $OCH_3$ | N | |
| 0 | S | S | H | $CH_3$ | $CH_3$ | H | $CH_3$ | $OCH_3$ | CH | |
| 0 | S | S | H | $CH_3$ | $CH_3$ | H | $OCH_3$ | $OCH_3$ | CH | |
| 0 | S | S | H | $OCH_3$ | $CH_3$ | H | $CH_3$ | $OCH_3$ | N | |
| 0 | S | S | H | $OCH_3$ | $CH_3$ | H | $CH_3$ | $OCH_3$ | CH | |
| 0 | S | S | H | $OCH_3$ | $CH_3$ | H | $OCH_3$ | $OCH_3$ | CH | |

Table Ia (Continued)

| n | Q | G | R | $R_1$ | $R_2$ | $R_3$ | X | Y | Z | m.p. (°C) |
|---|---|---|---|---|---|---|---|---|---|---|
| 0 | S | S | H | Cl | $CH_3$ | H | $CH_3$ | $OCH_3$ | N | |
| 0 | S | S | H | Cl | $CH_3$ | H | $CH_3$ | $OCH_3$ | CH | |
| 0 | S | S | H | Cl | $CH_3$ | H | $OCH_3$ | $OCH_3$ | CH | |
| 0 | S | S | H | Br | $CH_3$ | H | $CH_3$ | $OCH_3$ | N | |
| 0 | S | S | H | Br | $CH_3$ | H | $CH_3$ | $OCH_3$ | CH | |
| 0 | S | S | H | Br | $CH_3$ | H | $OCH_3$ | $OCH_3$ | CH | |
| 0 | S | S | H | $SCH_3$ | $CH_3$ | H | $CH_3$ | $OCH_3$ | N | |
| 0 | S | S | H | $SCH_3$ | $CH_3$ | H | $CH_3$ | $OCH_3$ | CH | |
| 0 | S | S | H | $SCH_3$ | $CH_3$ | H | $OCH_3$ | $OCH_3$ | CH | |
| 0 | S | S | H | $SO_2CH_3$ | $CH_3$ | H | $CH_3$ | $OCH_3$ | N | |
| 0 | S | S | H | $SO_2CH_3$ | $CH_3$ | H | $CH_3$ | $OCH_3$ | CH | |
| 0 | S | S | H | $SO_2CH_3$ | $CH_3$ | H | $OCH_3$ | $OCH_3$ | CH | |
| 0 | S | S | H | $NO_2$ | $CH_3$ | H | $CH_3$ | $OCH_3$ | N | |
| 0 | S | S | H | $NO_2$ | $CH_3$ | H | $CH_3$ | $OCH_3$ | CH | |
| 0 | S | S | H | $NO_2$ | $CH_3$ | H | $OCH_3$ | $OCH_3$ | CH | |
| 0 | $SO_2$ | S | H | H | $CH_2CH_3$ | H | $CH_3$ | $OCH_3$ | N | |
| 0 | $SO_2$ | S | H | H | $CH_2CH_3$ | H | $CH_3$ | $OCH_3$ | CH | |
| 0 | $SO_2$ | S | H | H | $CH_2CH_3$ | H | $OCH_3$ | $OCH_3$ | CH | |
| 0 | $SO_2$ | S | H | H | $CH_2CH_2CH_3$ | H | $OCH_3$ | $OCH_3$ | CH | |
| 0 | $SO_2$ | S | H | H | H | $CH(CH_3)_2$ | $OCH_3$ | $OCH_3$ | CH | |
| 0 | $SO_2$ | S | H | H | H | $C(CH_3)_3$ | $OCH_3$ | $OCH_3$ | CH | |
| 0 | $SO_2$ | S | $CH_3$ | H | H | $CH_3$ | $CH_3$ | $OCH_3$ | CH | |
| 0 | $SO_2$ | S | $CH_3$ | H | H | $CH_3$ | $OCH_3$ | $OCH_3$ | CH | |
| 0 | $SO_2$ | S | $CH_3$ | H | H | $CH_3$ | $CH_3$ | $OCH_3$ | N | |
| 0 | $SO_2$ | S | $CH_3$ | H | H | $CH_3$ | $OCH_3$ | $OCH_3$ | N | |
| 0 | $SO_2$ | S | H | H | $CH_3$ | H | $OCH_2CH_3$ | $OCH_3$ | CH | |
| 0 | $SO_2$ | S | H | H | $CH_3$ | H | $OCH_2CH_2CH_3$ | $OCH_3$ | CH | |
| 0 | $SO_2$ | S | H | H | $CH_3$ | H | $OCH(CH_3)_2$ | $OCH_3$ | CH | |
| 0 | $SO_2$ | S | H | H | $CH_3$ | H | F | $OCH_3$ | CH | |
| 0 | $SO_2$ | S | H | H | $CH_3$ | H | $CH_2F$ | $OCH_3$ | CH | |
| 0 | $SO_2$ | S | H | H | $CH_3$ | H | $OCHF_2$ | $OCH_3$ | CH | |

Table Ia (Continued)

| n | Q | G | R | $R_1$ | $R_2$ | $R_3$ | X | Y | Z | m.p. (°C) |
|---|---|---|---|---|---|---|---|---|---|---|
| 0 | $SO_2$ | S | H | H | $CH_3$ | H | $OCH_2CF_3$ | $OCH_3$ | CH | |
| 0 | $SO_2$ | S | H | H | $CH_3$ | H | $CF_3$ | $OCH_3$ | CH | |
| 0 | $SO_2$ | S | H | H | $CH_3$ | H | $OCH_3$ | H | CH | |
| 0 | $SO_2$ | S | H | H | $CH_3$ | H | $OCH_3$ | $OCH_2CH_3$ | CH | |
| 0 | $SO_2$ | S | H | H | $CH_3$ | H | $OCH_3$ | $CH_2CH_3$ | CH | |
| 0 | $SO_2$ | S | H | H | $CH_3$ | H | $OCH_3$ | $CH_2CH_2CH_3$ | CH | |
| 0 | $SO_2$ | S | H | H | $CH_3$ | H | $OCH_3$ | cyclo-propyl | N | |
| 0 | $SO_2$ | S | H | H | $CH_3$ | H | $OCH_3$ | $C\equiv CH$ | CH | |
| 0 | $SO_2$ | S | H | H | $CH_3$ | H | $OCH_3$ | $C\equiv CCH_3$ | CH | |
| 0 | $SO_2$ | S | H | H | $CH_3$ | H | $OCH_3$ | $CH_2C\equiv CCH_3$ | CH | |
| 0 | $SO_2$ | S | H | H | $CH_3$ | H | $OCH_3$ | $CH_2OCH_3$ | CH | |
| 0 | $SO_2$ | S | H | H | $CH_3$ | H | $OCH_3$ | $NH_2$ | CH | |
| 0 | $SO_2$ | S | H | H | $CH_3$ | H | $OCH_2CH_3$ | $NHCH_3$ | CH | |
| 0 | $SO_2$ | S | H | H | $CH_3$ | H | $OCH_3$ | $N(CH_3)_2$ | CH | |
| 0 | $SO_2$ | S | H | H | $CH_3$ | H | $OCH_3$ | $CF_3$ | CH | |
| 0 | $SO_2$ | S | H | H | $CH_3$ | H | $OCH_3$ | $SCH_3$ | CH | |
| 0 | $SO_2$ | S | H | H | $CH_3$ | H | $OCH_3$ | $OCH_2CH=CH_2$ | CH | |
| 0 | $SO_2$ | S | H | H | $CH_3$ | H | $OCH_3$ | $OCH_2C\equiv CH$ | CH | |
| 0 | $SO_2$ | S | H | H | $CH_3$ | H | $OCH_3$ | CH | CH | |
| 0 | $SO_2$ | S | H | H | $CH_3$ | H | $OCH_3$ | $N_3$ | CH | |
| 0 | $SO_2$ | S | H | H | $CH_3$ | H | $OCH_3$ | $OCH_2CH_2OCH_3$ | CH | |
| 0 | $SO_2$ | S | H | H | $CH_3$ | H | $OCH_3$ | $CH_2SCH_3$ | CH | |
| 0 | $SO_2$ | S | H | H | $CH_3$ | H | $OCH_3$ | $CH(OCH_3)_2$ | CH | |
| 0 | $SO_2$ | S | H | H | $CH_3$ | H | $OCH_3$ | $CH(SCH_3)_2$ | CH | |
| 0 | $SO_2$ | S | H | H | $CH_3$ | H | $OCH_3$ | $C(CH_3)(OCH_3)_2$ | CH | |
| 0 | $SO_2$ | S | H | H | $CH_3$ | H | $OCH_3$ | 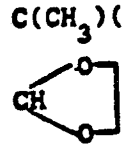 | CH | |
| 0 | $SO_2$ | S | H | H | $CH_3$ | H | $OCH_3$ | 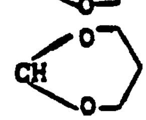 | CH | |
| 0 | $SO_2$ | S | H | H | $CH_3$ | H | $OCH_3$ | 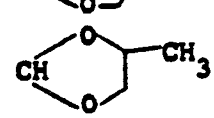 | CH | |

Table Ia (Continued)

| n | Q | G | R | $R_1$ | $R_2$ | $R_3$ | X | Y | Z | m.p. (°C) |
|---|---|---|---|---|---|---|---|---|---|---|
| 0 | $SO_2$ | S | H | H | $CH_3$ | H | $OCH_3$ | $CH(OCH_2CH_3)_2$ | CH | |
| 0 | $SO_2$ | S | H | H | $CH_3$ | H | $OCH_3$ | $OCF_2H$ | CH | |
| 0 | $SO_2$ | S | H | H | $CH_3$ | H | $OCH_3$ | $SCF_2H$ | CH | |
| 0 | $SO_2$ | S | H | H | $CH_3$ | H | $OCH_3$ | $OCF_2CHClF$ | CH | |
| 0 | $SO_2$ | S | H | H | $CH_3$ | H | $OCH_3$ | $OCF_2CHBrF$ | CH | |
| 0 | $SO_2$ | S | H | H | $CH_3$ | H | $OCH_3$ | $OCF_2CHFCF_3$ | CH | |
| 0 | $SO_2$ | S | H | H | $CH_3$ | H | $OCH_3$ | $OCH_3$ | $CCH_3$ | |
| 0 | $SO_2$ | S | H | H | $CH_3$ | H | $OCH_3$ | $OCH_3$ | $CC_2H_5$ | |
| 0 | $SO_2$ | S | H | H | $CH_3$ | H | $OCH_3$ | $OCH_3$ | CCl | |
| 0 | $SO_2$ | S | H | H | $CH_3$ | H | $OCH_3$ | $OCH_3$ | CBr | |
| 0 | $SO_2$ | S | H | H | $CH_3$ | H | $OCH_3$ | $(CH_2)_3CH_3$ | CH | |
| 0 | $SO_2$ | S | H | H | $CH_3$ | H | $OCH_3$ | $O(CH_2)_3CH_3$ | CH | |
| 0 | $SO_2$ | S | H | H | $CH_3$ | H | $OCH_3$ | $O(CH_2)_3CF_3$ | CH | |
| 0 | $SO_2$ | S | H | H | $CH_3$ | H | $OCH_3$ | $(CH_2)_3CF_3$ | CH | |
| 0 | $SO_2$ | S | H | H | $CH_3$ | H | $OCH_3$ | $S(CH_2)_3CF_3$ | CH | |
| 0 | $SO_2$ | S | H | H | $CH_3$ | H | $OCH_3$ | $S(CH_2)_2CH_3$ | CH | |
| 0 | $SO_2$ | S | H | H | $CH_3$ | H | $OCH_3$ | I | CH | |
| 0 | $SO_2$ | S | H | H | $CH_3$ | H | $OCH_3$ | $CH_2O(CH_2)_3CH_3$ | CH | |
| 0 | $SO_2$ | S | H | H | $CH_3$ | H | $OCH_3$ | $NH(CH_2)_3CH_3$ | CH | |
| 0 | $SO_2$ | S | H | H | $CH_3$ | H | $OCH_3$ | $N(CH_2CH_2CH_3)_2$ | CH | |
| 0 | $SO_2$ | S | H | H | $CH_3$ | H | $OCH_3$ | $OCH_2CH{=}CHCH_3$ | CH | |
| 0 | $SO_2$ | S | H | H | $CH_3$ | H | $OCH_3$ | $OCH_2C{\equiv}CCH_3$ | CH | |
| 0 | $SO_2$ | S | H | H | $CH_3$ | H | $OCH_3$ | $CH_2S(CH_2)_3CH_3$ | CH | |
| 0 | $SO_2$ | S | H | H | $CH_3$ | H | $OCH_3$ | $(CH_2)_4Cl$ | CH | |
| 0 | $SO_2$ | S | H | H | $CH_3$ | H | $OCH_3$ | cyclo-pentyl | CH | |
| 0 | $SO_2$ | S | H | H | $CH_3$ | H | $OCH_3$ | CHO | CH | |
| 0 | $SO_2$ | S | H | H | $CH_3$ | H | $OCH_3$ | $C(O)CH_3$ | CH | |
| 0 | $SO_2$ | S | H | H | $CH_3$ | H | $OCH_3$ | $CH(SCH_3)_2$ | CH | |
| 0 | $SO_2$ | S | H | H | $CH_3$ | H | $OCH_3$ | 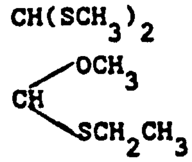 | CH | |

Table Ia (Continued)

| n | Q | G | R | $R_1$ | $R_2$ | $R_3$ | X | Y | Z | m.p. (°C) |
|---|---|---|---|---|---|---|---|---|---|---|
| O | $SO_2$ | S | H | H | $CH_3$ | H | $OCH_3$ | 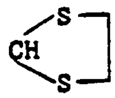 | CH | |
| O | $SO_2$ | S | H | H | $CH_3$ | H | $OCH_3$ | 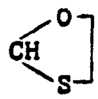 | CH | |
| O | $SO_2$ | S | H | H | $CH_3$ | H | $OCH_3$ | $N(OCH_3)CH_3$ | CH | |
| O | $SO_2$ | S | H | H | $CH_3$ | H | $CH_2CH_3$ | $OCH_3$ | CH | |
| O | $SO_2$ | S | H | H | $CH_3$ | H | $(CH_2)_3CH_3$ | $OCH_3$ | CH | |
| O | $SO_2$ | S | H | H | $CH_3$ | H | $O(CH_2)_3CH_3$ | $OCH_3$ | CH | |
| O | $SO_2$ | S | H | H | $CH_3$ | H | $O(CH_2)_3CCl_3$ | $OCH_3$ | CH | |
| O | $SO_2$ | S | H | H | $CH_3$ | H | $(CH_2)_4F$ | $OCH_3$ | CH | |
| O | $SO_2$ | S | H | H | $CH_3$ | H | $S(CH_2)_4Br$ | $OCH_3$ | CH | |
| O | $SO_2$ | S | H | H | $CH_3$ | H | $SC(CH_3)_3$ | $OCH_3$ | CH | |
| O | $SO_2$ | S | H | H | $CH_3$ | H | $(CH_2)_4OCH_3$ | $OCH_3$ | CH | |
| O | $SO_2$ | S | H | H | $CH_3$ | H | $O(CH_2)_4OCH_3$ | $OCH_3$ | CH | |
| O | $SO_2$ | S | H | H | $CH_3$ | H | $NH_2$ | $OCH_3$ | CH | |
| O | $SO_2$ | S | H | H | $CH_3$ | H | $NHCH(CH_3)_2$ | $OCH_3$ | CH | |
| O | $SO_2$ | S | H | H | $CH_3$ | H | $N[CH(CH_3)_2]_2$ | $OCH_3$ | CH | |

Table Ib

Structure Ib

| n | Q | R | $R_1'$ | $R_2$ | $R_3$ | X | Y | Z | m.p. (°C) |
|---|---|---|---|---|---|---|---|---|---|
| 0 | $SO_2$ | H | H | $CH_3$ | H | $CH_3$ | $CH_3$ | CH | |
| 0 | $SO_2$ | H | H | $CH_3$ | H | $CH_3$ | $OCH_3$ | CH | |
| 0 | $SO_2$ | H | H | $CH_3$ | H | $OCH_3$ | $OCH_3$ | CH | |
| 0 | $SO_2$ | H | H | $CH_3$ | H | Cl | $OCH_3$ | CH | |
| 0 | $SO_2$ | H | H | $CH_3$ | H | Br | $OCH_3$ | CH | |
| 0 | $SO_2$ | H | H | $CH_3$ | H | $CH_3$ | $OCH_3$ | N | |
| 0 | $SO_2$ | H | H | $CH_3$ | H | $OCH_3$ | $OCH_3$ | N | |
| 0 | S | H | H | $CH_3$ | H | $CH_3$ | $CH_3$ | CH | |
| 0 | S | H | H | $CH_3$ | H | $CH_3$ | $OCH_3$ | CH | |
| 0 | S | H | H | $CH_3$ | H | $OCH_3$ | $OCH_3$ | CH | |
| 0 | S | H | H | $CH_3$ | H | Cl | $OCH_3$ | CH | |
| 0 | S | H | H | $CH_3$ | H | Br | $OCH_3$ | CH | |
| 0 | S | H | H | $CH_3$ | H | $CH_3$ | $OCH_3$ | N | |
| 0 | S | H | H | $CH_3$ | H | $OCH_3$ | $OCH_3$ | N | |
| 0 | $SO_2$ | H | H | H | H | $CH_3$ | $OCH_3$ | N | |
| 0 | $SO_2$ | H | H | H | H | $CH_3$ | $OCH_3$ | CH | |
| 0 | $SO_2$ | H | H | H | H | $OCH_3$ | $OCH_3$ | CH | |
| 0 | $SO_2$ | H | H | $CH_3$ | $CH_3$ | $CH_3$ | $OCH_3$ | N | |
| 0 | $SO_2$ | H | H | $CH_3$ | $CH_3$ | $CH_3$ | $OCH_3$ | CH | |
| 0 | $SO_2$ | H | H | $CH_3$ | $CH_3$ | $OCH_3$ | $OCH_3$ | CH | |
| 0 | S | H | H | H | H | $CH_3$ | $OCH_3$ | N | |
| 0 | S | H | H | H | H | $CH_3$ | $OCH_3$ | CH | |
| 0 | S | H | H | H | H | $OCH_3$ | $OCH_3$ | CH | |
| 0 | S | H | H | $CH_3$ | $CH_3$ | $CH_3$ | $OCH_3$ | N | |
| 0 | S | H | H | $CH_3$ | $CH_3$ | $CH_3$ | $OCH_3$ | CH | |
| 0 | S | H | H | $CH_3$ | $CH_3$ | $OCH_3$ | $OCH_3$ | CH | |
| 1 | $SO_2$ | H | H | $CH_3$ | H | $CH_3$ | $OCH_3$ | N | |
| 1 | $SO_2$ | H | H | $CH_3$ | H | $CH_3$ | $OCH_3$ | CH | |
| 1 | $SO_2$ | H | H | $CH_3$ | H | $OCH_3$ | $OCH_3$ | CH | |
| 0 | O | H | H | $CH_3$ | H | $CH_3$ | $OCH_3$ | N | |

Table Ib (Continued)

| n | Q | R | $R_1'$ | $R_2$ | $R_3$ | X | Y | Z | m.p. (°C) |
|---|---|---|---|---|---|---|---|---|---|
| 0 | O | H | H | $CH_3$ | H | $CH_3$ | $OCH_3$ | CH | |
| 0 | O | H | H | $CH_3$ | H | $OCH_3$ | $OCH_3$ | CH | |
| 0 | $CH_2$ | H | H | H | H | $CH_3$ | $OCH_3$ | N | |
| 0 | $CH_2$ | H | H | H | H | $CH_3$ | $OCH_3$ | CH | |
| 0 | $CH_2$ | H | H | H | H | $OCH_3$ | $OCH_3$ | CH | |
| 1 | $CH_2$ | H | H | H | H | $CH_3$ | $OCH_3$ | N | |
| 1 | $CH_2$ | H | H | H | H | $CH_3$ | $OCH_3$ | CH | |
| 1 | $CH_2$ | H | H | H | H | $OCH_3$ | $OCH_3$ | CH | |
| 0 | $SO_2$ | H | Cl | $CH_3$ | H | $CH_3$ | $OCH_3$ | N | |
| 0 | $SO_2$ | H | Cl | $CH_3$ | H | $CH_3$ | $OCH_3$ | CH | |
| 0 | $SO_2$ | H | Cl | $CH_3$ | H | $OCH_3$ | $OCH_3$ | CH | |
| 0 | $SO_2$ | H | $CH_3$ | $CH_3$ | H | $CH_3$ | $OCH_3$ | N | |
| 0 | $SO_2$ | H | $CH_3$ | $CH_3$ | H | $CH_3$ | $OCH_3$ | CH | |
| 0 | $SO_2$ | H | $CH_3$ | $CH_3$ | H | $OCH_3$ | $OCH_3$ | CH | |

## Table Ic

### Structure Ic

| n | G | R | $R_1$ | $R_2$ | $R_3$ | X | Y | Z | m.p. (°C) |
|---|---|---|---|---|---|---|---|---|---|
| 1 | S | H | $CH_3$ | H | H | $CH_3$ | $CH_3$ | CH | |
| 1 | S | H | $CH_3$ | H | H | $CH_3$ | $OCH_3$ | CH | |
| 1 | S | H | $CH_3$ | H | H | $OCH_3$ | $OCH_3$ | CH | |
| 1 | S | H | $CH_3$ | H | H | Cl | $OCH_3$ | CH | |
| 1 | S | H | $CH_3$ | H | H | Br | $OCH_3$ | CH | |
| 1 | S | H | $CH_3$ | H | H | $CH_3$ | $OCH_3$ | N | |
| 1 | S | H | $CH_3$ | H | H | $OCH_3$ | $OCH_3$ | N | |
| 1 | O | H | $CH_3$ | H | H | $CH_3$ | $CH_3$ | CH | |
| 1 | O | H | $CH_3$ | H | H | $CH_3$ | $OCH_3$ | CH | |
| 1 | O | H | $CH_3$ | H | H | $OCH_3$ | $OCH_3$ | CH | |
| 1 | O | H | $CH_3$ | H | H | Cl | $OCH_3$ | CH | |
| 1 | O | H | $CH_3$ | H | H | Br | $OCH_3$ | CH | |
| 1 | O | H | $CH_3$ | H | H | $CH_3$ | $OCH_3$ | N | |
| 1 | O | H | $CH_3$ | H | H | $OCH_3$ | $OCH_3$ | N | |
| 1 | NH | H | $CH_3$ | H | H | $CH_3$ | $CH_3$ | CH | |
| 1 | NH | H | $CH_3$ | H | H | $CH_3$ | $OCH_3$ | CH | |
| 1 | NH | H | $CH_3$ | H | H | $OCH_3$ | $OCH_3$ | CH | |
| 1 | NH | H | $CH_3$ | H | H | Cl | $OCH_3$ | CH | |
| 1 | NH | H | $CH_3$ | H | H | Br | $OCH_3$ | CH | |
| 1 | NH | H | $CH_3$ | H | H | $CH_3$ | $OCH_3$ | N | |
| 1 | NH | H | $CH_3$ | H | H | $OCH_3$ | $OCH_3$ | N | |
| 1 | $NCH_3$ | H | $CH_3$ | H | H | $CH_3$ | $CH_3$ | CH | |
| 1 | $NCH_3$ | H | $CH_3$ | H | H | $CH_3$ | $OCH_3$ | CH | |
| 1 | $NCH_3$ | H | $CH_3$ | H | H | Cl | $OCH_3$ | CH | |
| 1 | $NCH_3$ | H | $CH_3$ | H | H | Br | $OCH_3$ | CH | |
| 1 | $NCH_3$ | H | $CH_3$ | H | H | $CH_3$ | $OCH_3$ | N | |
| 1 | $NCH_3$ | H | $CH_3$ | H | H | $OCH_3$ | $OCH_3$ | N | |
| 0 | S | H | $CH_3$ | H | H | $CH_3$ | $CH_3$ | CH | |
| 0 | S | H | $CH_3$ | H | H | $CH_3$ | $OCH_3$ | CH | |
| 0 | S | H | $CH_3$ | H | H | $OCH_3$ | $OCH_3$ | CH | |

Table Ic (Continued)

| n | G | R | $R_1$ | $R_2$ | $R_3$ | X | Y | Z | m.p. (°C) |
|---|---|---|---|---|---|---|---|---|---|
| 0 | S | H | $CH_3$ | H | H | Cl | $OCH_3$ | CH | |
| 0 | S | H | $CH_3$ | H | H | Br | $OCH_3$ | CH | |
| 0 | S | H | $CH_3$ | H | H | $CH_3$ | $OCH_3$ | N | |
| 0 | S | H | $CH_3$ | H | H | $OCH_3$ | $OCH_3$ | N | |
| 1 | S | H | H | H | H | $CH_3$ | $CH_3$ | CH | |
| 1 | S | H | H | H | H | $CH_3$ | $OCH_3$ | CH | |
| 1 | S | H | H | H | H | $OCH_3$ | $OCH_3$ | CH | |
| 1 | S | H | H | H | H | Cl | $OCH_3$ | CH | |
| 1 | S | H | H | H | H | Br | $OCH_3$ | CH | |
| 1 | S | H | H | H | H | $CH_3$ | $OCH_3$ | N | |
| 1 | S | H | H | H | H | $OCH_3$ | $OCH_3$ | N | |
| 1 | S | H | Cl | H | H | $CH_3$ | $CH_3$ | CH | |
| 1 | S | H | Cl | H | H | $CH_3$ | $OCH_3$ | CH | |
| 1 | S | H | Cl | H | H | $OCH_3$ | $OCH_3$ | CH | |
| 1 | S | H | Cl | H | H | Cl | $OCH_3$ | CH | |
| 1 | S | H | Cl | H | H | Br | $OCH_3$ | CH | |
| 1 | S | H | Cl | H | H | $CH_3$ | $OCH_3$ | N | |
| 1 | S | H | Cl | H | H | $OCH_3$ | $OCH_3$ | N | |
| 1 | S | H | $CH_3$ | $CH_3$ | H | $OCH_3$ | $OCH_3$ | CH | |
| 1 | S | H | $CH_3$ | $CH_3$ | $CH_3$ | $OCH_3$ | $OCH_3$ | CH | |
| 1 | S | H | $CH_3$ | Cl | Cl | $OCH_3$ | $OCH_3$ | CH | |

Table Id

Structure Id

| n | R | $R_1'$ | $R_2$ | $R_3$ | X | Y | Z | m.p. (°C) |
|---|---|---|---|---|---|---|---|---|
| 1 | H | H | H | H | $CH_3$ | $CH_3$ | CH | |
| 1 | H | H | H | H | $CH_3$ | $OCH_3$ | CH | |
| 1 | H | H | H | H | $OCH_3$ | $OCH_3$ | CH | |
| 1 | H | H | H | H | Cl | $OCH_3$ | CH | |
| 1 | H | H | H | H | Br | $OCH_3$ | CH | |
| 1 | H | H | H | H | $CH_3$ | $OCH_3$ | N | |
| 1 | H | H | H | H | $OCH_3$ | $OCH_3$ | N | |
| 1 | H | H | $CH_3$ | H | $OCH_3$ | $OCH_3$ | CH | |
| 1 | H | H | $CH_3$ | $CH_3$ | $OCH_3$ | $OCH_3$ | CH | |
| 1 | H | H | Cl | Cl | $OCH_3$ | $OCH_3$ | CH | |
| 1 | H | $CH_3$ | H | H | $OCH_3$ | $OCH_3$ | CH | |
| 1 | H | Cl | H | H | $OCH_3$ | $OCH_3$ | CH | |
| 0 | H | H | H | H | $CH_3$ | $CH_3$ | CH | |
| 0 | H | H | H | H | $CH_3$ | $OCH_3$ | CH | |
| 0 | H | H | H | H | $OCH_3$ | $OCH_3$ | CH | |
| 0 | H | H | H | H | Cl | $OCH_3$ | CH | |
| 0 | H | H | H | H | Br | $OCH_3$ | CH | |
| 0 | H | H | H | H | $CH_3$ | $OCH_3$ | N | |
| 0 | H | H | H | H | $OCH_3$ | $OCH_3$ | N | |

Table Ie

Structure Ie

| n | G | R | $R_1$ | $R_4$ | $R_5$ | X | Y | Z | m.p. (°C) |
|---|---|---|---|---|---|---|---|---|---|
| 1 | S | H | $CH_3$ | H | H | $CH_3$ | $CH_3$ | CH | |
| 1 | S | H | $CH_3$ | H | H | $CH_3$ | $OCH_3$ | CH | |
| 1 | S | H | $CH_3$ | H | H | $OCH_3$ | $OCH_3$ | CH | |
| 1 | S | H | $CH_3$ | H | H | Cl | $OCH_3$ | CH | |
| 1 | S | H | $CH_3$ | H | H | Br | $OCH_3$ | CH | |
| 1 | S | H | $CH_3$ | H | H | $CH_3$ | $OCH_3$ | N | |
| 1 | S | H | $CH_3$ | H | H | $OCH_3$ | $OCH_3$ | N | |
| 1 | O | H | $CH_3$ | H | H | $CH_3$ | $CH_3$ | CH | |
| 1 | O | H | $CH_3$ | H | H | $CH_3$ | $OCH_3$ | CH | |
| 1 | O | H | $CH_3$ | H | H | $OCH_3$ | $OCH_3$ | CH | |
| 1 | O | H | $CH_3$ | H | H | Cl | $OCH_3$ | CH | |
| 1 | O | H | $CH_3$ | H | H | Br | $OCH_3$ | CH | |
| 1 | O | H | $CH_3$ | H | H | $CH_3$ | $OCH_3$ | N | |
| 1 | O | H | $CH_3$ | H | H | $OCH_3$ | $OCH_3$ | N | |
| 1 | NH | H | $CH_3$ | H | H | $CH_3$ | $CH_3$ | CH | |
| 1 | NH | H | $CH_3$ | H | H | $CH_3$ | $OCH_3$ | CH | |
| 1 | NH | H | $CH_3$ | H | H | $OCH_3$ | $OCH_3$ | CH | |
| 1 | NH | H | $CH_3$ | H | H | Cl | $OCH_3$ | CH | |
| 1 | NH | H | $CH_3$ | H | H | Br | $OCH_3$ | CH | |
| 1 | NH | H | $CH_3$ | H | H | $CH_3$ | $OCH_3$ | N | |
| 1 | NH | H | $CH_3$ | H | H | $OCH_3$ | $OCH_3$ | N | |
| 1 | $NCH_3$ | H | $CH_3$ | H | H | $CH_3$ | $CH_3$ | CH | |
| 1 | $NCH_3$ | H | $CH_3$ | H | H | $CH_3$ | $OCH_3$ | CH | |
| 1 | $NCH_3$ | H | $CH_3$ | H | H | $OCH_3$ | $OCH_3$ | CH | |
| 1 | $NCH_3$ | H | $CH_3$ | H | H | Cl | $OCH_3$ | CH | |
| 1 | $NCH_3$ | H | $CH_3$ | H | H | Br | $OCH_3$ | CH | |
| 1 | $NCH_3$ | H | $CH_3$ | H | H | $CH_3$ | $OCH_3$ | N | |
| 1 | $NCH_3$ | H | $CH_3$ | H | H | $OCH_3$ | $OCH_3$ | N | |
| 1 | S | H | H | H | H | $OCH_3$ | $OCH_3$ | CH | |
| 1 | S | H | Cl | H | H | $OCH_3$ | $OCH_3$ | CH | |
| 1 | S | H | H | $CH_3$ | H | $OCH_3$ | $OCH_3$ | CH | |

Table Ie (Continued)

| n | G | R | $R_1$ | $R_4$ | $R_5$ | X | Y | Z | m.p. (°C) |
|---|---|---|---|---|---|---|---|---|---|
| 1 | S | H | H | CH | $CH_3$ | $OCH_3$ | $OCH_3$ | CH 3 | |
| 1 | S | H | H | $CH_2CH_3$ | H | $OCH_3$ | $OCH_3$ | CH | |
| 1 | S | H | H | $(CH_2)_3CH_3$ | H | $OCH_3$ | $OCH_3$ | CH | |
| O | S | H | H | $CH_3$ | H | $CH_3$ | $CH_3$ | CH | |
| O | S | H | H | $CH_3$ | H | $CH_3$ | $OCH_3$ | CH | |
| O | S | H | H | $CH_3$ | H | $OCH_3$ | $OCH_3$ | CH | |
| O | S | H | H | $CH_3$ | H | Cl | $OCH_3$ | CH | |
| O | S | H | H | $CH_3$ | H | Br | $OCH_3$ | CH | |
| O | S | H | H | $CH_3$ | H | $CH_3$ | $OCH_3$ | N | |
| O | S | H | H | $CH_3$ | H | $OCH_3$ | $OCH_3$ | N | |

Table If

Structure If

| n | R | $R_1'$ | $R_4$ | $R_5$ | X | Y | Z | m.p. (°C) |
|---|---|---|---|---|---|---|---|---|
| 1 | H | H | H | H | $CH_3$ | $CH_3$ | CH | |
| 1 | H | H | H | H | $CH_3$ | $OCH_3$ | CH | |
| 1 | H | H | H | H | $OCH_3$ | $OCH_3$ | CH | |
| 1 | H | H | H | H | Cl | $OCH_3$ | CH | |
| 1 | H | H | H | H | Br | $OCH_3$ | CH | |
| 1 | H | H | H | H | $CH_3$ | $OCH_3$ | N | |
| 1 | H | H | H | H | $OCH_3$ | $OCH_3$ | N | |
| 0 | H | H | H | H | $CH_3$ | $CH_3$ | CH | |
| 0 | H | H | H | H | $CH_3$ | $OCH_3$ | CH | |
| 0 | H | H | H | H | $OCH_3$ | $OCH_3$ | CH | |
| 0 | H | H | H | H | Cl | $OCH_3$ | CH | |
| 0 | H | H | H | H | Br | $OCH_3$ | CH | |
| 0 | H | H | H | H | $CH_3$ | $OCH_3$ | N | |
| 0 | H | H | H | H | $OCH_3$ | $OCH_3$ | N | |
| 1 | H | $CH_3$ | H | H | $OCH_3$ | $OCH_3$ | CH | |
| 0 | H | $CH_3$ | H | H | $OCH_3$ | $OCH_3$ | CH | |
| 0 | H | Cl | H | H | $OCH_3$ | $OCH_3$ | CH | |
| 1 | H | Cl | H | H | $CH_3$ | $CH_3$ | CH | |
| 1 | H | Cl | H | H | $CH_3$ | $OCH_3$ | CH | |
| 1 | H | Cl | H | H | $OCH_3$ | $OCH_3$ | CH | |
| 1 | H | Cl | H | H | Cl | $OCH_3$ | CH | |
| 1 | H | Cl | H | H | Br | $OCH_3$ | CH | |
| 1 | H | Cl | H | H | $CH_3$ | $OCH_3$ | N | |
| 1 | H | Cl | H | H | $OCH_3$ | $OCH_3$ | N | |

Table Ig

Structure Ig

| n | G | R | $R_1$ | $R_5$ | $R_6$ | X | Y | Z | m.p. (°C) |
|---|---|---|---|---|---|---|---|---|---|
| 0 | S | H | H | H | H | $CH_3$ | $CH_3$ | CH | |
| 0 | S | H | H | H | H | $CH_3$ | $OCH_3$ | CH | |
| 0 | S | H | H | H | H | $OCH_3$ | $OCH_3$ | CH | |
| 0 | S | H | H | H | H | Cl | $OCH_3$ | CH | |
| 0 | S | H | H | H | H | Br | $OCH_3$ | CH | |
| 0 | S | H | H | H | H | $CH_3$ | $OCH_3$ | N | |
| 0 | S | H | H | H | H | $OCH_3$ | $OCH_3$ | N | |
| 0 | S | H | H | H | $CH_3$ | $CH_3$ | $CH_3$ | CH | |
| 0 | S | H | H | H | $CH_3$ | $CH_3$ | $OCH_3$ | CH | |
| 0 | S | H | H | H | $CH_3$ | $OCH_3$ | $OCH_3$ | CH | |
| 0 | S | H | H | H | $CH_3$ | Cl | $OCH_3$ | CH | |
| 0 | S | H | H | H | $CH_3$ | Br | $OCH_3$ | CH | |
| 0 | S | H | H | H | $CH_3$ | $CH_3$ | $OCH_3$ | N | |
| 0 | S | H | H | H | $CH_3$ | $OCH_3$ | $OCH_3$ | N | |
| 0 | S | H | H | H | $CH_2CH_3$ | $CH_3$ | $CH_3$ | N | |
| 0 | S | H | H | H | $CH_2CH_3$ | $CH_3$ | $OCH_3$ | CH | |
| 0 | S | H | H | H | $CH_2CH_3$ | $OCH_3$ | $OCH_3$ | CH | |
| 0 | S | H | H | H | $CH_2CH_2CH_3$ | $CH_3$ | $OCH_3$ | N | |
| 0 | S | H | H | H | $CH_2CH_2CH_3$ | $CH_3$ | $OCH_3$ | CH | |
| 0 | S | H | H | H | $CH_2CH_2CH_3$ | $OCH_3$ | $OCH_3$ | CH | |
| 0 | S | H | H | H | $CH(CH_3)_2$ | $CH_3$ | $OCH_3$ | N | |
| 0 | S | H | H | H | $CH(CH_3)_2$ | $CH_3$ | $OCH_3$ | CH | |
| 0 | S | H | H | H | $CH(CH_3)_2$ | $OCH_3$ | $OCH_3$ | CH | |
| 0 | S | H | H | H | $CH_2CH_2CH_2CH_3$ | $CH_3$ | $CH_3$ | CH | |
| 0 | S | H | H | H | $CH_2CH_2CH_2CH_3$ | $CH_3$ | $OCH_3$ | CH | |
| 0 | S | H | H | H | $CH_2CH_2CH_2CH_3$ | $OCH_3$ | $OCH_3$ | CH | |
| 0 | S | H | H | H | $CH_2CH_2CH_2CH_3$ | Cl | $OCH_3$ | CH | |
| 0 | S | H | H | H | $CH_2CH_2CH_2CH_3$ | Br | $OCH_3$ | CH | |
| 0 | S | H | H | H | $CH_2CH_2CH_2CH_3$ | $CH_3$ | $OCH_3$ | N | |
| 0 | S | H | H | H | $CH_2CH_2CH_2CH_3$ | $OCH_3$ | $OCH_3$ | N | |

Table Ig (Continued)

| n | G | R | $R_1$ | $R_5$ | $R_6$ | X | Y | Z | m.p. (°C) |
|---|---|---|---|---|---|---|---|---|---|
| 0 | S | H | H | H | $CH_2CH(CH_3)_2$ | $CH_3$ | $OCH_3$ | N | |
| 0 | S | H | H | H | $CH_2CH(CH_3)_2$ | $CH_3$ | $OCH_3$ | CH | |
| 0 | S | H | H | H | $CH_2CH(CH_3)_2$ | $OCH_3$ | $OCH_3$ | CH | |
| 0 | S | H | H | H | $CH(CH_3)CH_2CH_3$ | $CH_3$ | $OCH_3$ | N | |
| 0 | S | H | H | H | $CH(CH_3)CH_2CH_3$ | $CH_3$ | $OCH_3$ | CH | |
| 0 | S | H | H | H | $CH(CH_3)CH_2CH_3$ | $OCH_3$ | $OCH_3$ | CH | |
| 0 | S | H | H | H | $C(CH_3)_3$ | $CH_3$ | $OCH_3$ | N | |
| 0 | S | H | H | H | $C(CH_3)_3$ | $CH_3$ | $OCH_3$ | CH | |
| 0 | S | H | H | H | $C(CH_3)_3$ | $OCH_3$ | $OCH_3$ | CH | |
| 0 | S | H | H | H | $(CH_2)_5CH_3$ | $CH_3$ | $OCH_3$ | N | |
| 0 | S | H | H | H | $(CH_2)_5CH_3$ | $CH_3$ | $OCH_3$ | CH | |
| 0 | S | H | H | H | $(CH_2)_5CH_3$ | $OCH_3$ | $OCH_3$ | CH | |
| 0 | S | H | H | H | $CH_2CH=CH_2$ | $CH_3$ | $OCH_3$ | N | |
| 0 | S | H | H | H | $CH_2CH=CH_2$ | $CH_3$ | $OCH_3$ | CH | |
| 0 | S | H | H | H | $CH_2CH=CH_2$ | $OCH_3$ | $OCH_3$ | CH | |
| 0 | S | H | H | H | $CH_2CH=CH_2CH_3$ | $CH_3$ | $OCH_3$ | N | |
| 0 | S | H | H | H | $CH_2CH=CH_2CH_3$ | $CH_3$ | $OCH_3$ | CH | |
| 0 | S | H | H | H | $CH_2CH=CH_2CH_3$ | $OCH_3$ | $OCH_3$ | CH | |
| 0 | S | H | H | H | $CH_2C(CH_3)=C(CH_3)_2$ | $CH_3$ | $OCH_3$ | N | |
| 0 | S | H | H | H | $CH_2C(CH_3)=C(CH_3)_2$ | $CH_3$ | $OCH_3$ | CH | |
| 0 | S | H | H | H | $CH_2C(CH_3)=C(CH_3)_2$ | $OCH_3$ | $OCH_3$ | CH | |
| 0 | S | H | H | H | 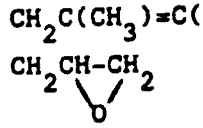 | $OCH_3$ | $OCH_3$ | CH | |
| 0 | S | H | H | H | $CH_2C\equiv CH$ | $CH_3$ | $OCH_3$ | N | |
| 0 | S | H | H | H | $CH_2C\equiv CH$ | $CH_3$ | $OCH_3$ | CH | |
| 0 | S | H | H | H | $CH_2C\equiv CH$ | $OCH_3$ | $OCH_3$ | CH | |
| 0 | S | H | H | H | $CH_2C\equiv CCH_3$ | $CH_3$ | $OCH_3$ | N | |
| 0 | S | H | H | H | $CH_2C\equiv CCH_3$ | $CH_3$ | $OCH_3$ | CH | |
| 0 | S | H | H | H | $CH_2C\equiv CCH_3$ | $OCH_3$ | $OCH_3$ | CH | |
| 0 | S | H | H | H | $(CH_2)_4C\equiv CH$ | $OCH_3$ | $OCH_3$ | CH | |
| 0 | S | H | H | H | cyclopropyl | $OCH_3$ | $OCH_3$ | CH | |

Table Ig (Continued)

| n | G | R | $R_1$ | $R_5$ | $R_6$ | X | Y | Z | m.p. (°C) |
|---|---|---|---|---|---|---|---|---|---|
| 0 | S | H | H | H | cyclopentyl | $OCH_3$ | $OCH_3$ | CH | |
| 0 | S | H | H | H | cyclohexyl | $OCH_3$ | $OCH_3$ | CH | |
| 0 | S | H | H | H | $CH_2$-cyclo-propyl | $OCH_3$ | $OCH_3$ | CH | |
| 0 | S | H | H | H | $CH_2$-cyclo-pentyl | $OCH_3$ | $OCH_3$ | CH | |
| 0 | S | H | H | H | phenyl | $OCH_3$ | $OCH_3$ | CH | |
| 0 | S | H | H | H | p-nitrophenyl | $OCH_3$ | $OCH_3$ | CH | |
| 0 | S | H | H | H | phenylthio | $OCH_3$ | $OCH_3$ | CH | |
| 0 | S | H | H | H | $SO_2CH_3$ | $OCH_3$ | $OCH_3$ | CH | |
| 0 | S | H | H | H | $OCH_3$ | $OCH_3$ | $OCH_3$ | CH | |
| 0 | S | H | H | H | $C(O)CH_3$ | $OCH_3$ | $OCH_3$ | CH | |
| 0 | S | H | H | H | $C(O)CH_2CH_3$ | $OCH_3$ | $OCH_3$ | CH | |
| 0 | S | H | H | H | $C(O)(CH_2)_5CH_3$ | $OCH_3$ | $OCH_3$ | CH | |
| 0 | S | H | H | H | $CO_2CH_3$ | $OCH_3$ | $OCH_3$ | CH | |
| 0 | S | H | H | H | $CO_2CH_2CH_3$ | $OCH_3$ | $OCH_3$ | CH | |
| 0 | S | H | H | H | $CO_2CH_2CH{=}CH_2$ | $OCH_3$ | $OCH_3$ | CH | |
| 0 | S | H | H | H | $N(CH_3)_2$ | $OCH_3$ | $OCH_3$ | CH | |
| 0 | S | H | H | H | CN | $OCH_3$ | $OCH_3$ | CH | |
| 0 | S | H | H | H | $Si(CH_3)_3$ | $OCH_3$ | $OCH_3$ | CH | |
| 0 | S | H | H | H | $Si(CH_3)_2C(CH_3)_3$ | $OCH_3$ | $OCH_3$ | CH | |
| 0 | S | H | H | H | $Si(CH_3)_2Ph$ | $OCH_3$ | $OCH_3$ | CH | |
| 0 | S | H | H | H | $Si(CH_3)_2CH_2Ph$ | $OCH_3$ | $OCH_3$ | CH | |
| 0 | S | H | H | H | $CH_2Cl$ | $OCH_3$ | $OCH_3$ | CH | |
| 0 | S | H | H | H | $CH_2CH_2Cl$ | $OCH_3$ | $OCH_3$ | CH | |
| 0 | S | H | H | H | $CH_2CH_2CH_2F$ | $OCH_3$ | $OCH_3$ | CH | |
| 0 | S | H | H | H | $CF_2H$ | $OCH_3$ | $OCH_3$ | CH | |
| 0 | S | H | H | H | $CH_2CH_2Br$ | $OCH_3$ | $OCH_3$ | CH | |
| 0 | S | H | H | H | $CH_2CF_3$ | $OCH_3$ | $OCH_3$ | CH | |
| 0 | S | H | H | H | $CH_2C(Cl){=}CH_2$ | $OCH_3$ | $OCH_3$ | CH | |
| 0 | S | H | H | H | $CH_2CH_2OH$ | $OCH_3$ | $OCH_3$ | CH | |

Table Ig (Continued)

| n | G | R | $R_1$ | $R_5$ | $R_6$ | X | Y | Z | m.p. (°C) |
|---|---|---|---|---|---|---|---|---|---|
| 0 | S | H | H | H | $CH_2CH_2OCH_3$ | $OCH_3$ | $OCH_3$ | CH | |
| 0 | S | H | H | H | $CH_2CH_2OCO_2(CH_2)_5CH_3$ | $OCH_3$ | $OCH_3$ | CH | |
| 0 | S | H | H | H | $CH_2CH_2OC(O)NHCH_3$ | $OCH_3$ | $OCH_3$ | CH | |
| 0 | S | H | H | H | $CH_2CH_2OSO_2Ph$ | $OCH_3$ | $OCH_3$ | CH | |
| 0 | S | H | H | H | $CH_2CH_2OSi(CH_3)_3$ | $OCH_3$ | $OCH_3$ | CH | |
| 0 | S | H | H | H | $CH_2Si(CH_3)_3$ | $OCH_3$ | $OCH_3$ | CH | |
| 0 | S | H | H | H | $CH_2SCH_2CH_3$ | $OCH_3$ | $OCH_3$ | CH | |
| 0 | S | H | H | H | $CH_2CH_2SOCH_3$ | $OCH_3$ | $OCH_3$ | CH | |
| 0 | S | H | H | H | $CH_2CH_2SO_2CH_3$ | $OCH_3$ | $OCH_3$ | CH | |
| 0 | S | H | H | H | $CH_2SCN$ | $OCH_3$ | $OCH_3$ | CH | |
| 0 | S | H | H | H | $CH_2CN$ | $OCH_3$ | $OCH_3$ | CH | |
| 0 | S | H | H | H | $CH_2CH_2CN$ | $OCH_3$ | $OCH_3$ | CH | |
| 0 | S | H | H | H | $CH_2NO_2$ | $OCH_3$ | $OCH_3$ | CH | |
| 0 | S | H | H | H | $CH_2C(O)CH_3$ | $OCH_3$ | $OCH_3$ | CH | |
| 0 | S | H | H | H | $CH_2CH_2C(O)CH_3$ | $OCH_3$ | $OCH_3$ | CH | |
| 0 | S | H | H | H | $CH_2CO_2CH_3$ | $OCH_3$ | $OCH_3$ | CH | |
| 0 | S | H | H | H | $CH_2CO_2C(CH_3)_3$ | $OCH_3$ | $OCH_3$ | CH | |
| 0 | S | H | H | H | $CH_2C(O)N(CH_3)_2$ | $OCH_3$ | $OCH_3$ | CH | |
| 0 | S | H | H | H | benzyl | $OCH_3$ | $OCH_3$ | CH | |
| 0 | S | H | H | H | p-chlorobenzyl | $OCH_3$ | $OCH_3$ | CH | |
| 0 | S | H | H | H | m-fluorobenzyl | $OCH_3$ | $OCH_3$ | CH | |
| 0 | S | H | H | H | o-bromobenzyl | $OCH_3$ | $OCH_3$ | CH | |
| 0 | S | H | H | H | p-methylbenzyl | $OCH_3$ | $OCH_3$ | CH | |
| 0 | S | H | H | H | p-methoxybenzyl | $OCH_3$ | $OCH_3$ | CH | |
| 0 | S | H | H | H | p-cyanobenzyl | $OCH_3$ | $OCH_3$ | CH | |
| 0 | S | H | H | H | p-nitrobenzyl | $OCH_3$ | $OCH_3$ | CH | |
| 0 | S | H | H | H | p-methylthiobenzyl | $OCH_3$ | $OCH_3$ | CH | |
| 0 | S | H | H | H | p-methyl-sulfonylbenzyl | $OCH_3$ | $OCH_3$ | CH | |
| 0 | S | H | H | H | m-trifluro-methylbenzyl | $OCH_3$ | $OCH_3$ | CH | |
| 0 | S | H | H | H | $CH_2CO_2$phenyl | $OCH_3$ | $OCH_3$ | CH | |

Table Ig (Continued)

| n | G | R | $R_1$ | $R_5$ | $R_6$ | X | Y | Z | m.p. (°C) |
|---|---|---|---|---|---|---|---|---|---|
| 0 | S | H | H | H | $CH_2CO_2$-cyclopentyl | $OCH_3$ | $OCH_3$ | CH | |
| 0 | S | H | H | H | $CH_2CO_2CH_2CH_2Cl$ | $OCH_3$ | $OCH_3$ | CH | |
| 0 | S | H | H | H | $CH_2CO_2CH_2C{\equiv}CH$ | $OCH_3$ | $OCH_3$ | CH | |
| 0 | S | H | H | H | $CH_2CH(OCH_3)_2$ | $OCH_3$ | $OCH_3$ | CH | |
| 0 | S | H | H | H | $CH_2CH(CN)SO_2CH_3$ | $OCH_3$ | $OCH_3$ | CH | |
| 0 | S | H | H | H | 2-pyridylmethyl | $OCH_3$ | $OCH_3$ | CH | |
| 0 | S | H | H | H | 3-pyridylmethyl | $OCH_3$ | $OCH_3$ | CH | |
| 0 | S | H | H | H | 4-pyridylmethyl | $OCH_3$ | $OCH_3$ | CH | |
| 0 | S | H | H | H | 2-thienomethyl | $OCH_3$ | $OCH_3$ | CH | |
| 0 | S | H | H | H | 3-thienomethyl | $OCH_3$ | $OCH_3$ | CH | |
| 0 | S | H | H | H | 2-furanylmethyl | $OCH_3$ | $OCH_3$ | CH | |
| 0 | S | H | H | $CH_3$ | $CH_3$ | $OCH_3$ | $OCH_3$ | CH | |
| 0 | S | $CH_3$ | H | H | $CH_3$ | $OCH_3$ | $OCH_3$ | CH | |
| 0 | S | H | $CH_3$ | H | $CH_3$ | $CH_3$ | $CH_3$ | CH | |
| 0 | S | H | $CH_3$ | H | $CH_3$ | $CH_3$ | $OCH_3$ | CH | |
| 0 | S | H | $CH_3$ | H | $CH_3$ | $OCH_3$ | $OCH_3$ | CH | |
| 0 | S | H | $CH_3$ | H | $CH_3$ | Cl | $OCH_3$ | CH | |
| 0 | S | H | $CH_3$ | H | $CH_3$ | Br | $OCH_3$ | CH | |
| 0 | S | H | $CH_3$ | H | $CH_3$ | $CH_3$ | $OCH_3$ | N | |
| 0 | S | H | $CH_3$ | H | $CH_3$ | $OCH_3$ | $OCH_3$ | N | |
| 0 | S | H | Cl | H | $CH_3$ | $CH_3$ | $CH_3$ | CH | |
| 0 | S | H | Cl | H | $CH_3$ | $CH_3$ | $OCH_3$ | CH | |
| 0 | S | H | Cl | H | $CH_3$ | $OCH_3$ | $OCH_3$ | CH | |
| 0 | S | H | Cl | H | $CH_3$ | Cl | $OCH_3$ | CH | |
| 0 | S | H | Cl | H | $CH_3$ | Br | $OCH_3$ | CH | |
| 0 | S | H | Cl | H | $CH_3$ | $CH_3$ | $OCH_3$ | N | |
| 0 | S | H | Cl | H | $CH_3$ | $OCH_3$ | $OCH_3$ | N | |
| 0 | S | H | $CH_3$ | H | $CH_2CH_2CH_2CH_3$ | $CH_3$ | $OCH_3$ | CH | |
| 0 | S | H | $CH_3$ | H | $CH_2CH_2CH_2CH_3$ | $OCH_3$ | $OCH_3$ | CH | |
| 0 | S | H | Cl | H | $CH_2CH_2CH_2CH_3$ | $CH_3$ | $OCH_3$ | CH | |
| 0 | S | H | Cl | H | $CH_2CH_2CH_2CH_3$ | $OCH_3$ | $OCH_3$ | CH | |

Table Ig (Continued)

| n | G | R | $R_1$ | $R_5$ | $R_6$ | X | Y | Z | m.p. (°C) |
|---|---|---|---|---|---|---|---|---|---|
| 1 | S | H | H | H | $CH_3$ | $CH_3$ | $OCH_3$ | N | |
| 1 | S | H | H | H | $CH_3$ | $CH_3$ | $OCH_3$ | CH | |
| 1 | S | H | H | H | $CH_3$ | $OCH_3$ | $OCH_3$ | CH | |
| 1 | S | H | H | H | H | $CH_3$ | $OCH_3$ | N | |
| 1 | S | H | H | H | H | $CH_3$ | $OCH_3$ | CH | |
| 1 | S | H | H | H | H | $OCH_3$ | $OCH_3$ | CH | |
| 0 | O | H | $CH_3$ | H | $CH_3$ | $CH_3$ | $OCH_3$ | N | |
| 0 | O | H | $CH_3$ | H | $CH_3$ | $CH_3$ | $OCH_3$ | CH | |
| 0 | O | H | $CH_3$ | H | $CH_3$ | $OCH_3$ | $OCH_3$ | CH | |
| 0 | NH | H | $CH_3$ | H | $CH_3$ | $CH_3$ | $OCH_3$ | N | |
| 0 | NH | H | $CH_3$ | H | $CH_3$ | $CH_3$ | $OCH_3$ | CH | |
| 0 | NH | H | $CH_3$ | H | $CH_3$ | $OCH_3$ | $OCH_3$ | CH | |
| 0 | $NCH_3$ | H | $CH_3$ | H | $CH_3$ | $CH_3$ | $OCH_3$ | N | |
| 0 | $NCH_3$ | H | $CH_3$ | H | $CH_3$ | $CH_3$ | $OCH_3$ | CH | |
| 0 | $NCH_3$ | H | $CH_3$ | H | $CH_3$ | $OCH_3$ | $OCH_3$ | CH | |
| 0 | O | H | $CH_3$ | H | $CH_2CH_2CH_2CH_3$ | $CH_3$ | $OCH_3$ | CH | |
| 0 | O | H | $CH_3$ | H | $CH_2CH_2CH_2CH_3$ | $OCH_3$ | $OCH_3$ | CH | |
| 0 | NH | H | $CH_3$ | H | $CH_2CH_2CH_2CH_3$ | $CH_3$ | $OCH_3$ | CH | |
| 0 | NH | H | $CH_3$ | H | $CH_2CH_2CH_2CH_3$ | $OCH_3$ | $OCH_3$ | CH | |
| 0 | $NCH_3$ | H | $CH_3$ | H | $CH_2CH_2CH_2CH_3$ | $CH_3$ | $OCH_3$ | CH | |
| 0 | $NCH_3$ | H | $CH_3$ | H | $CH_2CH_2CH_2CH_3$ | $OCH_3$ | $OCH_3$ | CH | |
| 0 | O | H | H | H | $CH_2CH_2CH_2CH_3$ | $CH_3$ | $OCH_3$ | CH | |
| 0 | O | H | H | H | $CH_2CH_2CH_2CH_3$ | $OCH_3$ | $OCH_3$ | CH | |
| 0 | NH | H | H | H | $CH_2CH_2CH_2CH_3$ | $CH_3$ | $OCH_3$ | CH | |
| 0 | NH | H | H | H | $CH_2CH_2CH_2CH_3$ | $OCH_3$ | $OCH_3$ | CH | |
| 0 | $NCH_3$ | H | H | H | $CH_2CH_2CH_2CH_3$ | $CH_3$ | $OCH_3$ | CH | |
| 0 | $NCH_3$ | H | H | H | $CH_2CH_2CH_2CH_3$ | $OCH_3$ | $OCH_3$ | CH | |
| 0 | S | H | H | H | 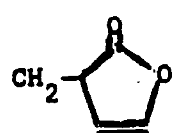 | $OCH_3$ | $OCH_3$ | CH | |
| 0 | S | H | H | H | 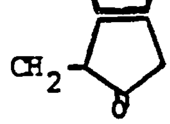 | $OCH_3$ | $OCH_3$ | CH | |

Table Ih

Structure Ih

| n | R | $R_1'$ | $R_5$ | $R_6$ | X | Y | Z | m.p. (°C) |
|---|---|---|---|---|---|---|---|---|
| 0 | H | H | H | H | $CH_3$ | $CH_3$ | CH | |
| 0 | H | H | H | H | $CH_3$ | $OCH_3$ | CH | |
| 0 | H | H | H | H | $OCH_3$ | $OCH_3$ | CH | |
| 0 | H | H | H | H | Cl | $OCH_3$ | CH | |
| 0 | H | H | H | H | Br | $OCH_3$ | CH | |
| 0 | H | H | H | H | $CH_3$ | $OCH_3$ | N | |
| 0 | H | H | H | H | $OCH_3$ | $OCH_3$ | N | |
| 0 | H | H | H | $CH_3$ | $CH_3$ | $CH_3$ | CH | |
| 0 | H | H | H | $CH_3$ | $CH_3$ | $OCH_3$ | CH | |
| 0 | H | H | H | $CH_3$ | $OCH_3$ | $OCH_3$ | CH | |
| 0 | H | H | H | $CH_3$ | Cl | $OCH_3$ | CH | |
| 0 | H | H | H | $CH_3$ | Br | $OCH_3$ | CH | |
| 0 | H | H | H | $CH_3$ | $CH_3$ | $OCH_3$ | N | |
| 0 | H | H | H | $CH_3$ | $OCH_3$ | $OCH_3$ | N | |
| 0 | H | H | H | $CH_2CH_2CH_2CH_3$ | $CH_3$ | $CH_3$ | CH | |
| 0 | H | H | H | $CH_2CH_2CH_2CH_3$ | $CH_3$ | $OCH_3$ | CH | |
| 0 | H | H | H | $CH_2CH_2CH_2CH_3$ | $OCH_3$ | $OCH_3$ | CH | |
| 0 | H | H | H | $CH_2CH_2CH_2CH_3$ | Cl | $OCH_3$ | CH | |
| 0 | H | H | H | $CH_2CH_2CH_2CH_3$ | Br | $OCH_3$ | CH | |
| 0 | H | H | H | $CH_2CH_2CH_2CH_3$ | $CH_3$ | $OCH_3$ | N | |
| 0 | H | H | H | $CH_2CH_2CH_2CH_3$ | $OCH_3$ | $OCH_3$ | N | |
| 0 | H | Cl | H | H | $CH_3$ | $CH_3$ | CH | |
| 0 | H | Cl | H | H | $CH_3$ | $OCH_3$ | CH | |
| 0 | H | Cl | H | H | $OCH_3$ | $OCH_3$ | CH | |
| 0 | H | Cl | H | H | Cl | $OCH_3$ | CH | |
| 0 | H | Cl | H | H | Br | $OCH_3$ | CH | |
| 0 | H | Cl | H | H | $CH_3$ | $OCH_3$ | N | |
| 0 | H | Cl | H | H | $OCH_3$ | $OCH_3$ | N | |
| 0 | H | Cl | H | $CH_3$ | $CH_3$ | $CH_3$ | CH | |
| 0 | H | Cl | H | $CH_3$ | $CH_3$ | $OCH_3$ | CH | |

Table Ih (Continued)

| n | R | $R_1$' | $R_5$ | $R_6$ | X | Y | Z | m.p. (°C) |
|---|---|---|---|---|---|---|---|---|
| 0 | H | Cl | H | $CH_3$ | $OCH_3$ | $OCH_3$ | CH | |
| 0 | H | Cl | H | $CH_3$ | Cl | $OCH_3$ | CH | |
| 0 | H | Cl | H | $CH_3$ | Br | $OCH_3$ | CH | |
| 0 | H | Cl | H | $CH_3$ | $CH_3$ | $OCH_3$ | N | |
| 0 | H | Cl | H | $CH_3$ | $OCH_3$ | $OCH_3$ | N | |
| 0 | H | Cl | H | $CH_2CH_2CH_2CH_3$ | $CH_3$ | $CH_3$ | CH | |
| 0 | H | Cl | H | $CH_2CH_2CH_2CH_3$ | $CH_3$ | $OCH_3$ | CH | |
| 0 | H | Cl | H | $CH_2CH_2CH_2CH_3$ | $OCH_3$ | $OCH_3$ | CH | |
| 0 | H | Cl | H | $CH_2CH_2CH_2CH_3$ | Cl | $OCH_3$ | CH | |
| 0 | H | Cl | H | $CH_2CH_2CH_2CH_3$ | Br | $OCH_3$ | CH | |
| 0 | H | Cl | H | $CH_2CH_2CH_2CH_3$ | $CH_3$ | $OCH_3$ | N | |
| 0 | H | Cl | H | $CH_2CH_2CH_2CH_3$ | $OCH_3$ | $OCH_3$ | N | |
| 1 | H | H | H | $CH_3$ | $OCH_3$ | $OCH_3$ | CH | |
| 0 | H | H | H | $C(O)CH_3$ | $OCH_3$ | $OCH_3$ | CH | |
| 0 | H | H | H | $CO_2CH_3$ | $OCH_3$ | $OCH_3$ | CH | |
| 0 | H | H | H | $CO_2Ph$ | $OCH_3$ | $OCH_3$ | CH | |
| 0 | H | H | H | $CH_2CH{=}CH_2$ | $OCH_3$ | $OCH_3$ | CH | |
| 0 | H | H | H | $CH_2CH{=}CHCH_3$ | $OCH_3$ | $OCH_3$ | CH | |
| 0 | H | H | H | $CH_2C{\equiv}CH$ | $OCH_3$ | $OCH_3$ | CH | |
| 0 | H | H | H | $CH_2C{\equiv}CCH_3$ | $OCH_3$ | $OCH_3$ | CH | |

Table Ii

Structure Ii

| W | R | $R_1$ | $R_7$ | X | Y | Z | m.p. (°C) |
|---|---|---|---|---|---|---|---|
| O | H | H | H | $CH_3$ | $CH_3$ | CH | |
| O | H | H | H | $CH_3$ | $OCH_3$ | CH | |
| O | H | H | H | $OCH_3$ | $OCH_3$ | CH | |
| O | H | H | H | Cl | $OCH_3$ | CH | |
| O | H | H | H | Br | $OCH_3$ | CH | |
| O | H | H | H | $CH_3$ | $OCH_3$ | N | |
| O | H | H | H | $OCH_3$ | $OCH_3$ | N | |
| O | H | H | $CH_3$ | $CH_3$ | $CH_3$ | CH | 191-192 |
| O | H | H | $CH_3$ | $CH_3$ | $OCH_3$ | CH | 175-176 |
| O | H | H | $CH_3$ | $OCH_3$ | $OCH_3$ | CH | 200-201 |
| O | H | H | $CH_3$ | Cl | $OCH_3$ | CH | 189-190 |
| O | H | H | $CH_3$ | Br | $OCH_3$ | CH | |
| O | H | H | $CH_3$ | $CH_3$ | $OCH_3$ | N | 176-177 |
| O | H | H | $CH_3$ | $OCH_3$ | $OCH_3$ | N | 193-194 |
| O | H | $CH_3$ | $CH_3$ | $CH_3$ | $CH_3$ | CH | |
| O | H | $CH_3$ | $CH_3$ | $CH_3$ | $OCH_3$ | CH | |
| O | H | $CH_3$ | $CH_3$ | $OCH_3$ | $OCH_3$ | CH | |
| O | H | $CH_3$ | $CH_3$ | Cl | $OCH_3$ | CH | |
| O | H | $CH_3$ | $CH_3$ | Br | $OCH_3$ | CH | |
| O | H | $CH_3$ | $CH_3$ | $CH_3$ | $OCH_3$ | N | |
| O | H | $CH_3$ | $CH_3$ | $OCH_3$ | $OCH_3$ | N | |
| O | H | H | $CH_2CH_3$ | $CH_3$ | $OCH_3$ | N | |
| O | H | H | $CH_2CH_3$ | $CH_3$ | $OCH_3$ | CH | |
| O | H | H | $CH_2CH_3$ | $OCH_3$ | $OCH_3$ | CH | |
| O | H | H | $(CH_2)_5CH_3$ | $OCH_3$ | $OCH_3$ | CH | |
| O | H | Cl | Cl | $CH_3$ | $CH_3$ | CH | |
| O | H | Cl | Cl | $CH_3$ | $OCH_3$ | CH | |
| O | H | Cl | Cl | $OCH_3$ | $OCH_3$ | CH | |
| O | H | Cl | Cl | Cl | $OCH_3$ | CH | |
| O | H | Cl | Cl | Br | $OCH_3$ | CH | |
| O | H | Cl | Cl | $CH_3$ | $OCH_3$ | N | |

Table Ii (Continued)

| W | R | $R_1$ | $R_7$ | X | Y | Z | m.p. (°C) |
|---|---|---|---|---|---|---|---|
| O | H | Cl | Cl | $OCH_3$ | $OCH_3$ | N | |
| O | H | H | Br | $CH_3$ | $OCH_3$ | N | |
| O | H | H | Br | $CH_3$ | $OCH_3$ | CH | |
| O | H | H | Br | $OCH_3$ | $OCH_3$ | CH | |
| O | H | H | CN | $CH_3$ | $OCH_3$ | N | |
| O | H | H | CN | $CH_3$ | $OCH_3$ | CH | |
| O | H | H | CN | $OCH_3$ | $OCH_3$ | CH | |
| O | H | H | $NO_2$ | $CH_3$ | $OCH_3$ | N | |
| O | H | H | $NO_2$ | $CH_3$ | $OCH_3$ | CH | |
| O | H | H | $NO_2$ | $OCH_3$ | $OCH_3$ | CH | |
| O | H | H | $SCH_3$ | $CH_3$ | $OCH_3$ | N | |
| O | H | H | $SCH_3$ | $CH_3$ | $OCH_3$ | CH | |
| O | H | H | $SCH_3$ | $OCH_3$ | $OCH_3$ | CH | |
| O | H | H | $SO_2CH_3$ | $CH_3$ | $OCH_3$ | N | |
| O | H | H | $SO_2CH_3$ | $CH_3$ | $OCH_3$ | CH | |
| O | H | H | $SO_2CH_3$ | $OCH_3$ | $OCH_3$ | CH | |
| O | H | H | $CO_2CH_3$ | $CH_3$ | $OCH_3$ | N | |
| O | H | H | $CO_2CH_3$ | $CH_3$ | $OCH_3$ | CH | |
| O | H | H | $CO_2CH_3$ | $OCH_3$ | $OCH_3$ | CH | |
| O | H | H | $C(O)CH_3$ | $CH_3$ | $OCH_3$ | N | |
| O | H | H | $C(O)CH_3$ | $CH_3$ | $OCH_3$ | CH | |
| O | H | H | $C(O)CH_3$ | $OCH_3$ | $OCH_3$ | CH | |
| O | H | H | $C(O)(CH_2)_3CH_3$ | $OCH_3$ | $OCH_3$ | CH | |
| O | H | H | $CO_2CH_2Ph$ | $OCH_3$ | $OCH_3$ | CH | |
| O | H | H | $SO_2Ph$ | $OCH_3$ | $OCH_3$ | CH | |
| S | H | H | $CH_3$ | $OCH_3$ | $OCH_3$ | CH | |

**Table Ij**

**Structure Ij**

| W | R | $R_1'$ | $R_7$ | X | Y | Z | m.p. (°C) |
|---|---|---|---|---|---|---|---|
| O | H | H | H | $CH_3$ | $CH_3$ | CH | |
| O | H | H | H | $CH_3$ | $OCH_3$ | CH | |
| O | H | H | H | $OCH_3$ | $OCH_3$ | CH | |
| O | H | H | H | Cl | $OCH_3$ | CH | |
| O | H | H | H | Br | $OCH_3$ | CH | |
| O | H | H | H | $CH_3$ | $OCH_3$ | N | |
| O | H | H | H | $OCH_3$ | $OCH_3$ | N | |
| O | H | H | $CH_3$ | $CH_3$ | $CH_3$ | CH | |
| O | H | H | $CH_3$ | $CH_3$ | $OCH_3$ | CH | |
| O | H | H | $CH_3$ | $OCH_3$ | $OCH_3$ | CH | |
| O | H | H | $CH_3$ | Cl | $OCH_3$ | CH | |
| O | H | H | $CH_3$ | Br | $OCH_3$ | CH | |
| O | H | H | $CH_3$ | $CH_3$ | $OCH_3$ | N | |
| O | H | H | $CH_3$ | $OCH_3$ | $OCH_3$ | N | |
| O | H | H | $CO_2CH_3$ | $CH_3$ | $OCH_3$ | N | |
| O | H | H | $CO_2CH_3$ | $CH_3$ | $OCH_3$ | CH | |
| O | H | H | $CO_2CH_3$ | $OCH_3$ | $OCH_3$ | CH | |
| S | H | H | $CH_3$ | $OCH_3$ | $OCH_3$ | CH | |

Table Ik

Structure Ik

| n | G | R | $R_1$ | $R_4$ | $R_5$ | X | Y | Z | m.p. (°C) |
|---|---|---|---|---|---|---|---|---|---|
| 0 | S | H | H | H | H | $CH_3$ | $OCH_3$ | N | |
| 0 | S | H | H | H | H | $CH_3$ | $OCH_3$ | CH | |
| 0 | S | H | H | H | H | $OCH_3$ | $OCH_3$ | CH | |
| 0 | S | H | H | $CH_3$ | H | $CH_3$ | $OCH_3$ | N | |
| 0 | S | H | H | $CH_3$ | H | $CH_3$ | $OCH_3$ | CH | |
| 0 | S | H | H | $CH_3$ | H | $OCH_3$ | $OCH_3$ | CH | |
| 0 | S | H | H | $(CH_2)_3CH_3$ | H | $OCH_3$ | $OCH_3$ | CH | |
| 1 | S | H | H | $CH_3$ | H | $OCH_3$ | $OCH_3$ | CH | |
| 1 | S | H | H | $CH_3$ | $CH_3$ | $OCH_3$ | $OCH_3$ | CH | |
| 0 | S | H | $CH_3$ | $CH_3$ | H | $OCH_3$ | $OCH_3$ | CH | |
| 0 | O | H | $CH_3$ | $CH_3$ | H | $OCH_3$ | $OCH_3$ | CH | |
| 0 | NH | H | $CH_3$ | $CH_3$ | H | $OCH_3$ | $OCH_3$ | CH | |
| 0 | $NCH_3$ | H | $CH_3$ | $CH_3$ | H | $OCH_3$ | $OCH_3$ | CH | |

Table Il

Structure Il

| n | R | $R_1'$ | $R_4$ | $R_5$ | X | Y | Z | m.p. (°C) |
|---|---|---|---|---|---|---|---|---|
| 0 | H | H | H | H | $CH_3$ | $OCH_3$ | N | |
| 0 | H | H | H | H | $CH_3$ | $OCH_3$ | CH | |
| 0 | H | H | H | H | $OCH_3$ | $OCH_3$ | CH | |
| 0 | H | H | $CH_3$ | H | $CH_3$ | $OCH_3$ | N | |
| 0 | H | H | $CH_3$ | H | $CH_3$ | $OCH_3$ | CH | |
| 0 | H | H | $CH_3$ | H | $OCH_3$ | $OCH_3$ | CH | |
| 0 | H | H | $CH_3$ | $CH_3$ | $OCH_3$ | $OCH_3$ | CH | |
| 1 | H | H | $CH_3$ | H | $OCH_3$ | $OCH_3$ | CH | |

Table Im

Structure Im

| n | G | R | $R_1$ | $R_5$ | X | Y | Z | m.p. (°C) |
|---|---|---|---|---|---|---|---|---|
| 1 | S | H | H | H | $CH_3$ | $OCH_3$ | N | |
| 1 | S | H | H | H | $CH_3$ | $OCH_3$ | CH | |
| 1 | S | H | H | H | $OCH_3$ | $OCH_3$ | CH | |
| 1 | S | H | $CH_3$ | H | $CH_3$ | $OCH_3$ | N | |
| 1 | S | H | $CH_3$ | H | $CH_3$ | $OCH_3$ | CH | |
| 1 | S | H | $CH_3$ | H | $OCH_3$ | $OCH_3$ | CH | |
| 1 | O | H | $CH_3$ | H | $CH_3$ | $OCH_3$ | N | |
| 1 | O | H | $CH_3$ | H | $CH_3$ | $OCH_3$ | CH | |
| 1 | O | H | $CH_3$ | H | $OCH_3$ | $OCH_3$ | CH | |
| 1 | NH | H | $CH_3$ | H | $CH_3$ | $OCH_3$ | N | |
| 1 | NH | H | $CH_3$ | H | $CH_3$ | $OCH_3$ | CH | |
| 1 | NH | H | $CH_3$ | H | $OCH_3$ | $OCH_3$ | CH | |
| 1 | $NCH_3$ | H | $CH_3$ | H | $CH_3$ | $OCH_3$ | N | |
| 1 | $NCH_3$ | H | $CH_3$ | H | $CH_3$ | $OCH_3$ | CH | |
| 1 | $NCH_3$ | H | $CH_3$ | H | $OCH_3$ | $OCH_3$ | CH | |
| 1 | S | H | H | $CH_3$ | $OCH_3$ | $OCH_3$ | CH | |
| 0 | S | H | H | H | $OCH_3$ | $OCH_3$ | CH | |

Table In

Structure In

| n | R | $R_1'$ | $R_5$ | X | Y | Z | m.p. (°C) |
|---|---|---|---|---|---|---|---|
| 0 | H | H | H | $CH_3$ | $OCH_3$ | N | |
| 0 | H | H | H | $CH_3$ | $OCH_3$ | CH | |
| 0 | H | H | H | $OCH_3$ | $OCH_3$ | CH | |
| 1 | H | H | H | $CH_3$ | $OCH_3$ | N | |
| 1 | H | H | H | $CH_3$ | $OCH_3$ | CH | |
| 1 | H | H | H | $OCH_3$ | $OCH_3$ | CH | |
| 1 | H | H | $CH_3$ | $OCH_3$ | $OCH_3$ | CH | |

Table Io

Structure Io

| J | G | R | $R_1$ | $R_1'$ | $R_4$ | $R_5$ | $R_6$ | X | Y | Z | m.p. (°C) |
|---|---|---|---|---|---|---|---|---|---|---|---|
| J-9 | S | H | H | - | H | - | - | $OCH_3$ | $OCH_3$ | CH | |
| J-9 | S | H | $CH_3$ | - | H | - | - | $OCH_3$ | $OCH_3$ | CH | |
| J-9 | O | H | $CH_3$ | - | H | - | - | $OCH_3$ | $OCH_3$ | CH | |
| J-9 | NH | H | $CH_3$ | - | H | - | - | $OCH_3$ | $OCH_3$ | CH | |
| J-9 | $NCH_3$ | H | $CH_3$ | - | H | - | - | $OCH_3$ | $OCH_3$ | CH | |
| J-10 | - | H | - | H | H | - | - | $OCH_3$ | $OCH_3$ | CH | |
| J-11 | S | H | H | - | - | H | $CH_3$ | $OCH_3$ | $OCH_3$ | CH | |
| J-11 | S | H | $CH_3$ | - | - | H | $CH_3$ | $OCH_3$ | $OCH_3$ | CH | |
| J-11 | O | H | $CH_3$ | - | - | H | $CH_3$ | $OCH_3$ | $OCH_3$ | CH | |
| J-11 | NH | H | $CH_3$ | - | - | H | $CH_3$ | $OCH_3$ | $OCH_3$ | CH | |
| J-11 | $NCH_3$ | H | $CH_3$ | - | - | H | $CH_3$ | $OCH_3$ | $OCH_3$ | CH | |
| J-12 | - | H | - | H | - | H | $CH_3$ | $OCH_3$ | $OCH_3$ | CH | |
| J-19 | S | H | H | - | $CH_3$ | H | - | $OCH_3$ | $OCH_3$ | CH | |
| J-19 | S | H | $CH_3$ | - | $CH_3$ | H | - | $OCH_3$ | $OCH_3$ | CH | |
| J-19 | O | H | $CH_3$ | - | $CH_3$ | H | - | $OCH_3$ | $OCH_3$ | CH | |
| J-19 | NH | H | $CH_3$ | - | $CH_3$ | H | - | $OCH_3$ | $OCH_3$ | CH | |
| J-19 | $NCH_3$ | H | $CH_3$ | - | $CH_3$ | H | - | $OCH_3$ | $OCH_3$ | CH | |
| J-20 | - | H | - | H | $CH_3$ | H | - | $OCH_3$ | $OCH_3$ | CH | |
| J-21 | S | H | H | - | - | H | - | $OCH_3$ | $OCH_3$ | CH | |
| J-21 | S | H | $CH_3$ | - | - | H | - | $OCH_3$ | $OCH_3$ | CH | |
| J-21 | O | H | $CH_3$ | - | - | H | - | $OCH_3$ | $OCH_3$ | CH | |
| J-21 | NH | H | $CH_3$ | - | - | H | - | $OCH_3$ | $OCH_3$ | CH | |
| J-21 | $NCH_3$ | H | $CH_3$ | - | - | H | - | $OCH_3$ | $OCH_3$ | CH | |
| J-22 | - | H | - | H | - | H | - | $OCH_3$ | $OCH_3$ | CH | |

Table Ip

Structure Ip

| J | G | n | Q | $R_1(R_1')$ | $R_2$ | $R_3$ | X | Y | Z | m.p. (°C) |
|---|---|---|---|---|---|---|---|---|---|---|
| J-1 | S | 1 | S | H | H | H | $OCH_3$ | $OCH_3$ | CH | |
| J-1 | S | 0 | S | H | $CH_3$ | H | $OCH_3$ | $OCH_3$ | CH | |
| J-1 | S | 0 | $SO_2$ | H | $CH_3$ | H | $OCH_3$ | $OCH_3$ | CH | |
| J-1 | O | 0 | $SO_2$ | $CH_3$ | H | H | $OCH_3$ | $OCH_3$ | CH | |
| J-1 | NH | 0 | $SO_2$ | $CH_3$ | H | H | $OCH_3$ | $OCH_3$ | CH | |
| J-1 | $NCH_3$ | 0 | $SO_2$ | $CH_3$ | H | H | $OCH_3$ | $OCH_3$ | CH | |
| J-1 | S | 0 | $SO_2$ | H | $CH_3$ | H | $CH_3$ | $OCH_3$ | N | |
| J-2 | - | 0 | $SO_2$ | H | $CH_3$ | H | $OCH_3$ | $OCH_3$ | CH | |
| J-3 | S | 1 | - | H | H | H | $OCH_3$ | $OCH_3$ | CH | |
| J-3 | S | 1 | - | $CH_3$ | H | H | $OCH_3$ | $OCH_3$ | CH | |
| J-3 | O | 1 | - | $CH_3$ | H | H | $OCH_3$ | $OCH_3$ | CH | |
| J-3 | NH | 1 | - | $CH_3$ | H | H | $OCH_3$ | $OCH_3$ | CH | |
| J-3 | $NCH_3$ | 1 | - | $CH_3$ | H | H | $OCH_3$ | $OCH_3$ | CH | |
| J-4 | - | 1 | - | H | H | H | $OCH_3$ | $OCH_3$ | CH | |
| J-4 | - | 0 | - | H | H | H | $OCH_3$ | $OCH_3$ | CH | |

Table Iq

Structure Iq

| J | G | n | $R_1 (R_1')$ | $R_4$ | $R_5$ | $R_6$ | X | Y | Z | m.p. (°C) |
|---|---|---|---|---|---|---|---|---|---|---|
| J-5 | S | 1 | H | H | H | - | $OCH_3$ | $OCH_3$ | CH | |
| J-5 | S | 1 | $CH_3$ | H | H | - | $OCH_3$ | $OCH_3$ | CH | |
| J-5 | O | 1 | $CH_3$ | H | H | - | $OCH_3$ | $OCH_3$ | CH | |
| J-5 | NH | 1 | $CH_3$ | H | H | - | $OCH_3$ | $OCH_3$ | CH | |
| J-5 | $NCH_3$ | 1 | $CH_3$ | H | H | - | $OCH_3$ | $OCH_3$ | CH | |
| J-5 | S | O | H | H | H | - | $CH_3$ | $OCH_3$ | N | |
| J-6 | - | 1 | H | H | H | - | $OCH_3$ | $OCH_3$ | CH | |
| J-6 | - | 1 | Cl | H | H | - | $OCH_3$ | $OCH_3$ | CH | |
| J-7 | S | O | H | - | H | $CH_3$ | $OCH_3$ | $OCH_3$ | CH | |
| J-7 | O | O | H | - | H | $CH_3$ | $OCH_3$ | $OCH_3$ | CH | |
| J-7 | S | 1 | H | - | H | $CH_3$ | $OCH_3$ | $OCH_3$ | CH | |
| J-8 | - | O | H | - | H | $CH_3$ | $OCH_3$ | $OCH_3$ | CH | |
| J-9 | S | - | $CH_3$ | H | - | - | $OCH_3$ | $OCH_3$ | CH | |
| J-9 | O | - | $CH_3$ | H | - | - | $OCH_3$ | $OCH_3$ | CH | |
| J-9 | NH | - | $CH_3$ | H | - | - | $OCH_3$ | $OCH_3$ | CH | |
| J-9 | $NCH_3$ | - | $CH_3$ | H | - | - | $OCH_3$ | $OCH_3$ | CH | |
| J-9 | S | - | H | H | - | - | $CH_3$ | $OCH_3$ | N | |
| J-10 | - | - | H | H | - | - | $OCH_3$ | $OCH_3$ | CH | |
| J-11 | S | - | H | - | H | $CH_3$ | $OCH_3$ | $OCH_3$ | CH | |
| J-12 | - | - | H | - | H | $CH_3$ | $OCH_3$ | $OCH_3$ | CH | |
| J-15 | S | 1 | H | $CH_3$ | H | - | $OCH_3$ | $OCH_3$ | CH | |
| J-15 | S | O | H | $CH_3$ | H | - | $OCH_3$ | $OCH_3$ | CH | |
| J-16 | - | O | H | $CH_3$ | H | - | $OCH_3$ | $OCH_3$ | CH | |
| J-17 | S | - | H | - | H | - | $OCH_3$ | $OCH_3$ | CH | |
| J-18 | - | - | H | - | H | - | $OCH_3$ | $OCH_3$ | CH | |
| J-19 | S | - | H | $CH_3$ | H | - | $OCH_3$ | $OCH_3$ | CH | |
| J-20 | - | - | H | $CH_3$ | H | - | $OCH_3$ | $OCH_3$ | CH | |
| J-21 | S | - | H | - | H | - | $OCH_3$ | $OCH_3$ | CH | |
| J-22 | - | - | H | - | H | - | $OCH_3$ | $OCH_3$ | CH | |

## Table IIa

### Structure IIa

| J | G | Q | n | R | $R_1$($R_1$') | $R_2$ | $R_3$ | $X_1$ | $Y_1$ | m.p. (°C) |
|---|---|---|---|---|---|---|---|---|---|---|
| $J_1$ | S | O | 0 | H | H | H | H | $CH_3$ | O | |
| $J_1$ | S | O | 0 | H | H | H | $CH_3$ | $OCH_3$ | $CH_2$ | |
| $J_1$ | S | O | 0 | H | H | $CH_3$ | $CH_3$ | $OCH_2CH_3$ | O | |
| $J_1$ | S | O | 1 | H | H | H | H | $OCF_2H$ | $CH_2$ | |
| $J_1$ | S | O | 1 | H | H | H | $CH_3$ | $CH_3$ | $CH_2$ | |
| $J_1$ | S | O | 1 | H | H | $CH_3$ | $CH_3$ | $OCH_3$ | O | |
| $J_1$ | S | S | 0 | H | H | H | H | $OCH_2CH_3$ | $CH_2$ | |
| $J_1$ | S | S | 0 | H | H | H | $CH_3$ | $OCF_2H$ | O | |
| $J_1$ | S | S | 0 | H | H | $CH_3$ | $CH_3$ | $CH_3$ | O | |
| $J_1$ | S | S | 1 | H | H | H | H | $OCH_3$ | $CH_2$ | |
| $J_1$ | S | S | 1 | H | H | H | $CH_3$ | $OCH_2CH_3$ | O | |
| $J_1$ | S | S | 1 | H | H | $CH_3$ | $CH_3$ | $OCF_2H$ | $CH_2$ | |
| $J_1$ | S | $SO_2$ | 0 | H | H | H | H | $CH_3$ | $CH_2$ | |
| $J_1$ | O | $SO_2$ | 0 | H | H | H | $CH_3$ | $OCH_3$ | O | |
| $J_1$ | NH | $SO_2$ | 0 | H | H | $CH_3$ | $CH_3$ | $OCH_2CH_3$ | $CH_2$ | |
| $J_1$ | S | $SO_2$ | 1 | H | H | H | H | $OCF_2H$ | O | |
| $J_1$ | S | $SO_2$ | 1 | H | H | H | $CH_3$ | $CH_3$ | O | |
| $J_1$ | S | $SO_2$ | 1 | H | H | $CH_3$ | $CH_3$ | $OCH_3$ | $CH_2$ | |
| $J_2$ | - | O | 0 | H | H | H | H | $OCH_2CH_3$ | O | |
| $J_2$ | - | O | 0 | H | H | H | $CH_3$ | $OCF_2H$ | $CH_2$ | |
| $J_2$ | - | O | 0 | H | H | $CH_3$ | $CH_3$ | $CH_3$ | $CH_2$ | |
| $J_2$ | - | O | 1 | H | H | H | H | $OCH_3$ | O | |
| $J_2$ | - | O | 1 | H | H | H | $CH_3$ | $OCH_2CH_3$ | $CH_2$ | |
| $J_2$ | - | O | 1 | H | H | $CH_3$ | $CH_3$ | $OCF_2H$ | O | |
| $J_2$ | - | S | 0 | H | H | H | H | $CH_3$ | O | |
| $J_2$ | - | S | 0 | H | H | H | $CH_3$ | $OCH_3$ | $CH_2$ | |

Table IIa (continued)

| J | G | Q | n | R | $R_1$($R_1$') | $R_2$ | $R_3$ | $X_1$ | $Y_1$ | m.p. (°C) |
|---|---|---|---|---|---|---|---|---|---|---|
| $J_2$ | - | S | 0 | H | H | $CH_3$ | $CH_3$ | $OCH_2CH_3$ | O | |
| $J_2$ | - | S | 1 | H | H | H | H | $OCF_2H$ | $CH_2$ | |
| $J_2$ | - | S | 1 | H | H | H | $CH_3$ | $CH_3$ | $CH_2$ | |
| $J_2$ | - | S | 1 | H | H | $CH_3$ | $CH_3$ | $OCH_3$ | O | |
| $J_2$ | - | $SO_2$ | 0 | H | H | H | H | $OCH_2CH_3$ | $CH_2$ | |
| $J_2$ | - | $SO_2$ | 0 | H | H | H | $CH_3$ | $OCF_2H$ | O | |
| $J_2$ | - | $SO_2$ | 0 | H | H | $CH_3$ | $CH_3$ | $CH_3$ | O | |
| $J_2$ | - | $SO_2$ | 1 | H | H | H | H | $OCH_3$ | $CH_2$ | |
| $J_2$ | - | $SO_2$ | 1 | H | H | H | $CH_3$ | $OCH_2CH_3$ | O | |
| $J_2$ | - | $SO_2$ | 1 | H | H | $CH_3$ | $CH_3$ | $OCF_2H$ | $CH_2$ | |
| $J_3$ | S | - | 0 | H | H | H | H | $CH_3$ | $CH_2$ | |
| $J_3$ | S | - | 0 | H | H | H | $CH_3$ | $OCH_3$ | O | |
| $J_3$ | S | - | 0 | H | H | $CH_3$ | $CH_3$ | $OCH_2CH_3$ | $CH_2$ | |
| $J_3$ | S | - | 1 | H | H | H | H | $OCF_2H$ | O | |
| $J_3$ | S | - | 1 | H | H | H | $CH_3$ | $CH_3$ | O | |
| $J_3$ | S | - | 1 | H | H | $CH_3$ | $CH_3$ | $OCH_3$ | $CH_2$ | |
| $J_4$ | - | - | 0 | H | H | H | H | $OCH_2CH_3$ | O | |
| $J_4$ | - | - | 0 | H | H | H | $CH_3$ | $OCF_2H$ | $CH_2$ | |
| $J_4$ | - | - | 0 | H | H | $CH_3$ | $CH_3$ | $CH_3$ | $CH_2$ | |
| $J_4$ | - | - | 1 | H | H | H | H | $OCH_3$ | O | |
| $J_4$ | - | - | 1 | H | H | H | $CH_3$ | $OCH_2CH_3$ | $CH_2$ | |
| $J_4$ | - | - | 1 | H | H | $CH_3$ | $CH_3$ | $OCF_2H$ | O | |
| $J_1$ | S | $CH_2$ | 0 | H | H | H | H | $CH_3$ | O | |
| $J_1$ | S | $CH_2$ | 0 | H | H | H | $CH_3$ | $OCH_3$ | $CH_2$ | |
| $J_1$ | S | $CHCH_3$ | 0 | H | H | H | H | $OCH_2CH_3$ | O | |
| $J_1$ | S | $CH_2$ | 1 | H | H | H | H | $OCF_2H$ | $CH_2$ | |
| $J_1$ | S | $CH_2$ | 1 | H | H | H | $CH_3$ | $CH_3$ | $CH_2$ | |
| $J_1$ | S | $CHCH_3$ | 1 | H | H | H | H | $OCH_3$ | O | |
| $J_{13}$ | S | - | - | H | H | H | - | $OCH_2CH_3$ | $CH_2$ | |
| $J_{13}$ | S | - | | H | H | Cl | - | $OCH_2CH_3$ | $CH_2$ | |
| $J_{14}$ | - | - | - | H | H | H | - | $OCF_2H$ | O | |
| $J_{14}$ | - | - | - | H | H | Cl | - | $OCF_2H$ | O | |

Table IIb

Structure IIb

| J | G | n | R | $R_1$ ($R_1'$) | $R_4$ | $R_5$ | $R_6$ | $X_1$ | $Y_1$ | m.p. (°C) |
|---|---|---|---|---|---|---|---|---|---|---|
| $J_5$ | S | 0 | H | H | H | H | - | $CH_3$ | $CH_2$ | |
| $J_5$ | S | 0 | H | H | H | $CH_3$ | - | $OCH_3$ | O | |
| $J_5$ | S | 0 | H | H | H | $CH_3$ | - | $OCH_2CH_3$ | $CH_2$ | |
| $J_5$ | S | 1 | H | H | H | H | - | $OCF_2H$ | O | |
| $J_5$ | S | 1 | H | H | $CH_3$ | H | - | $CH_3$ | O | |
| $J_5$ | S | 1 | H | H | $CH_3$ | $CH_3$ | - | $OCH_3$ | $CH_2$ | |
| $J_6$ | - | 0 | H | H | H | H | - | $OCH_2CH_3$ | O | |
| $J_6$ | - | 0 | H | H | H | $CH_3$ | - | $OCF_2H$ | $CH_2$ | |
| $J_6$ | - | 0 | H | H | H | $CH_3$ | - | $CH_3$ | $CH_2$ | |
| $J_6$ | - | 1 | H | H | H | H | - | $OCH_3$ | O | |
| $J_6$ | - | 1 | H | H | $CH_3$ | H | - | $OCH_2CH_3$ | $CH_2$ | |
| $J_6$ | - | 1 | H | H | $CH_3$ | $CH_3$ | - | $OCF_2H$ | O | |
| $J_7$ | S | 0 | H | H | - | H | $CH_3$ | $CH_3$ | O | |
| $J_7$ | S | 0 | H | H | - | $CH_3$ | $CH_3$ | $OCH_3$ | $CH_2$ | |
| $J_7$ | S | 1 | H | H | - | H | $CH_3$ | $OCH_2CH_3$ | O | |
| $J_7$ | S | 1 | H | H | - | $CH_3$ | $CH_3$ | $OCF_2H$ | $CH_2$ | |
| $J_8$ | - | 0 | H | H | - | H | $CH_3$ | $CH_3$ | $CH_2$ | |
| $J_8$ | - | 0 | H | H | - | $CH_3$ | $CH_3$ | $OCH_3$ | O | |
| $J_8$ | - | 1 | H | H | - | H | $CH_3$ | $OCH_2CH_3$ | $CH_2$ | |
| $J_8$ | - | 1 | H | H | - | $CH_3$ | $CH_3$ | $OCF_2H$ | O | |
| $J_9$ | S | - | H | H | H | - | - | $CH_3$ | O | |
| $J_9$ | S | - | H | H | $CH_3$ | - | - | $OCH_3$ | $CH_2$ | |
| $J_{10}$ | - | - | H | H | H | - | - | $OCH_2CH_3$ | O | |
| $J_{10}$ | - | - | H | H | $CH_3$ | - | - | $OCF_2H$ | $CH_2$ | |
| $J_{11}$ | S | - | H | H | - | H | $CH_3$ | $CH_3$ | $CH_2$ | |
| $J_{11}$ | S | - | H | H | - | $CH_3$ | $CH_3$ | $OCH_3$ | O | |

## Table IIb (continued)

| J | G | n | R | $(R'_1)_1$ | $R_4$ | $R_5$ | $R_6$ | $X_1$ | $Y_1$ | m.p. (°C) |
|---|---|---|---|---|---|---|---|---|---|---|
| $J_{12}$ | - | - | H | H | - | H | $CH_3$ | $OCH_2CH_3$ | $CH_2$ | |
| $J_{12}$ | - | - | H | H | - | $CH_3$ | $CH_3$ | $OCF_2H$ | O | |
| $J_{15}$ | S | 1 | H | H | $CH_3$ | H | - | $OCH_3$ | O | |
| $J_{16}$ | - | 1 | H | H | $CH_3$ | H | - | $OCH_3$ | O | |
| $J_{17}$ | S | - | H | H | - | H | - | $OCH_3$ | O | |
| $J_{18}$ | - | - | H | H | - | H | - | $OCH_3$ | O | |
| $J_{19}$ | S | - | H | H | $CH_3$ | H | - | $OCH_3$ | O | |
| $J_{20}$ | - | - | H | H | $CH_3$ | H | - | $OCH_3$ | O | |
| $J_{21}$ | S | - | H | H | - | H | - | $OCH_3$ | O | |
| $J_{22}$ | - | - | H | H | - | H | - | $OCH_3$ | O | |

Table IIIa

Structure IIIa

| J | G | Q | n | R | $R_1(R_1')$ | $R_2$ | $R_3$ | $X_1$ | m.p. (°C) |
|---|---|---|---|---|---|---|---|---|---|
| $J_1$ | S | O | 0 | H | H | H | H | $CH_3$ | |
| $J_1$ | S | O | 0 | H | H | H | $CH_3$ | $OCH_3$ | |
| $J_1$ | S | O | 0 | H | H | $CH_3$ | $CH_3$ | $OCH_2CH_3$ | |
| $J_1$ | S | O | 1 | H | H | H | H | $OCF_2H$ | |
| $J_1$ | S | O | 1 | H | H | H | $CH_3$ | $CH_3$ | |
| $J_1$ | S | O | 1 | H | H | $CH_3$ | $CH_3$ | $OCH_3$ | |
| $J_1$ | S | S | 0 | H | H | H | H | $OCH_2CH_3$ | |
| $J_1$ | S | S | 0 | H | H | H | $CH_3$ | $OCF_2H$ | |
| $J_1$ | S | S | 0 | H | H | $CH_3$ | $CH_3$ | $CH_3$ | |
| $J_1$ | S | S | 1 | H | H | H | H | $OCH_3$ | |
| $J_1$ | S | S | 1 | H | H | H | $CH_3$ | $OCH_2CH_3$ | |
| $J_1$ | S | S | 1 | H | H | $CH_3$ | $CH_3$ | $OCF_2H$ | |
| $J_1$ | S | $SO_2$ | 0 | H | H | H | H | $CH_3$ | |
| $J_1$ | S | $SO_2$ | 0 | H | H | H | $CH_3$ | $OCH_3$ | |
| $J_1$ | S | $SO_2$ | 0 | H | H | $CH_3$ | $CH_3$ | $OCH_2CH_3$ | |
| $J_1$ | S | $SO_2$ | 1 | H | H | H | H | $OCF_2H$ | |
| $J_1$ | S | $SO_2$ | 1 | H | H | H | $CH_3$ | $CH_3$ | |
| $J_1$ | S | $SO_2$ | 1 | H | H | $CH_3$ | $CH_3$ | $OCH_3$ | |
| $J_2$ | - | O | 0 | H | H | H | H | $OCH_2CH_3$ | |
| $J_2$ | - | O | 0 | H | H | H | $CH_3$ | $OCF_2H$ | |
| $J_2$ | - | O | 0 | H | H | $CH_3$ | $CH_3$ | $CH_3$ | |
| $J_2$ | - | O | 1 | H | H | H | H | $OCH_3$ | |
| $J_2$ | - | O | 1 | H | H | H | $CH_3$ | $OCH_2CH_3$ | |
| $J_2$ | - | O | 1 | H | H | $CH_3$ | $CH_3$ | $OCF_2H$ | |
| $J_2$ | - | S | 0 | H | H | H | H | $CH_3$ | |
| $J_2$ | - | S | 0 | H | H | H | $CH_3$ | $OCH_3$ | |

Table IIIa (continued)

| J | G | Q | n | R | $R_1(R_1')$ | $R_2$ | $R_3$ | $X_1$ | m.p. (°C) |
|---|---|---|---|---|---|---|---|---|---|
| $J_2$ | - | S | 0 | H | H | $CH_3$ | $CH_3$ | $OCH_2CH_3$ | |
| $J_2$ | - | S | 1 | H | H | H | H | $OCF_2H$ | |
| $J_2$ | - | S | 1 | H | H | H | $CH_3$ | $CH_3$ | |
| $J_2$ | - | S | 1 | H | H | $CH_3$ | $CH_3$ | $OCH_3$ | |
| $J_2$ | - | $SO_2$ | 0 | H | H | H | H | $OCH_2CH_3$ | |
| $J_2$ | - | $SO_2$ | 0 | H | H | H | $CH_3$ | $OCF_2H$ | |
| $J_2$ | - | $SO_2$ | 0 | H | H | $CH_3$ | $CH_3$ | $CH_3$ | |
| $J_2$ | - | $SO_2$ | 1 | H | H | H | H | $OCH_3$ | |
| $J_2$ | - | $SO_2$ | 1 | H | H | H | $CH_3$ | $OCH_2CH_3$ | |
| $J_2$ | - | $SO_2$ | 1 | H | H | $CH_3$ | $CH_3$ | $OCF_2H$ | |
| $J_3$ | S | - | 0 | H | H | H | H | $CH_3$ | |
| $J_3$ | S | - | 0 | H | H | H | $CH_3$ | $OCH_3$ | |
| $J_3$ | S | - | 0 | H | H | $CH_3$ | $CH_3$ | $OCH_2CH_3$ | |
| $J_3$ | S | - | 1 | H | H | H | H | $OCF_2H$ | |
| $J_3$ | S | - | 1 | H | H | H | $CH_3$ | $CH_3$ | |
| $J_3$ | S | - | 1 | H | H | $CH_3$ | $CH_3$ | $OCH_3$ | |
| $J_4$ | - | - | 0 | H | H | H | H | $OCH_2CH_3$ | |
| $J_4$ | - | - | 0 | H | H | H | $CH_3$ | $OCF_2H$ | |
| $J_4$ | - | - | 0 | H | H | $CH_3$ | $CH_3$ | $CH_3$ | |
| $J_4$ | - | - | 1 | H | H | H | H | $OCH_3$ | |
| $J_4$ | - | - | 1 | H | H | H | $CH_3$ | $OCH_2CH_3$ | |
| $J_4$ | - | - | 1 | H | H | $CH_3$ | $CH_3$ | $OCF_2H$ | |
| $J_1$ | S | $CH_2$ | 0 | H | H | H | H | $CH_3$ | |
| $J_1$ | S | $CH_2$ | 0 | H | H | H | $CH_3$ | $OCH_3$ | |
| $J_1$ | S | $CHCH_3$ | 0 | H | H | H | H | $OCH_2CH_3$ | |
| $J_1$ | S | $CH_2$ | 1 | H | H | H | H | $OCF_2H$ | |
| $J_1$ | S | $CH_2$ | 1 | H | H | H | $CH_3$ | $CH_3$ | |
| $J_1$ | S | $CHCH_3$ | 1 | H | H | H | H | $OCH_3$ | |
| $J_{13}$ | S | - | - | H | H | H | - | $OCH_2CH_3$ | |
| $J_{13}$ | S | - | . | H | H | Cl | - | $OCH_2CH_3$ | |
| $J_{14}$ | - | - | - | H | H | H | - | $OCF_2H$ | |
| $J_{14}$ | - | - | - | H | H | Cl | - | $OCF_2H$ | |

## Table IIIb

## Structure IIIb

| J | G | n | R | $R_1$($R_1$') | $R_4$ | $R_5$ | $R_6$ | $X_1$ | m.p.(°C) |
|---|---|---|---|---|---|---|---|---|---|
| $J_5$ | S | 0 | H | H | H | H | - | $CH_3$ | |
| $J_5$ | S | 0 | H | H | H | $CH_3$ | - | $OCH_3$ | |
| $J_5$ | S | 0 | H | H | H | $CH_3$ | - | $OCH_2CH_3$ | |
| $J_5$ | S | 1 | H | H | H | H | - | $OCF_2H$ | |
| $J_5$ | S | 1 | H | H | $CH_3$ | H | - | $CH_3$ | |
| $J_5$ | S | 1 | H | H | $CH_3$ | $CH_3$ | - | $OCH_3$ | |
| $J_6$ | - | 0 | H | H | H | H | - | $OCH_2CH_3$ | |
| $J_6$ | - | 0 | H | H | H | $CH_3$ | - | $OCF_2H$ | |
| $J_6$ | - | 0 | H | H | H | $CH_3$ | - | $CH_3$ | |
| $J_6$ | - | 1 | H | H | H | H | - | $OCH_3$ | |
| $J_6$ | - | 1 | H | H | $CH_3$ | H | - | $OCH_2CH_3$ | |
| $J_6$ | - | 1 | H | H | $CH_3$ | $CH_3$ | - | $OCF_2H$ | |
| $J_7$ | S | 0 | H | H | - | H | $CH_3$ | $CH_3$ | |
| $J_7$ | S | 0 | H | H | - | $CH_3$ | $CH_3$ | $OCH_3$ | |
| $J_7$ | S | 1 | H | H | - | H | $CH_3$ | $OCH_2CH_3$ | |
| $J_7$ | S | 1 | H | H | - | $CH_3$ | $CH_3$ | $OCF_2H$ | |
| $J_8$ | - | 0 | H | H | - | H | $CH_3$ | $CH_3$ | |
| $J_8$ | - | 0 | H | H | - | $CH_3$ | $CH_3$ | $OCH_3$ | |
| $J_8$ | - | 1 | H | H | - | H | $CH_3$ | $OCH_2CH_3$ | |
| $J_8$ | - | 1 | H | H | - | $CH_3$ | $CH_3$ | $OCF_2H$ | |
| $J_9$ | S | - | H | H | H | - | - | $CH_3$ | |
| $J_9$ | S | - | H | H | $CH_3$ | - | - | $OCH_3$ | |
| $J_{10}$ | - | - | H | H | H | - | - | $OCH_2CH_3$ | |
| $J_{10}$ | - | - | H | H | $CH_3$ | - | - | $OCF_2H$ | |
| $J_{11}$ | S | - | H | H | | H | $CH_3$ | $CH_3$ | |
| $J_{11}$ | S | - | H | H | - | $CH_3$ | $CH_3$ | $OCH_3$ | |

Table IIIb (continued)

| J | G | n | R | $R_1(R_1')$ | $R_4$ | $R_5$ | $R_6$ | $X_1$ | m.p.(°C) |
|---|---|---|---|---|---|---|---|---|---|
| $J_{12}$ | - | - | H | H | - | H | $CH_3$ | $OCH_2CH_3$ | |
| $J_{12}$ | - | - | H | H | - | $CH_3$ | $CH_3$ | $OCF_2H$ | |
| $J_{15}$ | S | 1 | H | H | $CH_3$ | H | - | $OCH_3$ | |
| $J_{16}$ | - | 1 | H | H | $CH_3$ | H | - | $OCH_3$ | |
| $J_{17}$ | S | - | H | H | - | H | - | $OCH_3$ | |
| $J_{18}$ | - | - | H | H | - | H | - | $OCH_3$ | |
| $J_{19}$ | S | - | H | H | $CH_3$ | H | - | $OCH_3$ | |
| $J_{20}$ | - | - | H | H | $CH_3$ | H | - | $OCH_3$ | |
| $J_{21}$ | S | - | H | H | - | H | - | $OCH_3$ | |
| $J_{22}$ | - | - | H | H | - | H | - | $OCH_3$ | |

Table IVa

Structure IVa

| J | G | Q | n | R | $R_1(R_1')$ | $R_2$ | $R_3$ | $X_1$ | m.p.(°C) |
|---|---|---|---|---|---|---|---|---|---|
| $J_1$ | S | O | 0 | H | H | H | H | $CH_3$ | |
| $J_1$ | S | O | 0 | H | H | H | $CH_3$ | $OCH_3$ | |
| $J_1$ | S | O | 0 | H | H | $CH_3$ | $CH_3$ | $OCH_2CH_3$ | |
| $J_1$ | S | O | 1 | H | H | H | H | $OCF_2H$ | |
| $J_1$ | S | O | 1 | H | H | H | $CH_3$ | $CH_3$ | |
| $J_1$ | S | O | 1 | H | H | $CH_3$ | $CH_3$ | $OCH_3$ | |
| $J_1$ | S | S | 0 | H | H | H | H | $OCH_2CH_3$ | |
| $J_1$ | S | S | 0 | H | H | H | $CH_3$ | $OCF_2H$ | |
| $J_1$ | S | S | 0 | H | H | $CH_3$ | $CH_3$ | $CH_3$ | |
| $J_1$ | S | S | 1 | H | H | H | H | $OCH_3$ | |
| $J_1$ | S | S | 1 | H | H | H | $CH_3$ | $OCH_2CH_3$ | |
| $J_1$ | S | S | 1 | H | H | $CH_3$ | $CH_3$ | $OCF_2H$ | |
| $J_1$ | S | $SO_2$ | 0 | H | H | H | H | $CH_3$ | |
| $J_1$ | S | $SO_2$ | 0 | H | H | H | $CH_3$ | $OCH_3$ | |
| $J_1$ | S | $SO_2$ | 0 | H | H | $CH_3$ | $CH_3$ | $OCH_2CH_3$ | |
| $J_1$ | S | $SO_2$ | 1 | H | H | H | H | $OCF_2H$ | |
| $J_1$ | S | $SO_2$ | 1 | H | H | H | $CH_3$ | $CH_3$ | |
| $J_1$ | S | $SO_2$ | 1 | H | H | $CH_3$ | $CH_3$ | $OCH_3$ | |
| $J_2$ | – | O | 0 | H | H | H | H | $OCH_2CH_3$ | |
| $J_2$ | – | O | 0 | H | H | H | $CH_3$ | $OCF_2H$ | |
| $J_2$ | – | O | 0 | H | H | $CH_3$ | $CH_3$ | $CH_3$ | |
| $J_2$ | – | O | 1 | H | H | H | H | $OCH_3$ | |
| $J_2$ | – | O | 1 | H | H | H | $CH_3$ | $OCH_2CH_3$ | |
| $J_2$ | – | O | 1 | H | H | $CH_3$ | $CH_3$ | $OCF_2H$ | |
| $J_2$ | – | S | 0 | H | H | H | H | $CH_3$ | |
| $J_2$ | | S | 0 | H | H | H | $CH_3$ | $OCH_3$ | |

Table IVa (continued)

| J | G | Q | n | R | $R_1(R_1')$ | $R_2$ | $R_3$ | $X_1$ | m.p.(°C) |
|---|---|---|---|---|---|---|---|---|---|
| $J_2$ | - | S | 0 | H | H | $CH_3$ | $CH_3$ | $OCH_2CH_3$ | |
| $J_2$ | - | S | 1 | H | H | H | H | $OCF_2H$ | |
| $J_2$ | - | S | 1 | H | H | H | $CH_3$ | $CH_3$ | |
| $J_2$ | - | S | 1 | H | H | $CH_3$ | $CH_3$ | $OCH_3$ | |
| $J_2$ | - | $SO_2$ | 0 | H | H | H | H | $OCH_2CH_3$ | |
| $J_2$ | - | $SO_2$ | 0 | H | H | H | $CH_3$ | $OCF_2H$ | |
| $J_2$ | - | $SO_2$ | 0 | H | H | $CH_3$ | $CH_3$ | $CH_3$ | |
| $J_2$ | - | $SO_2$ | 1 | H | H | H | H | $OCH_3$ | |
| $J_2$ | - | $SO_2$ | 1 | H | H | H | $CH_3$ | $OCH_2CH_3$ | |
| $J_2$ | - | $SO_2$ | 1 | H | H | $CH_3$ | $CH_3$ | $OCF_2H$ | |
| $J_3$ | S | - | 0 | H | H | H | H | $CH_3$ | |
| $J_3$ | S | - | 0 | H | H | H | $CH_3$ | $OCH_3$ | |
| $J_3$ | S | - | 0 | H | H | $CH_3$ | $CH_3$ | $OCH_2CH_3$ | |
| $J_3$ | S | - | 1 | H | H | H | H | $OCF_2H$ | |
| $J_3$ | S | - | 1 | H | H | H | $CH_3$ | $CH_3$ | |
| $J_3$ | S | - | 1 | H | H | $CH_3$ | $CH_3$ | $OCH_3$ | |
| $J_4$ | - | - | 0 | H | H | H | H | $OCH_2CH_3$ | |
| $J_4$ | - | - | 0 | H | H | H | $CH_3$ | $OCF_2H$ | |
| $J_4$ | - | - | 0 | H | H | $CH_3$ | $CH_3$ | $CH_3$ | |
| $J_4$ | - | - | 1 | H | H | H | H | $OCH_3$ | |
| $J_4$ | - | - | 1 | H | H | H | $CH_3$ | $OCH_2CH_3$ | |
| $J_4$ | - | - | 1 | H | H | $CH_3$ | $CH_3$ | $OCF_2H$ | |
| $J_1$ | S | $CH_2$ | 0 | H | H | H | H | $CH_3$ | |
| $J_1$ | S | $CH_2$ | 0 | H | H | H | $CH_3$ | $OCH_3$ | |
| $J_1$ | S | $CHCH_3$ | 0 | H | H | H | H | $OCH_2CH_3$ | |
| $J_1$ | S | $CH_2$ | 1 | H | H | H | H | $OCF_2H$ | |
| $J_1$ | S | $CH_2$ | 1 | H | H | H | $CH_3$ | $CH_3$ | |
| $J_1$ | S | $CHCH_3$ | 1 | H | H | H | H | $OCH_3$ | |
| $J_{13}$ | S | - | - | H | H | H | - | $OCH_2CH_3$ | |
| $J_{13}$ | S | - | - | H | H | Cl | - | $OCH_2CH_3$ | |
| $J_{14}$ | - | - | - | H | H | H | - | $OCF_2H$ | |
| $J_{14}$ | - | - | - | H | H | Cl | - | $OCF_2H$ | |

Table IVb

Structure IVb

| J | G | n | R | $R_1$($R_1$') | $R_4$ | $R_5$ | $R_6$ | $X_1$ | m.p. (°C) |
|---|---|---|---|---|---|---|---|---|---|
| $J_5$ | S | 0 | H | H | H | H | - | $CH_3$ | |
| $J_5$ | S | 0 | H | H | H | $CH_3$ | - | $OCH_3$ | |
| $J_5$ | S | 0 | H | H | H | $CH_3$ | - | $OCH_2CH_3$ | |
| $J_5$ | S | 1 | H | H | H | H | - | $OCF_2H$ | |
| $J_5$ | S | 1 | H | H | $CH_3$ | H | - | $CH_3$ | |
| $J_5$ | S | 1 | H | H | $CH_3$ | $CH_3$ | - | $OCH_3$ | |
| $J_6$ | - | 0 | H | H | H | H | - | $OCH_2CH_3$ | |
| $J_6$ | - | 0 | H | H | H | $CH_3$ | - | $OCF_2H$ | |
| $J_6$ | - | 0 | H | H | H | $CH_3$ | - | $CH_3$ | |
| $J_6$ | - | 1 | H | H | H | H | - | $OCH_3$ | |
| $J_6$ | - | 1 | H | H | $CH_3$ | H | - | $OCH_2CH_3$ | |
| $J_6$ | - | 1 | H | H | $CH_3$ | $CH_3$ | - | $OCF_2H$ | |
| $J_7$ | S | 0 | H | H | - | H | $CH_3$ | $CH_3$ | |
| $J_7$ | S | 0 | H | H | - | $CH_3$ | $CH_3$ | $OCH_3$ | |
| $J_7$ | S | 1 | H | H | - | H | $CH_3$ | $OCH_2CH_3$ | |
| $J_7$ | S | 1 | H | H | - | $CH_3$ | $CH_3$ | $OCF_2H$ | |
| $J_8$ | - | 0 | H | H | - | H | $CH_3$ | $CH_3$ | |
| $J_8$ | - | 0 | H | H | - | $CH_3$ | $CH_3$ | $OCH_3$ | |
| $J_8$ | - | 1 | H | H | - | H | $CH_3$ | $OCH_2CH_3$ | |
| $J_8$ | - | 1 | H | H | - | $CH_3$ | $CH_3$ | $OCF_2H$ | |
| $J_9$ | S | - | H | H | H | - | - | $CH_3$ | |
| $J_9$ | S | - | H | H | $CH_3$ | - | - | $OCH_3$ | |
| $J_{10}$ | - | - | H | H | H | - | - | $OCH_2CH_3$ | |
| $J_{10}$ | - | - | H | H | $CH_3$ | - | - | $OCF_2H$ | |
| $J_{11}$ | S | - | H | H | - | H | $CH_3$ | $CH_3$ | |
| $J_{11}$ | S | - | H | H | - | $CH_3$ | $CH_3$ | $OCH_3$ | |

Table IVb (continued)

| J | G | n | R | $R_1(R_1')$ | $R_4$ | $R_5$ | $R_6$ | $X_1$ | m.p. (°C) |
|---|---|---|---|---|---|---|---|---|---|
| $J_{12}$ | - | - | H | - | - | H | $CH_3$ | $OCH_2CH_3$ | |
| $J_{12}$ | - | - | H | - | - | $CH_3$ | $CH_3$ | $OCF_2H$ | |
| $J_{15}$ | S | 1 | H | H | $CH_3$ | H | - | $OCH_3$ | |
| $J_{16}$ | - | 1 | H | H | $CH_3$ | H | - | $OCH_3$ | |
| $J_{17}$ | S | - | H | H | - | H | - | $OCH_3$ | |
| $J_{18}$ | - | - | H | H | - | H | - | $OCH_3$ | |
| $J_{19}$ | S | - | H | H | $CH_3$ | H | - | $OCH_3$ | |
| $J_{20}$ | - | - | H | H | $CH_3$ | H | - | $OCH_3$ | |
| $J_{21}$ | S | - | H | H | - | H | - | $OCH_3$ | |
| $J_{22}$ | - | - | H | H | - | H | - | $OCH_3$ | |

Table Va

Structure Va

| J | G | Q | n | R | $R_1$($R_1$') | $R_2$ | $R_3$ | $X_2$ | $Y_2$ | m.p. (°C) |
|---|---|---|---|---|---|---|---|---|---|---|
| $J_1$ | S | O | 0 | H | H | H | $H_3$ | $CH_3$ | OCH | |
| $J_1$ | S | O | 0 | H | H | $H_3$ | $CH_2$ | $_3$CH CH | $OCH_2CH_3$ | |
| $J_1$ | S | O | 0 | H | H | $CH_3$ | $CH_3$ | $CH_2CF_3$ | $SCH_3$ | |
| $J_1$ | S | O | 1 | H | H | H | H | $CH_3$ | $SCH_2CH_3$ | |
| $J_1$ | S | O | 1 | H | H | H | $CH_3$ | $CH_2CH_3$ | $CH_3$ | |
| $J_1$ | S | O | 1 | H | H | $CH_3$ | $CH_3$ | $CH_2CF_3$ | $CH_2CH_3$ | |
| $J_1$ | S | S | 0 | H | H | H | H | $CH_3$ | $CH_2CH_3$ | |
| $J_1$ | S | S | 0 | H | H | H | $CH_3$ | $CH_2CH_3$ | $CH_3$ | |
| $J_1$ | S | S | 0 | H | H | $CH_3$ | $CH_3$ | $CH_2CF_3$ | $SCH_2CH_3$ | |
| $J_1$ | S | S | 1 | H | H | H | H | $CH_3$ | $SCH_3$ | |
| $J_1$ | S | S | 1 | H | H | H | $CH_3$ | $CH_2CH_3$ | $OCH_2CH_3$ | |
| $J_1$ | S | S | 1 | H | H | $CH_3$ | $CH_3$ | $CH_2CF_3$ | $OCH_3$ | |
| $J_1$ | S | $SO_2$ | 0 | H | H | H | H | $CH_3$ | $OCH_3$ | |
| $J_1$ | S | $SO_2$ | 0 | H | H | H | $CH_3$ | $CH_2CH_3$ | $OCH_2CH_3$ | |
| $J_1$ | S | $SO_2$ | 0 | H | H | $CH_3$ | $CH_3$ | $CH_2CF_3$ | $SCH_3$ | |
| $J_1$ | S | $SO_2$ | 1 | H | H | H | H | $CH_3$ | $SCH_2CH_3$ | |
| $J_1$ | S | $SO_2$ | 1 | H | H | H | $CH_3$ | $CH_2CH_3$ | $CH_3$ | |
| $J_1$ | S | $SO_2$ | 1 | H | H | $CH_3$ | $CH_3$ | $CH_2CF_3$ | $CH_2CH_3$ | |
| $J_2$ | - | O | 0 | H | H | H | H | $CH_3$ | $CH_2CH_3$ | |
| $J_2$ | - | O | 0 | H | H | H | $CH_3$ | $CH_2CH_3$ | $CH_3$ | |
| $J_2$ | - | O | 0 | H | H | $CH_3$ | $CH_3$ | $CH_2CF_3$ | $SCH_3$ | |
| $J_2$ | - | O | 1 | H | H | H | H | $CH_3$ | $SCH_2CH_3$ | |
| $J_2$ | - | O | 1 | H | H | H | $CH_3$ | $CH_2CH_3$ | $OCH_3$ | |
| $J_2$ | - | O | 1 | H | H | $CH_3$ | $CH_3$ | $CH_2CF_3$ | $OCH_2CH_3$ | |
| $J_2$ | - | S | 0 | H | H | H | H | $CH_3$ | $OCH_3$ | |
| $J_2$ | - | S | 0 | H | H | H | $CH_3$ | $CH_2CH_3$ | $OCH_2CH_3$ | |

Table Va (continued)

| J | G | Q | n | R | $R_1(R_1')$ | $R_2$ | $R_3$ | $X_2$ | $Y_2$ | m.p. (°C) |
|---|---|---|---|---|---|---|---|---|---|---|
| $J_2$ | - | S | 0 | H | H | $CH_3$ | $CH_3$ | $CH_2CF_3$ | $SCH_3$ | |
| $J_2$ | - | S | 1 | H | H | H | H | $CH_3$ | $SCH_2CH_3$ | |
| $J_2$ | - | S | 1 | H | H | H | $CH_3$ | $CH_3$ | $OCH_3$ | |
| $J_2$ | - | S | 1 | H | H | $CH_3$ | $CH_3$ | $CH_2CH_3$ | $OCH_2CH_3$ | |
| $J_2$ | - | $SO_2$ | 0 | H | H | H | H | $CH_2CF_3$ | $SCH_3$ | |
| $J_2$ | - | $SO_2$ | 0 | H | H | H | $CH_3$ | $CH_3$ | $SCH_2CH_3$ | |
| $J_2$ | - | $SO_2$ | 0 | H | H | $CH_3$ | $CH_3$ | $CH_2CH_3$ | $CH_3$ | |
| $J_2$ | - | $SO_2$ | 1 | H | H | H | H | $CH_2CF_3$ | $CH_2CH_3$ | |
| $J_2$ | - | $SO_2$ | 1 | H | H | H | $CH_3$ | $CH_3$ | $CH_2CH_3$ | |
| $J_2$ | - | $SO_2$ | 1 | H | H | $CH_3$ | $CH_3$ | $CH_2CH_3$ | $CH_3$ | |
| $J_3$ | S | - | 0 | H | H | H | H | $CH_2CF_3$ | $SCH_2CH_3$ | |
| $J_3$ | S | - | 0 | H | H | H | $CH_3$ | $CH_3$ | $SCH_3$ | |
| $J_3$ | S | - | 0 | H | H | $CH_3$ | $CH_3$ | $CH_2CH_3$ | $OCH_2CH_3$ | |
| $J_3$ | S | - | 1 | H | H | H | H | $CH_2CF_3$ | $OCH_3$ | |
| $J_3$ | S | - | 1 | H | H | H | $CH_3$ | $CH_3$ | $OCH_3$ | |
| $J_3$ | S | - | 1 | H | H | $CH_3$ | $CH_3$ | $CH_2CH_3$ | $OCH_2CH_3$ | |
| $J_4$ | - | - | 0 | H | H | H | H | $CH_2CF_3$ | $SCH_3$ | |
| $J_4$ | - | - | 0 | H | H | H | $CH_3$ | $CH_3$ | $SCH_2CH_3$ | |
| $J_4$ | - | - | 0 | H | H | $CH_3$ | $CH_3$ | $CH_2CH_3$ | $CH_3$ | |
| $J_4$ | - | - | 1 | H | H | H | H | $CH_2CF_3$ | $CH_2CH_3$ | |
| $J_4$ | - | - | 1 | H | H | H | $CH_3$ | $CH_3$ | $CH_2CH_3$ | |
| $J_4$ | - | - | 1 | H | H | $CH_3$ | $CH_3$ | $CH_2CH_3$ | $CH_3$ | |
| $J_1$ | S | $CH_2$ | 0 | H | H | H | H | $CH_2CF_3$ | $SCH_3$ | |
| $J_1$ | S | $CH_2$ | 0 | H | H | H | $CH_3$ | $CH_3$ | $SCH_2CH_3$ | |
| $J_1$ | S | $CHCH_3$ | 0 | H | H | H | H | $CH_2CH_3$ | $OCH_3$ | |
| $J_1$ | S | $CH_2$ | 1 | H | H | H | H | $CH_2CF_3$ | $OCH_2CH_3$ | |
| $J_1$ | S | $CH_2$ | 1 | H | H | H | $CH_3$ | $CH_3$ | $OCH_3$ | |
| $J_1$ | S | $CHCH_3$ | 1 | H | H | H | H | $CH_2CH_3$ | $OCH_2CH_3$ | |
| $J_{13}$ | - | - | - | H | H | H | - | $CH_2CF_3$ | $SCH_3$ | |
| $J_{13}$ | - | - | - | H | H | Cl | - | $CH_2CF_3$ | $SCH_3$ | |
| $J_{14}$ | - | - | - | H | H | H | - | $CH_3$ | $SCH_2CH_3$ | |
| $J_{14}$ | - | - | - | H | H | Cl | - | $CH_3$ | $SCH_2CH_3$ | |

## Table Vb

## Structure Vb

| J | G | n | R | $R_1(R_1')$ | $R_4$ | $R_5$ | $R_6$ | $X_2$ | $Y_2$ | m.p. (°C) |
|---|---|---|---|---|---|---|---|---|---|---|
| $J_5$ | S | 0 | H | H | H | H | - | $CH_3$ | $OCH_3$ | |
| $J_5$ | S | 0 | H | H | H | $CH_3$ | - | $CH_2OCH_3$ | $OCH_2CH_3$ | |
| $J_5$ | S | 0 | H | H | H | $CH_3$ | - | $CH_2CF_3$ | $SCH_3$ | |
| $J_5$ | S | 1 | H | H | H | H | - | $CH_3$ | $SCH_2CH_3$ | |
| $J_5$ | S | 1 | H | H | $CH_3$ | H | - | $CH_2CH_3$ | $OCH_3$ | |
| $J_5$ | S | 1 | H | H | $CH_3$ | $CH_3$ | - | $OCH_2CF_3$ | $CH_2CH_3$ | |
| $J_6$ | - | 0 | H | H | H | H | - | $CH_3$ | $OCH_2CH_3$ | |
| $J_6$ | - | 0 | H | H | H | $CH_3$ | - | $CH_2CH_3$ | $CH_3$ | |
| $J_6$ | - | 0 | H | H | H | $CH_3$ | - | $CH_2CF_3$ | $SCH_2CH_3$ | |
| $J_6$ | | 1 | H | H | H | H | - | $CH_3$ | $SCH_3$ | |
| $J_6$ | - | 1 | H | H | $CH_3$ | H | - | $CH_2CH_3$ | $OCH_2CH_3$ | |
| $J_6$ | - | 1 | H | H | $CH_3$ | $CH_3$ | - | $CH_2CF_3$ | $OCH_3$ | |
| $J_7$ | S | 0 | H | H | - | H | $CH_3$ | $CH_3$ | $OCH_3$ | |
| $J_7$ | S | 0 | H | H | - | $CH_3$ | $CH_3$ | $CH_2CH_3$ | $OCH_2CH_3$ | |
| $J_7$ | S | 1 | H | H | - | H | $CH_3$ | $CH_2CF_3$ | $SCH_3$ | |
| $J_7$ | S | 1 | H | H | - | $CH_3$ | $CH_3$ | $CH_3$ | $SCH_2CH_3$ | |
| $J_8$ | - | 0 | H | H | - | H | $CH_3$ | $CH_2CH_3$ | $CH_3$ | |
| $J_8$ | - | 0 | H | H | - | $CH_3$ | $CH_3$ | $CH_2CF_3$ | $CH_2CH_3$ | |
| $J_8$ | - | 1 | H | H | - | H | $CH_3$ | $CH_3$ | $CH_2CH_3$ | |
| $J_8$ | - | 1 | H | H | - | $CH_3$ | $CH_3$ | $CH_2CH_3$ | $CH_3$ | |
| $J_9$ | S | - | H | H | H | - | - | $CH_2CF_3$ | $SCH_3$ | |
| $J_9$ | S | - | H | H | $CH_3$ | - | - | $CH_3$ | $SCH_2CH_3$ | |
| $J_{10}$ | - | - | H | H | H | - | - | $CH_2CH_3$ | $OCH_3$ | |
| $J_{10}$ | - | - | H | H | $CH_3$ | - | - | $CH_2CF_3$ | $OCH_3$ | |
| $J_{11}$ | S | - | H | H | - | H | $CH_3$ | $CH_3$ | $OCH_3$ | |
| $J_{11}$ | S | - | H | H | - | $CH_3$ | $CH_3$ | $CH_2CH_3$ | $OCH_2CH_3$ | |

Table Vb (continued)

| J | G | n | R | $R_1$ ($R_1'$) | $R_4$ | $R_5$ | $R_6$ | $X_2$ | $Y_2$ | m.p. (°C) |
|---|---|---|---|---|---|---|---|---|---|---|
| $J_{12}$ | - | - | H | H | - | H | $CH_3$ | $CH_2CF_3$ | $SCH_3$ | |
| $J_{12}$ | - | - | H | H | - | $CH_3$ | $CH_3$ | $CH_3$ | $SCH_2CH_3$ | |
| $J_{15}$ | S | 1 | H | H | $CH_3$ | H | - | $CH_3$ | $OCH_3$ | |
| $J_{16}$ | - | 1 | H | H | $CH_3$ | H | - | $CH_3$ | $OCH_3$ | |
| $J_{17}$ | S | - | H | H | - | H | - | $CH_3$ | $OCH_3$ | |
| $J_{18}$ | - | - | H | H | - | H | - | $CH_3$ | $OCH_3$ | |
| $J_{19}$ | S | - | H | H | $CH_3$ | H | - | $CH_3$ | $OCH_3$ | |
| $J_{20}$ | - | - | H | H | $CH_3$ | H | - | $CH_3$ | $OCH_3$ | |
| $J_{21}$ | S | - | H | H | - | H | - | $CH_3$ | $OCH_3$ | |
| $J_{22}$ | - | - | H | H | - | H | - | $CH_3$ | $OCH_3$ | |

Table VIa

Structure VIa

| J | G | Q | n | R | $R_1$ ($R_1$') | $R_2$ | $R_3$ | $X_3$ | m.p.(°C) |
|---|---|---|---|---|---|---|---|---|---|
| $J_1$ | S | O | 0 | H | H | H | H | $CH_3$ | |
| $J_1$ | S | O | 0 | H | H | H | $CH_3$ | $OCH_3$ | |
| $J_1$ | S | O | 0 | H | H | $CH_3$ | $CH_3$ | $CH_3$ | |
| $J_1$ | S | O | 1 | H | H | H | H | $OCH_3$ | |
| $J_1$ | S | O | 1 | H | H | H | $CH_3$ | $CH_3$ | |
| $J_1$ | S | O | 1 | H | H | $CH_3$ | $CH_3$ | $OCH_3$ | |
| $J_1$ | S | S | 0 | H | H | H | H | $CH_3$ | |
| $J_1$ | S | S | 0 | H | H | H | $CH_3$ | $OCH_3$ | |
| $J_1$ | S | S | 0 | H | H | $CH_3$ | $CH_3$ | $CH_3$ | |
| $J_1$ | S | S | 1 | H | H | H | H | $OCH_3$ | |
| $J_1$ | S | S | 1 | H | H | H | $CH_3$ | $CH_3$ | |
| $J_1$ | S | S | 1 | H | H | $CH_3$ | $CH_3$ | $OCH_3$ | |
| $J_1$ | S | $SO_2$ | 0 | H | H | H | H | $CH_3$ | |
| $J_1$ | S | $SO_2$ | 0 | H | H | H | $CH_3$ | $OCH_3$ | |
| $J_1$ | S | $SO_2$ | 0 | H | H | $CH_3$ | $CH_3$ | $CH_3$ | |
| $J_1$ | S | $SO_2$ | 1 | H | H | H | H | $OCH_3$ | |
| $J_1$ | S | $SO_2$ | 1 | H | H | H | $CH_3$ | $CH_3$ | |
| $J_1$ | S | $SO_2$ | 1 | H | H | $CH_3$ | $CH_3$ | $OCH_3$ | |
| $J_2$ | - | O | 0 | H | H | H | H | $CH_3$ | |
| $J_2$ | - | O | 0 | H | H | H | $CH_3$ | $OCH_3$ | |
| $J_2$ | - | O | 0 | H | H | $CH_3$ | $CH_3$ | $CH_3$ | |
| $J_2$ | - | O | 1 | H | H | H | H | $OCH_3$ | |
| $J_2$ | - | O | 1 | H | H | H | $CH_3$ | $CH_3$ | |
| $J_2$ | - | O | 1 | H | H | $CH_3$ | $CH_3$ | $OCH_3$ | |
| $J_2$ | - | S | 0 | H | H | H | H | $CH_3$ | |
| $J_2$ | - | S | 0 | H | H | H | $CH_3$ | $OCH_3$ | |

Table VIa (continued)

| J | G | Q | n | R | $R_1(R_1')$ | $R_2$ | $R_3$ | $X_3$ | m.p.(°C) |
|---|---|---|---|---|---|---|---|---|---|
| $J_2$ | - | S | 0 | H | H | $CH_3$ | $CH_3$ | $CH_3$ | |
| $J_2$ | - | S | 1 | H | H | H | H | $OCH_3$ | |
| $J_2$ | - | S | 1 | H | H | H | $CH_3$ | $CH_3$ | |
| $J_2$ | - | S | 1 | H | H | $CH_3$ | $CH_3$ | $OCH_3$ | |
| $J_2$ | - | $SO_2$ | 0 | H | H | H | H | $CH_3$ | |
| $J_2$ | - | $SO_2$ | 0 | H | H | H | $CH_3$ | $OCH_3$ | |
| $J_2$ | - | $SO_2$ | 0 | H | H | $CH_3$ | $CH_3$ | $CH_3$ | |
| $J_2$ | - | $SO_2$ | 1 | H | H | H | H | $OCH_3$ | |
| $J_2$ | - | $SO_2$ | 1 | H | H | H | $CH_3$ | $CH_3$ | |
| $J_2$ | - | $SO_2$ | 1 | H | H | $CH_3$ | $CH_3$ | $OCH_3$ | |
| $J_3$ | S | - | 0 | H | H | H | H | $CH_3$ | |
| $J_3$ | S | - | 0 | H | H | H | $CH_3$ | $OCH_3$ | |
| $J_3$ | S | - | 0 | H | H | $CH_3$ | $CH_3$ | $CH_3$ | |
| $J_3$ | S | - | 1 | H | H | H | H | $OCH_3$ | |
| $J_3$ | S | - | 1 | H | H | H | $CH_3$ | $CH_3$ | |
| $J_3$ | S | - | 1 | H | H | $CH_3$ | $CH_3$ | $OCH_3$ | |
| $J_4$ | - | - | 0 | H | H | H | H | $CH_3$ | |
| $J_4$ | - | - | 0 | H | H | H | $CH_3$ | $OCH_3$ | |
| $J_4$ | - | - | 0 | H | H | $CH_3$ | $CH_3$ | $CH_3$ | |
| $J_4$ | - | - | 1 | H | H | H | H | $OCH_3$ | |
| $J_4$ | - | - | 1 | H | H | H | $CH_3$ | $CH_3$ | |
| $J_4$ | - | - | 1 | H | H | $CH_3$ | $CH_3$ | $OCH_3$ | |
| $J_1$ | S | $CH_2$ | 0 | H | H | H | H | $CH_3$ | |
| $J_1$ | S | $CH_2$ | 0 | H | H | H | $CH_3$ | $OCH_3$ | |
| $J_1$ | S | $CHCH_3$ | 0 | H | H | H | H | $CH_3$ | |
| $J_1$ | S | $CH_2$ | 1 | H | H | H | H | $OCH_3$ | |
| $J_1$ | S | $CH_2$ | 1 | H | H | H | $CH_3$ | $CH_3$ | |
| $J_1$ | S | $CHCH_3$ | 1 | H | H | H | H | $OCH_3$ | |
| $J_{13}$ | - | - | - | H | H | H | - | $CH_3$ | |
| $J_{13}$ | - | - | - | H | H | Cl | - | $OCH_3$ | |
| $J_{14}$ | - | - | - | H | H | H | - | $CH_3$ | |
| $J_{14}$ | - | - | - | H | H | Cl | - | $OCH_3$ | |

Table VIb

Structure VIb

| J | G | n | R | $R_1(R_1')$ | $R_4$ | $R_5$ | $R_6$ | $X_3$ | m.p. (°C) |
|---|---|---|---|---|---|---|---|---|---|
| $J_5$ | S | 0 | H | H | H | H | - | $CH_3$ | |
| $J_5$ | S | 0 | H | H | H | $CH_3$ | - | $OCH_3$ | |
| $J_5$ | S | 0 | H | H | H | $CH_3$ | - | $CH_3$ | |
| $J_5$ | S | 1 | H | H | H | H | - | $OCH_3$ | |
| $J_5$ | S | 1 | H | H | $CH_3$ | H | - | $CH_3$ | |
| $J_5$ | S | 1 | H | H | $CH_3$ | $CH_3$ | - | $OCH_3$ | |
| $J_6$ | - | 0 | H | H | H | H | - | $CH_3$ | |
| $J_6$ | - | 0 | H | H | H | $CH_3$ | - | $OCH_3$ | |
| $J_6$ | - | 0 | H | H | H | $CH_3$ | - | $CH_3$ | |
| $J_6$ | - | 1 | H | H | H | H | - | $OCH_3$ | |
| $J_6$ | - | 1 | H | H | $CH_3$ | H | - | $CH_3$ | |
| $J_6$ | - | 1 | H | H | $CH_3$ | $CH_3$ | - | $OCH_3$ | |
| $J_7$ | S | 0 | H | H | - | H | $CH_3$ | $CH_3$ | |
| $J_7$ | S | 0 | H | H | - | $CH_3$ | $CH_3$ | $OCH_3$ | |
| $J_7$ | S | 1 | H | H | - | H | $CH_3$ | $CH_3$ | |
| $J_7$ | S | 1 | H | H | - | $CH_3$ | $CH_3$ | $OCH_3$ | |
| $J_8$ | - | 0 | H | H | - | H | $CH_3$ | $CH_3$ | |
| $J_8$ | - | 0 | H | H | - | $CH_3$ | $CH_3$ | $OCH_3$ | |
| $J_8$ | - | 1 | H | H | - | H | $CH_3$ | $CH_3$ | |
| $J_8$ | - | 1 | H | H | - | $CH_3$ | $CH_3$ | $OCH_3$ | |
| $J_9$ | S | - | H | H | H | - | - | $CH_3$ | |
| $J_9$ | S | - | H | H | $CH_3$ | - | - | $OCH_3$ | |
| $J_{10}$ | - | - | H | H | H | - | - | $CH_3$ | |
| $J_{10}$ | - | - | H | H | $CH_3$ | - | - | $OCH_3$ | |
| $J_{11}$ | S | - | H | H | - | H | $CH_3$ | $CH_3$ | |
| $J_{11}$ | S | - | H | H | - | $CH_3$ | $CH_3$ | $OCH_3$ | |

Table VIb (continued)

| J | G | n | R | $R_1(R_1')$ | $R_4$ | $R_5$ | $R_6$ | $X_3$ | m.p. (°C) |
|---|---|---|---|---|---|---|---|---|---|
| $J_{12}$ | - | - | H | - | - | H | $CH_3$ | $CH_3$ | |
| $J_{12}$ | - | - | H | - | - | $CH_3$ | $CH_3$ | $OCH_3$ | |
| $J_{15}$ | S | 1 | H | H | $CH_3$ | H | - | $OCH_3$ | |
| $J_{16}$ | - | 1 | H | H | $CH_3$ | H | - | $OCH_3$ | |
| $J_{17}$ | S | - | H | H | - | H | - | $OCH_3$ | |
| $J_{18}$ | - | - | H | H | - | H | - | $OCH_3$ | |
| $J_{19}$ | S | - | H | H | $CH_3$ | H | - | $OCH_3$ | |
| $J_{20}$ | - | - | H | H | $CH_3$ | H | - | $OCH_3$ | |
| $J_{21}$ | S | - | H | H | - | H | - | $OCH_3$ | |
| $J_{22}$ | - | - | H | H | - | H | - | $OCH_3$ | |

## Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain for instance about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

Table VII

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufacture, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0 ° C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

In the following examples, all parts are by weight unless otherwise indicated.

Example 6

Wettable Powder

| | |
|---|---|
| N-[(4-methoxy-6-methylpyrimidin-2-yl)aminocarbonyl]-6,7-dihydro-5H-thieno[3,2-B]thiopyran-3-sulfonamide, 4,4-dioxide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 7

Wettable Powder

| | |
|---|---|
| N-[(4,6-dimethoxypyrimidin-2-yl)aminocarbonyl]-6,7-dihydro-5H-thieno[3,2-B]thiopyran-3-sulfonamide, 4,4-dioxide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 8

Granule

| | |
|---|---|
| Wettable Powder of Example 7 | 5% |
| attapulgite granules (U.S.S. 20-40 mesh: 0.84-0.42 mm) | 95% |

A slurry of wettable powder containing 25% solids is sprayed on the surface of attapulgite granules while tumbling in a double-cone blender. The granules are dried and packaged.

Example 9

Extruded Pellet

| | |
|---|---|
| N-[(4,6-dimethoxypyrimidin-2-yl)aminocarbonyl]-5,6-dihydro-5-methylthieno[3,2-B]thiophene-3-sulfonamide, 4,4-dioxide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 10

Oil Suspension

| | |
|---|---|
| N-[(4-methoxy-6-methyltriazin-2-yl)aminocarbonyl]-5,6-dihydro-5-methylthieno[3,2-B]-thiophene sulfonamide, 4,4-dioxide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 11

Wettable Powder

| | |
|---|---|
| N-[(4-chloro-6-methoxypyrimidin-2-yl)aminocarbonyl]-5,6-dihydro-5-methylthieno[3,2-B]thiophene-3-sulfonamide, 4,4-dioxide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening)

and packaged.

Example 12

Low Strength Granule

| | |
|---|---|
| N-[(4-chloro-6-methoxypyrimidin-2-yl)aminocarbonyl]-5,6-dihydro-5-methylthieno[3,2-B]thiophene-3-sulfonamide, 4,4-dioxide | 1% |
| N,N-dimethylformamide | 9% |
| attapulgite granules (U.S.S. 20-40 sieve; 0.84-0.42 mm) | 90% |

The active ingredient is dissolved in the solvent and the solution is sprayed upon dedusted granules in a double cone blender. After spraying of the solution has been completed, the blender is allowed to run for a short period and then the granules are packaged.

Example 13

Aqueous Suspension

| | |
|---|---|
| N-[(4-methoxy-6-methylpyrimidin-2-yl)aminocarbonyl]-6,7-dihydro-5H-thieno[3,2-B]thiopyran-3-sulfonamide, 4,4-dioxide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 14

Solution

| | |
|---|---|
| N-[(4,6-dimethoxypyrimidin-2-yl)aminocarbonyl]-5,6-dihydro-5methylthieno[3,2-B]thiophene-3-sulfonamide, 4,4-dioxide, sodium salt | 5% |
| water | 95% |

The salt is added directly to the water with stirring to produce the solution, which may then be packaged for use.

Example 15

Low Strength Granule

| | |
|---|---|
| N-[(4,6-dimethoxypyrimidin-2-yl)aminocarbonyl]-6,7-dihydro-5H-thieno[3,2-B]thiopyran-3-sulfonamide, 4,4-dioxide | 0.1% |
| attapulgite granules (U.S.S. 20-40 mesh; 0.84-0.42mm) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 16

Granule

| | |
|---|---|
| N-[(4-methoxy-6-methyltriazin-2-yl)aminocarbonyl]-5,6-dihydro-5-methylthieno[3,2-B]-thiophene-3-sulfonamide-4,4-dioxide | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen (149 microns). This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

Example 17

| High Strength Concentrate | |
|---|---|
| N-[(4-chloro-6-methoxypyrimidin-2-yl)aminocarbonyl]-5,6-dihydro-5-methylthieno[3,2-B]thiophene-3-sulfonamide, 4,4-dioxide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 18

| Wettable Powder | |
|---|---|
| N-[(4-methoxy-6-methylpyrimidin-2-yl)aminocarbonyl]-6,7-dihydro-5H-thieno[3,2-B]thiopyran-3-sulfonamide, 4,4-dioxide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen (0.3mm opening) and then packaged.

Example 19

| Wettable Powder | |
|---|---|
| N-[(4,6-dimethoxypyrimidin-2-yl)aminocarbonyl]-6,7-dihydro-5H-thieno[3,2-B]thiopyran-3-sulfonamide, 4,4-dioxide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 20

Oil Suspension

| | |
|---|---|
| N-[(4,6-dimethoxypyrimidin-2-yl)aminocarbonyl]-5,6-dihydro-5-methylthieno[3,2-B]thiophene-3-sulfonamide, 4,4-dioxide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 21

Dust

| | |
|---|---|
| N-[(4-methoxy-6-methyltriazin-2-yl)aminocarbonyl]-5,6-dihydro-5-methylthieno[3,2-B]thiophene-3-sulfonamide, 4,4-dioxide | 10% |
| attapulgite | 10% |
| pyrophyllite | 80% |

The active ingredient is blended with attapulgite and then passed through a hammer-mill to produce particles substantially all below 200 microns. The ground concentrate is then blended with powdered pyrophyllite until homogeneous.

Utility

Test results indicate that the compounds of the present invention are active herbicides. They should have utility for broad-spectrum pre- and/or post-emergence weed control in areas where complete control of all vegetation is desired, such as around fuel storage tanks, ammunition depots, industrial storage areas, parking lots, drive-in theaters, around billboards, highway and railroad structures. Alternatively, many of the subject compounds should be useful for plant growth modification, such as growth retardation. For such use, an effective but substantially non-phytotoxin amount of the compound is applied.

The rates of application for the compounds of the invention are determined by a number of factors, including their use as selective or general herbicides, the crop species involved, the types of weeds to be controlled, weather and climate, formulations selected, mode of application, amount of foliage present, etc. In general terms, the subject compounds should be applied at levels of around 50 to 1000 g/ha, the lower rates being suggested for use on lighter soils and/or those having a low organic matter content, for plant growth modification or for situations where only short-term persistence is required.

The compounds of the invention may be used in combination with any other commercial herbicide examples of which are those of the triazine, triazole, uracil, urea, amide, diphenylether, carbamate and bipyridylium types. Combinations with mefluidide are useful as well.

The herbicidal and plant growth modifying properties of the subject compounds were discovered in the greenhouse. The test procedures and results follow.

Compounds

Compound 1

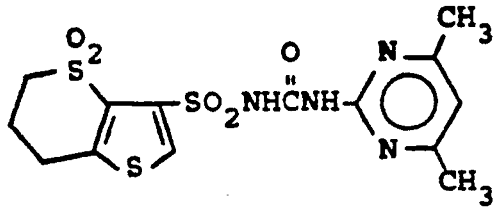

Compound 2

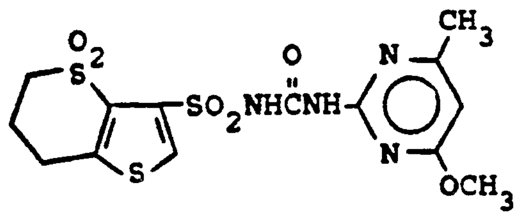

Compound 3

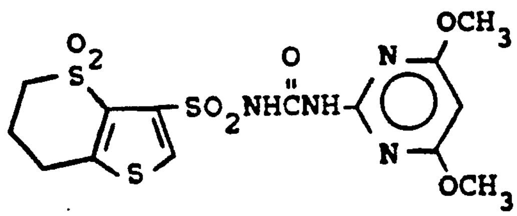

Compound 4

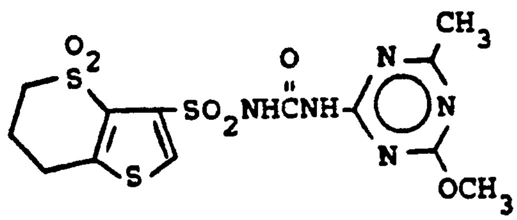

Compound 5

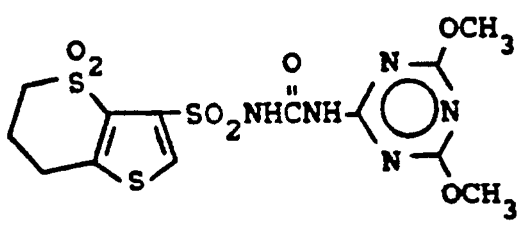

Compounds (continued)

Compound 6

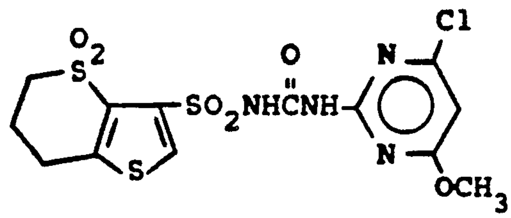

Compound 7

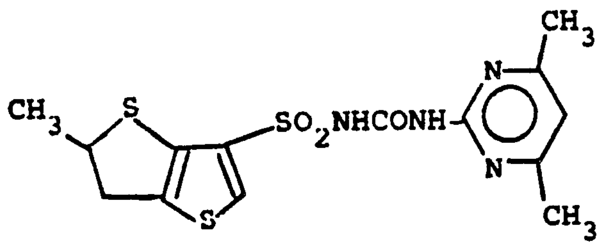

Compound 8

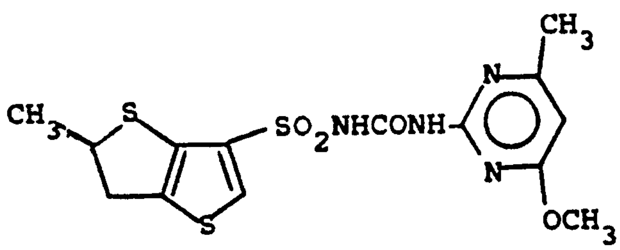

Compound 9

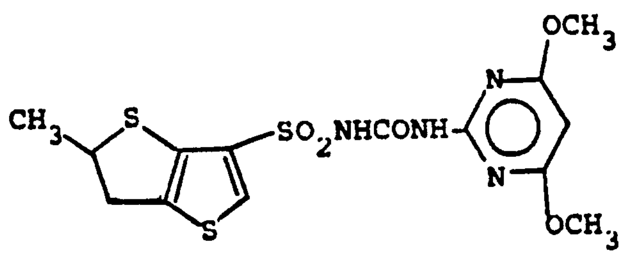

Compounds (continued)

Compound 10

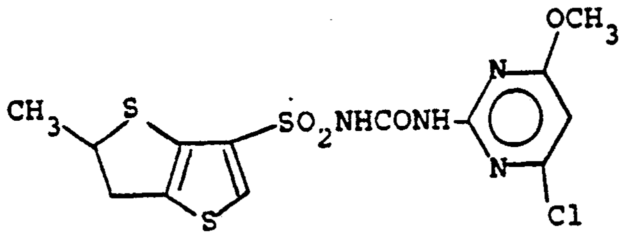

Compound 11

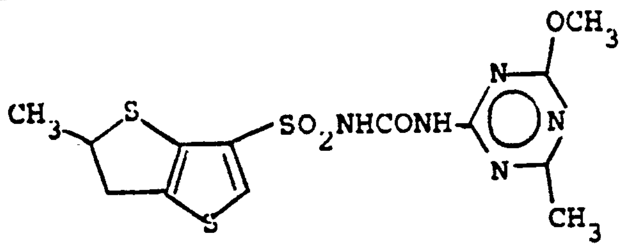

Compound 12

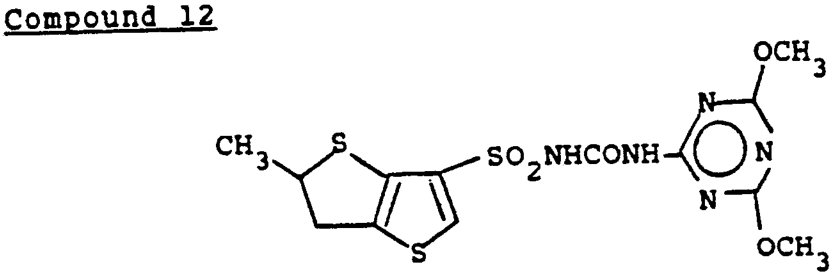

Compound 13

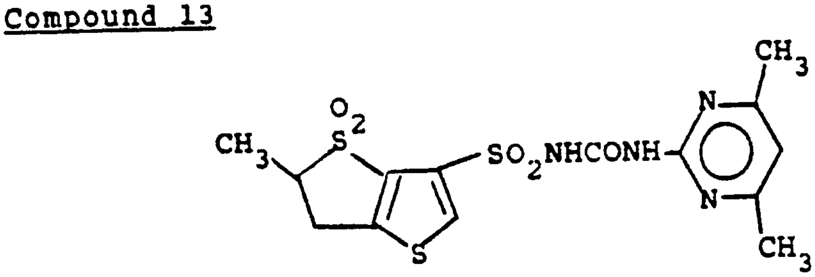

Compounds (continued)

Compound 14

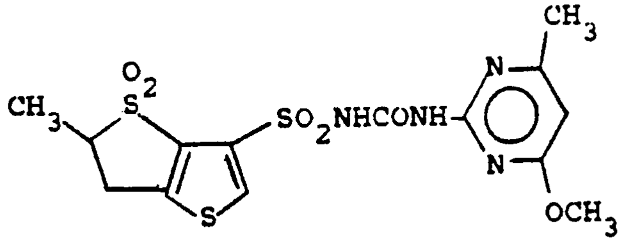

Compound 15

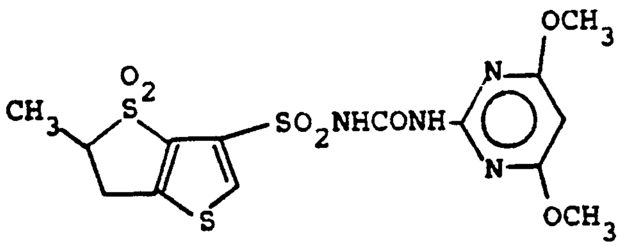

Compound 16

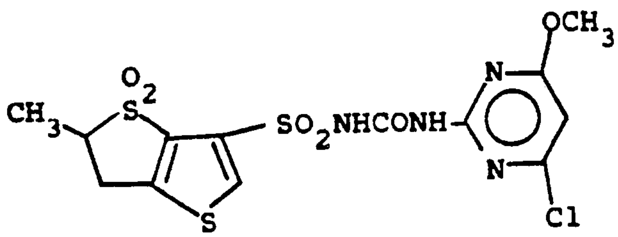

Compound 17

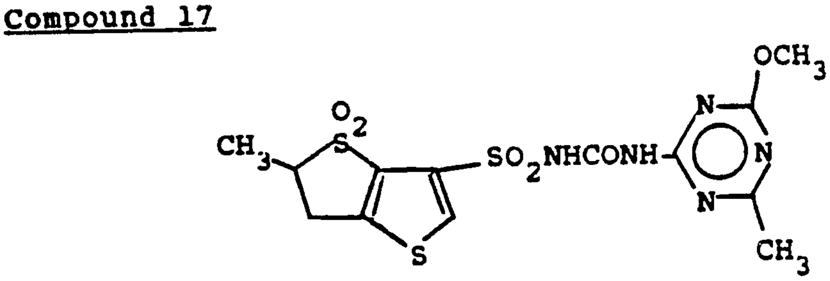

Compounds (continued)

Compound 18

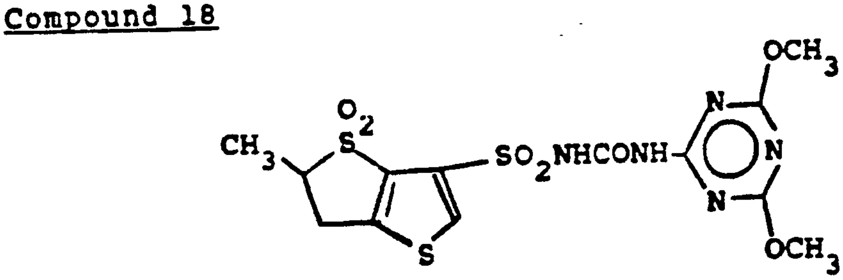

Compound 19

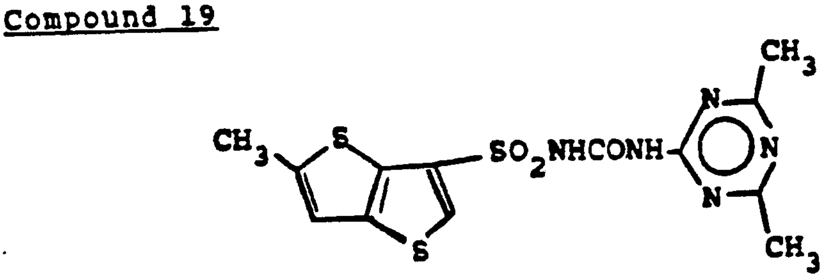

Compound 20

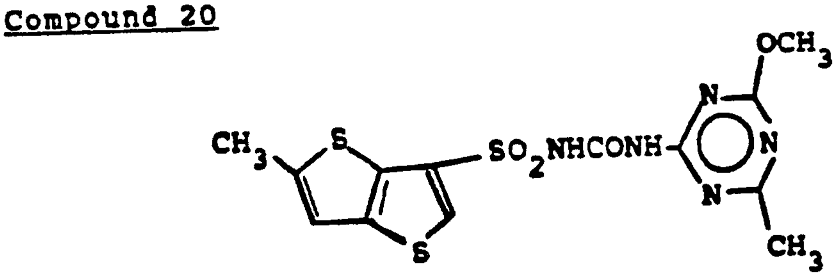

Compound 21

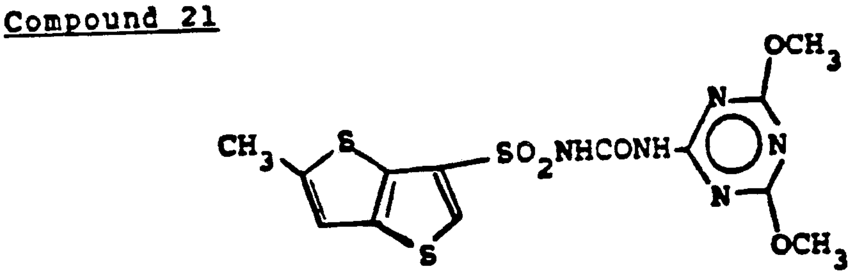

Compounds (continued)

Compound 22

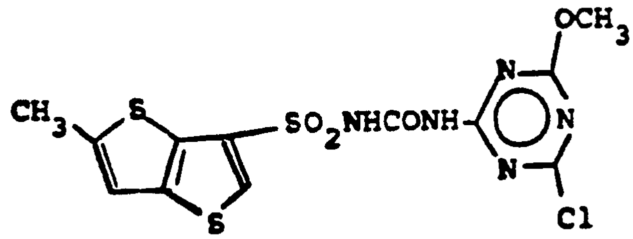

Compound 23

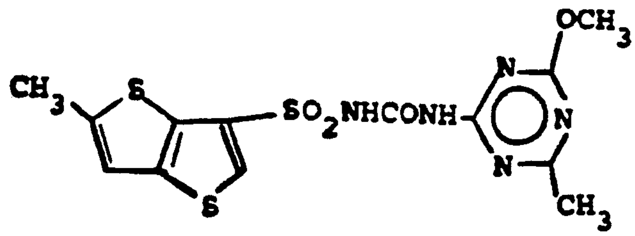

Compound 24

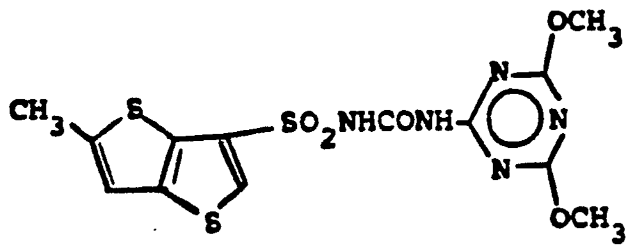

Test A

Seeds of crabgrass (Digitaria sp.), barnyardgrass (Echinochloa crusgalli), wild oats (Avena fatua), sicklepod (Cassia obtusifolia), morningglory (Ipomoea sp.), cocklebur (Xanthium pensylvanicum), sorghum, corn, soybean, cotton, sugar beet, rice, wheat and purple nutsedge (Cyperus rotundus) tubers were planted in a growth medium and treated pre-emergence with the chemicals dissolved in a non-phytotoxic solvent. It will be noted in the data that in some instances velvetleaf (Abutilon theophrasti) has been substituted for sicklepod (Cassia obtusifolia). At the same time, these crop and weed species were treated with a soil/foliage application. At the time of treatment, the plants ranged in height from 2 to 18 cm. Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

C = chlorosis or necrosis;
E = emergence inhibition;
G = growth retardation;
H = formative effect; and
U = unusual pigmentation.

It will be understood that treated plants sometimes exhibited more than one effect, in which case each effect was rated independently.

Table A

| | Compound 1 | Cmpd. 2 | Cmpd. 3 |
|---|---|---|---|
| Rate kg/ha | 2.0 | 0.05 | 0.05 |
| POST-EMERGENCE | | | |
| Cotton | 2C,9G | 4C,8H | 2C,9H |
| Morningglory | 1C | 4C,8H | 4C,8H |
| Cocklebur | 1C,2G | 4C,8H | 3C,8G |
| Sicklepod | 0 | 4C,6H | 4C,8H |
| Nutsedge | 0 | 0 | 2C,5G |
| Crabgrass | 0 | 2C,4H | 2C,7G |
| Barnyardgrass | 1H | 5C,9H | 5C,9H |
| Wild Oats | 1C,5G | 4C,9G | 5C,9G |
| Wheat | 2C,8G | 4C,9G | 5C,9G |
| Corn | 0 | 3U,9G | 4U,9G |
| Soybean | 0 | 4C,8G | 4H,9G |
| Rice | 5G | 5C,9G | 9C |
| Sorghum | 1C | 4C,9H | 4C,9H |
| Sugar beet | 2H | 4C,8H | 9C |
| PRE-EMERGENCE | | | |
| Morningglory | 2C | 1H | 7H |
| Cocklebur | 9G | 3H | 8H |
| Sicklepod | 2H | 0 | 7G |
| Nutsedge | 2C,5G | 0 | 0 |
| Crabgrass | 0 | 0 | 3G |
| Barnyardgrass | 0 | 0 | 2C |
| Wild Oats | 2C,7G | 0 | 4C,9G |
| Wheat | 8G | 0 | 9C |
| Corn | 2C,9H | 2C,6G | 3C,9H |
| Soybean | 2C | 2C | 8H |
| Rice | 3C,7H | 2G | 10E |
| Sorghum | 3C,8G | 3C,4G | 4C,9H |
| Sugar beet | 4C,8H | 2C | 2C,9G |
| Cotton | 6G | 2C | 9G |

Table A (continued)

| | Cmpd. 4 | Cmpd. 5 | Cmpd. 6 |
|---|---|---|---|
| Rate kg/ha | 2.0 | 2.0 | 0.05 |
| POST-EMERGENCE | | | |
| Cotton | 2C,7H | 1C,2G | 5G |
| Morningglory | 0 | 2G | 3G |
| Cocklebur | 0 | 3G | 5G |
| Sicklepod | 0 | 1C | 0 |
| Nutsedge | 0 | 0 | 0 |
| Crabgrass | 1C,3G | 1C,3G | 2G |
| Barnyardgrass | 3C,8H | 4C,9H | 9H |
| Wild Oats | 4C,9G | 5C,9G | 0 |
| Wheat | 4C,9G | 4C,9G | 0 |
| Corn | 2C,6H | 2U,9H | 2C,8H |
| Soybean | 2C,4H | 2H,5G | 2G |
| Rice | 4C,9G | 3C,9G | 5C,9G |
| Sorghum | 3C,7H | 3C,9H | 2C,9H |
| Sugar beet | 0 | 1C,3H | 5G |
| PRE-EMERGENCE | | | |
| Morningglory | 0 | 1C | 3C,5G |
| Cocklebur | 2H | 2H | 0 |
| Sicklepod | 0 | 3G | 0 |
| Nutsedge | 0 | 0 | 0 |
| Crabgrass | 0 | 0 | 0 |
| Barnyardgrass | 0 | 0 | 0 |
| Wild Oats | 2C,5G | 8G | 0 |
| Wheat | 7G | 1C,7G | 0 |
| Corn | 2C,8G | 3C,8H | 2G |
| Soybean | 0 | 1H | 0 |
| Rice | 2C,9G | 2C,5G | 5G |
| Sorghum | 2C | 0 | 2C,9H |
| Sugar beet | 0 | 0 | 0 |
| Cotton | 0 | 2G | 1C |

Table A (continued)

| | Cmpd. 7 | Cmpd. 8 |
|---|---|---|
| Rate (kg/ha) | 0.05 | 0.05 |
| POSTEMERGENCE | | |
| Morningglory | 4C, 8H | 5C, 9G |
| Cocklebur | 4C, 8H | 9C |
| Velvetleaf | 4C, 9G | 9C |
| Nutsedge | 2C, 8G | 4C, 8G |
| Crabgrass | 3C, 7G | 9C |
| Barnyardgrass | 3C, 7H | 9C |
| Wild Oats | 3C, 5G | 4C, 8G |
| Wheat | 2C, 6G | 4C, 9G |
| Corn | 3C, 9H | 10C |
| Soybean | 4C, 8H | 5C, 9G |
| Rice | 5C, 9G | 9C |
| Sorghum | 3C, 7G | 9C |
| Cheatgrass | 4C, 9G | 9C |
| Sugar Beets | 9C | 10C |
| Cotton | 4C, 9G | 10C |
| PREEMERGENCE | | |
| Morningglory | 7H | 9G |
| Cocklebur | -- | 8H |
| Velvetleaf | 7G | 10E |
| Nutsedge | 5G | 8G |
| Crabgrass | 2C, 5G | 4C, 8G |
| Barnyardgrass | 0 | 2C, 6H |
| Wild Oats | 0 | 0 |
| Wheat | 0 | 2G |
| Corn | 3C, 8H | 4C, 9G |
| Soybean | 2C, 5G | 4C, 7G |
| Rice | 3C, 7G | 10E |
| Sorghum | 3C, 8H | 4C, 9H |
| Cheatgrass | 2C, 7G | 4C, 9H |
| Sugar Beets | 8G | 4C, 9G |
| Cotton | 8G | 9G |

Table A (continued)

| | Cmpd. 9 | Cmpd. 10 |
|---|---|---|
| Rate (kg/ha) | 0.05 | 0.05 |
| **POSTEMERGENCE** | | |
| Morningglory | 10C | 4C, 9G |
| Cocklebur | 10C | 10C |
| Velvetleaf | 9C | 4C, 8G |
| Nutsedge | 9C | 0 |
| Crabgrass | 9C | 9C |
| Barnyardgrass | 10C | 9C |
| Wild Oats | 9C | 1C |
| Wheat | 10C | 2C, 4G |
| Corn | 9C | 5C, 9G |
| Soybean | 5C, 9G | 4C, 9G |
| Rice | 9C | 9C |
| Sorghum | 10C | 9C |
| Cheatgrass | 9C | 3C, 7G |
| Sugar Beets | 9C | 9C |
| Cotton | 9C | 5C, 9G |
| **PREEMERGENCE** | | |
| Morningglory | 9C | 9H |
| Cocklebur | 8H | 9H |
| Velvetleaf | 5C, 9G | 2C, 8G |
| Nutsedge | 10E | 3C, 5G |
| Crabgrass | 4C, 8G | 3C, 7G |
| Barnyardgrass | 4C, 9H | 3C, 9H |
| Wild Oats | 4C, 8G | 1C |
| Wheat | 10H | 1C |
| Corn | 4C, 9H | 2C, 9H |
| Soybean | 3C, 7G | 3C, 7G |
| Rice | 10E | 10E |
| Sorghum | 4C, 9H | 5C, 9H |
| Cheatgrass | 5C, 9H | 3C, 8G |
| Sugar Beets | 9C | 5C, 9G |
| Cotton | 9G | 9G |

Table A (continued)

| | Cmpd. 11 | Cmpd. 12 |
|---|---|---|
| Rate (kg/ha) | 0.05 | 0.05 |
| POSTEMERGENCE | | |
| Morningglory | 2C, 6G | 3G |
| Cocklebur | 2C, 9H | 0 |
| Velvetleaf | 4C, 9G | 4C, 9G |
| Nutsedge | 2C, 5G | 0 |
| Crabgrass | 0 | 0 |
| Barnyardgrass | 1C | 0 |
| Wild Oats | 0 | 0 |
| Wheat | 0 | 0 |
| Corn | 3C, 9G | 2C, 6H |
| Soybean | 4C, 9G | 4C, 9G |
| Rice | 4C, 9G | 2C, 8G |
| Sorghum | 3C, 7H | 2C, 4G |
| Cheatgrass | 1C | 0 |
| Sugar Beets | 9C | 3C, 7H |
| Cotton | 5C, 9G | 10C |
| PREEMERGENCE | | |
| Morningglory | 2C, 6G | 5G |
| Cocklebur | 6H | 0 |
| Velvetleaf | 6G | 2G |
| Nutsedge | 0 | 0 |
| Crabgrass | 0 | 0 |
| Barnyardgrass | 0 | 0 |
| Wild Oats | 0 | 0 |
| Wheat | 0 | 0 |
| Corn | 7G | 2C, 2G |
| Soybean | 2C, 4H | 0 |
| Rice | 3G | 2C, 5G |
| Sorghum | 0 | 0 |
| Cheatgrass | 0 | 0 |
| Sugar Beets | 8H | 6G |
| Cotton | 9G | 9G |

Table A (continued)

| | Cmpd. 13 | Cmpd. 14 |
|---|---|---|
| Rate (kg/ha) | 0.05 | 0.05 |
| POSTEMERGENCE | | |
| Morningglory | 9C | 10C |
| Cocklebur | 10C | 10C |
| Velvetleaf | 9C | 9C |
| Nutsedge | 9C | 9C |
| Crabgrass | 9C | 10C |
| Barnyardgrass | 9C | 9C |
| Wild Oats | 6C, 9G | 9C |
| Wheat | 9C | 9C |
| Corn | 10C | 10C |
| Soybean | 6C, 9G | 5C, 9G |
| Rice | 6C, 9G | 6C, 9G |
| Sorghum | 9C | 10C |
| Cheatgrass | 6C, 9G | 5C, 9G |
| Sugar Beets | 9C | 9C |
| Cotton | 9C | 9C |
| PREEMERGENCE | | |
| Morningglory | 9G | 9G |
| Cocklebur | 9H | 9H |
| Velvetleaf | 5C, 9G | 5C, 9G |
| Nutsedge | 10E | 10E |
| Crabgrass | 5C, 9G | 9C |
| Barnyardgrass | 3H | 2C, 9H |
| Wild Oats | 2C, 8G | 4C, 9H |
| Wheat | 9G | 7C, 9H |
| Corn | 3C, 9G | 10H |
| Soybean | 3C, 7H | 9H |
| Rice | 10E | 10E |
| Sorghum | 5C, 9H | 6C, 9H |
| Cheatgrass | 10E | 10E |
| Sugar Beets | 9C | 5C, 9G |
| Cotton | 9G | 9G |

Table A (continued)

| | Cmpd. 15 | Cmpd. 16 |
|---|---|---|
| Rate (kg/ha) | 0.05 | 0.05 |
| POSTEMERGENCE | | |
| Morningglory | 10C | 9C |
| Cocklebur | 10C | 10C |
| Velvetleaf | 9C | 9C |
| Nutsedge | 10C | 5C, 9G |
| Crabgrass | 10C | 9C |
| Barnyardgrass | 9C | 9C |
| Wild Oats | 6C, 9G | 2C, 5G |
| Wheat | 9C | 2C, 5G |
| Corn | 10C | 10C |
| Soybean | 6C, 9G | 4C, 9H |
| Rice | 9C | 9C |
| Sorghum | 9C | 10C |
| Cheatgrass | 9C | 5C, 9G |
| Sugar Beets | 9C | 9C |
| Cotton | 9C | 9C |
| PREEMERGENCE | | |
| Morningglory | 9C | 9H |
| Cocklebur | 9H | 9H |
| Velvetleaf | 10C | 5C, 9G |
| Nutsedge | 10E | 10E |
| Crabgrass | 9C | 4C, 8G |
| Barnyardgrass | 3C, 9H | 5C, 9H |
| Wild Oats | 4C, 9H | 7G |
| Wheat | 7C, 9H | 6G |
| Corn | 5C, 9H | 5C, 9H |
| Soybean | 3C, 8H | 4C, 7H |
| Rice | 10E | 10E |
| Sorghum | 7C, 9H | 10H |
| Cheatgrass | 10E | 9G |
| Sugar Beets | 5C, 9G | 9C |
| Cotton | 9C | 9G |

Table A (continued)

| | Cmpd. 17 | Cmpd. 18 |
|---|---|---|
| Rate (kg/ha) | 0.05 | 0.05 |
| POSTEMERGENCE | | |
| Morningglory | 5G | 2G |
| Cocklebur | 2H, 7G | 4G |
| Velvetleaf | 3C, 8H | 3H |
| Nutsedge | 4G | 5G |
| Crabgrass | 2G | 0 |
| Barnyardgrass | 3C, 9H | 3C, 8H |
| Wild Oats | 4C, 9G | 5C, 9G |
| Wheat | 3C, 9G | 5C, 9G |
| Corn | 5C, 9G | 9C |
| Soybean | 2H, 3G | 3H, 5G |
| Rice | 5C, 9G | 5C, 9G |
| Sorghum | 5C, 9H | 4C, 9H |
| Cheatgrass | 3C, 8G | 2C, 7G |
| Sugar Beets | 3C, 6H | 3C, 7G |
| Cotton | 3C, 5G | 1C |
| PREEMERGENCE | | |
| Morningglory | 5G | 7G |
| Cocklebur | -- | 5G |
| Velvetleaf | 0 | 2C |
| Nutsedge | 0 | 0 |
| Crabgrass | 0 | 0 |
| Barnyardgrass | 0 | 0 |
| Wild Oats | 2C, 8G | 2C, 8G |
| Wheat | 8G | 5G |
| Corn | 4C, 9G | 4C, 9H |
| Soybean | 0 | 0 |
| Rice | 9H | 9H |
| Sorghum | 2C, 8H | 3C, 7H |
| Cheatgrass | 3G | 3G |
| Sugar Beets | 6G | 3C, 7G |
| Cotton | 0 | 5G |

Table A (continued)

| | Cmpd. 19 | Cmpd. 20 |
|---|---|---|
| Rate (kg/ha) | 0.05 | 0.05 |
| **POSTEMERGENCE** | | |
| Morningglory | 4G | 5C, 9G |
| Cocklebur | 2C, 8G | 9C |
| Velvetleaf | 4C, 9H | 10C |
| Nutsedge | 4G | 2C, 8G |
| Crabgrass | 2G | 2G |
| Barnyardgrass | 2H | 3C, 8H |
| Wild Oats | 0 | 1C |
| Wheat | 0 | 0 |
| Corn | 2G | 2C, 8H |
| Soybean | 2C, 6H | 5C, 9G |
| Rice | 7G | 4C, 9G |
| Sorghum | 2G | 2C, 8H |
| Cheatgrass | 0 | 4G |
| Sugar Beets | 4C, 9H | 9C |
| Cotton | 7G | 9C |
| **PREEMERGENCE** | | |
| Morningglory | 2C, 5G | 9G |
| Cocklebur | 5H | 3C, 8G |
| Velvetleaf | 5G | 9C |
| Nutsedge | 0 | 9G |
| Crabgrass | 0 | 5G |
| Barnyardgrass | 0 | 3C, 8H |
| Wild Oats | 0 | 2C, 5G |
| Wheat | 0 | 5G |
| Corn | 4G | 2C, 8G |
| Soybean | 2C, 2H | 3C, 7H |
| Rice | 2C, 7G | 4C, 9H |
| Sorghum | 2C | 4C, 8H |
| Cheatgrass | 5G | 5G |
| Sugar Beets | 9G | 10C |
| Cotton | 8G | 9G |

Table A (continued)

| | Cmpd. 21 | Cmpd. 22 |
|---|---|---|
| Rate (kg/ha) | 0.05 | 0.05 |
| POSTEMERGENCE | | |
| Morningglory | 5C, 9G | 2C, 8G |
| Cocklebur | 2C, 6G | 4G |
| Velvetleaf | 10C | 8G |
| Nutsedge | 10C | 2C, 6G |
| Crabgrass | 5G | 0 |
| Barnyardgrass | 2H | 0 |
| Wild Oats | 0 | 0 |
| Wheat | 0 | 0 |
| Corn | 0 | 0 |
| Soybean | 5C, 9G | 5H |
| Rice | 3C, 8G | 4G |
| Sorghum | 2H | 0 |
| Cheatgrass | 4G | 0 |
| Sugar Beets | 10C | 2C, 7G |
| Cotton | 9C | 4C, 9G |
| PREEMERGENCE | | |
| Morningglory | 9G | 8G |
| Cocklebur | 7G | 5H |
| Velvetleaf | 9G | 6G |
| Nutsedge | 10E | 0 |
| Crabgrass | 2C, 4G | 0 |
| Barnyardgrass | 3C, 7H | 0 |
| Wild Oats | 0 | 0 |
| Wheat | 2G | 0 |
| Corn | 9G | 5G |
| Soybean | 7H | 3G |
| Rice | 9H | 5G |
| Sorghum | 4C, 9H | 5G |
| Cheatgrass | 6G | 2G |
| Sugar Beets | 10E | 8G |
| Cotton | 9G | 7G |

Table A (continued)

| | Cmpd. 23 | Cmpd. 24 |
|---|---|---|
| Rate (kg/ha) | 0.05 | 0.05 |
| POSTEMERGENCE | | |
| Morningglory | 9C | 10C |
| Cocklebur | 9C | 2G |
| Velvetleaf | 9C | 10C |
| Nutsedge | 5G | 4G |
| Crabgrass | 0 | 0 |
| Barnyardgrass | 2C, 6H | 0 |
| Wild Oats | 0 | 0 |
| Wheat | 0 | 0 |
| Corn | 2C, 9H | 2C, 3H |
| Soybean | 5C, 9G | 5C, 9G |
| Rice | 4C, 9G | 5C, 9G |
| Sorghum | 3C, 8H | 2C, 5H |
| Cheatgrass | 4G | 0 |
| Sugar Beets | 9C | 5C, 9G |
| Cotton | 9C | 5C, 9G |
| PREEMERGENCE | | |
| Morningglory | 9G | 9G |
| Cocklebur | 9H | 6G |
| Velvetleaf | 9C | 2C, 9G |
| Nutsedge | 5G | 5G |
| Crabgrass | 0 | 0 |
| Barnyardgrass | 2G | 3H |
| Wild Oats | 0 | 2G |
| Wheat | 0 | 0 |
| Corn | 2C, 8G | 2C, 7G |
| Soybean | 3C, 7H | 3C, 5H |
| Rice | 10E | 9H |
| Sorghum | 3C, 8H | 2C, 6H |
| Cheatgrass | 3G | 0 |
| Sugar Beets | 9C | 9C |
| Cotton | 9G | 9G |

Test B

Post-emergence

Two round pans (25 cm diameter by 12.5 cm deep) were filled with Woodstown sandy loam soil. One pan was planted with blackgrass (Alopecurus myosuroides), sugar beets, nutsedge (Cyperus rotundus) tubers, crabgrass (Digitaria sanguinalis), sicklepod (Cassia obtusifolia), teaweed (Sida spinosa), jimsonweed (Datura stramonium), velvetleaf (Abutilon theophrasti), and giant foxtail (Setaria faberii). The other pan was planted with wheat, cotton, rice, corn, soybean, wild oats (Avena fatua), cocklebur (Xanthium pensylvanicum), morningglory (Ipomoea hederacea), johnsongrass (Sorghum halepense) and barnyardgrass (Echinochloa crusgalli). The plants were grown for approximately fourteen days, then sprayed post-emergence with the chemicals dissolved in a non-phytotoxic solvent.

Pre-emergence

Two round pans (25 cm diameter by 12.5 cm deep) were filled with Woodstown sandy loam soil. One pan was planted with blackgrass (Alopecurus myosuroides), sugar beets, nutsedge, crabgrass, sicklepod, teaweed, jimsonweed, velvetleaf, and giant foxtail. The other pan was planted with wheat, cotton, rice, corn, soybean, wild oats, cocklebur, morningglory johnsongrass and barnyardgrass. The two pans were sprayed pre-emergence with the chemicals dissolved in a non-phytotoxic solvent.

Treated plants and controls were maintained in the greenhouse for 28 days, then all treated plants were compared to controls and visually rated for plant response.

Response ratings are based on a scale of 0 to 10: where 0 = no effect, and 10 = complete control. The type of response is indicated by letters where G = growth retardation and C = chlorosis and/or necrosis.

Response ratings are contained in Table B.

Table B

Compound Number 2

Post-Emergence

| Rate g/ha | 4 | 16 | 62 |
|---|---|---|---|
| Corn | 3G | 5G | 9G |
| Wheat | 0 | 0 | 6G |
| Rice | 0 | 3G | 10G |
| Soybean | 0 | 3G | 5G |
| Cotton | 0 | 0 | 4G |
| Sugar beet | 0 | 2G | 5G |
| Crabgrass | 2G | 4G | 5G |
| Johnsongrass | 0 | 0 | 3G |
| Blackgrass | 0 | 0 | 3G |
| Barnyardgrass | 0 | 0 | 0 |
| Nutsedge | 0 | 0 | 0 |
| Giant foxtail | 0 | 0 | 4G |
| Wild Oats | 0 | 3G | 8G |
| Cocklebur | 0 | 0 | 0 |
| Morningglory | 0 | 0 | 3G |
| Teaweed | 0 | 3G | 7G |
| Sicklepod | 2G | 5G | 9G |
| Jimsonweed | 0 | 2G | 4G |
| Velvetleaf | 0 | 2G | 6G |

Table B

Compound Number 3

| | Pre-Emergence | | Post-Emergence | | |
|---|---|---|---|---|---|
| Rate g/ha | 16 | 62 | 4 | 16 | 62 |
| Corn | 0 | 0 | 7G | 10C | 10C |
| Wheat | 0 | 0 | 3G | 8C | 10C |
| Rice | 3G | 9G | 8G | 10G | 10C |
| Soybean | 0 | 2G | 2G | 8G | 10G |
| Cotton | 0 | 0 | 0 | 2G | 8G |
| Sugar beet | 0 | 5G | 5G | 9G | 10G |
| Crabgrass | 0 | 5G | 0 | 6G | 9G |
| Johnsongrass | 3G | 9G | 3G | 9G | 10C |
| Blackgrass | 4G | 8G | 3G | 9C | 10C |
| Barnyardgrass | 0 | 0 | 0 | 3G | 10C |
| Nutsedge | 4G | 9G | 0 | 0 | 0 |
| Giant foxtail | 5G | 8G | 0 | 3G | 10G |
| Wild Oats | 0 | 3G | 3G | 8G | 10C |
| Cocklebur | 0 | 0 | 0 | 3G | 9G |
| Morningglory | 0 | 0 | 0 | 2G | 8G |
| Teaweed | 0 | 0 | 2G | 7G | 8G |
| Sicklepod | 0 | 0 | 5G | 9G | 10G |
| Jimsonweed | 0 | 0 | 3G | 6G | 7G |
| Velvetleaf | 0 | 0 | 3G | 8G | 9G |

## Claims

1. A compound of the formula:

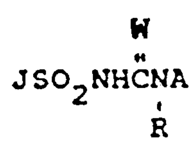

I

wherein
J is

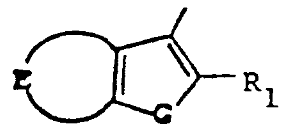 or 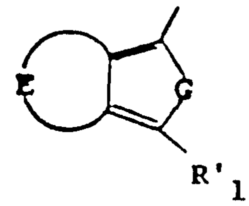

E is a bridge of 3 to 4 atoms, containing 0-2 heteroatoms selected from the group consisting of oxygen, sulfur and nitrogen and also containing 1 to 4 atoms of carbon, said bridge together with the two carbon attachment sites forming a non-aromatic 5 to 6 membered carbocyclic ring, an aromatic 5 to 6 membered heterocyclic ring, or a non-aromatic 5 to 6 membered heterocyclic ring, with the proviso that two oxygen atoms must be separated by at least one atom of carbon and that oxygen and sulfur are only linked to each other if the sulfur is in the form of -SO- or $-SO_2-$;
G is O, S or N-R;
W is O or S;
R is H or $CH_3$;
$R'_1$ is H, $CH_3$ or Cl when G is S and is H when G is O or N-R;
$R_1$ is H, $CH_3$, $OCH_3$, Cl, Br, $SCH_3$, $SO_2CH_3$ or $NO_2$; and
A is

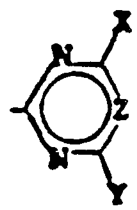 , 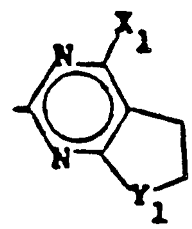 , 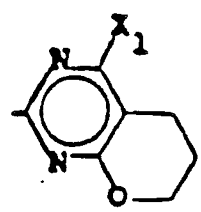

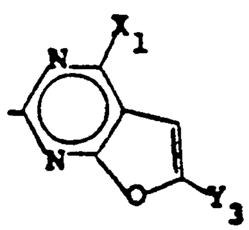 , 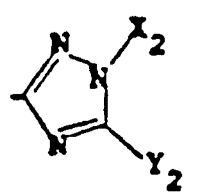 or 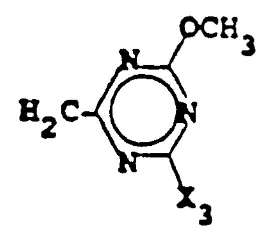 ;

A-4 A-5 A-6

wherein
X is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkythio, halogen, $C_2$-$C_5$ alkoxyalkoxy, amino, $C_1$-$C_3$ alkylamino or di($C_1$-$C_3$ alkyl)amino;
Y is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino di($C_1$-$C_3$ alkyl)amino, $C_3$-$C_4$ alkenyloxy, $C_3$-$C_4$ alkylnyloxy, $C_2$-$C_5$ alkylthioalkyl, $C_1$-$C_4$ haloalkyl, $C_3$-$C_5$ cycloalkyl, $C_2$-$C_4$ alkynyl;

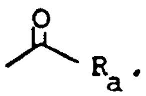 , 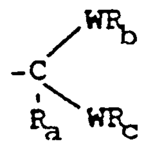 , 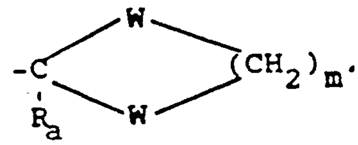 ,

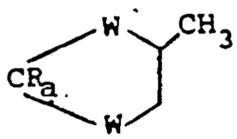

or $N(OCH_3)CH_3$ ;
W is O or S;
m is 2 or 3;
$R_a$ is H or $CH_3$;
$R_b$ is $C_1$-$C_2$ alkyl;
$R_c$ is $C_1$-$C_2$ alkyl;
Z is CH, N, $CCH_3$, $CC_2H_5$, CCl or CBr;
$Y_1$ is O or $CH_2$;
$X_1$ is $CH_3$, $OCH_3$, $OC_2H_5$ or $OCF_2H$;
$X_2$ is $CH_3$, $C_2H_5$ or $CH_2CF_3$;
$Y_2$ is $OCH_3$, $OC_2H_5$, $SCH_3$, $SC_2H_5$, $OCF_2H$, $SCF_2H$, $CH_3$ or $CH_2CH_3$;
$X_3$ is $CH_3$ or $OCH_3$; and $Y_3$ is H or $CH_3$;
provided that

a) when W is S, then R is H, A is A-1, Z is CH or N, and Y is $CH_3$, $OCH_3$, $OC_2H_5$, $CH_2OCH_3$, $C_2H_5$, $CF_3$, $SCH_3$, $OCH_2CH=CH_2$, $OCH_2C≡CH$, $OCH_2CH_2OCH_3$, $CH(OCH_3)_2$, $OCH_2CF_3$ or

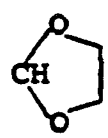 ;

c) when X is Cl, F or Br, then Z is CH and Y is $OCH_3$, $OC_2H_5$, $NH_2$, $NHCH_3$ $N(CH_3)_2$ or $OCF_2H$;
g) when X or Y is $OCF_2H$, then Z is CH; and their agriculturally suitable salts.

**2.** A compound of the formula

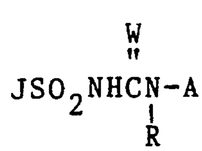

wherein J is

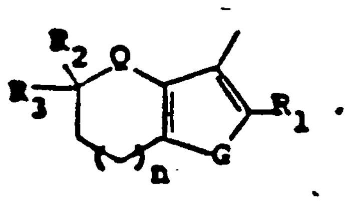

$J_1$

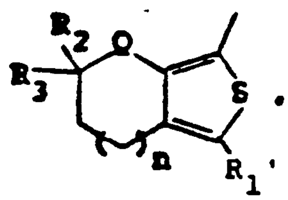

$J_2$

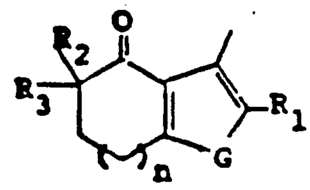

$J_3$

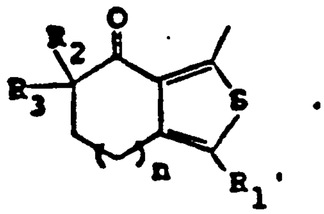

$J_4$

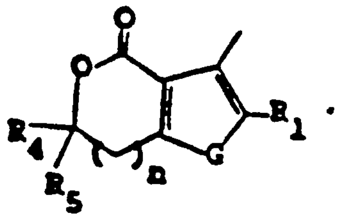

$J_5$

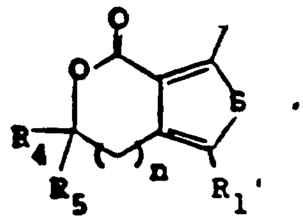

$J_6$

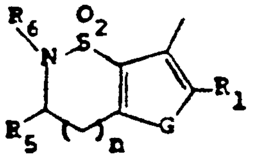

$J_7$

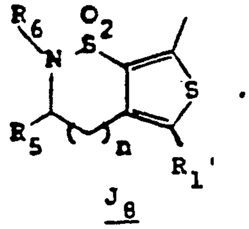

$J_8$

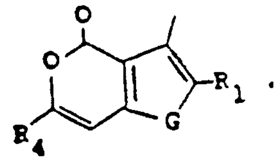

$J_9$

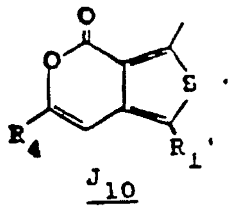

$J_{10}$

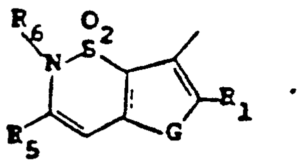

$J_{11}$

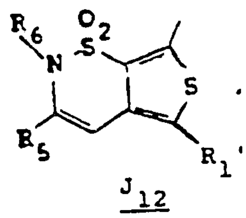

$J_{12}$

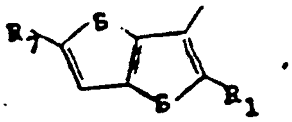

$J_{13}$

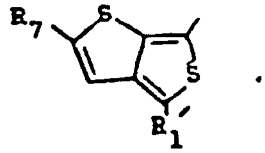

$J_{14}$

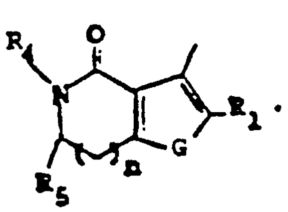

$J_{15}$

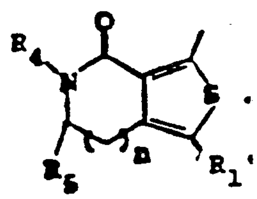

$J_{16}$

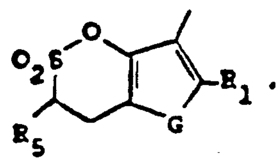

$J_{17}$

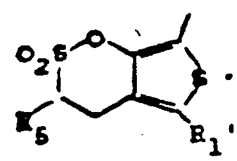

$J_{18}$

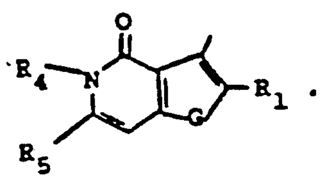

$J_{19}$

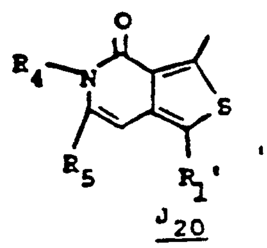

$J_{20}$

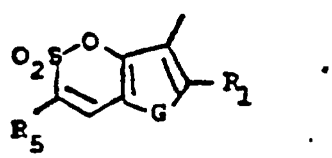

$J_{21}$

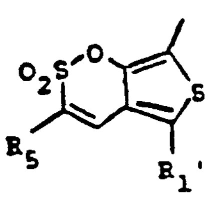

$J_{22}$

R is H or $CH_3$;
$R_1$' is H, $CH_3$ or Cl;
$R_1$ is H, $CH_3$, $OCH_3$, Cl, Br, $SCH_3$, $SO_2CH_3$, or $NO_2$;
$R_2$ is H, Cl or $C_1$-$C_4$ alkyl;
$R_3$ is H, Cl or $C_1$-$C_4$ alkyl;

$R_4$ is H or $C_1$-$C_4$ alkyl;
$R_5$ is H or $CH_3$;
$R_6$ is H, $R_8$, $SR_8$, $SO_2R_8$, $OR_8$, $C(O)R_8$, $CO_2R_8$, $NR_8R_4$, CN or $Si(CH_3)_2R_9$;
$R_7$ is H, $C_1$-$C_6$ alkyl, Cl, Br, CN, $NO_2$, $SR_9$, $SO_2R_9$, $CO_2R_9$ or $C(O)R_9$;
$R_8$ is $C_1$-$C_6$ alkyl, $C_2$-$C_6$ alkenyl, or $C_2$-$C_6$ alkynyl, each optionally substituted by one or more halogens and/or $R_{11}$, $C_2$-$C_6$ epoxyalkyl, $C_3$-$C_6$ cycloalkyl, $C_4$-$C_7$ cycloalkylalkyl, or

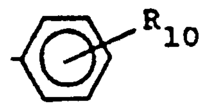 ;

$R_9$ is $C_1$-$C_4$ alkyl,

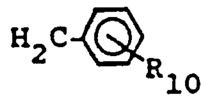 or 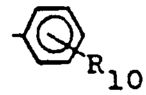

$R_{10}$ is H, F, Cl, Br, $CH_3$, $OCH_3$, CN, $NO_2$, $SCH_3$, $SO_2CH_3$ or $CF_3$;
$R_{11}$ is $OR_{12}$, $OC(O)R_{12}$, $OC(O)NR_9R_{12}$, $OSO_2R_{12}$, $OSi(CH_3)_2R_9$, $Si(CH_3)_2R_9$, $SR_{12}$, $SOR_{12}$, $SO_2R_{12}$, SCN, CN, $NO_2$, $C(O)R_{12}$, $C(O)OR_{12}$, $C(O)NR_4R_{12}$,

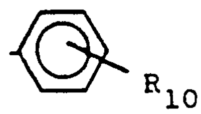 ;

or L;
$R_{12}$ is H, $C_1$-$C_6$ alkyl, $C_1$-$C_6$ haloalkyl, $C_2$-$C_6$ alkenyl, $C_2$-$C_6$ alkynyl, $C_3$-$C_6$ cycloalkyl or

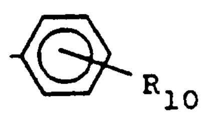

n is 0 or 1; Q is O, S, SO, $SO_2$, $CH_2$ or $CHCH_3$: and
L is a 5- or 6-membered aromatic heterocycle, a 5-or 6-membered dihydroaromatic heterocycle or a 5- or 6-membered tetrahydroaromatic heterocycle each containing 1-4 heteroatoms selected from the group consisting of 0-1 oxygen atoms, 0-1 sulfur atoms and 0-4 nitrogen atoms, also each optionally substituted with 1-4 $CH_3$, 1-2 $OCH_3$, 1 $SCH_3$, 1 Cl, 1 $N(CH_3)_2$ or 1 CN or L is a 5- or 6-membered lactone, lactam or cycloalkanone optionally substituted with 1-4 $CH_3$;
G is O, S or NR;
W is O or S;
$R_1$, $R'_1$ and A are as defined in claim 1;
said provisos a), c) and gl as defined in claim 1 apply; and provided further that
b)the total number of carbon atoms in $R_2$ and $R_3$ is less than or equal to 4;
d) when J is $J_1$ or $J_2$, then $R_2$ and $R_3$ are other than Cl;
e) the total number of carbons atoms in $R_4$ and $R_5$ is less than or equal to 4;
f) when J is $J_5$, $J_6$, $J_7$ or $J_8$ and n = O, then $R_4$ is H; and their agriculturally suitable salts.

**3.** A compound of claim 2 wherein J is

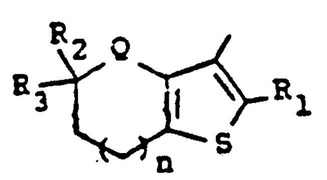,
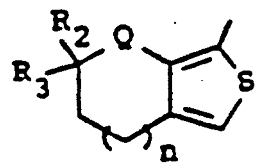,
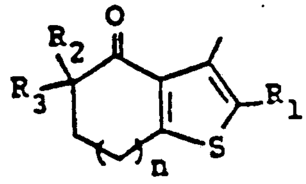

J1 J2 J3

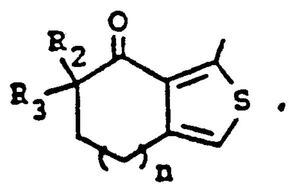,
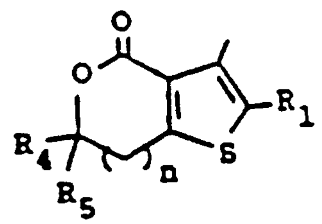,
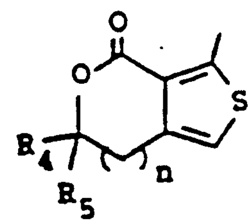

J4 J5 J6

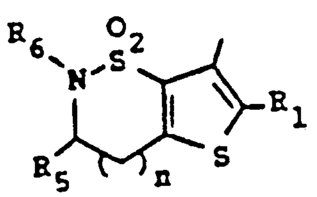,
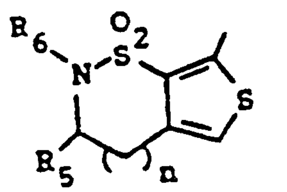,
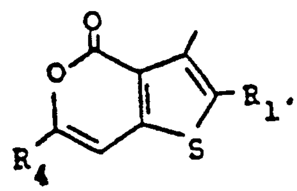,

J7 J8 J9

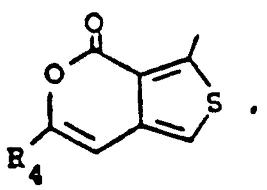,
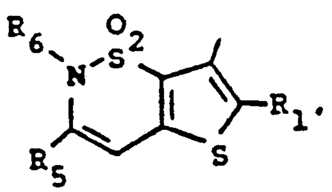,
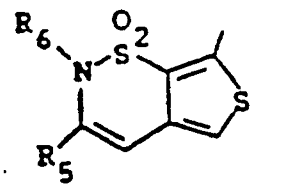,

J10 J11 J12

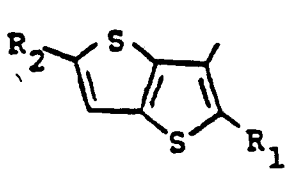

or

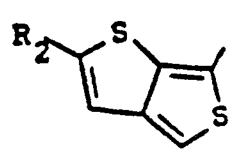

;

J13 J14

R is H or $CH_3$;
$R_1$ is H, $CH_3$, $OCH_3$, Cl or Br;
$R_2$ is H, Cl or $C_1$-$C_4$ alkyl;
$R_3$ is H, Cl or $C_1$-$C_4$ alkyl;
$R_4$ is H or $C_1$-$C_4$ alkyl;
$R_5$ is H or $CH_3$;
$R_6$ is H or $C_1$-$C_4$ alkyl;
Q is O, S, SO, $SO_2$, $CH_2$ or $CHCH_3$;
n is 0 or 1;
A is

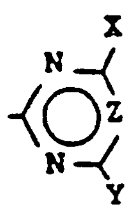 ,
A-1

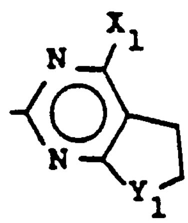 ,
A-2

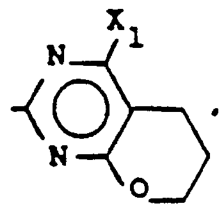 ,
A-3

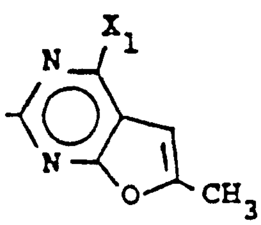 ,
A-4

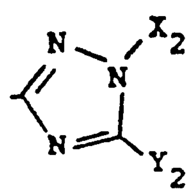 or
A-5

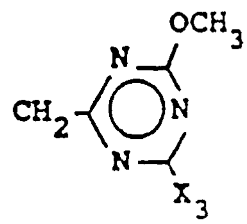 ;
A-6

X is $CH_3$, $OCH_3$, $OCH_2CH_3$, Cl, F, Br, $CH_2F$, $OCF_2H$ or $CF_3$;
Y is H, $CH_3$, $OCH_3$, $OC_2H_5$, $CH_2OCH_3$, $NH_2$, $NHCH_3$, $N(CH_3)_2$, $C_2H_5$, $CF_3$, $SCH_3$, $OCH_2CH=CH_2$, $OCH_2C{\equiv}CH$, $OCH_2CF_3$, CN, $N_3$, $OCH_2CH_2OCH_3$, $CH_2SCH_3$, $CR_a(WCH_3)_2$,

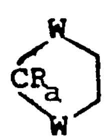 , 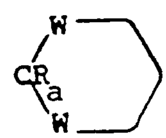 ,

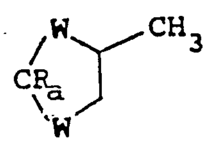 ,

$CR_a(WCH_2CH_3)_2$, or $WCF_2T$
wherein W is O or S, $R_a$ is H or $CH_3$ and T is H, CHClF, CHBrF or $CHFCF_3$;
Z is CH, N, $CCH_3$, $CC_2H_5$, CCl or CBr;
$Y_1$ is O or $CH_2$;
$X_1$ is $CH_3$, $OCH_3$, $OC_2H_5$ or $OCF_2H$;
$X_2$ is $CH_3$, $C_2H_5$ or $CH_2CF_3$;

$Y_2$ is $OCH_3$, $OC_2H_5$, $SCH_3$, $SC_2H_5$, $CH_3$ or $CH_2CH_3$;
$X_3$ is $CH_3$ or $OCH_3$;
and agriculturally suitable salts thereof.

**4.** A compound of Claim 3 wherein R is H, W is O, and G is O or S.

**5.** A compound of Claim 2 wherein R is H, W is is O and G is O or S.

**6.** A compound of Claim 5 wherein A is A-1, X is $CH_3$, $OCH_3$, Cl, Br, $OCH_2CF_3$ or $OCF_2H$; Y is $C_1$-$C_3$ alkyl, cyclopropyl, $C{\equiv}CH$, $OCH_3$, $OC_2H_5$, $CH_2OCH_3$, $NH_2$, $NHCH_3$, $N(CH_3)_2$, $C_2H_5$, $CF_3$, $SCH_3$, $OCH_2CH=CH_2$, $OCH_2C{\equiv}CH$, $OCH_2CH_2OCH_3$, $CR_a(OCH_3)_2$,

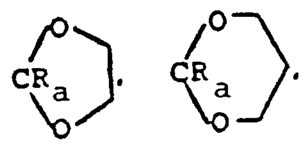

$CH_a(OCH_2CH_3)_2$ or $OCF_2H$; and Z is CH or N.

**7.** A compound of Claim 6 wherein $R_1$ is H, Cl, Br or $CH_3$; $R_2$, $R_3$ and $R_4$ are H or $C_1$-$C_3$ alkyl; $R_6$ is H, $R_8$, $C(O)R_8$ or $CO_2R_8$; $R_7$ is Cl, Br, $CO_2CH_3$ or $CO_2CH_2CH_3$; $R_8$ is $C_1$-$C_6$ alkyl, $C_1$-$C_6$ haloalkyl, $C_2$-$C_6$ alkenyl, $C_2$-$C_6$ alkynyl, $C_3$-$C_6$ cycloalkyl or $C_4$-$C_7$ cycloalkylalkyl and $R_{11}$ is $OCH_3$, $OCH_2CH_3$, CN, $CO_2(C_1$-$C_4$ alkyl), OH or $C(O)CH_3$.

**8.** A compound of Claim 7 wherein G is S, Y is $CH_3$ or $OCH_3$ and X is $CH_3$, $OCH_3$, Cl, or Br.

**9.** A compound of Claim 8 where J is J-1.

**10.** A compound of Claim 8 where J is J-3.

**11.** A compound of Claim 8 where J is J-5.

**12.** A compound of Claim 8 where J is J-7.

**13.** A compound of Claim 8 where J is J-9.

**14.** A compound of Claim 8 where J is J-11.

**15.** A compound of Claim 8 where J is J-13.

**16.** The compound of Claim 1 which is N-[(4-methoxy-6-methylpyrimidin-2-yl)aminocarbonyl]-6,7-dihydro-5H-thieno-[3,2-B]thiopyran-3-sulfonamide, 4.4-dioxide.

**17.** The compound of Claim 1 which is N-[(4,6-dimethoxypyrimidin-2-yl)aminocarbonyl]-6,7-dihydro-5H-thieno[3,2-B]thiopyran-3-sulfonamide, 4,4-dioxide.

**18.** The compound of Claim 1 which is N-[(4,6-dimethoxypyrimidin-2-yl)aminocarbonyl]-5,6-dihydro-5-methylthieno[3,2-B]-thiophene-3-sulfonamide-4,4-dioxide.

**19.** The compound of Claim 1 which is N-[(4-methoxy-6-methyl-1,3,5-triazin-2-yl)aminocarbonyl]-5,6-dihydro-5-methylthieno[3,2-B]-thiophene-3-sulfonamide-4,4-dioxide.

**20.** The compound of Claim 1 which is N-[(4-chloro-6-methoxypyrimidin-2-yl)aminocarbonyl]-5,6-dihydro-5-methylthieno[3,2-B]-thiophene-3-sulfonamide-4,4-dioxide.

**21.** A composition suitable for controlling the growth of undesired vegetation which comprises an effective amount of a compound of any of Claims 1 to 20 and at least one of the following: surfactant, solid or

liquid diluent.

**22.** A method for controlling the growth of undesired vegetation which comprises applying to the locus to be protected an effective amount of a compound of any of Claims 1 to 20.

**23.** A method for regulating the growth of plants which comprises applying to the locus of such plants an effective but substantially non-phytotoxic amount of a plant growth regulant selected from compounds of any of claims 1 to 20.

**24.** A method for the preparation of a compound of claim 1 or 2 which comprises:
(a) reacting an isocyanate or isothiocyanate of formula

$$J-SO_2N=C=W \qquad (II)$$

with an amine of formula

$$H\underset{R}{\underset{|}{N}}-A \qquad (III)$$

wherein J, A and R are as defined in claim 1 or 2;
(b) reacting a sulfonamide of formula

$$J-SO_2NH_2 \qquad (IV)$$

with a heterocyclic isothiocyanate of formula

$$S=C=N-A \qquad (V)$$

wherein J and A are as defined in claim 1 or 2, to obtain a product wherein W is S and R is H;
(c) reacting a sulfonamide of formula

$$J-SO_2NH_2 \qquad (IV)$$

wherein J is as defined in claim 1 or 2, other than $J_5$, $J_6$, $J_9$ and $J_{10}$ as defined in claim 2, with a methylcarbamate of formula

$$CH_3O\overset{O}{\overset{\|}{C}}-\underset{R}{\underset{|}{N}}-A$$

wherein R and A are as defined in claim 1 or 2;
(d) reacting a sulfonylcarbamate of formula

$$J-SO_2NH\overset{O}{\overset{\|}{C}}OC_6H_5 \qquad (VII)$$

wherein J is as defined in claim 1, with said amine of formula III;
(e) reacting a sulfonamide of formula

$$J-SO_2NH_2 \qquad (IV)$$

wherein J is as defined in claim 1 or 2, with a phenylcarbamate of formula

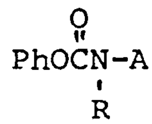

(VIII)

wherein L and A are as defined in claim 1 or 2; or
(f) reacting a bicyclic heterocycle of formula

$$J-H \qquad (IX)$$

with a sulfamoyl chloride of formula

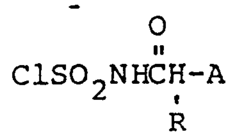

(X)

wherein J, A and R are as defined in claim 1 or 2.

Claims for the following Contracting State : AT

**1.** A process of the preparation of a compound of the formula:

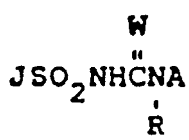

1

wherein
J is

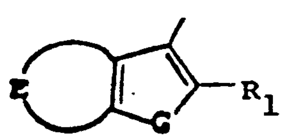 or 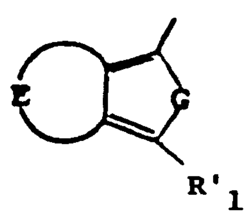

E is a bridge of 3 to 4 atoms, containing 0-2 heteroatoms selected from the group consisting of oxygen, sulfur and nitrogen and also containing 1 to 4 atoms of carbon, said bridge together with the two carbon attachment sites forming a non-aromatic 5 to 6 membered carbocyclic ring, an aromatic 5 to 6

membered heterocyclic ring, or a non-aromatic 5 to 6 membered heterocyclic ring, with the proviso that two oxygen atoms must be separated by at least one atom of carbon and that oxygen and sulfur are only linked to each other if the sulfur is in the form of -SO- or $-SO_2-$;
G is O, S or N-R;
W is O or S;
R is H or $CH_3$;
$R'_1$ is H, $CH_3$ or Cl when G is S and is H when G is O or N-R;
$R_1$ is H, $CH_3$, $OCH_3$, Cl, Br, $SCH_3$, $SO_2CH_3$ or $NO_2$; and
A is

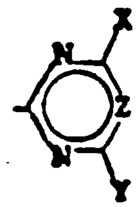 , 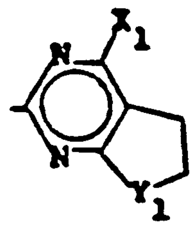 , 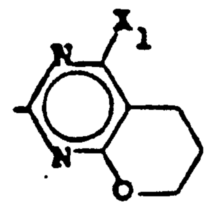

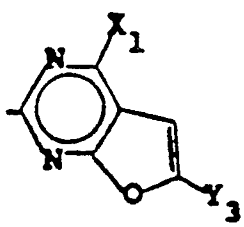 , 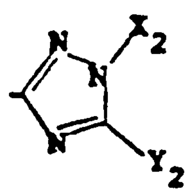 or 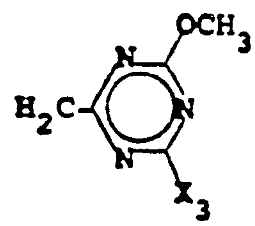 ;

A-4 A-5 A-6

wherein
X is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkythio, halogen, $C_2$-$C_5$ alkoxyalkoxy, amino, $C_1$-$C_3$ alkylamino or di($C_1$-$C_3$ alkyl)amino;
Y is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino di($C_1$-$C_3$ alkyl)amino, $C_3$-$C_4$ alkenyloxy, $C_3$-$C_4$ alkylnyloxy, $C_2$-$C_5$ alkylthioalkyl, $C_1$-$C_4$ haloalkyl, $C_3$-$C_5$ cycloalkyl, $C_2$-$C_4$ alkynyl;

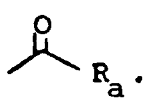 , 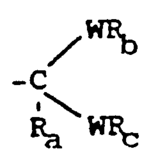 , 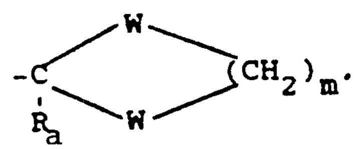 ,

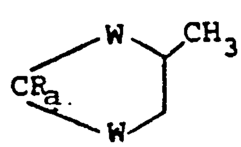

or $N(OCH_3)CH_3$ ;
W is O or S;
m is 2 of 3;
$R_a$ is H or $CH_3$;
$R_b$ is $C_1$-$C_2$ alkyl;
$R_c$ is $C_1$-$C_2$ alkyl;
Z is CH, N, $CCH_3$, $CC_2H_5$, CCl or CBr;

$Y_1$ is O or $CH_2$;
$X_1$ is $CH_3$, $OCH_3$, $OC_2H_5$ or $OCF_2H$;
$X_2$ is $CH_3$, $C_2H_5$ or $CH_2CF_3$;
$Y_2$ is $OCH_3$, $OC_2H_5$, $SCH_3$, $SC_2H_5$, $OCF_2H$, $SCF_2H$, $CH_3$ or $CH_2CH_3$;
$X_3$ is $CH_3$ or $OCH_3$; and $Y_3$ is H or $CH_3$;
provided that

a) when W is S, then R is H, A is A-1, Z is CH or N, and Y is $CH_3$, $OCH_3$, $OC_2H_5$, $CH_2OCH_3$, $C_2H_5$, $CF_3$, $SCH_3$, $OCH_2CH=CH_2$, $OCH_2C{\equiv}CH$, $OCH_2CH_2OCH_3$, $CH(OCH_3)_2$, $OCH_2CF_3$ or

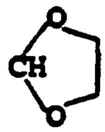 :

c) when X is Cl, F or Br, then Z is CH;
g) when X or Y is $OCF_2H$, then Z is CH; or an agriculturally suitable salt thereof;
which comprises:
(a) reacting an isocyanate or isothiocyanate of formula

$$J{-}SO_2N{=}C{=}W \qquad (II)$$

with an amine of formula

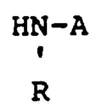 (III)

wherein J, A and R are as defined above;
(b) reacting a sulfonamide of formula

$$J{-}SO_2NH_2 \qquad (IV)$$

with a heterocyclic isothiocyanate of formula

$$S{=}C{=}N{-}A \qquad (V)$$

wherein J and A are as defined above, to obtain a product wherein W is S and R is H;
(c) reacting a sulfonamide of formula

$$J{-}SO_2NH_2 \qquad (IV)$$

wherein J is as defined above, other than $J_5$, $J_6$, $J_9$ and $J_{10}$ as defined in claim 2 hereinafter with a methylcarbamate of formula

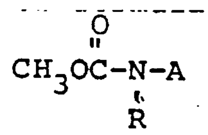

wherein R and A are as defined above;

(d) reacting a sulfonylcarbamate of formula

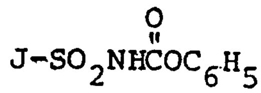 (VII)

wherein J is as defined above, with said amine of formula III;

(e) reacting a sulfonamide of formula

$$J-SO_2NH_2 \qquad (IV)$$

wherein J is as defined above, with a phenylcarbamate of formula

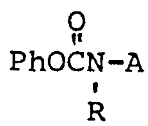 (VIII)

wherein L and A are as defined above; or

(f) reacting a bicyclic heterocycle of formula

$$J-H \qquad (IX)$$

with a sulfamoyl chloride of formula

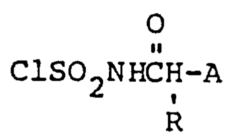 (X)

wherein J, A and R are as defined above.

**2.** A process for the preparation of a compound of the formula:

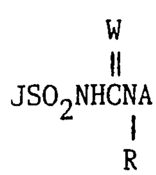

wherein J is

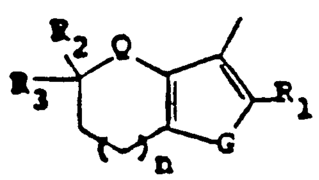

$J_1$

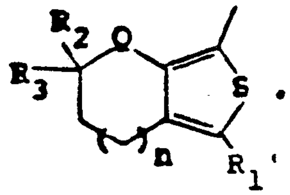

$J_2$

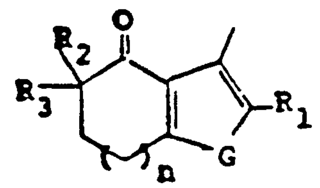

$J_3$

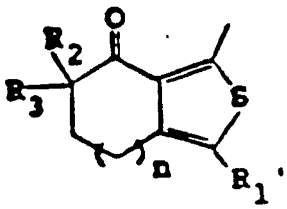

$J_4$

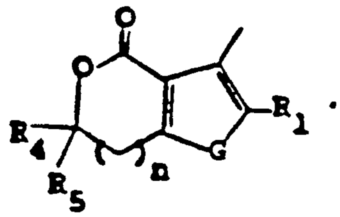

$J_5$

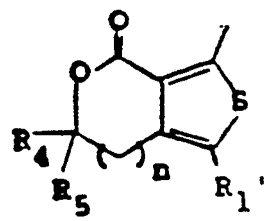

$J_6$

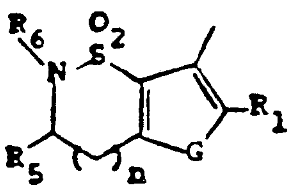

$J_7$

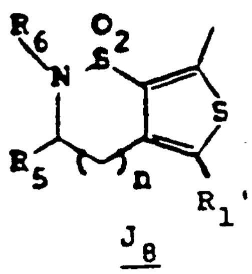

$J_8$

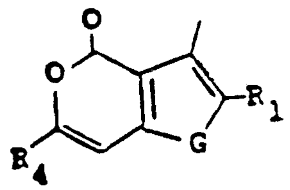

$J_9$

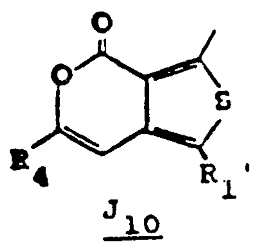

$J_{10}$

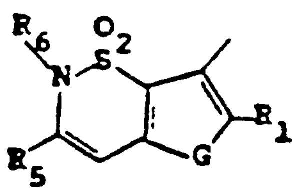

$J_{11}$

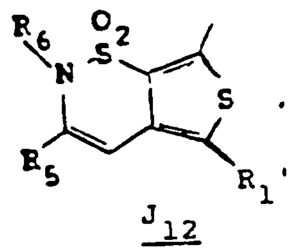

$J_{12}$

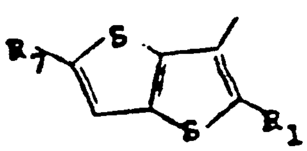

$J_{13}$

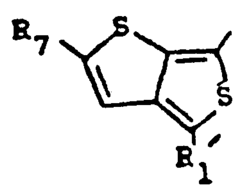

$J_{14}$

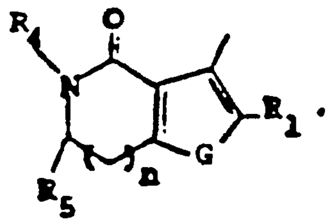

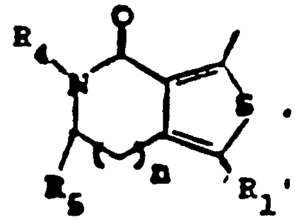

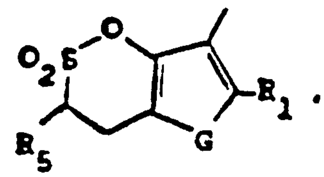

$J_{15}$ $J_{16}$ $J_{17}$

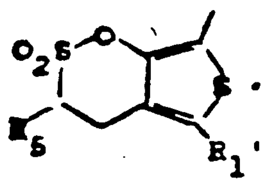

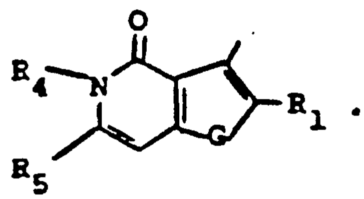

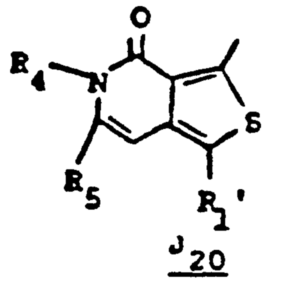

$J_{18}$ $J_{19}$ $J_{20}$

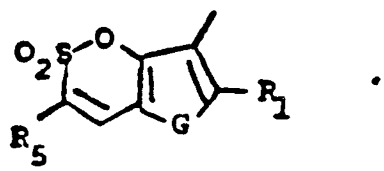

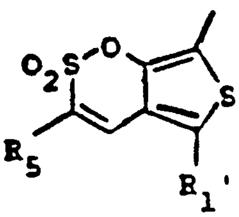

$J_{21}$ $J_{22}$

R is H or $CH_3$;
$R_1'$ is H, $CH_3$ or Cl;
$R_1$ is H, $CH_3$, $OCH_3$, Cl, Br, $SCH_3$, $SO_2CH_3$, or $NO_2$;
$R_2$ is H, Cl or $C_1$-$C_4$ alkyl;
$R_3$ is H, Cl or $C_1$-$C_4$ alkyl;
$R_4$ is H or $C_1$-$C_4$ alkyl;
$R_5$ is H or $CH_3$;
$R_6$ is H, $R_8$, $SR_8$, $SO_2R_8$, $OR_8$, $C(O)R_8$, $CO_2R_8$, $NR_8R_4$, CN or $Si(CH_3)_2R_9$;
$R_7$ is H, $C_1$-$C_6$ alkyl, Cl, Br, CN, $NO_2$, $SR_9$, $SO_2R_9$, $CO_2R_9$ or $C(O)R_9$;
$R_8$ is $C_1$-$C_6$ alkyl, $C_2$-$C_6$ alkenyl, or $C_2$-$C_6$ alkynyl, each optionally substituted by one or more halogens and/or $R_{11}$, $C_2$-$C_6$ epoxyalkyl, $C_3$-$C_6$ cycloalkyl, $C_4$-$C_7$ cycloalkylalkyl, or

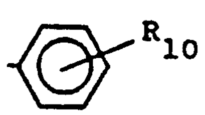

;

$R_9$ is $C_1$-$C_4$ alkyl,

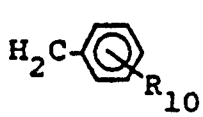 or 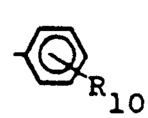

$R_{10}$ is H, F, Cl, Br, $CH_3$, $OCH_3$, CN, $NO_2$, $SCH_3$, $SO_2CH_3$ or $CF_3$;
$R_{11}$ is $OR_{12}$, $OC(O)R_{12}$, $OC(O)NR_9R_{12}$, $OSO_2R_{12}$, $OSi(CH_3)_2R_9$, $Si(CH_3)_2R_9$, $SR_{12}$, $SOR_{12}$, $SO_2R_{12}$, SCN, CN, $NO_2$, $C(O)R_{12}$, $C(O)OR_{12}$, $C(O)NR_4R_{12}$,

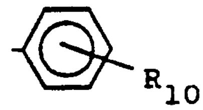

or L;
$R_{12}$ is H, $C_1$-$C_6$ alkyl, $C_1$-$C_6$ haloalkyl, $C_2$-$C_6$ alkenyl, $C_2$-$C_6$ alkynyl, $C_3$-$C_6$ cycloalkyl or

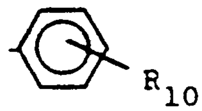

;

n is 0 or 1; Q is O, S, SO, $SO_2$, $CH_2$ or $CHCH_3$: and
L is a 5- or 6-membered aromatic heterocycle, a 5-or 6-membered dihydroaromatic heterocycle or a 5- or 6-membered tetrahydroaromatic heterocycle each containing 1-4 heteroatoms selected from the group consisting of 0-1 oxygen atoms, 0-1 sulfur atoms and 0-4 nitrogen atoms, also each optionally substituted with 1-4 $CH_3$, 1-2 $OCH_3$, 1 $SCH_3$, 1 Cl, 1 $N(CH_3)_2$ or 1 CN or L is a 5- or 6-membered lactone, lactam or cycloalkanone optionally substituted with 1-4 $CH_3$;
G is O, S or NR;
W is O or S;
$R_1$, $R'_1$ and A are as defined in claim 1;
said provisos a), c) and g) as defined in claim 1 apply;
and provided further that

b) the total number of carbon atoms in $R_2$ and $R_3$ is less than or equal to 4;
d) when J is $J_1$ or $J_2$, then $R_2$ and $R_3$ are other than Cl;
e) the total number of carbons atoms in $R_4$ and $R_5$ is less than or equal to 4;
f) when J is $J_5$, $J_6$, $J_7$ or $J_8$ and n = O, then $R_4$ is H;.

or an agriculturally suitable salt thereof; which comprises performing any of processes (a) to (f) as defined in claim 1, wherein J, R and A are as defined above, except that J is not $J_5$, $J_6$, $J_9$ or $J_{10}$ in process (c).

**3.** A process of claim 2 wherein J is

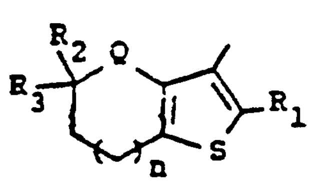,

J1

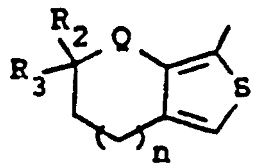,

J2

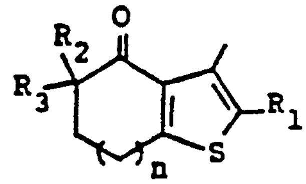,

J3

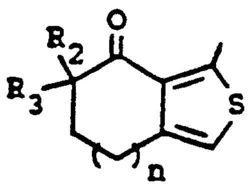,

J4

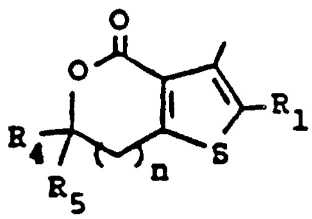,

J5

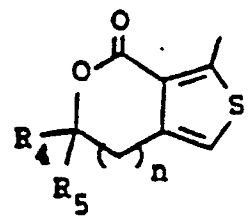,

J6

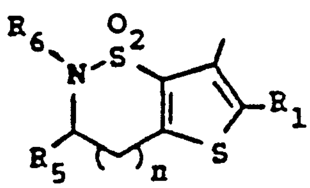,

J7

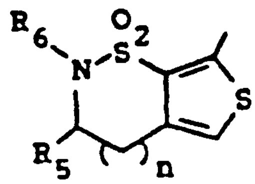,

J8

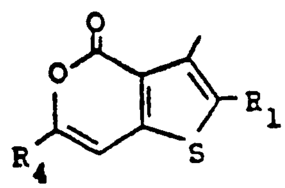,

J9

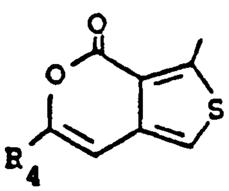,

J10

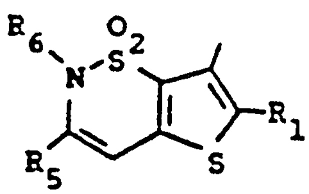,

J11

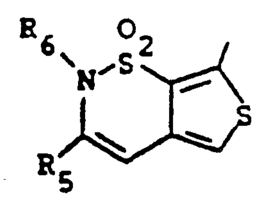,

J12

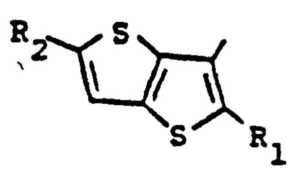

J13

or

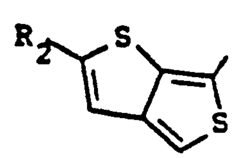;

J14

R is H or $CH_3$;
$R_1$ is H, $CH_3$, $OCH_3$, Cl or Br;
$R_2$ is H, Cl or $C_1$-$C_4$ alkyl;
$R_3$ is H, Cl or $C_1$-$C_4$ alkyl;
$R_4$ i s H or $C_1$-$C_4$ alkyl;
$R_5$ is H or $CH_3$;
$R_6$ is H or $C_1$-$C_4$ alkyl;
Q is O, S, SO, $SO_2$, $CH_2$ or $CHCH_3$;
n is 0 or 1;
A is

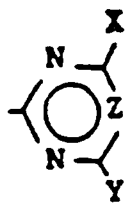,

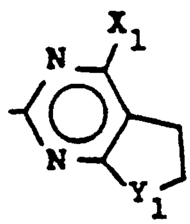,

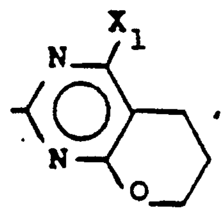,

A-1 A-2 A-3

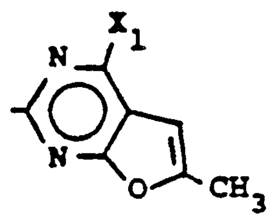,

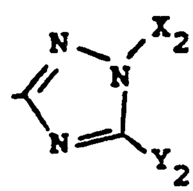

or

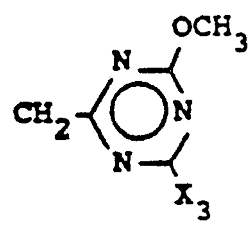;

A-4 A-5 A-6

X is $CH_3$, $OCH_3$, $OCH_2CH_3$, Cl, F, Br, $CH_2F$, $OCF_2H$ or $CF_3$;
Y is H, $CH_3$, $OCH_3$, $OC_2H_5$, $CH_2OCH_3$, $NH_2$, $NHCH_3$, $N(CH_3)_2$, $C_2H_5$, $CF_3$, $SCH_3$, $OCH_2CH=CH_2$, $OCH_2C \equiv CH$, $OCH_2CF_3$, CN, $N_3$, $OCH_2CH_2OCH_3$, $CH_2SCH_3$, $CR_a(WCH_3)_2$,

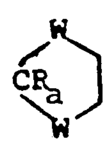,

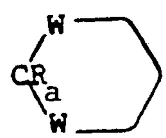,

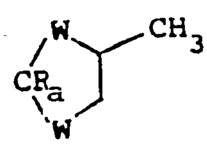,

$CR_a(WCH_2CH_3)_2$, or $WCF_2T$
wherein W is O or S, $R_a$ is H or $CH_3$ and T is H, CHClF, CHBrF or $CHFCF_3$;
Z is CH, N, $CCH_3$, $CC_2H_5$, CCl or CBr;
$Y_1$ is O or $CH_2$;
$X_1$ is $CH_3$, $OCH_3$, $OC_2H_5$ or $OCF_2H$;
$X_2$ is $CH_3$, $C_2H_5$ or $CH_2CF_3$;
$Y_2$ is $OCH_3$, $OC_2H_5$, $SCH_3$, $SC_2H_5$, $CH_3$ or $CH_2CH_3$;
$X_3$ is $CH_3$ or $OCH_3$;
and agriculturally suitable salts thereof.

4. A process of Claim 3 wherein R is H, W is O, and G is O or S.

5. A process of Claim 2 wherein R is H, W is is O and G is O or S.

6. A process of Claim 5 wherein A is A-1, X is $CH_3$, $OCH_3$ Cl, Br, $OCH_2CF_3$ or $OCF_2H$; Y is $C_1$-$C_3$ alkyl, cyclopropyl, C≡CH, $OCH_3$, $OC_2H_5$, $CH_2OCH_3$, $NH_2$, $NHCH_3$, $N(CH_3)_2$, $C_2H_5$, $CF_3$, $SCH_3$, $OCH_2CH=CH_2$, $OCH_2C≡CH$, $OCH_2CH_2OCH_3$, $CR_a(OCH_3)_2$,

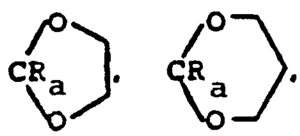

$CH_a(OCH_2CH_3)_2$ or $OCF_2H$; and Z is CH or N.

7. A process of Claim 6 wherein $R_1$ is H, Cl, Br or $CH_3$; $R_2$, $R_3$ and $R_4$ are H or $C_1$-$C_3$ alkyl; $R_6$ is H, $R_8$, $C(O)R_8$ or $CO_2R_8$; $R_7$ is Cl, Br, $CO_2CH_3$ or $CO_2CH_2CH_3$; $R_8$ is $C_1$-$C_6$ alkyl, $C_1$-$C_6$ haloalkyl, $C_2$-$C_6$ alkenyl, $C_2$-$C_6$ alkynyl, $C_3$-$C_6$ cycloalkyl or $C_4$-$C_7$ cycloalkylalkyl and $R_{11}$ is $OCH_3$, $OCH_2CH_3$, CN, $CO_2$($C_1$-$C_4$ alkyl), OH or $C(O)CH_3$.

8. A process of Claim 7 wherein G is S, Y is $CH_3$ or $OCH_3$ and X is $CH_3$, $OCH_3$, Cl, or Br.

9. A process of claim 8 where J is selected from J-1, J-3, J-5, J-7, J-9, J-11 and J-13.

10. A process of Claim 1 wherein the product is a compound selected from N-[(4-methoxy-6-methylpyrimidin-2-yl)aminocarbonyl]-6,7-dihydro-5H-thieno-[3,2-B]thioyran-3-sulfonamide, 4,4-dioxide; N-[(4,6-dimethoxypyrmidin-2-yl)aminocarbonyl]-6,7-dihydro-5H-thieno[3,2-B]thiopyran-3-sulfonamide, 4,4-dioxide; N-[4,6-dimethoxypyrimidin-2-yl)aminocarbonyl]-5,6-dihydro-5-methylthieno[3,2-B]-thiophene-3-sulfonamide-4,4-dioxide; N-[(4-methoxy-6-methyl-1,3,5-triazin-2-yl)aminocarbonyl]-5,6-dihydro-5-methylthieno[3,2-B]-thiophene-3-sulfonamide-4,4-dioxide, and N-[(4-chloro-6-methoxy pyrimidin-2-yl) aminocarbonyl]-5, 6-dihydro-5-methylthieno[3,2-B]-thiophene-3-sulfonamide-4,4-dioxide.

11. A composition suitable for controlling the growth of undesired vegetation which comprises an effective amount of a compound of formula (I) as defined in any of Claims 1 to 10 and at least one of the following: surfactant, solid or liquid diluent.

12. A method for controlling the growth of undesired vegetation which comprises applying to the locus to be protected an effective amount of a compound of formula (I) as defined in any of Claims 1 to 10.

13. A method of Claim 12 wherein a compound defined in Claim 10 is applied, or an agriculturally suitable salt thereof.

14. A method for regulating the growth of plants which comprises applying to the locus of such plants an effective but substantially non-phytotoxic amount of a plant growth regulant selected from compounds of formula (I) as defined in any of Claims 1 to 10.

**Revendications**

1. Un composé de la formule :

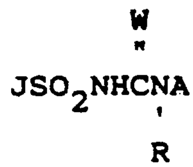

I

dans laquelle
J est

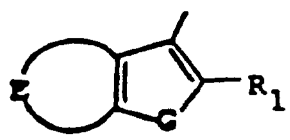 ou 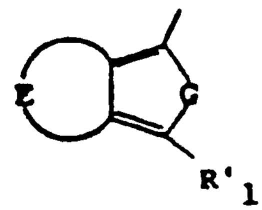

E est un pont de 3 ou 4 atomes, contenant 0 à 2 hétéroatomes choisis dans le groupe formé par l'oxygène, le soufre et l'azote, et oontenant également 1 à 4 atomes de carbone, ledit pont, pris avec les deux sites de fixation carbonés, formant un carbocycle penta- ou hexagonal non aromatique, un hétérocycle penta- ou hexagonal aromatique, ou un hétérocycle penta- ou hexagonal non aromatique, avec la condition que deux atomes d'oxygène soient séparés par au moins un atome de carbone et que l'oxygène et le soufre ne soient liés l'un à l'autre que si le soufre est sous la forme de -SO- ou $-SO_2-$ ;
G est O, S ou N-R ;
W est O ou S ;
R est H ou $CH_3$ ;
$R_1$' est H, $CH_3$ ou Cl si G est S, et est H si G est O ou N-R ;
$R_1$ est H, $CH_3$, $OCH_3$, Cl, Br, $SCH_3$, $SO_2CH_3$ ou $NO_2$ ; et A est

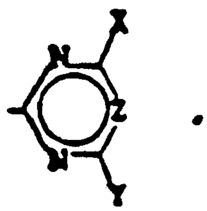 , 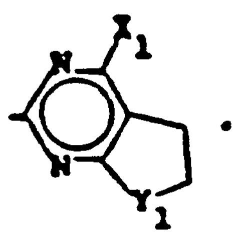 , 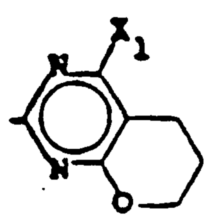

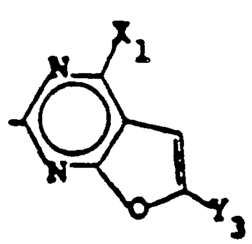 , 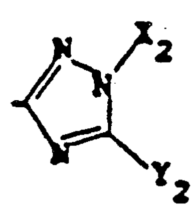 ou 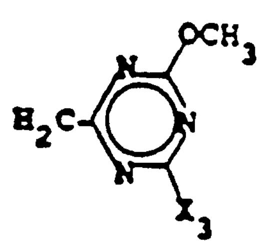 ;

A-4 A-5 A-6

où
X est H, un groupe alkyle en $C_1$-$C_4$, alcoxy en $C_1$-$C_4$, halogénalcoxy en $C_1$-$C_4$, halogénalkyle en $C_1$-$C_4$, halogénalkylthio en $C_1$-$C_4$, alkylthio en $C_1$-$C_4$, halogéno, alcoxyalcoxy en $C_2$-$C_5$, amino, alkylamino en $C_1$-$C_3$ ou di(alkyle en $C_1$-$C_3$)amino ;

Y est H, un groupe alkyle en $C_1$-$C_4$, alcoxy en $C_1$-$C_4$, halogénalcoxy en $C_1$-$C_4$, halogénalkylthio en $C_1$-$C_4$, alkylthio en $C_1$-$C_4$, halogéno, alcoxyalkyle en $C_2$-$C_5$, alcoxyalcoxy en $C_2$-$C_5$, amino, alkylamino en $C_1$-$C_3$, di(alkyle en $C_1$-$C_3$)amino, alcényloxy en $C_3$-$C_4$, alcynyloxy en $C_3$-$C_4$, alkylthioalkyle en $C_2$-$C_5$, halogénalkyle en $C_1$-$C_4$, cycloalkyle en $C_3$-$C_5$, alcynyle en $C_2$-$C_4$ ;

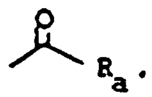 , 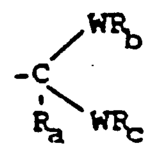 , 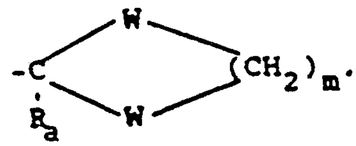

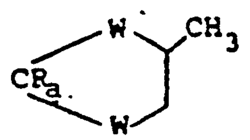

ou $N(OCH_3)CH_3$ ;
W est O ou S ;
m est 2 ou 3 ;
$R_a$ est H ou $CH_3$ ;
$R_b$ est un groupe alkyle en $C_1$-$C_2$ ;
$R_c$ est un groupe alkyle en $C_1$-$C_2$ ;
Z est CH, N, $CCH_3$, $CC_2H_5$, CCl ou CBr ;
$Y_1$ est O ou $CH_2$ ;
$X_1$ est $CH_3$, $OCH_3$, $OC_2H_5$ ou $OCF_2H$ ;
$X_2$ est $CH_3$, $C_2H_3$ ou $CH_2CF_3$ ;
$Y_2$ est $OCH_3$, $OC_2H_5$, $SCH_3$, $SC_2H_5$, $OCF_2H$, $SCF_2H$, $CH_3$ ou $CH_2CH_3$ ;
$X_3$ est $CH_3$ ou $OCH_3$ ; et $Y_3$ est H ou $CH_3$ ;
avec les conditions suivantes

a) si W est S, alors R est H, A est A-1, Z est CH ou N, et Y est $CH_3$, $OCH_3$, $OC_2H_5$, $CH_2OCH_3$, $C_2H_5$, $CF_3$, $SCH_3$, $OCH_2CH=CH_2$, $OCH_2C{\equiv}CH$, $OCH_2CH_2OCH_3$, $CH(OCH_3)_2$, $OCH_2CF_3$ ou

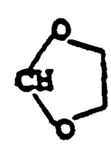 ;

c) si X est Cl, F ou Br, alors Z est CH et Y est $OCH_3$, $OC_2H_5$, $NH_2$, $NHCH_3$, $N(CH_3)_2$ ou $OCF_2H$ ;

g) si X ou Y est $OCF_2H$, alors Z est CH ; et ses sels convenant pour l'agriculture.

**2.** Un composé de la formule :

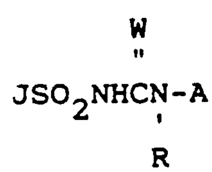

dans laquelle J est

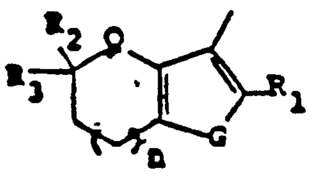

,

J1

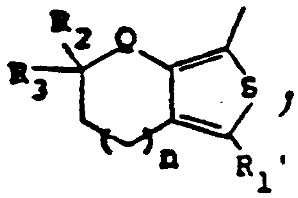

,

J2

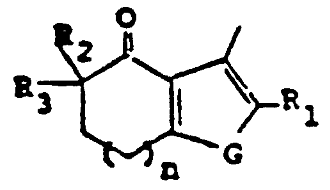

,

J3

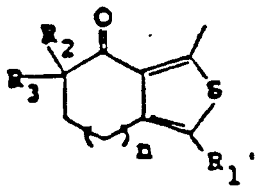

$J_4$

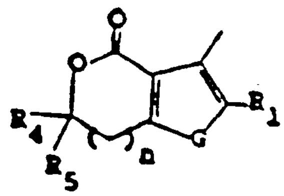

$J_5$

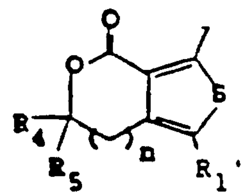

$J_6$

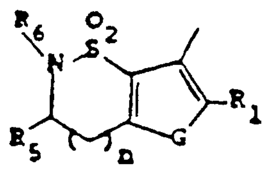

$J_7$

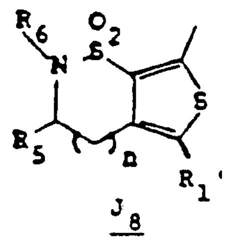

$J_8$

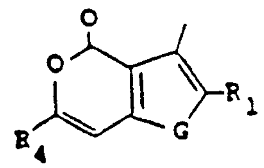

$J_9$

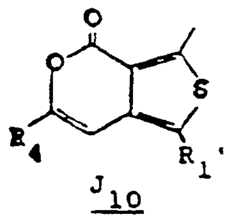

$J_{10}$

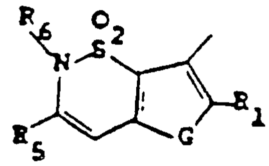

$J_{11}$

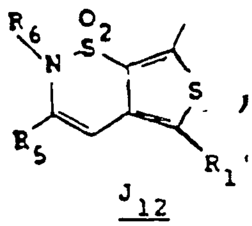

$J_{12}$

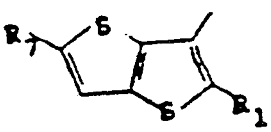

$J_{13}$

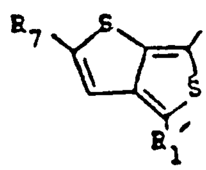

$J_{14}$

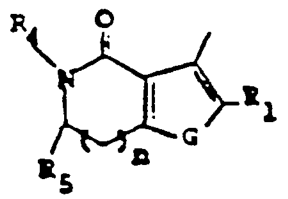

$J_{15}$

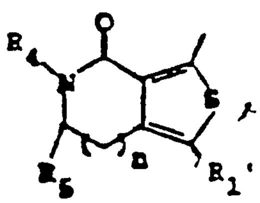

$J_{16}$

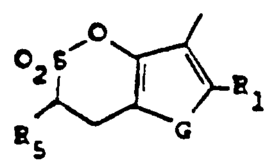

$J_{17}$

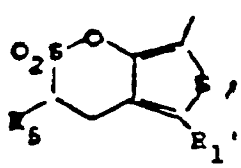

$J_{18}$

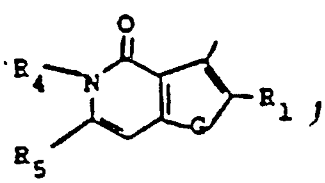

$J_{19}$

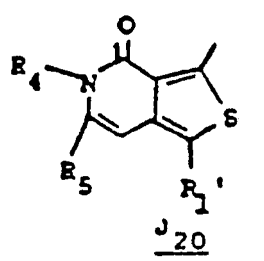

$J_{20}$

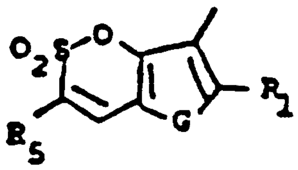 , 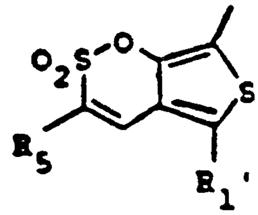

$J_{21}$ $J_{22}$

R est H ou $CH_3$ ;
$R_1$' est H, $CH_3$ ou Cl ;
$R_1$ est H, $CH_3$, $OCH_3$, Cl, Br, $SCH_3$, $SO_2CH_3$ ou $NO_2$ ;
$R_2$ est H, Cl ou un groupe alkyle en $C_1$-$C_4$ ;
$R_3$ est H, Cl ou un groupe alkyle en $C_1$-$C_4$ ;
$R_4$ est H ou un groupe alkyle en $C_1$-$C_4$ ;
$R_5$ est H ou $CH_3$ ;
$R_6$ est H, $R_8$, $SR_8$, $SO_2R_8$, $OR_8$, $C(O)R_8$, $CO_2R_8$, $NR_8R_4$, CN ou $Si(CH_3)_2R_9$ ;
$R_7$ est H, un groupe alkyle en $C_1$-$C_6$, Cl, Br, CN, $NO_2$, $SR_9$, $SO_2R_9$, $CO_2R_9$ ou $C(O)R_9$ ;
$R_8$ est un groupe alkyle en $C_1$-$C_6$, alcényle en $C_2$-$C_6$ ou alcynyle en $C_2$-$C_6$, chacun étant facultativement substitué par un ou plusieurs halogènes et/ou $R_{11}$, époxyalkyle en $C_2$-$C_6$, cycloalkyle en $C_3$-$C_6$, cycloalkylalkyle en $C_4$-$C_7$, ou

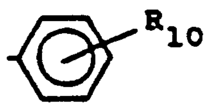 ;

$R_9$ est un groupe alkyle en $C_1$-$C_4$,

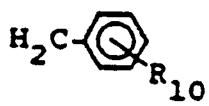

ou

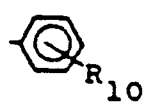 ;

$R_{10}$ est H, F, Cl, Br, $CH_3$, $OCH_3$, CN, $NO_2$, $SCH_3$, $SO_2CH_3$ ou $CF_3$ ;
$R_{11}$ est $OR_{12}$, $OC(O)R_{12}$, $OC(O)NR_9R_{12}$, $OSO_2R_{12}$, $OSi(CH_3)_2R_9$, $Si(CH_3)_2R_9$, $SR_{12}$, $SOR_{12}$, $SO_2R_{12}$, SCN, CN, $NO_2$, $C(O)R_{12}$, $C(O)OR_{12}$, $C(O)NR_4R_{12}$,

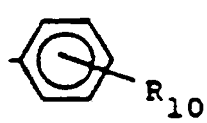

ou L ;
$R_{12}$ est H, un groupe alkyle en $C_1$-$C_6$, halogénalkyle en $C_1$-$C_6$, alcényle en $C_2$-$C_6$, alcynyle en $C_2$-$C_6$, cycloalkyle en $C_3$-$C_6$ ou

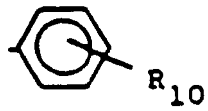 ;

n est 0 ou 1 ;
Q est O, S, SO, $SO_2$, $CH_2$ ou $CHCH_3$ ; et
L est un hétérocycle aromatique penta- ou hexagonal, un hétérocycle dihydroaromatique penta- ou hexagonal ou un hétérocycle tétrahydroaromatique penta- ou hexagonal, chacun contenant 1 à 4 hétéroatomes choisis dans le groupe formé par 0 ou 1 atome d'oxygène, 0 ou 1 atome de soufre et 0 à 4 atomes d'azote, chacun étant également facultativement substitué par 1 à 4 $CH_3$, 1 à 2 $OCH_3$, 1 $SCH_3$, 1 Cl, 1 $N(CH_3)_2$ ou 1 CN, ou bien L est une lactone, un lactame ou une cycloalcanone, penta- ou hexagonal, facultativement substitué par 1 à 4 $CH_3$ ;
G est O, S ou NR ;
W est O ou S ;
$R_1$, $R_1'$ et A sont tels que définis dans la revendication 1 ;
lesdites conditions a), c) et g) telles que définies dans la revendication 1 étant appliquées ;
et avec les conditions supplémentaires suivantes
b) le nombre total d'atomes de carbone dans $R_2$ et $R_3$ est inférieur ou égal à 4 ;
d) si J est $J_1$ ou $J_2$, alors $R_2$ et $R_3$ sont autre chose que Cl ;
e) le nombre total d'atomes de carbone dans $R_4$ et $R_5$ est inférieur ou égal à 4 ;
f) si J est $J_5$, $J_6$, $J_7$ ou $J_8$ et n = 0, alors $R_4$ est H ; et ses sels convenant pour l'agriculture.

**3.** Un composé de la revendication 2, dans lequel J est

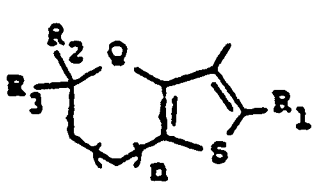 , 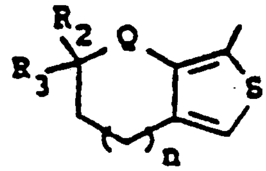 , 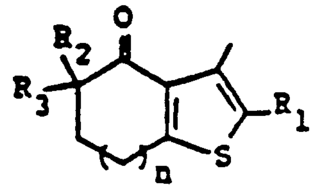 ,

$\underline{J_1}$ $\underline{J_2}$ $\underline{J_3}$

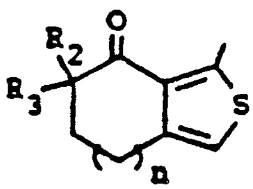 , 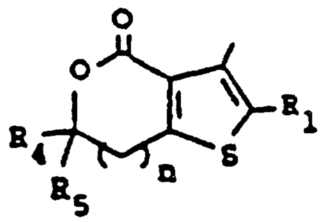 , 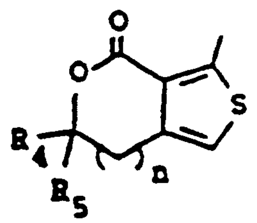 ,

$\underline{J_4}$ $\underline{J_5}$ $\underline{J_6}$

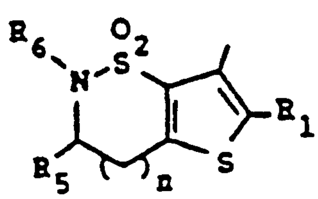 , 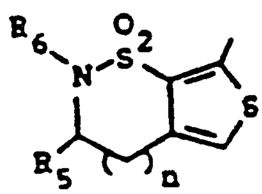 , 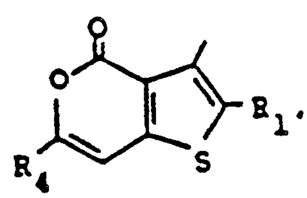 ,

$\underline{J_7}$ $\underline{J_8}$ $\underline{J_9}$

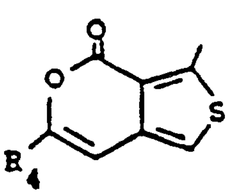 , 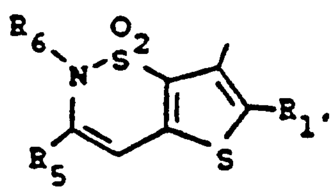 , 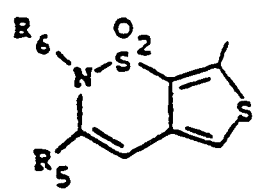 ,

$\underline{J_{10}}$ $\underline{J_{11}}$ $\underline{J_{12}}$

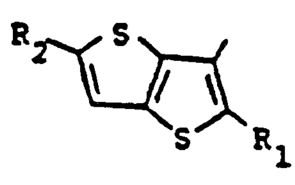 ou 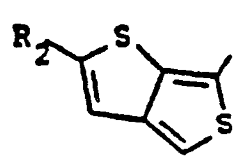 ;

$\underline{J_{13}}$ $\underline{J_{14}}$

R est H ou $CH_3$ ;
$R_1$ est H, $CH_3$, $OCH_3$, Cl ou Br ;
$R_2$ est H, Cl ou un groupe alkyle en $C_1$-$C_4$ ;
$R_3$ est H, Cl ou un groupe alkyle en $C_1$-$C_4$ ;
$R_4$ est H ou un groupe alkyle en $C_1$-$C_4$ ;
$R_5$ est H ou $CH_3$ ;
$R_6$ est H ou un groupe alkyle en $C_1$-$C_4$ ;
Q est O, S, SO, $SO_2$, $CH_2$ ou $CHCH_3$ ;
n est 0 ou 1 ;
A est

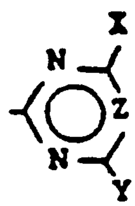
A-1
,
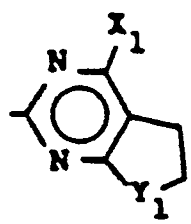
A-2
,
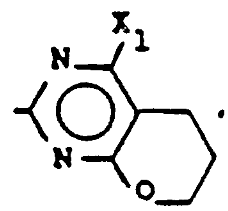
A-3
,
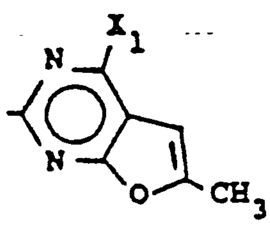
A-4
,
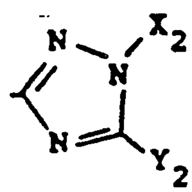
A-5
ou
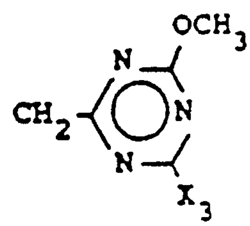
A-6
;

X est $CH_3$, $OCH_3$, $OCH_2CH_3$, Cl, F, Br, $CH_2F$, $OCF_2H$ ou $CF_3$ ;
Y est H, $CH_3$, $OCH_3$, $OC_2H_5$, $CH_2OCH_3$, $NH_2$, $NHCH_3$, $N(CH_3)_2$, $C_2H_5$, $CF_3$, $SCH_3$, $OCH_2CH=CH_2$, $OCH_2C\equiv CH$, $OCH_2CF_3$, CN, $N_3$, $OCH_2CH_2OCH_3$, $CH_2SCH_3$, $CR_a(WCH_3)_2$,

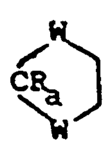
,
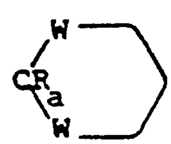
,

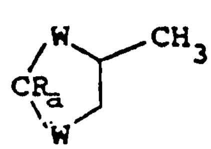
,

$CR_a(WCH_2CH_3)_2$, ou $WCF_2T$
où W est O ou S, $R_a$ est H ou $CH_3$ et T est H, CHClF, CHBrF ou $CHFCF_3$ ;
Z est CH, N, $CCH_3$, $CC_2H_5$, CCl ou CBr ;
$Y_1$ est O ou $CH_2$ ;
$X_1$ est $CH_3$, $OCH_3$, $OC_2H_5$ ou $OCF_2H$ ;
$X_2$ est $CH_3$, $C_2H_5$ ou $CH_2CF_3$ ;
$Y_2$ est $OCH_3$, $OC_2H_5$, $SCH_3$, $SC_2H_5$, $CH_3$, ou $CH_2CH_3$ ;
$X_3$ est $CH_3$ ou $OCH_3$ ;

et ses sels convenant pour l'agriculture.

**4.** Un composé de la revendication 3, dans lequel R est H, W est O et G est O ou S.

**5.** Un composé de la revendication 2, dans lequel R est H, W est O et G est O ou S.

**6.** Un composé de la revendication 5, dans lequel A est A-1, X est $CH_3$, $OCH_3$, Cl, Br, $OCH_2CF_3$ ou $OCF_2H$ ; Y est un groupe alkyle en $C_1$-$C_3$, cyclopropyle, C≡CH, $OCH_3$, $OC_2H_5$, $CH_2OCH_3$, $NH_2$, $NHCH_3$, $N(CH_3)_2$, $C_2H_5$, $CF_3$, $SCH_3$, $OCH_2CH=CH_2$, $OCH_2C≡CH$, $OCH_2CH_2OCH_3$, $CR_a(OCH_3)_2$,

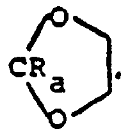

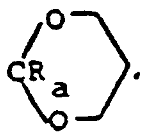

$CH_a(OCH_2CH_3)_2$ ou $OCF_2H$; et Z est CH ou N.

**7.** Un composé de la revendication 6, dans lequel $R_1$ est H, Cl, Br ou $CH_3$ ; $R_2$, $R_3$ et $R_1$ sont H ou un groupe alkyle en $C_1$-$C_3$ ; $R_6$ est H, $R_8$, $C(O)R_8$ ou $CO_2R_8$ ; $R_7$ est Cl, Br, $CO_2CH_3$ ou $CO_2CH_2CH_3$ ; $R_8$ est un groupe alkyle en $C_1$-$C_6$, halogénalkyle en $C_1$-$C_6$, alcényle en $C_2$-$C_6$, alcynyle en $C_2$-$C_6$, cycloalkyle en $C_3$-$C_6$ ou cycloalkylalkyle en $C_4$-$C_7$ et $R_{11}$ est $OCH_3$, $OCH_2CH_3$, CN, $CO_2$(alkyle en $C_1$-$C_4$), OH ou $C(O)CH_3$.

**8.** Un composé de la revendication 7, dans lequel G est S, Y est $CH_3$ ou $OCH_3$ et X est $CH_3$, $OCH_3$, Cl ou Br.

**9.** Un composé de la revendication 8, dans lequel J est J-1.

**10.** Un composé de la revendication 8, dans lequel J est J-3.

**11.** Un composé de la revendication 8, dans lequel J est J-5.

**12.** Un composé de la revendication 8, dans lequel J est J-7.

**13.** Un composé de la revendication 8, dans lequel J est J-9.

**14.** Un composé de la revendication 8, dans lequel J est J-11.

**15.** Un composé de la revendication 8, dans lequel J est J-13.

**16.** Le composé de la revendication 1, qui est le 4,4-dioxyde de N-[(4-méthoxy-6-méthylpyrimidine-2-yl)-aminocarbonyl]-6,7-dihydro-5H-thiéno[3,2-B]thiopyranne-3-sulfonamide.

**17.** Le composé de la revendication 1, qui est le 4,4-dioxyde de N-[(4,6-diméthoxypyrimidine-2-yl)-aminocarbonyl]-6,7-dihydro-5H-thiéno[3,2-B]thiopyranne-3-sulfonamide.

**18.** Le composé de la revendication 1, qui est le 4,4-dioxyde de N-[(4,6-diméthoxypyrimidine-2-yl)-aminocarbonyl]-5,6-dihydro-5-méthylthiéno[3,2-B]thiophène-3-sulfonamide.

**19.** Le composé de la revendication 1, qui est le 4,4-dioxyde de N-[(4-méthoxy-6-méthyl-1,3,5-triazine-2-yl)-

aminocarbonyl]-5,6-dihydro-5-méthylthiéno[3,2-B]thiophène-3-sulfonamide.

**20.** Le composé de la revendication 1, qui est le 4,4-dioxyde de N-[(4-chloro-6-méthoxypyrimidine-2-yl)-aminocarbonyl]-5,6-dihydro-5-méthylthiéno[3,2-B]thiophène-3-sulfonamide.

**21.** Une composition convenant pour lutter contre la croissance d'une végétation indésirable, qui comprend une quantité efficace d'un composé de l'une quelconque des revendications 1 à 20 et au moins l'un des ingrédients suivants : agent tensio-actif, diluant solide ou liquide.

**22.** Un procédé pour lutter contre la croissance d'une végétation indésirable, qui consiste à appliquer au site à protéger une quantité efficace d'un composé de l'une quelconque des revendications 1 à 20.

**23.** Un procédé pour réguler la croissance de plantes, qui consiste à appliquer au site où se trouvent ces plantes une quantité efficace, mais sensiblement non phytotoxique, d'un régulateur de croissance de plantes choisi parmi les composés de l'une quelconque des revendications 1 à 20.

**24.** Un procédé pour la préparation d'un composé de la revendication 1 ou 2, qui consiste à :
(a) faire réagir un isocyanate ou isothiocyanate de formule

$$J\text{-}SO_2N{=}C{=}W \qquad (II)$$

avec une amine de formule

$$\underset{\displaystyle R}{HN}\text{-}A \qquad (III)$$

où J, A et R sont tels que définis dans la revendication 1 ou 2 ;
(b) faire réagir un sulfonamide de formule

$$J\text{-}SO_2NH_2 \qquad (IV)$$

avec un isothiocyanate hétérocyclique de formule

$$S{=}C{=}N\text{-}A \qquad (V)$$

où J et A sont tels que définis dans la revendication 1 ou 2, pour obtenir un produit dans lequel W est S et R est H ;
(c) faire réagir un sulfonamide de formule

$$J\text{-}SO_2NH_2 \qquad (IV)$$

où J est tel que défini dans la revendication 1 ou 2, autre que $J_5$, $J_6$, $J_9$ et $J_{10}$ tels que définis dans la revendication 2, avec un méthylcarbamate de formule

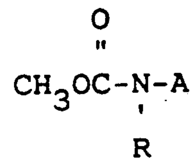

où R et A sont tels que définis dans la revendication 1 ou 2 ;
(d) faire réagir un sulfonylcarbamate de formule

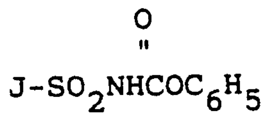 (VII)

où J est tel que défini à la revendication 1, avec ladite amine de formule III ;
(e) faire réagir un sulfonamide de formule

$$J\text{-}SO_2NH_2 \qquad (IV)$$

où J est tel que défini dans la revendication 1 ou 2, avec un phénylcarbamate de formule

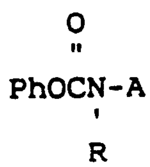 (VIII)

où L et A sont tels que définis dans la revendication 1 ou 2 ; ou
(f) faire réagir un hétérocycle bicyclique de formule

$$J\text{-}H \qquad (IX)$$

avec un chlorure de sulfamyle de formule

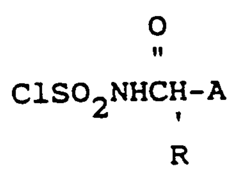 (X)

où J, A et R sont tels que définis dans la revendication 1 ou 2.

Revendication pour l'Etat contractant suivant : AT

1. Un procédé de préparation d'un composé de la formule :

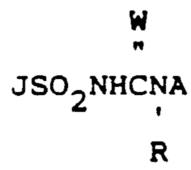

I

dans laquelle
J est

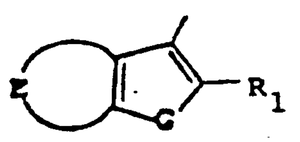

ou

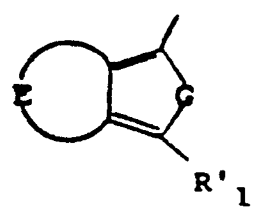

E est un pont de 3 ou 4 atomes, contenant 0 à 2 hétéroatomes choisis dans le groupe formé par l'oxygène, le soufre et l'azote, et contenant également 1 à 4 atomes de carbone, ledit pont, pris avec les deux sites de fixation carbonés, formant un carbocycle penta- ou hexagonal non aromatique, un hétérocycle penta- ou hexagonal aromatique, ou un hétérocycle penta- ou hexagonal non aromatique, avec la condition que deux atomes d'oxygène soient séparés par au moins un atome de carbone et que l'oxygène et le soufre ne soient liés l'un à l'autre que si le soufre est sous la forme de -SO- ou $-SO_2-$ ;
G est O, S ou N-R ;
W est O ou S ;
R est H ou $CH_3$ ;
$R_1$' est H, $CH_3$ ou Cl si G est S, et est H si G est O ou N-R ;
$R_1$ est H, $CH_3$, $OCH_3$, Cl, Br, $SCH_3$, $SO_2CH_3$ ou $NO_2$ ; et
A est

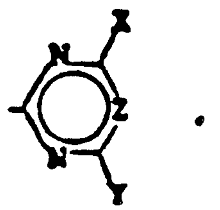

,

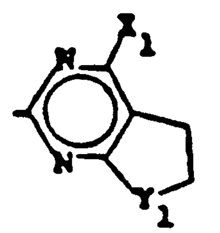

,

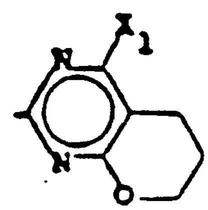

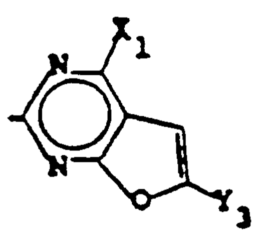

A-4

,

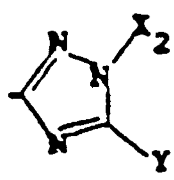

A-5

ou

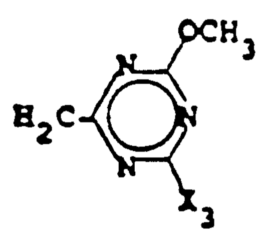

A-6

;

X est H, un groupe alkyle en $C_1$-$C_4$, alcoxy en $C_1$-$C_4$, halogénalcoxy en $C_1$-$C_4$, halogénalkyle en $C_1$-$C_4$, halogénalkylthio en $C_1$-$C_4$, alkylthio en $C_1$-$C_4$, halogéno, alcoxyalcoxy en $C_2$-$C_5$, amino, alkylamino en $C_1$-$C_3$ ou di(alkyle en $C_1$-$C_3$)amino ;
Y est H, un groupe alkyle en $C_1$-$C_4$, alcoxy en $C_1$-$C_4$, halogénalcoxy en $C_1$-$C_4$, halogénalkylthio en $C_1$-

$C_4$, alkylthio en $C_1$-$C_4$, halogéno, alcoxyalkyle en $C_2$-$C_5$, alcoxyalcoxy en $C_2$-$C_5$, amino, alkylamino en $C_1$-$C_3$, di(alkyle en $C_1$-$C_3$)amino, alcényloxy en $C_3$-$C_4$, alcynyloxy en $C_3$-$C_4$, alkylthioalkyle en $C_2$-$C_5$, halogénalkyle en $C_1$-$C_4$, cycloalkyle en $C_3$-$C_5$, alcynyle en $C_2$-$C_4$ ;

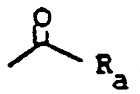 , 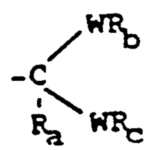 , 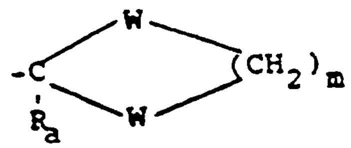 ,

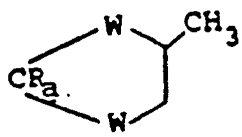

ou $N(OCH_3)CH_3$ ;
W est O ou S ;
m est 2 ou 3 ;
$R_a$ est H ou $CH_3$ ;
$R_b$ est un groupe alkyle en $C_1$-$C_2$ ;
$R_c$ est un groupe alkyle en $C_1$-$C_2$ ;
Z est CH, N, $CCH_3$, $CC_2H_5$, CCl ou CBr ;
$Y_1$ est O ou $CH_2$ ;
$X_1$ est $CH_3$, $OCH_3$, $OC_2H_5$ ou $OCF_2H$ ;
$X_2$ est $CH_3$, $C_2H_5$ ou $CH_2CF_3$ ;
$Y_2$ est $OCH_3$, $OC_2H_5$, $SCH_3$, $SC_2H_5$, $OCF_2H$, $SCF_2H$, $CH_3$ ou $CH_2CH_3$ ;
$X_3$ est $CH_3$ ou $OCH_3$ ; et $Y_3$ est H ou $CH_3$ ;
avec les conditions suivantes

a) si W est S, alors R est H, A est A-1, Z est CH ou N, et Y est $CH_3$, $OCH_3$, $OC_2H_5$, $CH_2OCH_3$, $C_2H_5$, $CF_3$, $SCH_3$, $OCH_2CH=CH_2$, $OCH_2C\equiv CH$, $OCH_2CH_2OCH_3$, $CH(OCH_3)_2$, $OCH_2CF_3$ ou

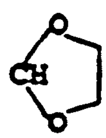 ;

c) si X est Cl, F ou Br, alors Z est CH;

g) si X ou Y est $OCF_2H$, alors Z est CH; ou d'un de ses sels convenant pour l'agriculture; qui consiste à :

(a) faire réagir un isocyanate ou isothiocyanate de formule

$$J\text{-}SO_2N{=}C{=}W \qquad (II)$$

avec une amine de formule

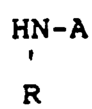 (III)

où J, A et R sont tels que définis ci-dessus;

(b) faire réagir un sulfonamide de formule

$$J\text{-}SO_2NH_2 \qquad (IV)$$

avec un isothiocyanate hétérocyclique de formule

$$S{=}C{=}N\text{-}A \qquad (V)$$

où J et A sont tels que définis ci-dessus, pour obtenir un produit dans lequel W est S et R est H ;
(c) faire réagir un sulfonamide de formule

$$J\text{-}SO_2NH_2 \qquad (IV)$$

où J est tel que défini ci-dessus, autre que $J_5$, $J_6$, $J_9$ et $J_{10}$ tels que définis dans la revendication 2 ci-après, avec un méthylcarbamate de formule

$$CH_3O\overset{O}{\overset{\|}{C}}\text{-}\underset{R}{\underset{|}{N}}\text{-}A$$

où R et A sont tels que définis ci-dessus;
(d) faire réagir un sulfonylcarbamate de formule

$$J\text{-}SO_2NH\overset{O}{\overset{\|}{C}}OC_6H_5 \qquad (VII)$$

où J est tel que défini ci-dessus, avec ladite amine de formule III ;
(e) faire réagir un sulfonamide de formule

$$J\text{-}SO_2NH_2 \qquad (IV)$$

où J est tel que défini ci-dessus, avec un phénylcarbamate de formule

$$PhO\overset{O}{\overset{\|}{C}}\underset{R}{\underset{|}{N}}\text{-}A \qquad (VIII)$$

où L et A sont tels que définis ci-dessus ; ou
(f) faire réagir un hétérocycle bicyclique de formule

$$J\text{-}H \qquad (IX)$$

avec un chlorure de sulfamyle de formule

$$\begin{array}{c} O \\ \| \\ ClSO_2NHC\underset{|}{N}\text{-}A \\ R \end{array} \qquad (X)$$

où J, A et R sont tels que définis ci-dessus.

**2.** Un procédé de préparation d'un composé de la formule :

$$\begin{array}{c} W \\ \| \\ JSO_2NHC\underset{|}{N}\text{-}A \\ R \end{array}$$

dans laquelle J est

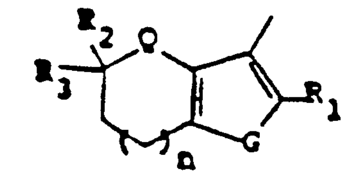

J1 ,

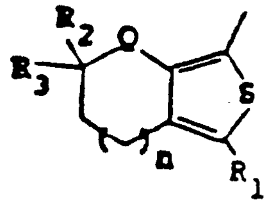

J2 ,

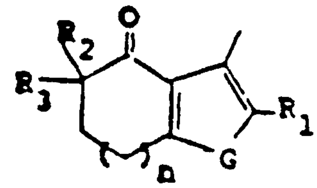

J3 ,

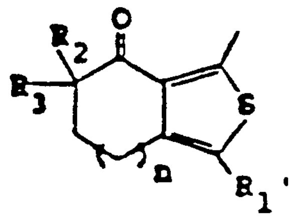

J4 ,

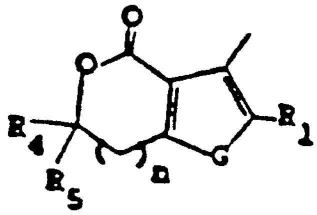

J5 ,

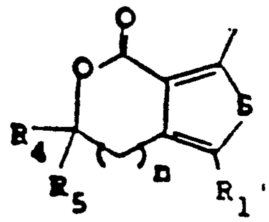

J6 ,

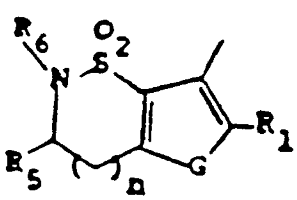

J7 ,

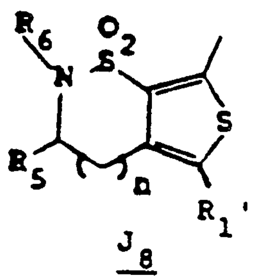

J8 ,

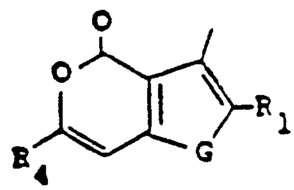

J9 ,

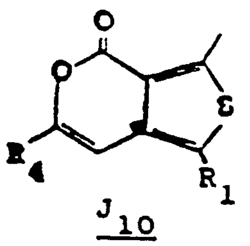

J10 ,

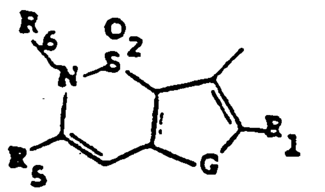

J11 ,

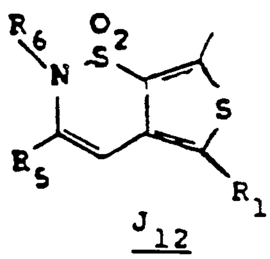

J12 ,

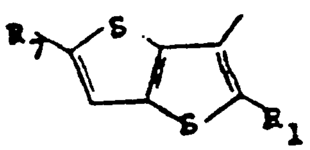

J13 ,

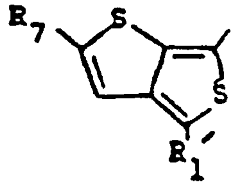

J14 ,

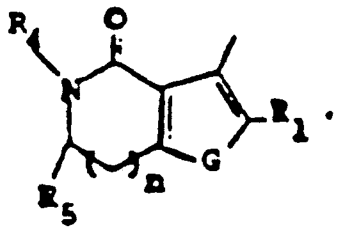 ,

$J_{15}$

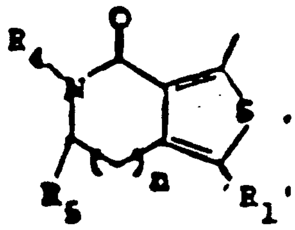 ,

$J_{16}$

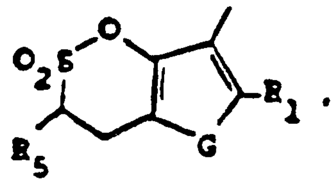 ,

$J_{17}$

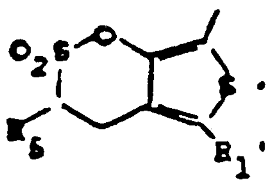 ,

$J_{18}$

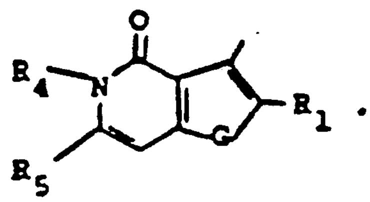 ,

$J_{19}$

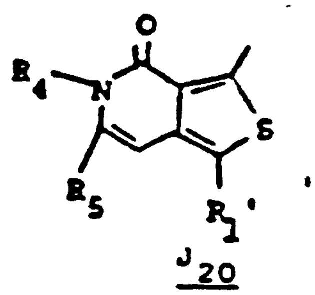 ,

$J_{20}$

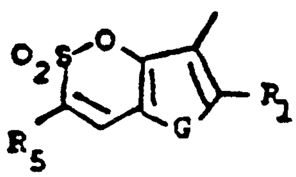 ,

$J_{21}$

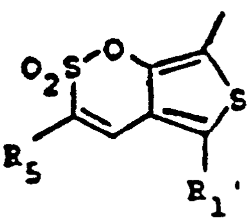

$J_{22}$

R est H ou $CH_3$ ;
$R_1$' est H, $CH_3$ ou Cl ;
$R_1$ est H, $CH_3$, $OCH_3$, Cl, Br, $SCH_3$, $SO_2CH_3$ ou $NO_2$ ;
$R_2$ est H, Cl ou un groupe alkyle en $C_1$-$C_4$ ;
$R_3$ est H, Cl ou un groupe alkyle en $C_1$-$C_4$ ;
$R_4$ est H ou un groupe alkyle en $C_1$-$C_4$ ;
$R_5$ est H ou $CH_3$ ;
$R_6$ est H, $R_8$, $SR_8$, $SO_2R_8$, $OR_8$, $C(O)R_8$, $CO_2R_8$, $NR_8R_4$, CN ou $Si(CH_3)_2R_9$ ;
$R_7$ est H, un groupe alkyle en $C_1$-$C_6$, Cl, Br, CN, $NO_2$, $SR_9$, $SO_2R_9$, $CO_2R_9$ ou $C(O)R_9$ ;
$R_8$ est un groupe alkyle en $C_1$-$C_6$, alcényle en $C_2$-$C_6$ ou alcynyle en $C_2$-$C_6$, chacun étant facultativement substitué par un ou plusieurs halogènes et/ou $R_{11}$, époxyalkyle en $C_2$-$C_6$, cycloalkyle en $C_3$-$C_6$, cycloalkylalkyle en $C_4$-$C_7$, ou

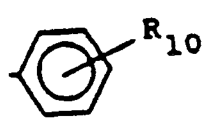 ;

$R_9$ est un groupe alkyle en $C_1$-$C_4$,

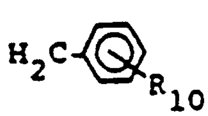

ou

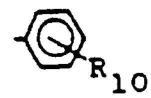 ;

$R_{10}$ est H, F, Cl, Br, $CH_3$, $OCH_3$, CN, $NO_2$, $SCH_3$, $SO_2CH_3$ ou $CF_3$ ;
$R_{11}$ est $OR_{12}$, $OC(O)R_{12}$, $OC(O)NR_9R_{12}$, $OSO_2R_{12}$, $OSi(CH_3)_2R_9$, $Si(CH_3)_2R_9$, $SR_{12}$, $SOR_{12}$, $SO_2R_{12}$, SCN, CN, $NO_2$, $C(O)R_{12}$, $C(O)OR_{12}$, $C(O)NR_4R_{12}$,

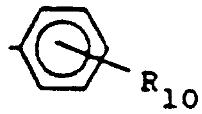

ou L ;
$R_{12}$ est H, un groupe alkyle en $C_1$-$C_6$, halogénalkyle en $C_1$-$C_6$, alcényle en $C_2$-$C_6$, alcynyle en $C_2$-$C_6$, cycloalkyle en $C_3$-$C_6$ ou

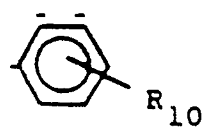 ;

n est 0 ou 1 ;
Q est O, S, SO, $SO_2$, $CH_2$ ou $CHCH_3$ ; et
L est un hétérocycle aromatique penta- ou hexagonal, un hétérocycle dihydroaromatique penta- ou hexagonal ou un hétérocycle tétrahydroaromatique penta- ou hexagonal, chacun contenant 1 à 4 hétéroatomes choisis dans le groupe formé par 0 ou 1 atome d'oxygène, 0 ou 1 atome de soufre et 0 à 4 atomes d'azote, chacun étant également facultativement substitué par 1 à 4 $CH_3$, 1 à 2 $OCH_3$, 1 $SCH_3$, 1 Cl, 1 $N(CH_3)_2$ ou 1 CN, ou bien L est une lactone, un lactame ou une cycloalcanone, penta- ou hexagonal, facultativement substitué par 1 à 4 $CH_3$ ;
G est O, S ou NR ;
W est O ou S ;
$R_1$, $R_1'$ et A sont tels que définis dans la revendication 1 ;
lesdites conditions a), c) et g) telles que définies dans la revendication 1 étant appliquées ;
et avec les conditions supplémentaires suivantes

b) le nombre total d'atomes de carbone dans $R_2$ et $R_3$ est inférieur ou égal à 4 ;
d) si J est $J_1$ ou $J_2$, alors $R_2$ et $R_3$ sont autre chose que Cl ;
e) le nombre total d'atomes de carbone dans $R_4$ et $R_5$ est inférieur ou égal à 4 ;
f) si J est $J_5$, $J_6$, $J_7$ ou $J_8$ et n = 0, alors $R_4$ est H ; ou d'un de ses sels convenant pour l'agriculture; qui consiste à mettre en oeuvre l'un quelconque des procédés (a) à (f) définis dans la revendication 1, où J, R et A sont tels que définis ci-dessus, excepté que J n'est pas $J_5$, $J_6$, $J_9$ ou $J_{10}$ dans le procédé (c).

**3.** Un procédé selon la revendication 2, dans lequel J est

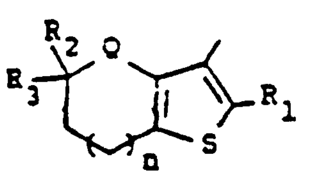 , 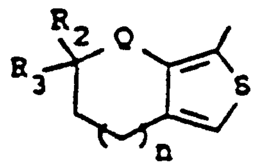 , 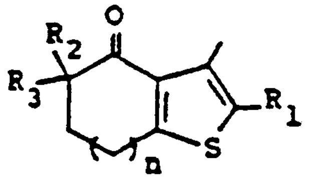 ,

$J_1$ $J_2$ $J_3$

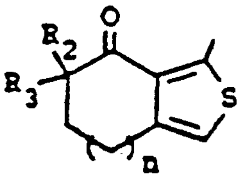 , 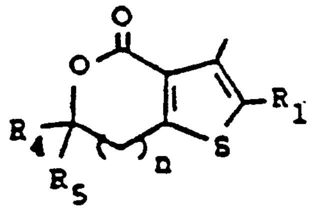 , 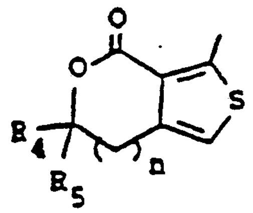 ,

$J_4$ $J_5$ $J_6$

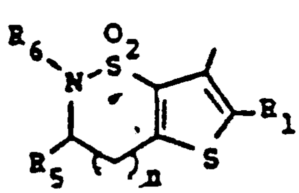 , 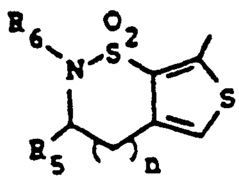 , 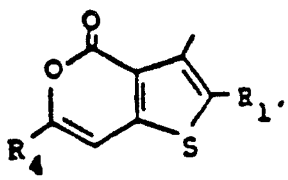 ,

$J_7$ $J_8$ $J_9$

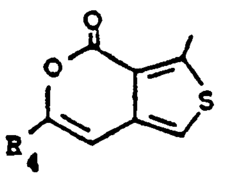 , 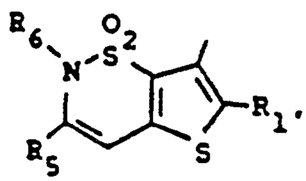 , 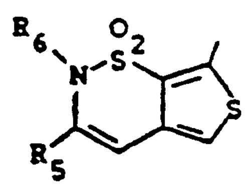 ,

$J_{10}$ $J_{11}$ $J_{12}$

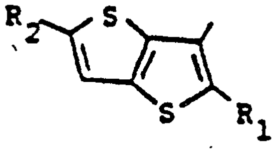 ou 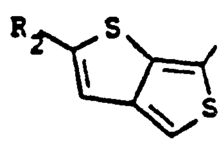 ;

$J_{13}$ $J_{14}$

R est H ou $CH_3$ ;
$R_1$ est H, $CH_3$, $OCH_3$, Cl ou Br ;
$R_2$ est H, Cl ou un groupe alkyle en $C_1$-$C_4$ ;

$R_3$ est H, Cl ou un groupe alkyle en $C_1$-$C_4$ ;
$R_4$ est H ou un groupe alkyle en $C_1$-$C_4$ ;
$R_5$ est H ou $CH_3$ ;
$R_6$ est H ou un groupe alkyle en $C_1$-$C_4$ ;
Q est O, S, SO, $SO_2$, $CH_2$ ou $CHCH_3$ ;
n est 0 ou 1 ;
A est

A est 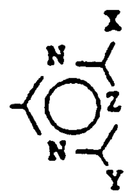 , 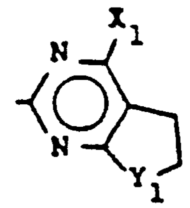 , 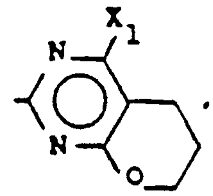 ,

A-1 A-2 A-3

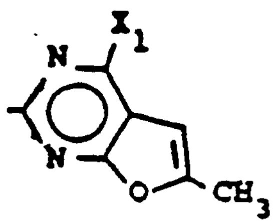 , 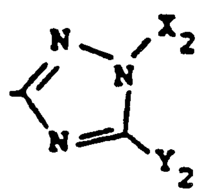 ou 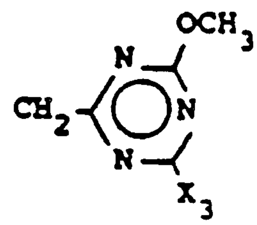 ;

X est $CH_3$, $OCH_3$, $OCH_2CH_3$, Cl, F, Br, $CH_2F$, $OCF_2H$ ou $CF_3$ ;
Y est H, $CH_3$, $OCH_3$, $OC_2H_5$, $CH_2OCH_3$, $NH_2$, $NHCH_3$, $N(CH_3)_2$, $C_2H_5$, $CF_3$, $SCH_3$, $OCH_2CH=CH_2$, $OCH_2C \equiv CH$, $OCH_2CF_3$, CN, $N_3$, $OCH_2CH_2OCH_3$, $CH_2SCH_3$, $CR_a(WCH_3)_2$,

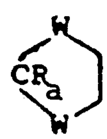 , 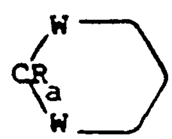 ,

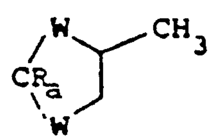 ,

$CR_a(WCH_2CH_3)_2$, ou $WCF_2T$
où W est O ou S, $R_a$ est H ou $CH_3$ et T est H, CHClF, CHBrF ou $CHFCF_3$ ;
Z est CH, N, $CCH_3$, $CC_2H_5$, CCl ou CBr ;
$Y_1$ est O ou $CH_2$ ;
$X_1$ est $CH_3$, $OCH_3$ , $OC_2H_5$ ou $OCF_2H$ ;
$X_2$ est $CH_3$, $C_2H_5$ ou $CH_2CF_3$ ;
$Y_2$ est $OCH_3$, $OC_2H_5$, $SCH_3$, $SC_2H_5$, $CH_3$, ou $CH_2CH_3$ ;
$X_3$ est $CH_3$ ou $OCH_3$ ;
et de ses sels convenant pour l'agriculture.

**4.** Un procédé selon la revendication 3, dans lequel R est H, W est O et G est O ou S.

**5.** Un procédé selon la revendication 2, dans lequel R est H, W est O et G est O ou S.

**6.** Un procédé selon la revendication 5, dans lequel A est A-1, X est $CH_3$, $OCH_3$, Cl, Br, $OCH_2CF_3$ ou $OCF_2H$ ; Y est un groupe alkyle en $C_1$-$C_3$, cyclopropyle, C≡CH, $OCH_3$, $OC_2H_5$, $CH_2OCH_3$, $NH_2$, $NHCH_3$, $N(CH_3)_2$, $C_2H_5$, $CF_3$, $SCH_3$, $OCH_2CH=CH_2$, $OCH_2C≡CH$, $OCH_2CH_2OCH_3$, $CR_a(OCH_3)_2$,

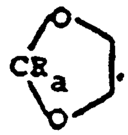

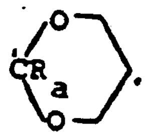

$CH_a(OCH_2CH_3)_2$ ou $OCF_2H$; et Z est CH ou N.

**7.** Un procédé selon la revendication 6, dans 3lequel $R_1$ est H, Cl, Br ou $CH_3$ ; $R_2$, $R_3$ et $R_4$ sont H ou un groupe alkyle en $C_1$-$C_3$ ; $R_6$ est H, $R_8$, $C(O)R_8$ ou $CO_2R_8$ ; $R_7$ est Cl, Br, $CO_2CH_3$ ou $CO_2CH_2CH_3$ ; $R_8$ est un groupe alkyle en $C_1$-$C_6$, halogénalkyle en $C_1$-$C_6$, alcényle en $C_2$-$C_6$, alcynyle en $C_2$-$C_6$, cycloalkyle en $C_3$-$C_6$ ou cycloalkylalkyle en $C_4$-$C_7$ et $R_{11}$ est $OCH_3$, $OCH_2CH_3$, CN, $CO_2$(alkyle en $C_1$-$C_4$), OH ou $C(O)CH_3$.

**8.** Un procédé selon la revendication 7, dans lequel G est S, Y est $CH_3$ ou $OCH_3$ et X est $CH_3$, $OCH_3$, Cl ou Br.

**9.** Un procédé selon la revendication 8, dans lequel J est choisi parmi J-1, J-3, J-5, J-7, J-9, J-11 et J-13.

**10.** Un procédé selon la revendication 1, dans lequel le produit est un composé choisi parmi le 4,4-dioxyde de N-[(4-méthoxy-6-méthylpyrimidine-2-yl)aminocarbonyl]-6,7-dihydro-5H-thiéno[3,2-B]thiopyranne-3-sulfonamide; le 4,4-dioxyde de N-[(4,6-diméthoxypyrimidine-2-yl)aminocarbonyl]-6,7-dihydro-5H-thiéno-[3,2-B]thiopyranne-3-sulfonamide; le 4,4-dioxyde de N-[(4,6-diméthoxypyrimidine-2-yl)aminocarbonyl]-5,6-dihydro-5-méthylthiéno[3,2-B]thiophène-3-sulfonamide; le 4,4-dioxyde de N-[(4-méthoxy-6-méthyl-1,3,5-triazine-2-yl)aminocarbonyl]-5,6-dihydro-5-méthylthiéno[3,2-B]thiophène-3-sulfonamide; et le 4,4-dioxyde de N-[(4-chloro-6-méthoxypyrimidine-2-yl)aminocarbonyl]-5,6-dihydro-5-méthylthiéno[3,2-B]-thiophène-3-sulfonamide.

**11.** Une composition convenant pour lutter contre la croissance d'une végétation indésirable, qui comprend une quantité efficace d'un composé de formule (I) tel que défini à l'une quelconque des revendications 1 à 10 et au moins l'un des ingrédients suivants : agent tensio-actif, diluant solide ou liquide.

**12.** Un procédé pour lutter contre la croissance d'une végétation indésirable, qui consiste à appliquer au site à protéger une quantité efficace d'un composé de formule (I) tel que défini à l'une quelconque des revendications 1 à 10.

**13.** Un procédé selon la revendication 12, dans lequel on applique un composé tel que défini à la revendication 10, ou un de ses sels convenant pour l'agriculture.

**14.** Un procédé pour réguler la croissance de plantes, qui consiste à appliquer au site où se trouvent ces plantes une quantité efficace, mais sensiblement non phytotoxique, d'un régulateur de croissance de plantes choisi parmi les composés de formule (I) tel que définis à l'une quelconque des revendications 1 à 10.

**Ansprüche**

**1.** Verbindung der Formel

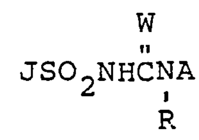

I

worin J

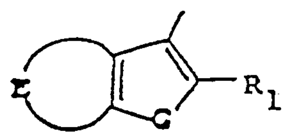

oder

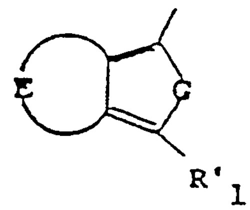

ist;
E eine Brücke aus 3 bis 4 Atomen ist, die 0 bis 2 aus der aus Sauerstoff, Schwefel und Stickstoff bestehenden Gruppe ausgewählte Heteroatome enthält und weiterhin 1 bis 4 Kohlenstoffatome enthält, wobei die Brücke zusammen mit den beiden Kohlenstoff-Bindungsstellen einen nicht aromatischen 5- bis 6-gliedrigen carbocyclischen Ring, einen aromatischen 5- bis 6-gliedrigen heterocyclischen Ring oder einen nicht aromatischen 5- bis 6-gliedrigen heterocyclischen Ring bildet, mit der Maßgabe, daß zwei Sauerstoffatome durch wenigstens ein Kohlenstoffatom getrennt sein müssen und daß Sauerstoff und Schwefel nur dann aneinander gebunden sind, wenn der Schwefel in Form von -SO- oder $-SO_2-$ vorliegt;
G O, S oder N-R ist;
W O oder S ist;
R H oder $CH_3$ ist;
$R'_1$ H, $CH_3$ oder Cl ist, wenn G S ist und H ist, wenn G O oder N-R ist;
$R_1$ H, $CH_3$, $OCH_3$, Cl, Br, $SCH_3$, $SO_2CH_3$ oder $NO_2$ ist;
und

A

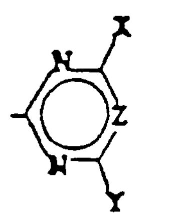

,

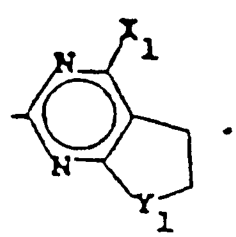

,

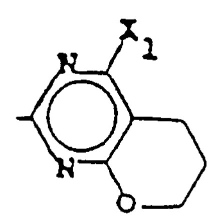

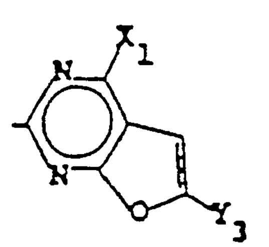

,

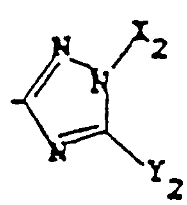

oder

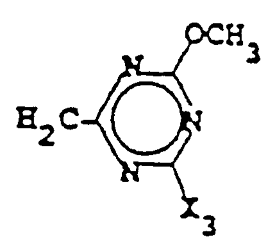

A-4 A-5 A-6

ist;
worin X H, $C_1$-$C_4$-Alkyl, $C_1$-$C_4$-Alkoxy, $C_1$-$C_4$-Halogenalkoxy, $C_1$-$C_4$-Halogenalkyl, $C_1$-$C_4$-Halogenalkylthio, $C_1$-$C_4$-Alkylthio, Halogen, $C_2$-$C_5$-Alkoxyalkoxy, Amino, $C_1$-$C_3$-Alkylamino oder Di($C_1$-$C_3$-alkyl)amino

ist;
Y H, $C_1$-$C_4$-Alkyl, $C_1$-$C_4$-Alkoxy, $C_1$-$C_4$-Halogenalkoxy, $C_1$-$C_4$-Halogenalkylthio, $C_1$-$C_4$-Alkylthio, Halogen, $C_2$-$C_5$-Alkoxyalkyl, $C_2$-$C_5$-Alkoxyalkoxy, Amino, $C_1$-$C_3$-Alkylamino, Di($C_1$-$C_3$-alkyl)amino, $C_3$-$C_4$-Alkenyloxy, $C_3$-$C_4$-Alkinyloxy, $C_2$-$C_5$-Alkylthioalkyl, $C_1$-$C_4$-Halogenalkyl, $C_3$-$C_5$-Cycloalkyl, $C_2$-$C_4$-Alkinyl,

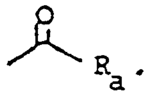 , 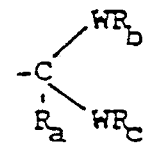 , 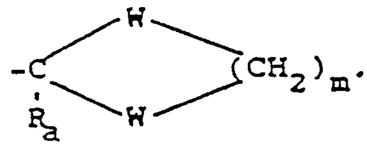

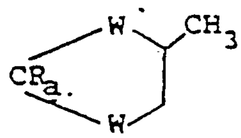

oder $N(OCH_3)CH_3$ ist; W O oder S ist;
m 2 oder 3 ist;
$R_a$ H oder $CH_3$ ist;
$R_b$ $C_1$-$C_2$-Alkyl ist;
$R_c$ $C_1$-$C_2$-Alkyl ist;
Z CH, N, $CCH_3$, $CC_2H_5$, CCl oder CBr ist;
$Y_1$ O oder $CH_2$ ist;
$X_1$ $CH_3$, $OCH_3$, $OC_2H_5$ oder $OCF_2H$ ist;
$X_2$ $CH_3$, $C_2H_5$ oder $CH_2CF_3$ ist;
$Y_2$ $OCH_3$, $OC_2H_5$, $SCH_3$, $SC_2H_5$, $OCF_2H$, $SCF_2H$, $CH_3$ oder $CH_2CH_3$ ist;
$X_3$ $CH_3$ oder $OCH_3$ ist; und
$Y_3$ H oder $CH_3$ ist;
mit der Maßgabe, daß

a) wenn W S ist, dann R H ist, A A-1 ist, Z CH oder N ist und Y $CH_3$, $OCH_3$, $OC_2H_5$, $CH_2OCH_3$, $C_2H_5$, $CF_3$, $SCH_3$, $OCH_2CH=CH_2$, $OCH_2C{\equiv}CH$, $OCH_2CH_2OCH_3$, $CH(OCH_3)_2$, $OCH_2CF_3$ oder

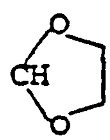

ist;

c) wenn X Cl, F oder Br ist, dann Z CH ist und Y $OCH_3$, $OC_2H_5$, $NH_2$, $NHCH_3$, $N(CH_3)_2$ oder $OCF_2H$ ist;

g) wenn X oder Y $OCF_2H$ ist, dann Z CH ist; sowie ihre landwirtschaftlich geeigneten Salze.

**2.** Verbindung der Formel

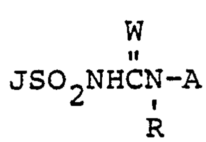

worin J

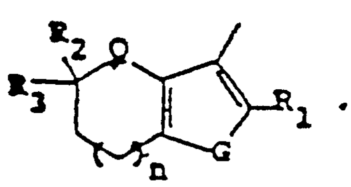

J1

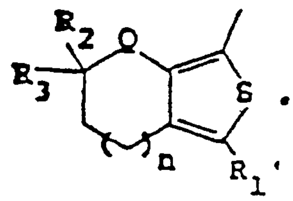

J2

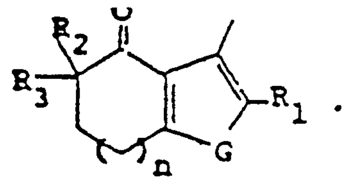

J3

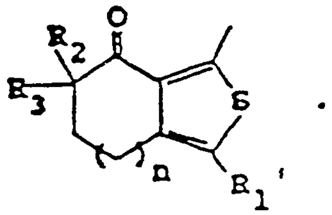

J4

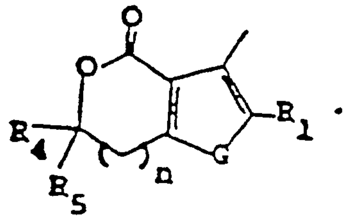

J5

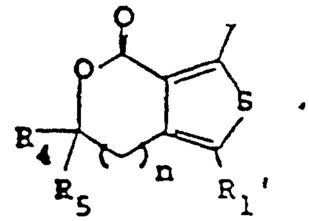

J6

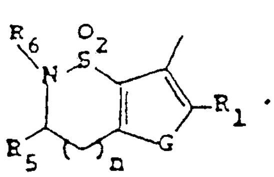

$J_7$

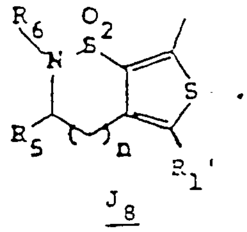

$J_8$

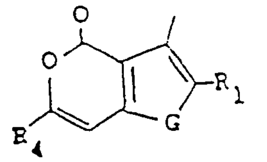

$J_9$

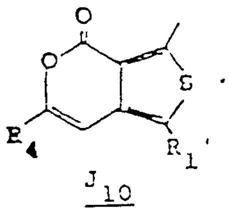

$J_{10}$

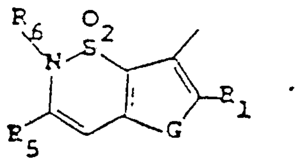

$J_{11}$

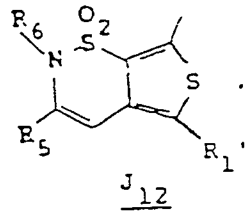

$J_{12}$

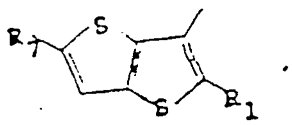

$J_{13}$

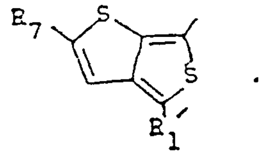

$J_{14}$

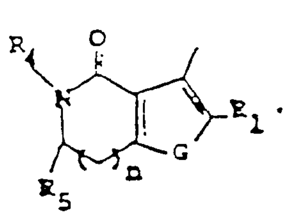

$J_{15}$

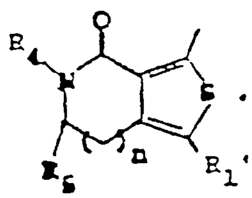

$J_{16}$

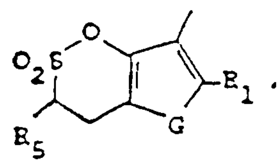

$J_{17}$

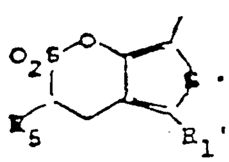

$J_{18}$

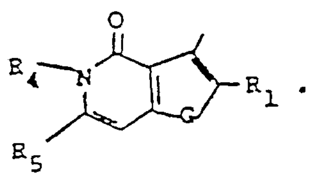

$J_{19}$

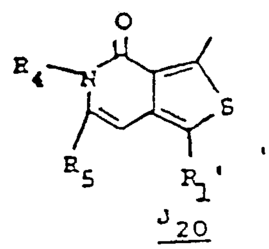

$J_{20}$

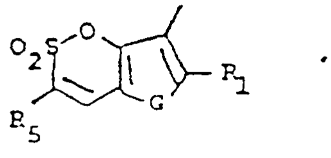

$J_{21}$

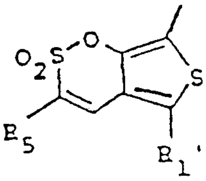 ist;

$J_{22}$

ist;
R H oder $CH_3$ ist;
$R_1$' H, $CH_3$ oder Cl ist;
$R_1$ H, $CH_3$, $OCH_3$, Cl, Br, $SCH_3$, $SO_2CH_3$ oder $NO_2$ ist;
$R_2$ H, Cl oder $C_1$-$C_4$-Alkyl ist;

$R_3$ H, Cl oder $C_1$-$C_4$-Alkyl ist;
$R_4$ H oder $C_1$-$C_4$-Alkyl ist;
$R_5$ H oder $CH_3$ ist;
$R_6$ H, $R_8$, $SR_8$, $SO_2R_8$, $OR_8$, $C(O)R_8$, $CO_2R_8$, $NR_8R_4$, CN oder $Si(CH_3)_2R_9$ ist;
$R_7$ H, $C_1$-$C_6$-Alkyl, Cl, Br, CN, $NO_2$, $SR_9$, $SO_2R_9$, $CO_2R_9$ oder $C(O)R_9$ ist;
$R_8$ $C_1$-$C_6$-Alkyl, $C_2$-$C_6$-Alkenyl oder $C_2$-$C_6$-Alkinyl ist, jedes gegebenenfalls durch ein oder mehrere Halogene und/oder $R_{11}$, $C_2$-$C_6$-Epoxyalkyl, $C_3$-$C_6$-Cycloalkyl, $C_4$-$C_7$-Cycloalkylalkyl oder

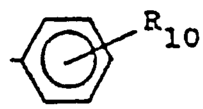

substituiert;
$R_9$ $C_1$-$C_4$-Alkyl,

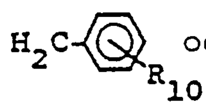 oder 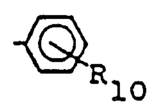

ist;
$R_{10}$ H, F, Cl, Br, $CH_3$, $OCH_3$, CN, $NO_2$, $SCH_3$, $SO_2CH_3$ oder $CF_3$ ist;
$R_{11}$ $OR_{12}$, $OC(O)R_{12}$, $OC(O)NR_9R_{12}$, $OSO_2R_{12}$, $OSi(CH_3)_2R_9$, $Si(CH_3)_2R_9$, $SR_{12}$, $SOR_{12}$, $SO_2R_{12}$, SCN, CN, $NO_2$, $C(O)R_{12}$, $C(O)OR_{12}$, $C(O)NR_4R_{12}$,

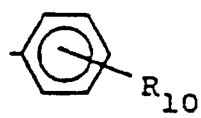

oder L ist;
$R_{12}$ H, $C_1$-$C_6$-Alkyl, $C_1$-$C_6$-Halogenalkyl, $C_2$-$C_6$-Alkenyl, $C_2$-$C_6$-Alkinyl, $C_3$-$C_6$-Cycloalkyl oder

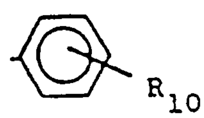

ist;
n 0 oder 1 ist;
Q O, S, SO, $SO_2$, $CH_2$ oder $CHCH_3$ ist; und
L ein 5- oder 6-gliedriger aromatischer Heterozyklus, ein 5- oder 6-gliedriger dihydroaromatischer Heterozyklus oder ein 5- oder 6-gliedriger tetrahydroaromatischer Heterozyklus ist, der jeweils 1 bis 4 aus der aus 0 bis 1 Sauerstoffatomen, 0 bis 1 Schwefelatomen und 0 bis 4 Stickstoffatomen bestehenden Gruppe ausgewählte Heteroatome enthält, und weiterhin jeweils gegebenenfalls mit 1 bis 4 $CH_3$, 1 bis 2 $OCH_3$, 1 $SCH_3$, 1 Cl, 1 $N(CH_3)_2$ oder 1 CN substituiert ist, oder worin L ein 5- oder 6-gliedriges Lacton, Lactam oder Cycloalkanon ist, das gegebenenfalls mit 1 bis 4 $CH_3$ substituiert ist;
G O, S oder NR ist;
W O oder S ist;
$R_1$, $R'_1$ und A wie in Anspruch 1 definiert sind, die Maßgaben a), c) und g), wie in Anspruch 1 definiert, zutreffen;
mit der weiteren Maßgabe, daß
b) die Gesamtzahl der Kohlenstoffatome in $R_2$ und $R_3$ kleiner oder gleich 4 ist;
d) wenn J $J_1$ oder $J_2$ ist, dann $R_2$ und $R_3$ von Cl verschieden sind;
e) die Gesamtzahl der Kohlenstoffatome in $R_4$ und $R_5$ kleiner als oder gleich 4 ist;
f) wenn J $J_5$, $J_6$, $J_7$ oder $J_8$ ist und n = 0 ist, dann $R_4$ H ist; und ihre landwirtschaftlich geeigneten

Salze.

3. Verbindung nach Anspruch 2, worin J

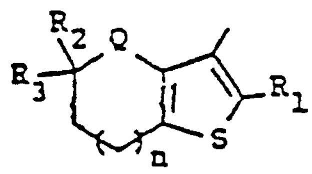 ,

$J_1$

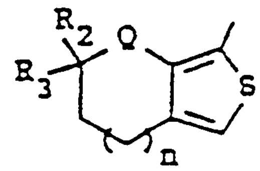 ,

$J_2$

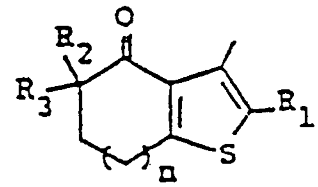 ,

$J_3$

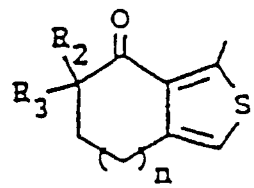 ,

$J_4$

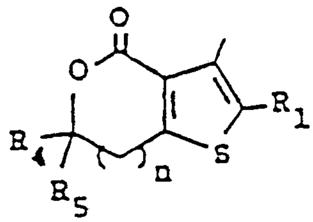 ,

$J_5$

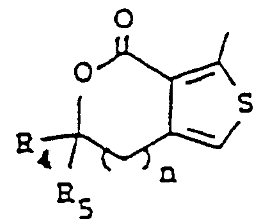 .

$J_6$

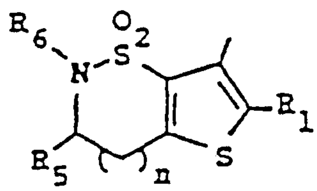,
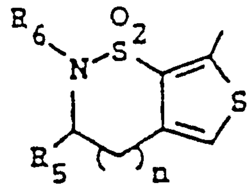,
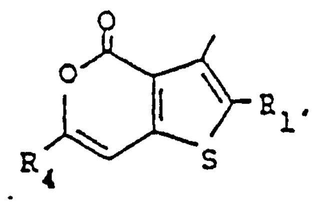,

$J_7$ $J_8$ $J_9$

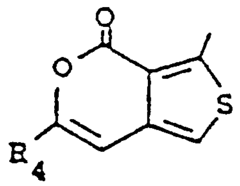,
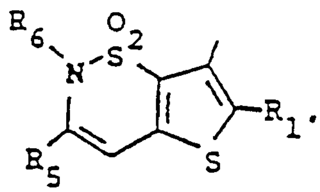,
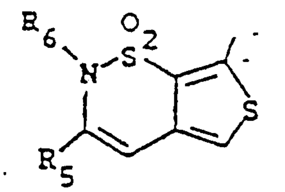,

$J_{10}$ $J_{11}$ $J_{12}$

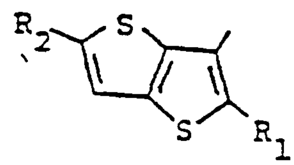
oder
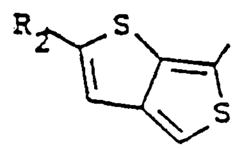

$J_{13}$ $J_{14}$

ist;
R H oder $CH_3$ ist;
$R_1$ H, $CH_3$, $OCH_3$, Cl oder Br ist;
$R_2$ H, Cl oder $C_1$-$C_4$-Alkyl ist;
$R_3$ H, Cl oder $C_1$-$C_4$-Alkyl ist;
$R_4$ H oder $C_1$-$C_4$-Alkyl ist;
$R_5$ H oder $CH_3$ ist;
$R_6$ H oder $C_1$-$C_4$-Alkyl ist;
Q O, S, SO, $SO_2$, $CH_2$ oder $CHCH_3$ ist;
n 0 oder 1 ist;
A

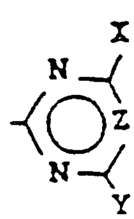,
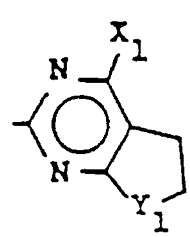,
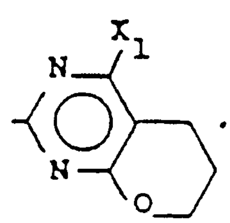,

A-1 A-2 A-3

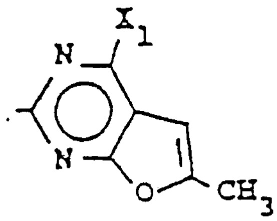 , 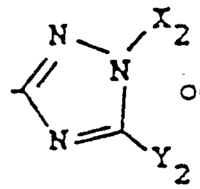 oder 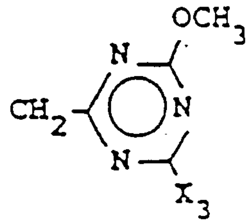

A-4 A-5 A-6

ist;

X $CH_3$, $OCH_3$, $OCH_2CH_3$, Cl, F, Br, $CH_2F$, $OCF_2H$ oder $CF_3$ ist;

Y H, $CH_3$, $OCH_3$, $OC_2H_5$, $CH_2OCH_3$, $NH_2$, $NHCH_3$, $N(CH_3)_2$, $C_2H_5$, $CF_3$, $SCH_3$, $OCH_2CH=CH_2$, $OCH_2C{\equiv}CH$, $OCH_2CF_3$, CN, $N_3$, $OCH_2CH_2OCH_3$, $CH_2SCH_3$, $CR_a(WCH_3)_2$,

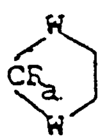 , 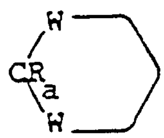 , 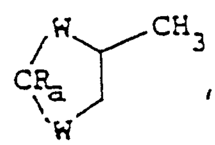 ,

$CR_a$ $(WCH_2CH_3)_2$ oder $WCF_2T$ ist,

worin W O oder S ist, $R_a$ H oder $CH_3$ ist und T H, CHClF, CHBrF oder $CHFCF_3$ ist;

Z CH, N, $CCH_3$, $CC_2H_5$, CCl oder CBr ist;

$Y_1$ O oder $CH_2$ ist;

$X_1$ $CH_3$, $OCH_3$, $OC_2H_5$ oder $OCF_2H$ ist;

$X_2$ $CH_3$, $C_2H_5$ oder $CH_2CF_3$ ist;

$Y_2$ $OCH_3$, $OC_2H_5$, $SCH_3$, $SC_2H_5$, $CH_3$ oder $CH_2CH_3$ ist;

$X_3$ $CH_3$ oder $OCH_3$ ist;

und landwirtschaftlich geeignete Salze davon.

4. Verbindung nach Anspruch 3, worin R H ist, W O ist und G O oder S ist.

5. Verbindung nach Anspruch 2, worin R H ist, W O ist und G O oder S ist.

6. Verbindung nach Anspruch 5, worin A A-1 ist, X $CH_3$, $OCH_3$, Cl, Br, $OCH_2CF_3$ oder $OCF_2H$ ist; Y $C_1$-$C_3$-Alkyl, Cyclopropyl, $C{\equiv}CH$, $OCH_3$, $OC_2H_5$, $CH_2OCH_3$, $NH_2$, $NHCH_3$, $N(CH_3)_2$, $C_2H_5$, $CF_3$, $SCH_3$, $OCH_2CH=CH_2$, $OCH_2C{\equiv}CH$, $OCH_2CH_2OCH_3$, $CR_a(OCH_3)_2$,

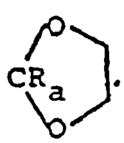 , 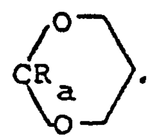 ,

$CR_a(OCH_2CH_3)_2$ oder $OCF_2H$ ist und S CH oder N ist.

7. Verbindung nach Anspruch 6, worin $R_1$ H, Cl, Br oder $CH_3$ ist; $R_2$, $R_3$ und $R_4$ H oder $C_1$-$C_3$-Alkyl sind; $R_6$ H, $R_8$, $C(O)R_8$ oder $CO_2R_8$ ist; $R_7$ Cl, Br, $CO_2CH_3$ oder $CO_2CH_2CH_3$ ist; $R_8$ $C_1$-$C_6$-Alkyl, $C_1$-$C_6$-Halogenalkyl, $C_2$-$C_6$-Alkenyl, $C_2$-$C_6$-Alkinyl, $C_3$-$C_6$-Cycloalkyl oder $C_4$-$C_7$-Cycloalkylalkyl ist und $R_{11}$ $OCH_3$, $OCH_2CH_3$, CN, $CO_2(C_1$-$C_4$-alkyl), OH oder $C(O)CH_3$ ist.

8. Verbindung nach Anspruch 7, worin G S ist, Y $CH_3$ oder $OCH_3$ ist und X $CH_3$, $OCH_3$, Cl oder Br ist.

9. Verbindung nach Anspruch 8, worin J J-1 ist.

**10.** Verbindung nach Anspruch 8, worin J J-3 ist.

**11.** Verbindung nach Anspruch 8, worin J J-5 ist.

**12.** Verbindung nach Anspruch 8, worin J J-7 ist.

**13.** Verbindung nach Anspruch 8, worin J J-9 ist.

**14.** Verbindung nach Anspruch 8, worin J J-11 ist.

**15.** Verbindung nach Anspruch 8, worin J J-13 ist.

**16.** Verbindung nach Anspruch 1, welche N-[(4-Methoxy-6-methylpyrimidin-2-yl)aminocarbonyl]6,7-dihydro-5H-thieno-[3,2-B]thiopyran-3-sulfonamid-4,4-dioxid ist.

**17.** Verbindung nach Anspruch 1, welche N-[(4,6-Dimethoxypyrimidin-2-yl)aminocarbonyl]-6,7-dihydro-5H-thieno[3,2-B]thiopyran-3-sulfonamid-4,4-dioxid ist.

**18.** Verbindung nach Anspruch 1, welche N-[(4,6-Dimethoxypyrimidin-2-yl)aminocarbonyl]-5,6-dihydro-5-methylthieno[3,2-B]thiophen-3-sulfonamid-4,4-dioxid ist.

**19.** Verbindung nach Anspruch 1, welche N-[(4-Methoxy-6-methyl-1 , 3, 5-triazin-2-yl)aminocarbonyl]-5,6-dihydro-5-methylthieno[3,2-B]thiophen-3-sulfonamid-4,4-dioxid ist.

**20.** Verbindung nach Anspruch 1, welche N-[(4-Chlor-6-methoxypyrimidin-2-yl)aminocarbonyl]-5,6-dihydro-5-methylthieno[3,2-B]thiophen-3-sulfonamid-4,4-dioxid ist.

**21.** Zur Bekämpfung des Wachstums unerwünschter Vegetation geeignete Zusammensetzung, welche eine wirksame Menge einer Verbindung nach einem der Ansprüche 1 bis 20 und wenigstens eines der folgenden umfaßt: oberflächenaktives Mittel, festes oder flüssiges Verdünnungsmittel.

**22.** Verfahren zur Bekämpfung des Wachstums unerwünschter Vegetation durch Anwenden einer wirksamen Menge einer Verbindung nach einem der Ansprüche 1 bis 20 auf den zu schützenden Ort.

**23.** Verfahren zur Steuerung des Wachstums von Pflanzen durch Anwenden einer wirksamen, jedoch im wesentlichen nicht phytotoxischen Menge eines Pflanzenwachstumsregulans, ausgewählt aus Verbindungen nach einem der Ansprüche 1 bis 20, auf den Ort solcher Pflanzen.

**24.** Verfahren zur Herstellung einer Verbindung nach Anspruch 1 oder 2 durch
a) Umsetzen eines Isocyanats oder Isothiocyanats der Formel

$$J-SO_2N=C=W \qquad (II)$$

mit einem Amin der Formel

$$\underset{\displaystyle R}{\underset{|}{HN}}-A \qquad (III)$$

worin J, A und R wie in Anspruch 1 oder 2 definiert sind;
b) Umsetzen eines Sulfonamids der Formel

$$J-SO_2NH_2 \qquad (IV)$$

mit einem heterocyclischen Isothiocyanat der Formel

$$S=C=N-A \qquad (V)$$

worin J und A wie in Anspruch 1 oder 2 definiert sind, unter Erhalt eines Produkts, worin W S ist und R H ist;
c) Umsetzen eines Sulfonamids der Formel

$$J-SO_2NH_2 \qquad (IV)$$

worin J wie in Anspruch 1 oder 2 definiert ist, jedoch von $J_5$, $J_6$, $J_9$ und $J_{10}$, wie in Anspruch 2 definiert, verschieden ist, mit einem Methylcarbamat der Formel

$$CH_3O\overset{O}{\overset{\|}{C}}-\underset{R}{N}-A$$

worin R und A wie in Anspruch 1 oder 2 definiert sind;
d) Umsetzen eines Sulfonylcarbamats der Formel

$$J-SO_2NH\overset{O}{\overset{\|}{C}}OC_6H_5 \qquad (VII)$$

worin J wie in Anspruch 1 definiert ist, mit dem Amin der Formel III,
e) Umsetzen eines Sulfonamids der Formel

$$J-SO_2NH_2 \qquad (IV)$$

worin J wie in Anspruch 1 oder 2 definiert ist, mit einem Phenylcarbamat der Formel

$$PhO\overset{O}{\overset{\|}{C}}\underset{R}{N}-A \qquad (VIII)$$

worin L und A wie in Anspruch 1 oder 2 definiert sind; oder
f) Umsetzen eines bicyclischen Heterozyklus der Formel

$$J-H \qquad (IX)$$

mit einem Sulfamoylchlorid der Formel

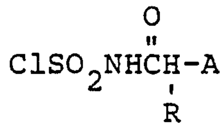 (X)

worin J, A und R wie in Anspruch 1 oder 2 definiert sind.

Patentansprüche für folgenden Vertragsstaat : AT

**1.** Verfahren zur Herstellung einer Verbindung der Formel

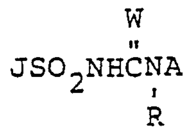 I

worin J

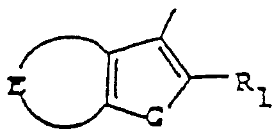 oder 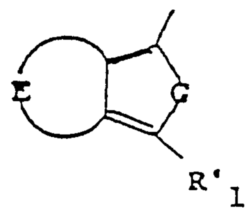

ist;
E eine Brücke aus 3 bis 4 Atomen ist, die 0 bis 2 aus der aus Sauerstoff, Schwefel und Stickstoff bestehenden Gruppe ausgewählte Heteroatome enthält und weiterhin 1 bis 4 Kohlenstoffatome enthält, wobei die Brücke zusammen mit den beiden Kohlenstoff-Bindungsstellen einen dicht aromatischen 5- bis 6-gliedrigen carbocyclischen Ring, einen aromatischen 5- bis 6-gliedrigen heterocyclischen Ring oder einen nicht aromatischen 5- bis 6-gliedrigen heterocyclischen Ring bildet, mit der Maßgabe, daß zwei Sauerstoffatome durch wenigstens ein Kohlenstoffatom getrennt sein müssen und daß Sauerstoff und Schwefel nur dann aneinander gebunden sind, wenn der Schwefel in Form von -SO- oder $-SO_2-$ vorliegt;
G O, S oder N-R ist;
W O oder S ist;
R H oder $CH_3$ ist;
$R'_1$ H, $CH_3$ oder Cl ist, wenn G S ist und H ist, wenn G O oder N-R ist;
$R_1$ H, $CH_3$, $OCH_3$, Cl, Br, $SCH_3$, $SO_2CH_3$ oder $NO_2$ ist;
und A

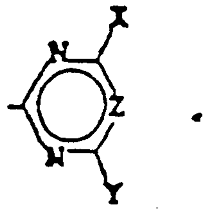,
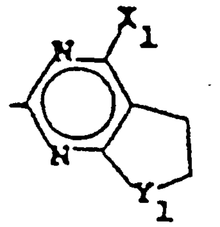,
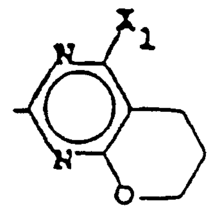

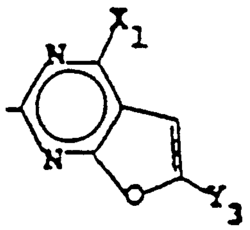,
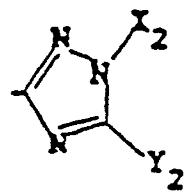
oder
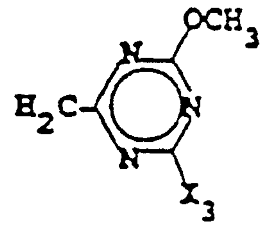

A-4 A-5 A-6

ist;
worin X H, $C_1$-$C_4$-Alkyl, $C_1$-$C_4$-Alkoxy, $C_1$-$C_4$-Halogenalkoxy, $C_1$-$C_4$-Halogenalkyl, $C_1$-$C_4$-Halogenalkylthio, $C_1$-$C_4$-Alkylthio, Halogen, $C_2$-$C_5$-Alkoxyalkoxy, Amino, $C_1$-$C_3$-Alkylamino oder Di($C_1$-$C_3$-alkyl)amino ist;
Y H, $C_1$-$C_4$-Alkyl, $C_1$-$C_4$-Alkoxy, $C_1$-$C_4$-Halogenalkoxy, $C_1$-$C_4$-Halogenalkylthio, $C_1$-$C_4$-Alkylthio, Halogen, $C_2$-$C_5$-Alkoxyalkyl, $C_2$-$C_5$-Alkoxyalkoxy, Amino, $C_1$-$C_3$-Alkylamino, Di($C_1$-$C_3$-alkyl)amino, $C_3$-$C_4$-Alkenyloxy, $C_3$-$C_4$-Alkinyloxy, $C_2$-$C_5$-Alkylthioalkyl, $C_1$-$C_4$-Halogenalkyl, $C_3$-$C_5$-Cycloalkyl, $C_2$-$C_4$-Alkinyl,

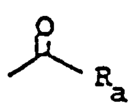,
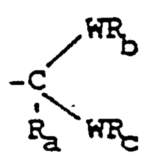,
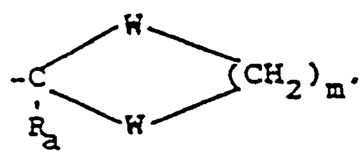,

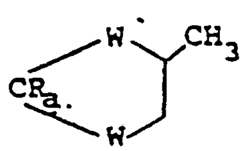

oder $N(OCH_3)CH_3$ ist;
W O oder S ist;
m 2 oder 3 ist;
$R_a$ H oder $CH_3$ ist;
$R_b$ $C_1$-$C_2$-Alkyl ist;
$R_c$ $C_1$-$C_2$-Alkyl ist;
Z CH, N, $CCH_3$, $CC_2H_5$, CCl oder CBr ist;
$Y_1$ O oder $CH_2$ ist;
$X_1$ $CH_3$, $OCH_3$, $OC_2H_5$ oder $OCF_2H$ ist;
$X_2$ $CH_3$, $C_2H_5$ oder $CH_2CF_3$ ist;
$Y_2$ $OCH_3$, $OC_2H_5$, $SCH_3$, $SC_2H_5$, $OCF_2H$, $SCF_2H$, $CH_3$ oder $CH_2CH_3$ ist;
$X_3$ $CH_3$ oder $OCH_3$ ist; und
$Y_3$ H oder $CH_3$ ist;
mit der Maßgabe, daß

a) wenn W S ist, dann R H ist, A A-1 ist, Z CH oder N ist und

Y $CH_3$, $OCH_3$, $OC_2H_5$, $CH_2OCH_3$, $C_2H_5$, $CF_3$, $SCH_3$, $OCH_2CH=CH_2$, $OCH_2C{\equiv}CH$, $OCH_2CH_2OCH_3$, $CH(OCH_3)_2$, $OCH_2CF_3$ oder

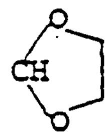

ist;

c) wenn X Cl, F oder Br ist, dann Z CH ist und Y $OCH_3$, $OC_2H_5$, $NH_2$, $NHCH_3$, $N(CH_3)_2$ oder $OCF_2H$ ist;

g) wenn x oder Y $OCF_2H$ ist, dann Z CH ist; oder eines landwirtschaftlich geeignetes Salzes davon durch

a) Umsetzen eines Isocyanats oder Isothiocyanats der Formel

$$J-SO_2N=C=W \qquad (II)$$

mit einem Amin der Formel

$$\begin{array}{l} HN-A \\ \;| \\ \;R \end{array} \qquad (III)$$

worin J, A und R wie oben definiert sind;

b) Umsetzen eines Sulfonamids der Formel

$$J-SO_2NH_2 \qquad (IV)$$

mit einem heterocyclischen Isothiocyanat der Formel

$$S=C=N-A \qquad (V)$$

worin J und A wie oben definiert sind, unter Erhalt eines Produkts, worin W S ist und R H ist;

c) Umsetzen eines Sulfonamids der Formel

$$J-SO_2NH_2 \qquad (IV)$$

worin J wie oben definiert ist, jedoch von $J_5$, $J_6$, $J_9$ und $J_{10}$, wie in Anspruch 2 nachstehend definiert, verschieden ist, mit einem Methylcarbamat der Formel

$$\begin{array}{l} \qquad\;\; O \\ \qquad\;\; \| \\ CH_3OC-N-A \\ \qquad\quad\;\, | \\ \qquad\quad\;\, R \end{array}$$

worin R und A wie oben definiert sind;

d) Umsetzen eines Sulfonylcarbamats der Formel

$$J-SO_2NH\overset{O}{\overset{\|}{C}}OC_6H_5 \qquad (VII)$$

worin J wie oben definiert ist, mit dem Amin der Formel III;

e) Umsetzen eines Sulfonamids der Formel

$$J-SO_2NH_2 \qquad (IV)$$

worin J wie oben definiert ist, mit einem Phenylcarbamat der Formel

$$PhO\overset{O}{\overset{\|}{C}}\underset{\underset{R}{|}}{N}-A \qquad (VIII)$$

worin L und A wie oben definiert sind; oder

f) Umsetzen eines bicyclischen Heterozyklus der Formel

$$J-H \qquad (IX)$$

mit einem Sulfamoylchlorid der Formel

$$ClSO_2NH\overset{O}{\overset{\|}{C}}\underset{\underset{R}{|}}{H}-A \qquad (X)$$

worin J, A und R wie oben definiert sind.

**2.** Verfahren zur Herstellung einer Verbindung der Formel

$$JSO_2NH\overset{W}{\overset{\|}{C}}\underset{\underset{R}{|}}{N}-A$$

worin J

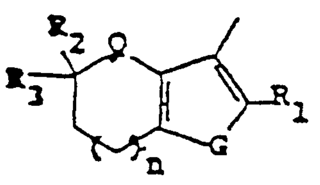 .

$J_1$

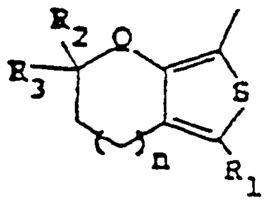 .

$J_2$

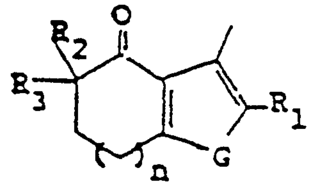 .

$J_3$

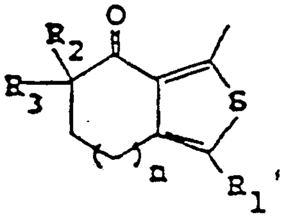 .

$J_4$

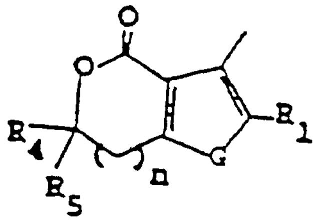 .

$J_5$

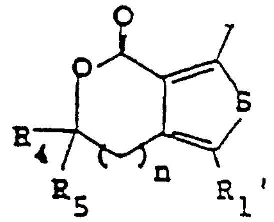 .

$J_6$

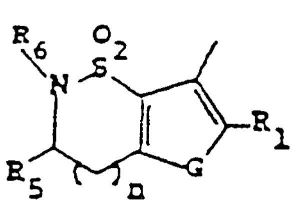 .

$J_7$

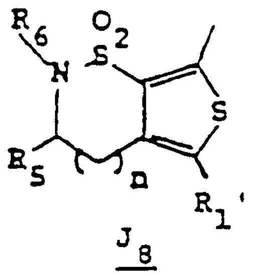 .

$J_8$

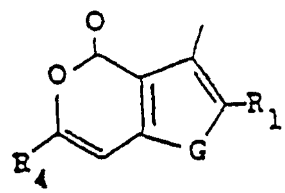 .

$J_9$

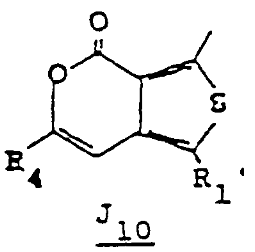 .

$J_{10}$

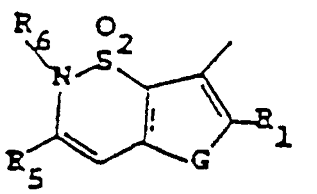 .

$J_{11}$

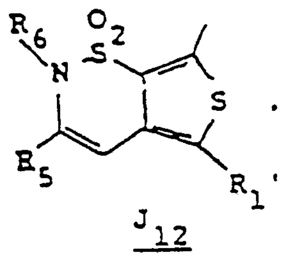 .

$J_{12}$

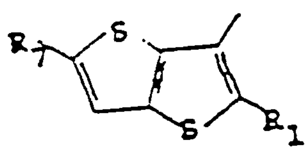 .

$J_{13}$

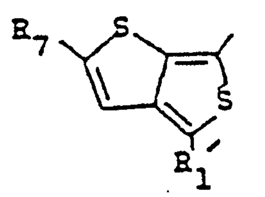 .

$J_{14}$

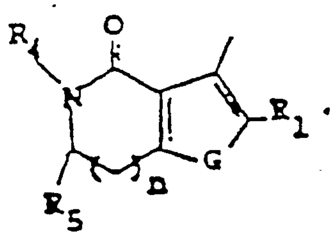

$J_{15}$

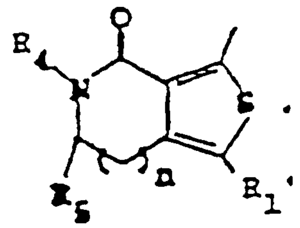

$J_{16}$

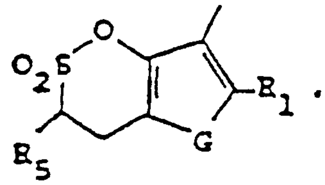

$J_{17}$

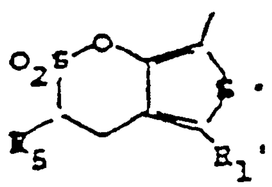

$J_{18}$

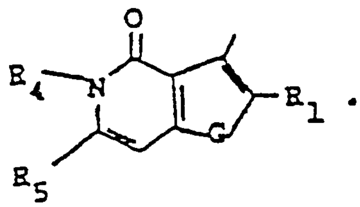

$J_{19}$

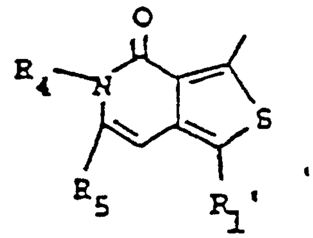

$J_{20}$

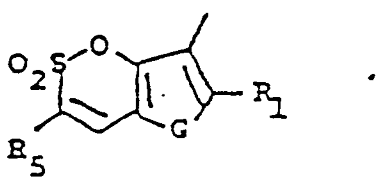

$J_{21}$

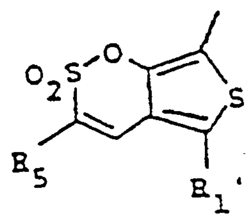

$J_{22}$

ist;
R H oder $CH_3$ ist;
$R_1$' H, $CH_3$ oder Cl ist;
$R_1$ H, $CH_3$, $OCH_3$, Cl, Br, $SCH_3$, $SO_2CH_3$ oder $NO_2$ ist;
$R_2$ H, Cl oder $C_1$-$C_4$-Alkyl ist;
$R_3$ H, Cl oder $C_1$-$C_4$-Alkyl ist;
$R_4$ H oder $C_1$-$C_4$-Alkyl ist;
$R_5$ H oder $CH_3$ ist;
$R_6$ H, $R_8$, $SR_8$, $SO_2R_8$, $OR_8$, $C(O)R_8$, $CO_2R_8$, $NR_8R_4$, CN oder $Si(CH_3)_2R_9$ ist;
$R_7$ H, $C_1$-$C_6$-Alkyl, Cl, Br, CN, $NO_2$, $SR_9$, $SO_2R_9$, $CO_2R_9$ oder $C(O)R_9$ ist;
$R_8$ $C_1$-$C_6$-Alkyl, $C_2$-$C_6$-Alkenyl oder $C_2$-$C_6$-Alkinyl ist, jedes gegebenenfalls durch ein oder mehrere Halogene und/oder $R_{11}$, $C_2$-$C_6$-Epoxyalkyl, $C_3$-$C_6$-Cycloalkyl, $C_4$-$C_7$-Cycloalkylalkyl oder

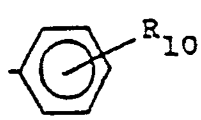

substituiert;
$R_9$ $C_1$-$C_4$-Alkyl,

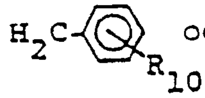 oder 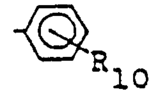

ist;

$R_{10}$ H, F, Cl, Br, $CH_3$, $OCH_3$, CN, $NO_2$, $SCH_3$, $SO_2CH_3$ oder $CF_3$ ist;

$R_{11}$ $OR_{12}$, $OC(O)R_{12}$, $OC(O)NR_9R_{12}$, $OSO_2R_{12}$, $OSi(CH_3)_2R_9$, $Si(CH_3)_2R_9$, $SR_{12}$, $SOR_{12}$, $SO_2R_{12}$, SCN, CN, $NO_2$, $C(O)R_{12}$, $C(O)OR_{12}$, $C(O)NR_4R_{12}$,

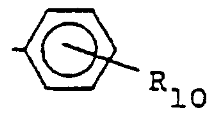

oder L ist;

$R_{12}$ H, $C_1$-$C_6$-Alkyl, $C_1$-$C_6$-Halogenalkyl, $C_2$-$C_6$-Alkenyl, $C_2$-$C_6$-Alkinyl, $C_3$-$C_6$-Cycloalkyl oder

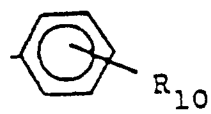

ist;

n 0 oder 1 ist;

Q O, S, SO, $SO_2$, $CH_2$ oder $CHCH_3$ ist; und

L ein 5- oder 6-gliedriger aromatischer Heterozyklus, ein 5- oder 6-gliedriger dihydroaromatischer Heterozyklus oder ein 5- oder 6-gliedriger tetrahydroaromatischer Heterozyklus ist, der jeweils 1 bis 4 aus der aus 0 bis 1 Sauerstoffatomen, 0 bis 1 Schwefelatomen und 0 bis 4 Stickstoffatomen bestehenden Gruppe ausgewählte Heteroatome enthält, und weiterhin jeweils gegebenenfalls mit 1 bis 4 $CH_3$, 1 bis 2 $OCH_3$, 1 $SCH_3$, 1 Cl, 1 $N(CH_3)_2$ oder 1 CN substituiert ist, oder worin L ein 5- oder 6-gliedriges Lacton, Lactam oder Cycloalkanon ist, das gegebenenfalls mit 1 bis 4 $CH_3$ substituiert ist;

G O, S oder NR ist;

W O oder S ist;

$R_1$, $R'_1$ und A wie in Anspruch 1 definiert sind, die Maßgaben a), c) und g), wie in Anspruch 1 definiert, zutreffen;

mit der weiteren Maßgabe, daß

b) die Gesamtzahl der Kohlenstoffatome in $R_2$ und $R_3$ kleiner oder gleich 4 ist;

d) wenn J $J_1$ oder $J_2$ ist, dann $R_2$ und $R_3$ von Cl verschieden sind;

e) die Gesamtzahl der Kohlenstoffatome in $R_4$ und $R_5$ kleiner als oder gleich 4 ist;

f) wenn J $J_5$, $J_6$, $J_7$ oder $J_8$ ist und n = 0 ist, dann $R_4$ H ist; oder eines landwirtschaftlich geeigneten Salzes davon durch Durchführen eines der Verfahren a) bis f), wie in Anspruch 1 definiert, worin J, R und A wie oben definiert sind, außer daß J nicht $J_5$, $J_6$, $J_9$ oder $J_{10}$ in Verfahren c) ist.

**3.** Verfahren nach Anspruch 2, worin J

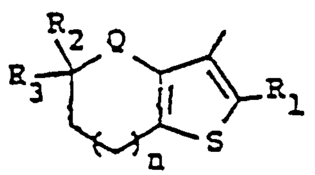 ,

J1

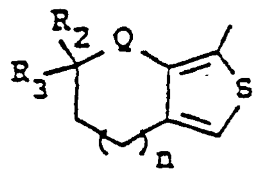 ,

J2

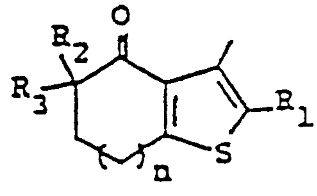 ,

J3

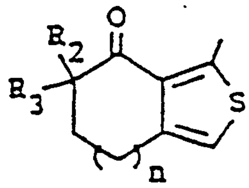 ,

J4

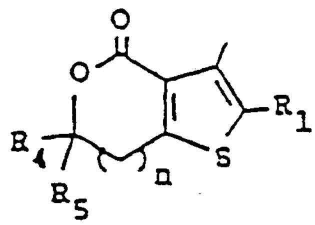 ,

J5

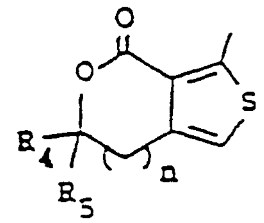 ,

J6

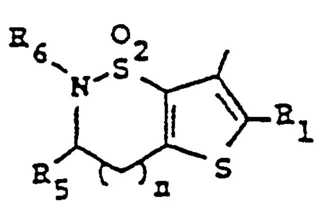 ,

J7

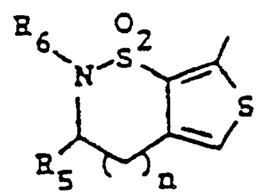 ,

J8

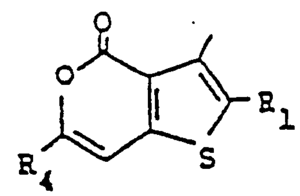 ,

J9

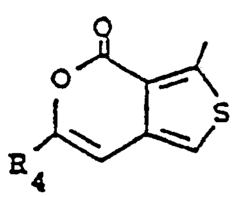 ,

J10

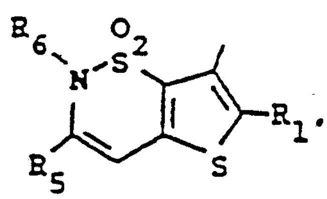 ,

J11

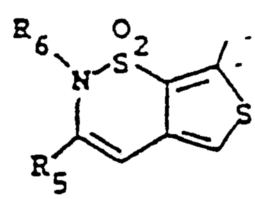 ,

J12

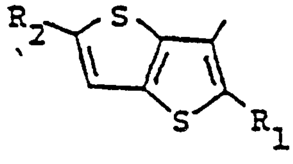

J13

oder

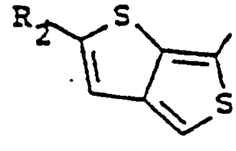

J14

ist;

R H oder $CH_3$ ist;

$R_1$ H, $CH_3$, $OCH_3$, Cl oder Br ist;

$R_2$ H, Cl oder $C_1$-$C_4$-Alkyl ist;
$R_3$ H, Cl oder $C_1$-$C_4$-Alkyl ist;
$R_4$ H oder $C_1$-$C_4$-Alkyl ist;
$R_5$ H oder $CH_3$ ist;
$R_6$ H oder $C_1$-$C_4$-Alkyl ist;
Q O, S, SO, $SO_2$, $CH_2$ oder $CHCH_3$ ist;
n 0 oder 1 ist;
A

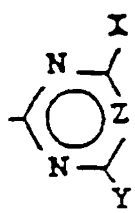
A-1
,
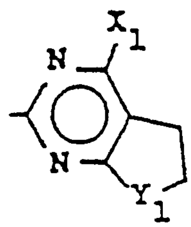
A-2
,
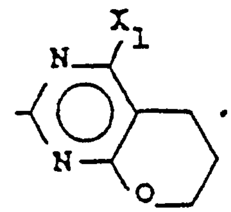
A-3
,

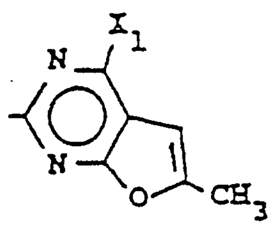
A-4
,
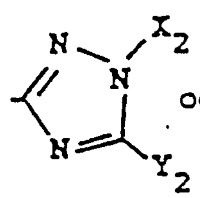
A-5
oder
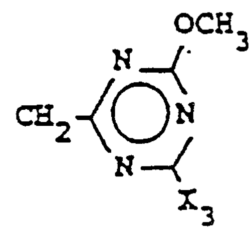
A-6

ist;
X $CH_3$, $OCH_3$, $OCH_2CH_3$, Cl, F, Br, $CH_2F$, $OCF_2H$ oder $CF_3$ ist;
Y H, $CH_3$, $OCH_3$, $OC_2H_5$, $CH_2OCH_3$, $NH_2$, $NHCH_3$, $N(CH_3)_2$, $C_2H_5$, $CF_3$, $SCH_3$, $OCH_2CH=CH_2$, $OCH_2C\equiv CH$, $OCH_2CF_3$, CN, $N_3$, $OCH_2CH_2OCH_3$, $CH_2SCH_3$, $CR_a(WCH_3)_2$,

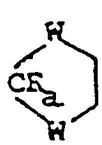
,
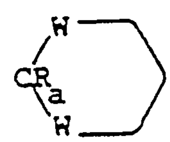
,
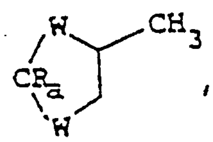
,

$CR_a$ $(WCH_2CH_3)_2$ oder $WCF_2T$ ist,
worin W O oder S ist, $R_a$ H oder $CH_3$ ist und T H, CHClF, CHBrF oder $CHFCF_3$ ist;
Z CH, N, $CCH_3$, $CC_2H_5$, CCl oder CBr ist;
$Y_1$ O oder $CH_2$ ist;
$X_1$ $CH_3$, $OCH_3$, $OC_2H_5$ oder $OCF_2H$ ist;
$X_2$ $CH_3$, $C_2H_5$ oder $CH_2CF_3$ ist;
$Y_2$ $OCH_3$, $OC_2H_5$, $SCH_3$, $SC_2H_5$, $CH_3$ oder $CH_2CH_3$ ist;
$X_3$ $CH_3$ oder $OCH_3$ ist;
und ihre landwirtschaftlich geeigneten Salze.

4. Verfahren nach Anspruch 3, worin R H ist, W O ist und G O oder S ist.

**5.** Verfahren nach Anspruch 2, worin R H ist, W O ist und G O oder S ist.

**6.** Verfahren nach Anspruch 5, worin A A-1 ist, X $CH_3$, $OCH_3$, Cl, Br, $OCH_2CF_3$ oder $OCF_2H$ ist; Y $C_1$-$C_3$-Alkyl, Cyclopropyl, C≡CH, $OCH_3$, $OC_2H_5$, $CH_2OCH_3$, $NH_2$, $NHCH_3$, $N(CH_3)_2$, $C_2H_5$, $CF_3$, $SCH_3$, $OCH_2CH=CH_2$, $OCH_2C≡CH$, $OCH_2CH_2OCH_3$, $CR_a(OCH_3)_2$,

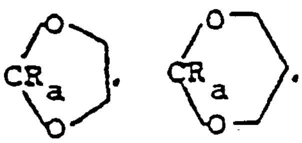

$CR_a(OCH_2CH_3)_2$ oder $OCF_2H$ ist; und Z CH oder N ist.

**7.** Verfahren nach Anspruch 6, worin $R_1$ H, Cl, Br oder $CH_3$ ist; $R_2$, $R_3$ und $R_4$ H oder $C_1$-$C_3$-Alkyl sind; $R_6$ H, $R_8$, $C(O)R_8$ oder $CO_2R_8$ ist; $R_7$ Cl, Br, $CO_2CH_3$ oder $CO_2CH_2CH_3$ ist; $R_8$ $C_1$-$C_6$-Alkyl, $C_1$-$C_6$-Halogenalkyl, $C_2$-$C_6$-Alkenyl, $C_2$-$C_6$-Alkinyl, $C_3$-$C_6$-Cycloalkyl oder $C_4$-$C_7$-Cycloalkylalkyl ist und $R_{11}$ $OCH_3$, $OCH_2CH_3$, CN, $CO_2(C_1$-$C_4$-alkyl), OH oder $C(O)CH_3$ ist.

**8.** Verfahren nach Anspruch 7, worin G S ist, Y $CH_3$ oder $OCH_3$ ist und X $CH_3$ $OCH_3$, Cl oder Br ist.

**9.** Verfahren nach Anspruch 8, worin J aus J-1, J-3, J-5, J-7, J-9, J-11 und J-13 ausgewählt ist.

**10.** Verfahren nach Anspruch 1, worin das Produkt eine aus N-[(4-Methoxy-6-methylpyrimidin-2-yl)-aminocarbonyl]-6,7-dihydro-5H-thieno-[3,2-B]thiopyran-3-sulfonamid-4,4-dioxid; N-[(4,6-Dimethoxypyrimidin-2-yl)aminocarbonyl]-6,7-dihydro-5H-thieno-[3,2-B]thiopyran-3-sulfonamid-4,4-dioxid; N-[4,6-Dimethoxypyrimidin-2-yl)aminocarbonyl]-5,6-dihydro-5-methylthieno[3,2-B]thiophen-3-sulfonamid-4,4-dioxid; N-[(4-Methoxy-6-methyl-1,3,5-triazin-2-yl)aminocarbonyl]-5,6-dihydro-5-methylthieno[3,2-B]thiophen-3-sulfonamid-4,4-dioxid und N-[(4-Chlor-6-methoxypyrimidin-2-yl)-aminocarbonyl]-5,6-dihydro-5-methylthieno[3,2-B]thiophen-3-sulfonamid-4,4-dioxid ausgewählte Verbindung ist.

**11.** Zur Bekämpfung des Wachstums unerwünschter Vegetation geeignete Zusammensetzung, welche eine wirksame Menge einer Verbindung der Formel (I), wie in einem der Ansprüche 1 bis 10 definiert, und wenigstens eines der folgenden umfaßt: oberflächenaktives Mittel, festes oder flüssiges Verdünnungsmittel.

**12.** Verfahren zur Bekämpfung des Wachstums unerwünschter Vegetation durch Anwenden einer wirksamen Menge einer Verbindung der Formel (I), wie in einem der Ansprüche 1 bis 10 definiert, auf den zu schützenden Ort.

**13.** Verfahren nach Anspruch 12, worin eine in Anspruch 10 definierte Verbindung oder ein landwirtschaftlich geeignetes Salz davon eingesetzt wird.

**14.** Verfahren zur Steuerung des Wachstums von Pflanzen durch Anwenden einer wirksamen, jedoch im wesentlichen nicht phytotoxischen Menge eines Pflanzenwachstumsregulans, ausgewählt aus Verbindungen der Formel (I), wie in einem der Ansprüche 1 bis 10 definiert, auf den Ort solcher Pflanzen.